(19) Europäisches Patentamt
European Patent Office
Office européen des brevets

(11) EP 2 727 939 A2

(12) **EUROPEAN PATENT APPLICATION**

(43) Date of publication:
**07.05.2014 Bulletin 2014/19**

(21) Application number: **13190391.6**

(22) Date of filing: **02.09.2011**

(51) Int Cl.:
***C07K 16/22*** *(2006.01)* ***A61K 39/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.09.2010 EP 10175318**

(62) Document number(s) of the earlier application(s) in accordance with Art. 76 EPC:
**11749454.2 / 2 611 830**

(71) Applicant: **Boehringer Ingelheim International GmbH**
**55216 Ingelheim am Rhein (DE)**

(72) Inventors:
- **Gschwind, Andreas**
**55216 Ingelheim am Rhein (DE)**
- **Borges, Eric**
**55216 Ingelheim am Rhein (DE)**
- **Boucneau, Joachim**
**9840 De Pinte (BE)**
- **De Tavernier, Evelyn**
**9080 Beervelde (BE)**
- **Kolkman, Joost**
**9830 Sint-Martens-Latem (BE)**
- **Merchiers, Pascal**
**2460 Kasterlee (BE)**

(74) Representative: **Simon, Elke Anna Maria et al**
**Boehringer Ingelheim GmbH**
**Corporate Patents**
**Binger Strasse 173**
**55216 Ingelheim am Rhein (DE)**

Remarks:
This application was filed on 25-10-2013 as a divisional application to the application mentioned under INID code 62.

# (54) VEGF-binding molecules

(57) VEGF-binding molecules, preferably VEGF-binding immunoglobulin single variable domains like VHHs and domain antibodies, pharmaceutical compositions containing same and their use in the treatment of diseases that are associated with VEGF-mediated effects on angiogenesis. Nucleic acids encoding VEGF-binding molecules, host cells and methods for preparing same.

EP 2 727 939 A2

SEQUENCE LISTING

<110> Boehringer Ingelheim International GmbH

<120> VEGF-BINDING MOLECULES

<130> 12-0319-EP-3

<160> 179

<170> PatentIn version 3.3

<210> 1
<211> 17
<212> PRT
<213> Lama glama

<220>
<221> MISC_FEATURE
<222> (5)..(5)
<223> Xaa may be Gly or Ala

<220>
<221> MISC_FEATURE
<222> (7)..(7)
<223> Xaa may be Ser or Gly

<220>
<221> MISC_FEATURE
<222> (12)..(12)
<223> Xaa may be Gly, Ala or Pro

<220>
<221> MISC_FEATURE
<222> (13)..(13)
<223> Xaa may be Asp or Gly

<220>
<221> MISC_FEATURE
<222> (16)..(16)
<223> Xaa may be Asp or Glu

<400> 1

```
Ser Arg Ala Tyr Xaa Ser Xaa Arg Leu Arg Leu Xaa Xaa Thr Tyr Xaa
1               5                   10                  15


Tyr
```

<210> 2
<211> 17
<212> PRT
<213> Lama glama

<400> 2

```
Ser Arg Ala Tyr Gly Ser Ser Arg Leu Arg Leu Gly Asp Thr Tyr Asp
1               5                   10                  15
```

Tyr

<210> 3
<211> 17
<212> PRT
<213> Lama glama

<400> 3

Ser Arg Ala Tyr Gly Ser Ser Arg Leu Arg Leu Ala Asp Thr Tyr Asp
1 5 10 15

Tyr

<210> 4
<211> 17
<212> PRT
<213> Lama glama

<400> 4

Ser Arg Ala Tyr Gly Ser Ser Arg Leu Arg Leu Ala Asp Thr Tyr Glu
1 5 10 15

Tyr

<210> 5
<211> 17
<212> PRT
<213> Lama glama

<400> 5

Ser Arg Ala Tyr Gly Ser Gly Arg Leu Arg Leu Ala Asp Thr Tyr Asp
1 5 10 15

Tyr

<210> 6
<211> 17
<212> PRT
<213> Lama glama

<400> 6

Ser Arg Ala Tyr Ala Ser Ser Arg Leu Arg Leu Ala Asp Thr Tyr Asp
1 5 10 15

Tyr

<210> 7
<211> 17
<212> PRT
<213> Lama glama

<400> 7

```
Ser Arg Ala Tyr Gly Ser Ser Arg Leu Arg Leu Pro Asp Thr Tyr Asp
1               5                   10                  15

Tyr
```

<210> 8
<211> 17
<212> PRT
<213> Lama glama

<400> 8

```
Ser Arg Ala Tyr Gly Ser Ser Arg Leu Arg Leu Pro Gly Thr Tyr Asp
1               5                   10                  15

Tyr
```

<210> 9
<211> 126
<212> PRT
<213> Lama glama

<400> 9

```
Glu Val Gln Leu Val Glu Ser Gly Gly Gly Leu Val Gln Pro Gly Asp
1               5                   10                  15

Ser Leu Lys Leu Ser Cys Ala Phe Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Ser Ser Gly Gly Tyr Ile Tyr Asp Ser Val Ser Leu
    50                  55                  60

Glu Gly Arg Phe Thr Ile Ser Arg Asp Asn Thr Lys Asn Thr Val Tyr
65                  70                  75                  80

Leu Gln Thr Pro Ser Leu Lys Pro Glu Asp Thr Ala Asp Tyr Tyr Cys
                85                  90                  95
```

Ala Ala Ser Arg Ala Tyr Gly Ser Ser Arg Leu Arg Leu Ala Asp Thr
100 105 110

Tyr Asp Tyr Trp Gly Gln Gly Thr Gln Val Thr Val Ser Ser
115 120 125

<210> 10
<211> 125
<212> PRT
<213> Lama glama

<400> 10

Glu Val Gln Leu Val Glu Ser Gly Gly Gly Leu Val Gln Pro Gly Asp
1 5 10 15

Ser Leu Lys Leu Ser Cys Ala Phe Ser Gly Arg Thr Phe Ser Ser Tyr
20 25 30

Ser Met Ala Trp Phe Arg Gln Ala Gln Gly Lys Glu Arg Glu Phe Val
35 40 45

Val Ala Ile Ser Ser Gly Gly Phe Ile Tyr Asp Ala Val Ser Leu Glu
50 55 60

Gly Arg Phe Thr Ile Ser Arg Asp Asn Thr Lys Asn Thr Val Tyr Leu
65 70 75 80

Gln Thr Pro Ser Leu Lys Pro Glu Asp Thr Ala Val Tyr Tyr Cys Ala
85 90 95

Ala Ser Arg Ala Tyr Gly Ser Ser Arg Leu Arg Leu Ala Asp Thr Tyr
100 105 110

Asp Tyr Trp Gly Gln Gly Thr Gln Val Thr Val Ser Ser
115 120 125

<210> 11
<211> 126
<212> PRT
<213> Lama glama

<400> 11

Glu Val Gln Leu Val Glu Ser Gly Gly Gly Leu Val Gln Pro Gly Asp
1 5 10 15

Ser Leu Lys Leu Ser Cys Ala Ala Ser Gly Arg Thr Phe Ser Ser Tyr
20 25 30

Ser Met Gly Trp Phe Arg Gln Ala Gln Gly Lys Glu Arg Glu Phe Val

35 40 45

Val Ala Ile Ser Ser Ser Gly Gly Tyr Ile Tyr Asp Ser Val Ser Leu
50 55 60

Glu Gly Arg Phe Thr Ile Ser Arg Asp Asn Thr Lys Asn Thr Val Tyr
65 70 75 80

Leu Gln Thr Pro Ser Leu Lys Pro Glu Asp Thr Ala Val Tyr Tyr Cys
85 90 95

Ala Ala Ser Arg Ala Tyr Gly Ser Ser Arg Leu Arg Leu Ala Asp Thr
100 105 110

Tyr Asp Tyr Trp Gly Gln Gly Thr Gln Val Thr Val Ser Ser
115 120 125

<210> 12
<211> 126
<212> PRT
<213> Lama glama

<400> 12

Glu Val Gln Leu Val Glu Ser Gly Gly Gly Leu Val Gln Ala Gly Asp
1 5 10 15

Ser Leu Arg Leu Ser Cys Ala Ala Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Ser Ser Gly Asn Tyr Lys Tyr Asp Ser Val Ser Leu
50 55 60

Glu Gly Arg Phe Thr Ile Ser Arg Asp Asn Thr Lys Asn Thr Val Tyr
65 70 75 80

Leu Gln Ile Asn Ser Leu Lys Pro Glu Asp Thr Ala Val Tyr Tyr Cys
85 90 95

Ala Ala Ser Arg Ala Tyr Gly Ser Ser Arg Leu Arg Leu Gly Asp Thr
100 105 110

Tyr Asp Tyr Trp Gly Gln Gly Thr Gln Val Thr Val Ser Ser
115 120 125

<210> 13

<211> 125
<212> PRT
<213> Lama glama

<400> 13

Glu Val Gln Leu Val Glu Ser Gly Gly Gly Leu Val Gln Pro Gly Asp
1 5 10 15

Ser Leu Lys Leu Ser Cys Val Ala Ser Gly Arg Thr Ser Ser Ser Tyr
20 25 30

Ser Met Gly Trp Phe Arg Gln Ala Gln Gly Lys Glu Arg Glu Phe Val
35 40 45

Val Ala Ile Ser Ser Gly Gly Ser Ile Tyr Asp Ser Val Ser Leu Gln
50 55 60

Gly Arg Phe Thr Ile Ser Arg Asp Asn Thr Lys Asn Thr Val Tyr Leu
65 70 75 80

Gln Thr Pro Ser Leu Lys Pro Glu Asp Thr Ala Val Tyr Tyr Cys Ala
85 90 95

Ala Ser Arg Ala Tyr Ala Ser Ser Arg Leu Arg Leu Ala Asp Thr Tyr
100 105 110

Asp Tyr Trp Gly Gln Gly Thr Gln Val Thr Val Ser Ser
115 120 125

<210> 14
<211> 125
<212> PRT
<213> Lama glama

<400> 14

Glu Val Gln Leu Val Glu Ser Gly Gly Gly Leu Val Gln Pro Gly Asp
1 5 10 15

Ser Leu Lys Leu Ser Cys Val Ala Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Ser Gly Gly Tyr Ile Tyr Asp Ser Val Ser Leu Gln
50 55 60

Gly Arg Phe Thr Ile Ser Arg Asp Asn Thr Lys Asn Thr Val Tyr Leu
65 70 75 80

Gln Thr Pro Ser Leu Lys Pro Glu Asp Thr Ala Val Tyr Tyr Cys Ala
85 90 95

Ala Ser Arg Ala Tyr Gly Ser Ser Arg Leu Arg Leu Ala Asp Thr Tyr
100 105 110

Asp Tyr Trp Gly Gln Gly Thr Gln Val Thr Val Ser Ser
115 120 125

<210> 15
<211> 125
<212> PRT
<213> Lama glama

<400> 15

Glu Val Gln Leu Val Glu Ser Gly Gly Gly Leu Val Gln Pro Gly Asp
1 5 10 15

Ser Leu Lys Leu Ser Cys Ala Phe Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Ser Gly Gly Phe Ile Tyr Asp Ala Val Ser Leu Glu
50 55 60

Gly Arg Phe Thr Ile Ser Arg Asp Asn Thr Lys Asn Thr Val Tyr Leu
65 70 75 80

Gln Thr Pro Ser Leu Lys Pro Glu Asp Thr Ala Val Tyr Tyr Cys Ala
85 90 95

Ala Ser Arg Ala Tyr Gly Ser Ser Arg Leu Arg Leu Ala Asp Thr Tyr
100 105 110

Asp Tyr Trp Gly Gln Gly Thr Gln Val Thr Val Ser Ser
115 120 125

<210> 16
<211> 125
<212> PRT
<213> Lama glama

<400> 16

Glu Val Gln Leu Val Glu Ser Gly Gly Gly Leu Val Gln Thr Gly Asp
1 5 10 15

Ser Leu Arg Leu Ser Cys Val Ala Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Asn Gly Gly Tyr Lys Tyr Asp Ser Val Ser Leu Glu
50 55 60

Gly Arg Phe Thr Ile Ser Arg Asp Asn Thr Lys Asn Thr Val Tyr Leu
65 70 75 80

Gln Ile Asn Ser Leu Lys Pro Glu Asp Thr Ala Val Tyr Tyr Cys Ala
85 90 95

Ala Ser Arg Ala Tyr Gly Ser Ser Arg Leu Arg Leu Ala Asp Thr Tyr
100 105 110

Asp Tyr Trp Gly Gln Gly Thr Gln Val Thr Val Ser Ser
115 120 125

<210> 17
<211> 126
<212> PRT
<213> Lama glama

<400> 17

Glu Val Gln Leu Val Glu Ser Gly Gly Gly Leu Val Gln Pro Gly Asp
1 5 10 15

Ser Leu Lys Leu Ser Cys Ala Phe Ser Gly Arg Thr Phe Gly Ser Tyr
20 25 30

Ser Met Gly Trp Phe Arg Gln Ala Gln Gly Lys Glu Arg Glu Phe Val
35 40 45

Val Ala Ile Ser Ser Ser Gly Gly Tyr Ile Tyr Asp Ser Val Ser Leu
50 55 60

Glu Gly Arg Phe Thr Ile Ser Arg Asp Asn Ser Lys Asn Thr Val Tyr
65 70 75 80

Leu Gln Thr Pro Ser Leu Lys Pro Glu Asp Thr Ala Val Tyr Tyr Cys
85 90 95

Ala Ala Ser Arg Ala Tyr Gly Ser Ser Arg Leu Arg Leu Pro Asp Thr
100 105 110

Tyr Asp Tyr Trp Gly Gln Gly Thr Gln Val Thr Val Ser Ser
115 120 125

<210> 18
<211> 125
<212> PRT
<213> Lama glama

<400> 18

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Lys Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Gln Val Thr Val Ser Ser
115 120 125

<210> 19
<211> 125
<212> PRT
<213> Lama glama

<400> 19

Glu Val Gln Leu Val Glu Ser Gly Gly Gly Leu Val Gln Pro Gly Asp
1 5 10 15

Ser Leu Arg Leu Ser Cys Ala Phe Ser Gly Arg Thr Phe Ser Ser Tyr
20 25 30

Ser Met Ala Trp Phe Arg Gln Ala Gln Gly Lys Glu Arg Glu Phe Val
35 40 45

```
Val Ala Ile Ser Ser Gly Gly Phe Ile Tyr Asp Ala Val Ser Leu Glu
    50                  55                  60

Gly Arg Phe Thr Ile Ser Arg Asp Asn Thr Lys Asn Thr Val Tyr Leu
65                  70                  75                  80

Gln Thr Pro Ser Leu Lys Pro Glu Asp Thr Ala Val Tyr Tyr Cys Ala
                85                  90                  95

Ala Ser Arg Ala Tyr Gly Ser Ser Arg Leu Arg Leu Ala Asp Thr Tyr
            100                 105                 110

Asp Tyr Trp Gly Gln Gly Thr Gln Val Thr Val Ser Ser
        115                 120                 125
```

<210>  20
<211>  125
<212>  PRT
<213>  Lama glama

<400>  20

```
Glu Val Gln Leu Val Glu Ser Glu Gly Gly Leu Val Gln Pro Gly Asp
1               5                   10                  15

Ser Leu Lys Leu Ser Cys Val Ala Ser Gly Arg Thr Ser Ser Ser Tyr
            20                  25                  30

Ser Met Gly Trp Phe Arg Gln Ala Gln Gly Lys Glu Arg Glu Phe Val
        35                  40                  45

Val Ala Ile Ser Ser Gly Gly Tyr Ile Tyr Asp Ser Val Ser Leu Gln
    50                  55                  60

Gly Arg Phe Thr Ile Ser Arg Asp Asn Thr Lys Asn Thr Val Tyr Leu
65                  70                  75                  80

Gln Thr Pro Ser Leu Lys Pro Glu Asp Thr Ala Val Tyr Tyr Cys Ala
                85                  90                  95

Ala Ser Arg Ala Tyr Gly Ser Ser Arg Leu Arg Leu Ala Asp Thr Tyr
            100                 105                 110

Asp Tyr Trp Gly Gln Gly Thr Gln Val Thr Val Ser Ser
        115                 120                 125
```

<210>  21
<211>  126
<212>  PRT
<213>  Lama glama

<400> 21

Glu Met Gln Leu Val Glu Ser Gly Gly Gly Leu Val Gln Pro Gly Asp
1 5 10 15

Ser Leu Lys Leu Ser Cys Ala Phe Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Ser Ser Gly Gly Tyr Ile Tyr Asp Ser Val Ser Leu
50 55 60

Glu Gly Arg Phe Thr Ile Ser Arg Asp Asn Thr Lys Asn Thr Val Tyr
65 70 75 80

Leu Gln Thr Pro Ser Leu Lys Pro Glu Asp Thr Ala Asp Tyr Tyr Cys
85 90 95

Ala Ala Ser Arg Ala Tyr Gly Ser Ser Arg Leu Arg Leu Ala Asp Thr
100 105 110

Tyr Asp Tyr Trp Gly Gln Gly Thr Gln Val Thr Val Ser Ser
115 120 125

<210> 22
<211> 126
<212> PRT
<213> Lama glama

<400> 22

Glu Val Gln Leu Val Glu Ser Gly Gly Gly Leu Val Gln Ala Gly Asp
1 5 10 15

Ser Leu Arg Leu Ser Cys Ala Ala Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Ser Ser Gly Asn Tyr Lys Tyr Asp Ser Val Ser Leu
50 55 60

Glu Gly Arg Phe Thr Ile Ser Arg Asp Asn Thr Lys Asn Thr Val Tyr
65 70 75 80

Leu Gln Ile Asn Ser Leu Lys Pro Lys Asp Thr Ala Val Tyr Tyr Cys

```
                85                  90                  95

Ala Ala Ser Arg Ala Tyr Gly Ser Ser Arg Leu Arg Leu Gly Asp Thr
            100                 105                 110

Tyr Asp Tyr Trp Gly Gln Gly Thr Gln Val Thr Val Ser Ser
        115                 120                 125


<210>  23
<211>  126
<212>  PRT
<213>  Lama glama

<400>  23

Glu Val Gln Leu Val Glu Ser Gly Gly Gly Leu Val Gln Pro Gly Asp
1               5                   10                  15

Ser Leu Lys Leu Ser Cys Ala Phe Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Ser Gly Gly Gly Tyr Ile Tyr Asp Ser Val Ser Leu
    50                  55                  60

Glu Gly Arg Phe Thr Ile Ser Arg Asp Asn Thr Lys Asn Thr Val Tyr
65                  70                  75                  80

Leu Gln Thr Pro Ser Leu Lys Pro Glu Asp Thr Ala Asp Tyr Tyr Cys
                85                  90                  95

Ala Ala Ser Arg Ala Tyr Gly Ser Ser Arg Leu Arg Leu Ala Asp Thr
            100                 105                 110

Tyr Asp Tyr Trp Gly Gln Gly Thr Gln Val Thr Val Ser Ser
        115                 120                 125


<210>  24
<211>  126
<212>  PRT
<213>  Lama glama

<400>  24

Glu Val Gln Leu Val Glu Ser Gly Gly Gly Leu Val Gln Pro Gly Asp
1               5                   10                  15

Ser Leu Lys Leu Ser Cys Ala Phe Ser Gly Arg Thr Phe Gly Ser Tyr
            20                  25                  30
```

Ser Met Gly Trp Phe Arg Gln Ala Gln Gly Lys Glu Arg Glu Phe Val
35 40 45

Val Ala Ile Ser Ser Ser Gly Gly Tyr Ile Tyr Asp Ser Val Ser Leu
50 55 60

Glu Gly Arg Phe Thr Ile Ser Arg Asp Asn Ser Lys Asn Thr Val Tyr
65 70 75 80

Leu Gln Thr Pro Ser Leu Lys Pro Glu Asp Thr Ala Val Tyr Tyr Cys
85 90 95

Ala Ala Ser Arg Ala Tyr Gly Ser Ser Arg Leu Arg Leu Pro Gly Thr
100 105 110

Tyr Asp Tyr Trp Gly Gln Gly Thr Gln Val Thr Val Ser Ser
115 120 125

<210> 25
<211> 125
<212> PRT
<213> Lama glama

<400> 25

Glu Val Gln Leu Val Glu Ser Gly Gly Gly Leu Val Gln Pro Gly Asp
1 5 10 15

Ser Leu Lys Leu Ser Cys Val Ala Ser Gly Arg Thr Ser Ser Ser Tyr
20 25 30

Ser Met Gly Trp Phe Arg Gln Ala Gln Gly Lys Glu Arg Glu Phe Val
35 40 45

Val Ala Ile Ser Ser Gly Gly Tyr Ile Tyr Asp Ser Val Ser Leu Gln
50 55 60

Gly Arg Phe Thr Ile Ser Arg Asp Asn Thr Lys Asn Thr Val Tyr Leu
65 70 75 80

Gln Thr Pro Ser Leu Lys Pro Glu Asp Thr Ala Val Tyr Tyr Cys Ala
85 90 95

Ala Ser Arg Ala Tyr Gly Ser Ser Arg Leu Arg Leu Ala Asp Thr Tyr
100 105 110

Asp Tyr Trp Gly Gln Gly Thr Gln Val Thr Val Ser Ser
115 120 125

<210> 26
<211> 125
<212> PRT
<213> Lama glama

<400> 26

```
Glu Val Gln Leu Val Glu Ser Gly Gly Gly Leu Val Gln Thr Gly Asp
1               5                   10                  15

Ser Leu Arg Leu Ser Cys Ala Ala Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Ser Gly Gly Tyr Lys Tyr Asp Ser Val Ser Leu Glu
    50                  55                  60

Gly Arg Phe Thr Ile Ser Arg Asp Asn Thr Gln Asn Thr Val Tyr Leu
65                  70                  75                  80

Gln Ile Asn Ser Leu Lys Pro Glu Asp Thr Ala Val Tyr Tyr Cys Ala
                85                  90                  95

Ala Ser Arg Ala Tyr Gly Ser Gly Arg Leu Arg Leu Ala Asp Thr Tyr
            100                 105                 110

Asp Tyr Trp Gly Gln Gly Thr Gln Val Thr Val Ser Ser
        115                 120                 125
```

<210> 27
<211> 125
<212> PRT
<213> Lama glama

<400> 27

```
Glu Val Gln Leu Val Glu Ser Gly Gly Gly Leu Val Gln Pro Gly Asp
1               5                   10                  15

Ser Leu Lys Leu Ser Cys Val Ala Ser Gly Arg Thr Ser Ser Ser Tyr
            20                  25                  30

Ser Met Gly Trp Phe Arg Gln Ala Gln Gly Gln Glu Arg Glu Phe Val
        35                  40                  45

Val Ala Ile Ser Ser Gly Gly Tyr Ile Tyr Asp Ser Val Ser Leu Gln
    50                  55                  60
```

```
Gly Arg Phe Thr Ile Ser Arg Asp Asn Thr Lys Asn Thr Val Tyr Leu
65                  70                  75                  80

Gln Thr Pro Ser Leu Lys Pro Glu Asp Thr Ala Val Tyr Tyr Cys Ala
                85                  90                  95

Ala Ser Arg Ala Tyr Gly Ser Ser Arg Leu Arg Leu Ala Asp Thr Tyr
            100                 105                 110

Asp Tyr Trp Gly Gln Gly Thr Gln Val Thr Val Ser Ser
        115                 120                 125


<210>  28
<211>  125
<212>  PRT
<213>  Lama glama

<400>  28

Glu Val Gln Leu Val Glu Ser Gly Gly Gly Leu Val Gln Pro Gly Asp
1               5                   10                  15

Ser Leu Lys Leu Ser Cys Ile Ala Ser Gly Arg Thr Ser Ser Ser Tyr
            20                  25                  30

Ser Met Gly Trp Phe Arg Gln Ala Gln Gly Gln Glu Arg Glu Phe Val
        35                  40                  45

Val Ala Ile Ser Ser Gly Gly Tyr Ile Tyr Asp Ser Val Ser Leu Gln
    50                  55                  60

Gly Arg Phe Thr Ile Ser Arg Asp Asn Thr Lys Asn Thr Val Tyr Leu
65                  70                  75                  80

Gln Thr Pro Ser Leu Lys Pro Glu Asp Thr Ala Val Tyr Tyr Cys Ala
                85                  90                  95

Ala Ser Arg Ala Tyr Gly Ser Ser Arg Leu Arg Leu Ala Asp Thr Tyr
            100                 105                 110

Asp Tyr Trp Gly Gln Gly Thr Gln Val Thr Val Ser Ser
        115                 120                 125


<210>  29
<211>  126
<212>  PRT
<213>  Lama glama

<400>  29
```

Glu Val Gln Leu Val Glu Ser Gly Gly Gly Leu Val Gln Ala Gly Asp
1               5                   10                  15

Ser Leu Arg Leu Ser Cys Ala Ala Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Ser Ser Gly Asn Tyr Lys Tyr Asp Ser Val Ser Leu
    50                  55                  60

Glu Gly Arg Phe Thr Ile Ser Arg Asp Asn Thr Lys Asn Thr Val Tyr
65                  70                  75                  80

Leu Gln Ile Asn Ser Leu Lys Pro Glu Asp Thr Ala Val Tyr Tyr Cys
                85                  90                  95

Ala Ala Ser Arg Ala Tyr Gly Ser Ser Arg Leu Arg Leu Gly Asp Thr
            100                 105                 110

Tyr Asp Tyr Trp Gly Gln Gly Thr Arg Val Thr Val Ser Ser
        115                 120                 125

<210> 30
<211> 126
<212> PRT
<213> Lama glama

<400> 30

Glu Val Gln Leu Val Glu Ser Gly Gly Gly Leu Val Gln Thr Gly Asp
1               5                   10                  15

Ser Leu Arg Leu Ser Cys Ala Ala Ser Gly Gly Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Ser Ser Gly Gly Tyr Ile Tyr Asp Ser Val Ser Leu
    50                  55                  60

Glu Gly Arg Phe Thr Ile Ser Arg Asp Asn Thr Lys Asn Thr Val Tyr
65                  70                  75                  80

Leu Gln Thr Pro Ser Leu Lys Pro Glu Asp Thr Ala Asp Tyr Tyr Cys
                85                  90                  95

```
Ala Ala Ser Arg Ala Tyr Gly Ser Ser Arg Leu Arg Leu Ala Asp Thr
            100                 105                 110

Tyr Asp Tyr Trp Gly Gln Gly Thr Gln Val Thr Val Ser Ser
        115                 120                 125
```

<210> 31
<211> 126
<212> PRT
<213> Lama glama

<400> 31

```
Glu Val Gln Leu Val Glu Ser Gly Gly Gly Leu Val Gln Pro Gly Asp
1               5                   10                  15

Pro Leu Lys Leu Ser Cys Ala Phe Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Ser Ser Gly Gly Tyr Ile Tyr Asp Ser Val Ser Leu
    50                  55                  60

Glu Gly Arg Phe Thr Ile Ser Arg Asp Asn Thr Lys Asn Thr Val Tyr
65                  70                  75                  80

Leu Gln Thr Pro Ser Leu Lys Pro Glu Asp Thr Ala Asp Tyr Tyr Cys
                85                  90                  95

Ala Ala Ser Arg Ala Tyr Gly Ser Ser Arg Leu Arg Leu Ala Asp Thr
            100                 105                 110

Tyr Asp Tyr Trp Gly Gln Gly Thr Gln Val Thr Val Ser Ser
        115                 120                 125
```

<210> 32
<211> 126
<212> PRT
<213> Lama glama

<400> 32

```
Glu Val Pro Leu Val Glu Ser Gly Gly Gly Leu Val Gln Ala Gly Asp
1               5                   10                  15

Ser Leu Arg Leu Ser Cys Ala Ala Ser Gly Arg Thr Phe Ser Ser Tyr
            20                  25                  30

Ser Met Gly Trp Phe Arg Gln Ala Gln Gly Lys Glu Arg Glu Phe Val
```

35 40 45

Val Ala Ile Ser Ser Ser Gly Asn Tyr Lys Tyr Asp Ser Ala Ser Leu
50 55 60

Glu Gly Arg Phe Thr Ile Ser Arg Asp Asn Thr Lys Asn Thr Val Tyr
65 70 75 80

Leu Gln Ile Asn Ser Leu Lys Pro Glu Asp Thr Ala Val Tyr Tyr Cys
85 90 95

Ala Ala Ser Arg Ala Tyr Gly Ser Ser Arg Leu Arg Leu Gly Asp Thr
100 105 110

Tyr Asp Tyr Trp Gly Gln Gly Thr Gln Val Thr Val Ser Ser
115 120 125

<210> 33
<211> 125
<212> PRT
<213> Lama glama

<400> 33

Glu Val Gln Leu Val Glu Ser Gly Gly Gly Leu Val Gln Pro Gly Asp
1 5 10 15

Ser Leu Lys Leu Ser Cys Ala Phe Ser Gly Arg Thr Phe Ser Ser Tyr
20 25 30

Ser Met Ala Trp Phe Arg Gln Ala Gln Gly Lys Glu Arg Glu Phe Val
35 40 45

Val Ala Ile Ser Ser Gly Gly Phe Ile Tyr Asp Ala Val Ser Leu Glu
50 55 60

Gly Arg Phe Thr Ile Ser Arg Asp Asn Thr Lys Asn Thr Val Tyr Leu
65 70 75 80

Gln Thr Pro Ser Leu Lys Pro Glu Gly Thr Ala Val Tyr Tyr Cys Ala
85 90 95

Ala Ser Arg Ala Tyr Gly Ser Ser Arg Leu Arg Leu Ala Asp Thr Tyr
100 105 110

Asp Tyr Trp Gly Gln Gly Thr Gln Val Thr Val Ser Ser
115 120 125

<210> 34

<211> 126
<212> PRT
<213> Lama glama

<400> 34

Glu Val Gln Leu Val Glu Ser Gly Gly Gly Leu Val Gln Pro Gly Asp
1 5 10 15

Ser Leu Lys Leu Ser Cys Ala Phe Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Ser Ser Gly Gly Tyr Ile Tyr Asp Ser Val Ser Leu
50 55 60

Glu Gly Arg Phe Thr Ile Ser Arg Asp Asn Thr Lys Asn Ala Val Tyr
65 70 75 80

Leu Gln Thr Pro Ser Leu Lys Pro Glu Asp Thr Ala Asp Tyr Tyr Cys
85 90 95

Ala Ala Ser Arg Ala Tyr Gly Ser Ser Arg Leu Arg Leu Ala Asp Thr
100 105 110

Tyr Asp Tyr Trp Gly Gln Gly Thr Gln Val Thr Val Ser Ser
115 120 125

<210> 35
<211> 126
<212> PRT
<213> Lama glama

<400> 35

Glu Val Gln Leu Met Glu Ser Gly Gly Gly Leu Val Gln Pro Gly Asp
1 5 10 15

Ser Leu Lys Leu Ser Cys Ala Phe Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Ser Ser Gly Gly Tyr Ile Tyr Asp Ser Val Ser Leu
50 55 60

Glu Gly Arg Phe Thr Ile Ser Arg Asp Asn Thr Lys Asn Thr Val Tyr
65 70 75 80

Leu Gln Thr Pro Ser Leu Lys Pro Glu Asp Thr Ala Asp Tyr Tyr Cys
85 90 95

Ala Ala Ser Arg Ala Tyr Gly Ser Ser Arg Leu Arg Leu Ala Asp Thr
100 105 110

Tyr Asp Tyr Trp Gly Gln Gly Thr Gln Val Thr Val Ser Ser
115 120 125

<210> 36
<211> 125
<212> PRT
<213> Lama glama

<400> 36

Glu Val Gln Leu Val Glu Ser Gly Gly Gly Leu Val Gln Pro Gly Gly
1 5 10 15

Ser Leu Arg Leu Ser Cys Ala Phe Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Ser Gly Gly Phe Ile Tyr Asp Ala Val Ser Leu Glu
50 55 60

Gly Arg Phe Thr Ile Ser Arg Asp Asn Thr Lys Asn Thr Val Tyr Leu
65 70 75 80

Gln Thr Pro Ser Leu Lys Pro Glu Asp Thr Ala Val Tyr Tyr Cys Ala
85 90 95

Ala Ser Arg Ala Tyr Gly Ser Ser Arg Leu Arg Leu Ala Asp Thr Tyr
100 105 110

Asp Tyr Trp Gly Gln Gly Thr Gln Val Thr Val Ser Ser
115 120 125

<210> 37
<211> 125
<212> PRT
<213> Lama glama

<400> 37

Glu Val Gln Leu Val Glu Ser Gly Gly Gly Leu Val Gln Pro Gly Asp
1 5 10 15

```
Ser Leu Lys Leu Ser Cys Ala Phe Ser Gly Arg Thr Phe Ser Ser Tyr
            20                  25                  30

Ser Met Ala Trp Phe Arg Gln Ala Gln Gly Lys Glu Arg Glu Phe Val
        35                  40                  45

Val Ala Ile Ser Ser Gly Gly Phe Ile Tyr Asp Ala Val Ser Leu Glu
    50                  55                  60

Gly Arg Phe Thr Ile Ser Arg Glu Asn Thr Lys Asn Thr Val Tyr Leu
65                  70                  75                  80

Gln Thr Pro Ser Leu Lys Pro Glu Asp Thr Ala Val Tyr Tyr Cys Ala
                85                  90                  95

Ala Ser Arg Ala Tyr Gly Ser Ser Arg Leu Arg Leu Ala Asp Thr Tyr
            100                 105                 110

Asp Tyr Trp Gly Gln Gly Thr Gln Val Thr Val Ser Ser
        115                 120                 125
```

```
<210>  38
<211>  126
<212>  PRT
<213>  Lama glama

<400>  38

Glu Val Gln Leu Val Glu Ser Gly Gly Gly Leu Val Gln Pro Gly Asp
1               5                   10                  15

Ser Leu Lys Leu Ser Cys Ala Phe Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Ser Ser Gly Gly Tyr Ile Tyr Asp Ser Val Ser Leu
    50                  55                  60

Glu Gly Arg Phe Thr Ile Ser Arg Asp Asn Thr Lys Asn Thr Val Tyr
65                  70                  75                  80

Leu Gln Met Pro Ser Leu Lys Pro Glu Asp Thr Ala Asp Tyr Tyr Cys
                85                  90                  95

Ala Ala Ser Arg Ala Tyr Gly Ser Ser Arg Leu Arg Leu Ala Asp Thr
            100                 105                 110
```

Tyr Asp Tyr Trp Gly Gln Gly Thr Gln Val Thr Val Ser Ser
115 120 125

<210> 39
<211> 126
<212> PRT
<213> Lama glama

<400> 39

Glu Val Gln Leu Val Glu Ser Gly Gly Gly Leu Val Gln Pro Gly Asp
1 5 10 15

Ser Leu Lys Leu Ser Cys Ala Phe Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Ser Ser Gly Gly Tyr Ile Tyr Asp Ser Val Ser Leu
50 55 60

Glu Gly Arg Phe Thr Ile Ser Arg Asp Asn Thr Lys Asn Thr Val Tyr
65 70 75 80

Leu Gln Thr Pro Ser Leu Lys Pro Glu Asp Thr Ala Val Tyr Tyr Cys
85 90 95

Ala Ala Ser Arg Ala Tyr Gly Ser Ser Arg Leu Arg Leu Ala Asp Thr
100 105 110

Tyr Asp Tyr Trp Gly Gln Gly Thr Gln Val Thr Val Ser Ser
115 120 125

<210> 40
<211> 125
<212> PRT
<213> Lama glama

<400> 40

Glu Val Gln Leu Val Glu Ser Gly Gly Gly Leu Val Gln Pro Gly Asp
1 5 10 15

Ser Leu Lys Leu Ser Cys Val Ala Ser Gly Arg Thr Ser Ser Ser Tyr
20 25 30

Ser Val Gly Trp Phe Arg Gln Ala Gln Gly Lys Glu Arg Glu Phe Val
35 40 45

```
Val Ala Ile Ser Ser Gly Gly Tyr Ile Tyr Asp Ser Val Ser Leu Gln
    50                  55                  60

Gly Arg Phe Thr Ile Ser Arg Asp Asn Thr Lys Asn Thr Val Tyr Leu
65                  70                  75                  80

Gln Thr Pro Ser Leu Lys Pro Glu Asp Thr Ala Val Tyr Tyr Cys Ala
                85                  90                  95

Ala Ser Arg Ala Tyr Gly Ser Ser Arg Leu Arg Leu Ala Asp Thr Tyr
            100                 105                 110

Asp Tyr Trp Gly Gln Gly Thr Gln Val Thr Val Ser Ser
        115                 120                 125


<210>  41
<211>  126
<212>  PRT
<213>  Lama glama

<400>  41

Glu Val Gln Leu Val Glu Ser Gly Gly Gly Leu Val Gln Pro Gly Asp
1               5                   10                  15

Ser Leu Lys Leu Ser Cys Ala Phe Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Ser Ser Gly Gly Tyr Ile Tyr Asp Ser Val Ser Leu
    50                  55                  60

Glu Gly Arg Phe Thr Ile Ser Arg Asp Asn Thr Lys Asn Thr Val Tyr
65                  70                  75                  80

Leu Gln Thr Pro Ser Leu Lys Pro Glu Asp Ala Ala Asp Tyr Tyr Cys
                85                  90                  95

Ala Ala Ser Arg Ala Tyr Gly Ser Ser Arg Leu Arg Leu Ala Asp Thr
            100                 105                 110

Tyr Asp Tyr Trp Gly Gln Gly Thr Gln Val Thr Val Ser Ser
        115                 120                 125


<210>  42
<211>  126
<212>  PRT
<213>  Lama glama
```

<400> 42

Glu Val Gln Leu Val Glu Ser Gly Gly Gly Leu Val Gln Pro Gly Asp
1               5                   10                  15

Ser Leu Lys Leu Ser Cys Ala Phe Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Ser Ser Gly Gly Tyr Ile Tyr Asp Ser Val Ser Leu
    50                  55                  60

Glu Gly Arg Phe Thr Ile Ser Arg Asp Asn Thr Arg Asn Thr Val Tyr
65                  70                  75                  80

Leu Gln Thr Pro Ser Leu Lys Pro Glu Asp Thr Ala Asp Tyr Tyr Cys
                85                  90                  95

Ala Ala Ser Arg Ala Tyr Gly Ser Ser Arg Leu Arg Leu Ala Asp Thr
            100                 105                 110

Tyr Asp Tyr Trp Gly Gln Gly Thr Gln Val Thr Val Ser Ser
        115                 120                 125

<210> 43
<211> 125
<212> PRT
<213> Lama glama

<400> 43

Glu Val Gln Leu Val Glu Ser Gly Gly Gly Leu Val Gln Ala Gly Gly
1               5                   10                  15

Ser Leu Arg Leu Ser Cys Ala Phe Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Ser Gly Gly Tyr Lys Tyr Asp Ala Val Ser Leu Glu
    50                  55                  60

Gly Arg Phe Thr Ile Ser Arg Asp Asn Thr Lys Asn Thr Val Tyr Leu
65                  70                  75                  80

Gln Thr Pro Ser Leu Lys Pro Glu Asp Thr Ala Val Tyr Tyr Cys Ala

85 90 95

Ala Ser Arg Ala Tyr Ala Ser Ser Arg Leu Arg Leu Ala Asp Thr Tyr
100 105 110

Asp Tyr Trp Gly Gln Gly Thr Gln Val Thr Val Ser Ser
115 120 125

<210> 44
<211> 126
<212> PRT
<213> Lama glama

<400> 44

Glu Val Gln Leu Val Glu Ser Gly Gly Asp Leu Val Gln Pro Gly Asp
1 5 10 15

Ser Leu Lys Leu Ser Cys Ala Ala Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Ser Ser Gly Gly Tyr Ile Tyr Asp Ser Val Ser Leu
50 55 60

Glu Gly Arg Phe Thr Ile Ser Arg Asp Asn Thr Lys Asn Thr Val Tyr
65 70 75 80

Leu Gln Thr Pro Ser Leu Lys Pro Glu Asp Thr Ala Val Tyr Tyr Cys
85 90 95

Ala Ala Ser Arg Ala Tyr Gly Ser Ser Arg Leu Arg Leu Ala Asp Thr
100 105 110

Tyr Asp Tyr Trp Gly Gln Gly Thr Gln Val Thr Val Ser Ser
115 120 125

<210> 45
<211> 126
<212> PRT
<213> Lama glama

<400> 45

Glu Val Gln Leu Val Glu Ser Glu Gly Gly Leu Val Gln Ala Gly Asp
1 5 10 15

Ser Leu Arg Leu Ser Cys Ala Ala Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Ser Ser Gly Asn Tyr Lys Tyr Asp Ser Val Ser Leu
50 55 60

Glu Gly Arg Phe Thr Ile Ser Arg Asp Asn Thr Lys Asn Thr Val Tyr
65 70 75 80

Leu Gln Ile Asn Ser Leu Lys Pro Glu Asp Thr Ala Val Tyr Tyr Cys
85 90 95

Ala Ala Ser Arg Ala Tyr Gly Ser Ser Arg Leu Arg Leu Gly Asp Thr
100 105 110

Tyr Asp Tyr Trp Gly Gln Gly Thr Gln Val Thr Val Ser Ser
115 120 125

<210> 46
<211> 125
<212> PRT
<213> Lama glama

<400> 46

Glu Val Gln Leu Val Glu Ser Gly Gly Gly Leu Val Gln Pro Gly Asp
1 5 10 15

Ser Leu Lys Leu Ser Cys Ala Phe Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ala Ser Gly Gly Tyr Ile Tyr Asp Ala Val Ser Leu Glu
50 55 60

Gly Arg Phe Thr Ile Ser Arg Asp Asn Thr Lys Asp Thr Val Tyr Leu
65 70 75 80

Gln Thr Pro Ser Leu Lys Pro Glu Asp Thr Ala Val Tyr Tyr Cys Ala
85 90 95

Ala Ser Arg Ala Tyr Gly Ser Ser Arg Leu Arg Leu Ala Asp Thr Tyr
100 105 110

Asp Tyr Trp Gly Gln Gly Thr Gln Val Thr Val Ser Ser
115 120 125

<210> 47
<211> 125
<212> PRT
<213> Artificial

<220>
<223> Mutated lama glama sequence

<400> 47

```
Glu Val Gln Leu Val Glu Ser Gly Gly Gly Leu Val Gln Thr Gly Gly
1               5                   10                  15

Ser Leu Arg Leu Ser Cys Glu Ala Ser Gly Arg Thr Phe Ser Ser Tyr
            20                  25                  30

Ser Met Gly Trp Phe Arg Gln Ala Pro Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Arg Asp Asn Ala Lys Asn Thr Val Tyr Leu
65                  70                  75                  80

Gln Ile Asn Ser Leu Arg Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Leu Val Thr Val Ser Ser
        115                 120                 125
```

<210> 48
<211> 125
<212> PRT
<213> Artificial

<220>
<223> Mutated lama glama sequence

<400> 48

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Arg Thr Phe Ser Ser Tyr
            20                  25                  30

Ser Met Gly Trp Phe Arg Gln Ala Pro Gly Lys Glu Arg Glu Phe Val
```

```
        35                  40                  45

Val Ala Ile Ser Lys Gly Gly Tyr Lys Tyr Asp Ser Val Ser Leu Glu
    50                  55                  60

Gly Arg Phe Thr Ile Ser Arg Asp Asn Ala Lys Asn Thr Val Tyr Leu
65                  70                  75                  80

Gln Met Asn Ser Leu Arg Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Leu Val Thr Val Ser Ser
        115                 120                 125


<210>  49
<211>  125
<212>  PRT
<213>  Artificial

<220>
<223>  Mutated lama glama sequence

<400>  49

Glu Val Gln Leu Val Glu Ser Gly Gly Gly Leu Val Gln Pro Gly Gly
1               5                   10                  15

Ser Leu Arg Leu Ser Cys Glu Ala Ser Gly Arg Thr Phe Ser Ser Tyr
            20                  25                  30

Ser Met Gly Trp Phe Arg Gln Ala Pro Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Arg Asp Asn Ala Lys Asn Thr Val Tyr Leu
65                  70                  75                  80

Gln Ile Asn Ser Leu Arg Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Leu Val Thr Val Ser Ser
        115                 120                 125
```

<210> 50
<211> 125
<212> PRT
<213> Artificial

<220>
<223> Mutated lama glama sequence

<400> 50

Glu Val Gln Leu Val Glu Ser Gly Gly Gly Leu Val Gln Thr Gly Gly
1 5 10 15

Ser Leu Arg Leu Ser Cys Glu Val Ser Gly Arg Thr Phe Ser Ser Tyr
20 25 30

Ser Met Gly Trp Phe Arg Gln Ala Pro Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Arg Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Leu Val Thr Val Ser Ser
115 120 125

<210> 51
<211> 125
<212> PRT
<213> Artificial

<220>
<223> Mutated lama glama sequence

<400> 51

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val

35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Met Asn Ser Leu Arg Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Leu Val Thr Val Ser Ser
115 120 125

<210> 52
<211> 125
<212> PRT
<213> Artificial

<220>
<223> Mutated lama glama sequence

<400> 52

Glu Val Gln Leu Val Glu Ser Gly Gly Gly Leu Val Gln Pro Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Pro Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Arg Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Leu Val Thr Val Ser Ser
115 120 125

<210> 53
<211> 125
<212> PRT
<213> Artificial

<220>
<223> Mutated lama glama sequence

<400> 53

Glu Val Gln Leu Val Glu Ser Gly Gly Gly Leu Val Gln Thr Gly Gly
1 5 10 15

Ser Leu Arg Leu Ser Cys Glu Val Ser Gly Arg Thr Phe Ser Ser Tyr
20 25 30

Ser Met Gly Trp Phe Arg Gln Ala Pro Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Arg Asp Asn Ala Lys Asn Thr Val Tyr Leu
65 70 75 80

Gln Ile Asn Ser Leu Arg Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Leu Val Thr Val Ser Ser
115 120 125

<210> 54
<211> 125
<212> PRT
<213> Artificial

<220>
<223> Mutated lama glama sequence

<400> 54

Glu Val Gln Leu Val Glu Ser Gly Gly Gly Leu Val Gln Thr Gly Asp
1 5 10 15

Ser Leu Arg Leu Ser Cys Ala Val Ser Gly Arg Thr Phe Ser Ser Tyr
20 25 30

Ser Met Gly Trp Phe Arg Gln Ala Gln Gly Lys Glu Arg Glu Phe Val

35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Arg Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Leu Val Thr Val Ser Ser
115 120 125

<210> 55
<211> 125
<212> PRT
<213> Artificial

<220>
<223> Mutated lama glama sequence

<400> 55

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Lys Gly Gly Tyr Lys Tyr Asp Ala Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Lys Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Gln Val Thr Val Ser Ser
115 120 125

<210> 56
<211> 125
<212> PRT
<213> Artificial

<220>
<223> Mutated lama glama sequence

<400> 56

```
Asp Val Gln Leu Val Glu Ser Gly Gly Gly Leu Val Gln Pro Gly Gly
1               5                   10                  15

Ser Leu Arg Leu Ser Cys Ala Ala Ser Gly Arg Thr Phe Ser Ser Tyr
            20                  25                  30

Ser Met Gly Trp Phe Arg Gln Ala Pro Gly Lys Glu Arg Glu Phe Val
        35                  40                  45

Val Ala Ile Ser Lys Gly Gly Tyr Lys Tyr Asp Ala Val Ser Leu Glu
    50                  55                  60

Gly Arg Phe Thr Ile Ser Arg Asp Asn Ala Lys Asn Thr Val Tyr Leu
65                  70                  75                  80

Gln Met Asn Ser Leu Arg Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Leu Val Thr Val Ser Ser
        115                 120                 125
```

<210> 57
<211> 125
<212> PRT
<213> Artificial

<220>
<223> Mutated lama glama sequence

<400> 57

```
Asp Val Gln Leu Val Glu Ser Gly Gly Gly Leu Val Gln Pro Gly Gly
1               5                   10                  15

Ser Leu Arg Leu Ser Cys Ala Ala Ser Gly Arg Thr Phe Ser Ser Tyr
            20                  25                  30

Ser Met Gly Trp Phe Arg Gln Ala Pro Gly Lys Glu Arg Glu Phe Val
```

35 40 45

Val Ala Ile Ser Lys Gly Gly Tyr Lys Tyr Asp Ala Val Ser Leu Glu
50 55 60

Gly Arg Phe Thr Ile Ser Arg Asp Asn Ala Lys Asn Thr Val Tyr Leu
65 70 75 80

Gln Ile Asn Ser Leu Arg Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Leu Val Thr Val Ser Ser
115 120 125

<210> 58
<211> 122
<212> PRT
<213> Lama glama

<400> 58

Glu Val Gln Leu Val Glu Ser Gly Gly Gly Leu Val Gln Ala Gly Gly
1 5 10 15

Ser Leu Arg Leu Ser Cys Thr Ala Ser Gly Gly Ser Phe Ser Ser Tyr
20 25 30

Gly Met Gly Trp Phe Arg Gln Ser Pro Gly Lys Glu Arg Glu Phe Val
35 40 45

Ser Ala Ile Ser Glu Tyr Ser Asn Thr Tyr Cys Ser Asp Ser Val Arg
50 55 60

Gly Arg Phe Thr Ile Ser Arg Asp Asn Thr Lys Asn Thr Val Tyr Leu
65 70 75 80

Gln Met Asn Ser Leu Thr Pro Asp Asp Thr Ala Ile Tyr Tyr Cys Ala
85 90 95

Ala Ser Pro Thr Ile Leu Leu Thr Thr Glu Gln Trp Tyr Lys Tyr Trp
100 105 110

Gly Gln Gly Thr Gln Val Thr Val Ser Ser
115 120

<210> 59

<211> 126
<212> PRT
<213> Lama glama

<400> 59

```
Glu Val Gln Leu Val Glu Ser Gly Gly Gly Leu Val Gln Ala Gly Asp
1               5                   10                  15

Ser Leu Arg Leu Ser Cys Val Ala Thr Gly Arg Thr Phe Arg Ala Ser
            20                  25                  30

Asp Met Gly Trp Phe Arg Gln Ala Pro Gly Lys Glu Arg Glu Phe Val
        35                  40                  45

Ala Ala Ile Asn Trp Ser Gly Leu Ser Thr Phe Tyr Thr Asp Ser Val
    50                  55                  60

Lys Gly Arg Phe Thr Ile Ser Arg Asp Asn Asp Asn Gly Ala Leu Tyr
65                  70                  75                  80

Leu Gln Met Asn Thr Leu Lys Pro Glu Asp Thr Ala Val Tyr Ser Cys
                85                  90                  95

Ala Ala Gly Arg Ile Pro Ser Ser Ser Arg Phe Ser Ser Pro Ala Ala
            100                 105                 110

Tyr Ala Ser Trp Gly Gln Gly Thr Gln Val Thr Val Ser Ser
        115                 120                 125
```

<210> 60
<211> 125
<212> PRT
<213> Lama glama

<400> 60

```
Glu Val Gln Leu Val Glu Ser Gly Gly Gly Leu Val Gln Ala Gly Gly
1               5                   10                  15

Ser Leu Arg Leu Ser Cys Thr Ala Ser Thr Ser Ile Tyr Thr Ile Thr
            20                  25                  30

Val Met Ala Trp Phe Arg Gln Ala Pro Gly Lys Glu Arg Glu Phe Val
        35                  40                  45

Ala Ala Ile Thr Trp Ser Ala Pro Thr Thr Tyr Tyr Ala Asp Ser Val
    50                  55                  60

Lys Gly Arg Phe Thr Ile Ser Arg Asp Asn Ala Lys Asn Thr Val Tyr
65                  70                  75                  80
```

Leu Arg Met Asn Ser Leu Lys Pro Glu Asp Ser Ala Ile Tyr Tyr Cys
85 90 95

Ala Ala Asp Arg Phe Lys Gly Arg Ser Ile Val Thr Pro Ser Asp Tyr
100 105 110

Arg Tyr Trp Gly Gln Gly Thr Gln Val Thr Val Ser Ser
115 120 125

<210> 61
<211> 111
<212> PRT
<213> Lama glama

<400> 61

Glu Val Gln Leu Val Glu Ser Gly Gly Gly Leu Val Gln Pro Gly Gly
1 5 10 15

Ser Leu Arg Leu Ser Cys Ala Ala Ser Gly Ser Ala Val Gly Asp Ile
20 25 30

Thr Val Ala Trp Tyr Arg Gln Ala Pro Gly Ile Gln Arg Gln Leu Val
35 40 45

Ala Thr Ile Thr Pro Ser Gly Tyr Thr Tyr Tyr Trp Asp Phe Val Lys
50 55 60

Gly Arg Phe Thr Ile Ser Arg Asp Asn Ser Lys Asn Ile Val Tyr Leu
65 70 75 80

Gln Met Asn Ser Leu Lys Pro Glu Asp Thr Ala Ala Tyr Tyr Cys Asn
85 90 95

Thr Gln Phe Tyr Trp Gly Gln Gly Thr Gln Val Thr Val Ser Ser
100 105 110

<210> 62
<211> 128
<212> PRT
<213> Lama glama

<400> 62

Glu Val Gln Leu Val Glu Ser Gly Gly Gly Leu Val Gln Ala Gly Gly
1 5 10 15

Ser Leu Arg Leu Ser Cys Ala Ala Ser Gly Arg Thr Phe Ser Thr Asp
20 25 30

Asp Val Gly Trp Phe Arg Gln Ala Pro Gly Lys Glu Arg Glu Phe Val
35 40 45

Ala Val Ile Arg Trp Ser Thr Gly Gly Thr Tyr Thr Ser Asp Ser Val
50 55 60

Lys Gly Arg Phe Thr Leu Ser Arg Asp Asn Ala Lys Asn Thr Met Tyr
65 70 75 80

Leu Gln Met Asn Ser Leu Lys Pro Glu Asp Thr Ala Val Tyr Tyr Cys
85 90 95

Ala Ala Arg Ser Arg Pro Leu Gly Ala Gly Ala Trp Tyr Ser Gly Glu
100 105 110

Lys His Tyr Asn Tyr Trp Gly Gln Gly Thr Gln Val Thr Val Ser Ser
115 120 125

<210> 63
<211> 121
<212> PRT
<213> Lama glama

<400> 63

Glu Val Gln Leu Val Glu Ser Gly Gly Gly Leu Ala Gln Ala Gly Asp
1 5 10 15

Ser Leu Arg Leu Ser Cys Ala Ala Ser Gly Arg Ser Phe Ser His Tyr
20 25 30

Asn Met Gly Trp Phe Arg Gln Ala Pro Gly Lys Glu Arg Glu Phe Val
35 40 45

Ala Ser Ile Arg Gly Gly Gly Gly Ser Thr Thr Tyr Ala Asn Ser Val
50 55 60

Lys Asp Arg Phe Thr Ile Ser Arg Glu Asn Ala Lys Asn Thr Val Tyr
65 70 75 80

Leu Gln Met Asn Ser Leu Lys Pro Glu Asp Thr Ala Val Tyr Tyr Cys
85 90 95

Ala Ala Thr Ala Phe Tyr Arg Gly Pro Tyr Asp Tyr Asp Tyr Trp Gly
100 105 110

Gln Gly Thr Gln Val Thr Val Ser Ser
115 120

<210> 64
<211> 113
<212> PRT
<213> Lama glama

<400> 64

Glu Val Gln Leu Val Glu Ser Gly Gly Gly Leu Val Gln Pro Gly Gly
1 5 10 15

Ser Leu Arg Leu Ser Cys Val Ala Ser Gly Ile Arg Phe Met Ser Met
20 25 30

Ala Trp Tyr Arg Gln Ala Pro Gly Lys His Arg Glu Leu Val Ala Arg
35 40 45

Ile Ser Ser Gly Gly Thr Thr Ala Tyr Val Asp Ser Val Lys Gly Arg
50 55 60

Phe Thr Ile Ser Arg Asp Asn Ser Lys Asn Thr Val Tyr Leu Gln Met
65 70 75 80

Asn Ser Leu Lys Ala Glu Asp Thr Ala Val Tyr Tyr Cys Asn Thr Phe
85 90 95

Ser Ser Arg Pro Asn Pro Trp Gly Ala Gly Thr Gln Val Thr Val Ser
100 105 110

Ser

<210> 65
<211> 116
<212> PRT
<213> Lama glama

<400> 65

Glu Val Gln Leu Val Glu Ser Gly Gly Gly Leu Val Gln Pro Gly Gly
1 5 10 15

Ser Leu Arg Leu Ser Cys Ala Ala Ser Gly Asn Ile Phe Ser Asn Asn
20 25 30

Ala Met Ala Trp Tyr Arg Gln Ala Pro Gly Lys Gln Arg Glu Leu Val
35 40 45

Ala Arg Ile Ser Ser Gly Gly Gly Phe Thr Tyr Tyr Leu Asp Ser Val
50 55 60

Lys Gly Arg Phe Thr Val Ser Arg Asp Asn Ala Lys Asn Thr Val Tyr
65 70 75 80

Leu Gln Met Asn Ser Leu Lys Pro Glu Asp Thr Ala Val Tyr Tyr Cys
85 90 95

Asn Ala Ala Tyr Arg Thr Tyr Asn Tyr Trp Gly Gln Gly Thr Gln Val
100 105 110

Thr Val Ser Ser
115

<210> 66
<211> 125
<212> PRT
<213> Lama glama

<400> 66

Glu Val Gln Leu Val Glu Ser Gly Gly Gly Leu Val Gln Ala Gly Gly
1 5 10 15

Ser Leu Arg Leu Ser Cys Ala Ala Ser Thr Ser Ile Tyr Ser Ile Thr
20 25 30

Val Met Ala Trp Phe Arg Gln Ala Pro Gly Lys Glu Ser Glu Phe Val
35 40 45

Ala Ala Ile Thr Trp Ser Ala Pro Ser Ser Tyr Tyr Ala Asp Ser Val
50 55 60

Lys Gly Arg Phe Thr Ile Ser Arg Asp Asn Ala Lys Asn Thr Val Tyr
65 70 75 80

Leu Gln Met Asn Ser Leu Lys Pro Glu Asp Ser Ala Ile Tyr Tyr Cys
85 90 95

Ala Ala Asp Arg Phe Lys Gly Arg Ser Ile Val Thr Arg Ser Asp Tyr
100 105 110

Lys Tyr Trp Gly Gln Gly Thr Gln Val Thr Val Ser Ser
115 120 125

<210> 67
<211> 125
<212> PRT
<213> Lama glama

<400> 67

Glu Val Gln Leu Val Glu Ser Gly Gly Gly Leu Val Gln Ala Gly Gly

1 5 10 15

Ser Leu Arg Leu Ser Cys Ala Val Ser Thr Ser Ile Tyr Ser Ile Ser
20 25 30

Val Met Ala Trp Phe Arg Gln Ala Pro Gly Lys Glu Arg Ala Phe Val
35 40 45

Ala Ala Ile Thr Trp Ser Ala Pro Thr Thr Tyr Tyr Ala Asp Ser Val
50 55 60

Lys Gly Arg Phe Thr Ile Ser Arg Asp Asn Ala Lys Asn Thr Val Tyr
65 70 75 80

Leu Gln Thr Asn Ser Leu Lys Pro Glu Asp Ser Ala Ile Tyr Tyr Cys
85 90 95

Ala Ala Asp Arg Phe Lys Gly Arg Ser Ile Val Thr Arg Ser Asp Tyr
100 105 110

Arg Tyr Trp Gly Gln Gly Thr Gln Val Thr Val Ser Ser
115 120 125

<210> 68
<211> 126
<212> PRT
<213> Lama glama

<400> 68

Glu Val Gln Leu Val Glu Ser Gly Gly Gly Leu Val Gln Thr Gly Gly
1 5 10 15

Ser Leu Arg Leu Ser Cys Ala Ala Ser Gly Arg Thr Phe Ser Asn Tyr
20 25 30

Ala Met Ala Trp Phe Arg Gln Ala Pro Gly Lys Glu Arg Glu Phe Val
35 40 45

Ser Ala Ile Asn Gln Arg Gly Ser Asn Thr Asn Tyr Ala Asp Ser Val
50 55 60

Lys Gly Arg Phe Thr Ile Ser Arg Asp Ser Ala Lys Asn Ser Val Phe
65 70 75 80

Leu Gln Met Asn Ser Leu Lys Pro Glu Asp Thr Ala Val Tyr Tyr Cys
85 90 95

Ala Ala Ser Thr Trp Tyr Gly Tyr Ser Thr Tyr Ala Arg Arg Glu Glu

100 105 110

Tyr Arg Tyr Trp Gly Gln Gly Thr Gln Val Thr Val Ser Ser
115 120 125

<210> 69
<211> 123
<212> PRT
<213> Lama glama

<400> 69

Glu Val Gln Leu Val Glu Ser Gly Gly Gly Leu Val Gln Ala Gly Gly
1 5 10 15

Ser Leu Arg Leu Ser Cys Ala Ala Ser Gly Arg Ser Phe Ser Asp Asn
20 25 30

Val Met Gly Trp Phe Arg Gln Ala Ala Gly Lys Glu Arg Glu Phe Val
35 40 45

Ala His Ile Ser Arg Gly Gly Ser Arg Thr Glu Tyr Ala Glu Ser Val
50 55 60

Lys Gly Arg Phe Thr Ile Ser Arg Asp Asn Thr Lys Lys Thr Met Tyr
65 70 75 80

Leu Gln Met Asn Ser Leu Lys Pro Glu Asp Thr Ala Val Tyr Tyr Cys
85 90 95

Ala Ala Ser Arg Ser Val Ala Leu Ala Thr Ala Arg Pro Tyr Asp Tyr
100 105 110

Trp Gly Gln Gly Thr Gln Val Thr Val Ser Ser
115 120

<210> 70
<211> 121
<212> PRT
<213> Lama glama

<400> 70

Glu Val Gln Leu Val Glu Ser Gly Gly Gly Leu Ala Gln Ala Gly Gly
1 5 10 15

Ser Leu Arg Leu Ser Cys Thr Thr Ser Gly Leu Thr Phe Ser Ser Tyr
20 25 30

Tyr Met Gly Trp Phe Arg Gln Ala Pro Gly Lys Glu Arg Glu Phe Val
35 40 45

```
Ala Thr Ile Ser Trp Asn Lys Ile Ser Thr Ile Tyr Thr Asp Ser Val
    50                  55                  60

Lys Gly Arg Phe Thr Val Ser Arg Asp Asn Asn Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Asp Ala Ser Arg Pro Thr Leu Arg Ile Pro Gln Tyr Trp Gly
            100                 105                 110

Gln Gly Thr Gln Val Thr Val Ser Ser
        115                 120
```

<210> 71
<211> 115
<212> PRT
<213> Lama glama

<400> 71

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Ser Ile Val Arg Ser Asp
            20                  25                  30

Val Met Gly Trp Tyr Arg Gln Ala Pro Gly Lys Gln Arg Glu Leu Val
        35                  40                  45

Ala Phe Ile Arg Ser Leu Gly Ser Thr Tyr Tyr Ala Gly Ser Val Lys
    50                  55                  60

Gly Arg Phe Thr Ile Ser Arg Asp Asp Ala Ala Asn Thr Val Tyr Leu
65                  70                  75                  80

Gln Met Asn Asn Leu Lys Pro Glu Asp Thr Ala Val Tyr Tyr Cys Asn
                85                  90                  95

Ala Arg Phe Ser Gly Glu Ser Tyr Trp Gly Gln Gly Thr Pro Val Thr
            100                 105                 110

Val Ser Ser
        115
```

<210> 72
<211> 124

<212> PRT
<213> Lama glama

<400> 72

Glu Val Gln Leu Val Glu Ser Gly Gly Gly Leu Val Gln Ala Gly Gly
1 5 10 15

Ser Leu Arg Leu Ser Cys Ala Val Ser Gly Ser Thr Phe Gly Leu Tyr
20 25 30

Ala Met Gly Trp Phe Arg Gln Ala Pro Gly Arg Glu Arg Glu Phe Leu
35 40 45

Ser Ala Ile Thr Trp Ser Ala Gly Asp Thr Gln Tyr Ala Asp Ser Val
50 55 60

Lys Gly Arg Phe Thr Ile Ser Arg Asp Asn Ala Arg Asn Thr Val Asn
65 70 75 80

Leu Gln Met Asn Gly Leu Lys Pro Glu Asp Thr Ala Val Tyr Tyr Cys
85 90 95

Ala Gly Arg Gln Trp Gly Gly Thr Tyr Tyr Tyr His Gly Ser Tyr Ala
100 105 110

Tyr Trp Gly Gln Gly Thr Gln Val Thr Val Ser Ser
115 120

<210> 73
<211> 113
<212> PRT
<213> Lama glama

<400> 73

Glu Val Gln Leu Val Glu Ser Gly Gly Gly Leu Val Gln Pro Gly Gly
1 5 10 15

Ser Leu Arg Leu Ser Cys Val Ala Ser Gly Ile Arg Phe Met Ser Met
20 25 30

Ala Trp Tyr Arg Gln Ala Pro Gly Lys His Arg Glu Leu Val Ala Arg
35 40 45

Ile Ser Ser Glu Gly Thr Thr Ala Tyr Val Asp Ser Val Lys Gly Arg
50 55 60

Phe Thr Ile Ser Arg Asp Asn Ser Lys Asn Thr Val Tyr Leu Gln Met
65 70 75 80

```
Asn Ser Leu Lys Ala Glu Asp Thr Ala Val Tyr Tyr Cys Asn Thr Phe
                85                  90                  95

Ser Ser Arg Pro Asn Pro Trp Gly Ala Gly Thr Thr Val Thr Val Ser
            100                 105                 110

Ser


<210>  74
<211>  128
<212>  PRT
<213>  Lama glama

<400>  74

Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Phe Ser Thr Asp
            20                  25                  30

Asp Val Gly Trp Phe Arg Gln Ala Pro Gly Lys Glu Arg Glu Phe Val
        35                  40                  45

Ala Val Ile Arg Trp Ser Thr Gly Gly Thr Tyr Thr Ser Asp Ser Val
    50                  55                  60

Ala Gly Arg Phe Thr Leu Ser Arg Asp Asn Ala Lys Asn Thr Met Tyr
65                  70                  75                  80

Leu Gln Met Asn Ser Leu Lys Pro Glu Asp Thr Ala Val Tyr Tyr Cys
                85                  90                  95

Ala Ala Arg Ser Arg Pro Leu Gly Ala Gly Ala Trp Tyr Thr Gly Glu
            100                 105                 110

Thr Arg Tyr Asp Ser Trp Gly Gln Gly Thr Gln Val Thr Val Ser Ser
        115                 120                 125


<210>  75
<211>  122
<212>  PRT
<213>  Lama glama

<400>  75

Glu Val Gln Leu Val Glu Ser Gly Gly Gly Leu Val Gln Pro Gly Asp
1               5                   10                  15
```

```
Ser Leu Arg Leu Ser Cys Ala Ala Ser Gly Leu Ser Phe Ser Arg Tyr
            20                  25                  30

Gly Met Gly Trp Phe Arg Gln Ala Pro Gly Lys Glu Arg Glu Phe Val
        35                  40                  45

Ile Ala Ile Ser Glu Tyr Asp Asn Val Tyr Thr Ala Asp Ser Val Arg
    50                  55                  60

Gly Arg Phe Thr Ile Ser Arg Asp Asn Ser Lys Ser Thr Val Tyr Leu
65                  70                  75                  80

Gln Met Asn Ser Leu Lys Ser Glu Asp Thr Ala Val Tyr Tyr Cys Ala
                85                  90                  95

Ala Ser Pro Thr Ile Leu Leu Ser Thr Asp Glu Trp Tyr Lys Tyr Trp
            100                 105                 110

Gly Arg Gly Thr Gln Val Thr Val Ser Ser
        115                 120
```

<210> 76
<211> 128
<212> PRT
<213> Lama glama

<400> 76

```
Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Phe Ser Thr Asp
            20                  25                  30

Asp Val Gly Trp Phe Arg Gln Ala Pro Gly Lys Glu Arg Glu Phe Val
        35                  40                  45

Ala Val Ile Arg Trp Ser Thr Gly Gly Thr Tyr Thr Ser Asp Ser Val
    50                  55                  60

Lys Gly Arg Phe Thr Leu Ser Arg Asp Asn Ala Lys Asn Thr Met Tyr
65                  70                  75                  80

Leu Gln Met Asn Ser Leu Lys Pro Glu Asp Thr Ala Val Tyr Tyr Cys
                85                  90                  95

Ala Ala Arg Ser Arg Pro Leu Gly Ala Gly Ala Trp Tyr Thr Gly Glu
            100                 105                 110
```

Thr Arg Tyr Asn Tyr Trp Gly Gln Gly Thr Gln Val Thr Val Ser Ser
115 120 125

<210> 77
<211> 127
<212> PRT
<213> Lama glama

<400> 77

Glu Val Gln Leu Val Glu Ser Gly Gly Gly Leu Val Gln Ala Gly Gly
1 5 10 15

Ser Leu Ser Leu Ser Cys Ala Ala Ser Ala Arg Ala Phe Ser Asn Tyr
20 25 30

Ala Met Gly Trp Phe Arg Gln Val Pro Gly Arg Glu Arg Glu Phe Val
35 40 45

Ala Val Ile Thr Arg Ser Pro Ser Asn Thr Tyr Tyr Thr Asp Ser Val
50 55 60

Lys Gly Arg Phe Thr Ile Ser Arg Asp Asn Ala Lys Asn Ile Val Tyr
65 70 75 80

Leu Gln Met Asn Ser Leu Lys Pro Glu Asp Thr Ala Val Tyr Tyr Cys
85 90 95

Ala Ala His Tyr Trp Asn Ser Asp Ser Tyr Thr Tyr Thr Asp Ser Arg
100 105 110

Trp Tyr Asn Tyr Trp Gly Gln Gly Thr Gln Val Thr Val Ser Ser
115 120 125

<210> 78
<211> 124
<212> PRT
<213> Lama glama

<400> 78

Glu Val Gln Leu Val Glu Ser Gly Gly Gly Leu Val Gln Ala Gly Gly
1 5 10 15

Ser Leu Arg Leu Ser Cys Ala Ala Ser Gly Arg Thr Phe Ser Asn Tyr
20 25 30

Ala Met Gly Trp Phe Arg Gln Ala Pro Gly Lys Glu Arg Val Leu Val
35 40 45

Ala Asp Ile Ser Ser Ser Gly Ile Asn Thr Tyr Val Ala Asp Ala Val

50 55 60

Lys Gly Arg Phe Thr Ile Ser Arg Asp Asn Ala Lys Asn Thr Val Tyr
65 70 75 80

Leu Gln Met Asn Ser Leu Lys Pro Glu Asp Thr Ala Val Tyr Tyr Cys
85 90 95

Ala Ala Ser Ala Trp Trp Tyr Ser Gln Met Ala Arg Asp Asn Tyr Arg
100 105 110

Tyr Trp Gly Gln Gly Thr Gln Val Thr Val Ser Ser
115 120

<210> 79
<211> 125
<212> PRT
<213> Lama glama

<400> 79

Glu Val Gln Leu Val Glu Ser Gly Gly Gly Leu Val Gln Ala Gly Gly
1 5 10 15

Ser Leu Arg Leu Ser Cys Ala Ala Ser Gly Asp Thr Leu Ser Arg Tyr
20 25 30

Ala Met Gly Trp Phe Arg Gln Ala Pro Gly Lys Glu Arg Glu Phe Val
35 40 45

Ala Ser Ile Asn Thr Ser Gly Lys Arg Thr Ser Tyr Ala Asp Ser Met
50 55 60

Lys Gly Arg Phe Ala Val Ser Arg Asp Asn Ala Lys Asn Thr Gly Tyr
65 70 75 80

Leu Gln Met Asn Ser Leu Lys Leu Glu Asp Thr Ala Thr Tyr Tyr Cys
85 90 95

Ala Ala Asp Arg Phe Phe Gly Ser Asp Ser Asn Glu Pro Arg Ala Tyr
100 105 110

Arg Tyr Trp Gly Gln Gly Thr Gln Val Thr Val Ser Ser
115 120 125

<210> 80
<211> 123
<212> PRT
<213> Lama glama

<400> 80

Glu Val Gln Leu Val Glu Ser Gly Gly Gly Leu Val Gln Ala Gly Glu
1               5                   10                  15

Ser Leu Arg Leu Ser Cys Val Ala Ser Gly Ile Thr Phe Ser Asn Tyr
            20                  25                  30

Asn Met Gly Trp Phe Arg Gln Ala Pro Gly Lys Glu Arg Glu Phe Val
        35                  40                  45

Ala Thr Ile Arg His His Gly Tyr Asp Thr Tyr Tyr Ala Glu Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Leu Tyr Ser Cys
                85                  90                  95

Ala Lys Lys Leu Phe Trp Asp Met Asp Pro Lys Thr Gly Phe Ser Ser
            100                 105                 110

Trp Gly Gln Gly Thr Gln Val Thr Val Ser Ser
        115                 120

<210> 81
<211> 126
<212> PRT
<213> Lama glama

<400> 81

Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Leu Ser Ser Tyr
            20                  25                  30

Gly Leu Gly Trp Phe Arg Gln Ala Pro Gly Lys Glu Arg Glu Phe Val
        35                  40                  45

Ala Ala Ile Gly Trp Ser Gly Ser Ser Thr Tyr Tyr Ala Asp Ser Val
    50                  55                  60

Lys Gly Arg Phe Thr Val Ser Val Asp Asn Ala Lys Asn Thr Val Tyr
65                  70                  75                  80

Leu Lys Met Asn Ser Leu Glu Pro Glu Asp Thr Ala Val Tyr Tyr Cys
                85                  90                  95

Ala Ala Lys Val Arg Asn Phe Asn Ser Asp Trp Asp Leu Leu Thr Ser
100 105 110

Tyr Asn Tyr Trp Gly Gln Gly Thr Gln Val Thr Val Ser Ser
115 120 125

<210> 82
<211> 125
<212> PRT
<213> Lama glama

<400> 82

Glu Val Gln Leu Val Glu Ser Gly Gly Gly Leu Val Gln Ala Gly Gly
1 5 10 15

Ser Leu Met Leu Ser Cys Ala Ala Ser Gly Arg Ala Leu Ser Ser Tyr
20 25 30

Ala Ile Gly Trp Phe Arg Gln Ala Pro Gly Arg Glu Arg Glu Phe Val
35 40 45

Ala Arg Ile Ser Trp Ser Gly Ala Asn Thr Tyr Tyr Ala Asp Ser Val
50 55 60

Lys Gly Arg Phe Thr Ile Ser Arg Gly Asn Ala Lys Asn Thr Val Tyr
65 70 75 80

Leu Gln Met Asn Ser Leu Lys Pro Glu Asp Thr Ala Ala Tyr Tyr Cys
85 90 95

Ala Ala Gln Thr Thr Ser Lys Tyr Asp Asn Tyr Asp Ala Arg Ala Tyr
100 105 110

Gly Tyr Trp Gly Gln Gly Thr Gln Val Thr Val Ser Ser
115 120 125

<210> 83
<211> 125
<212> PRT
<213> Lama glama

<400> 83

Glu Glu Gln Leu Val Glu Ser Gly Gly Gly Leu Val Gln Ala Gly Gly
1 5 10 15

Ser Leu Met Leu Ser Cys Ala Ala Ser Gly Arg Ala Leu Ser Ser Tyr
20 25 30

```
Ala Ile Gly Trp Phe Arg Gln Ala Pro Gly Arg Glu Arg Glu Phe Val
        35                  40                  45

Ala Arg Ile Ser Trp Ser Gly Ala Asn Thr Tyr Tyr Ala Asp Ser Val
    50                  55                  60

Lys Gly Arg Phe Thr Ile Ser Arg Gly Asn Ala Lys Asn Thr Val Tyr
65                  70                  75                  80

Leu Gln Met Asn Ser Leu Lys Pro Glu Asp Thr Ala Ala Tyr Tyr Cys
                85                  90                  95

Ala Ala Gln Thr Thr Ser Lys Tyr Asp Asn Tyr Asp Ala Arg Ala Tyr
            100                 105                 110

Gly Tyr Trp Gly Gln Gly Thr Gln Val Thr Val Ser Ser
        115                 120                 125


<210>  84
<211>  123
<212>  PRT
<213>  Lama glama

<400>  84

Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Phe Ser Ser Tyr
            20                  25                  30

Ala Met Gly Trp Phe Arg Gln Ala Pro Gly Lys Glu Arg Glu Phe Val
        35                  40                  45

Ala Thr Ile Ser Gln Ser Gly Tyr Ser Thr Tyr Tyr Ala Asp Ser Val
    50                  55                  60

Lys Gly Arg Phe Thr Ile Ser Arg Asp Asn Ala Lys Asn Thr Val Asn
65                  70                  75                  80

Leu Gln Met Asn Ser Leu Lys Pro Glu Asp Thr Ala Val Tyr Tyr Cys
                85                  90                  95

Ala Ala Asp Pro Phe Tyr Ser Tyr Gly Ser Pro Ser Pro Tyr Arg Tyr
            100                 105                 110

Trp Gly Gln Gly Thr Gln Val Thr Val Ser Ser
        115                 120
```

<210> 85
<211> 125
<212> PRT
<213> Lama glama

<400> 85

Glu Val Gln Leu Val Glu Ser Gly Gly Gly Leu Val Gln Pro Gly Gly
1 5 10 15

Ser Leu Arg Leu Ser Cys Ala Ser Ser Gly Arg Leu Phe Ser Phe Ser
20 25 30

Ala Met Gly Trp Phe Arg Gln Ala Pro Gly Lys Glu Arg Glu Phe Val
35 40 45

Ala Ala Phe Lys Trp Ser Gly Ser Thr Thr Tyr Tyr Ala Asp Tyr Val
50 55 60

Lys Gly Arg Phe Thr Ile Ser Thr Asp Asn Ala Lys Asn Ile Leu Phe
65 70 75 80

Leu Gln Met Asn Ser Leu Lys Pro Glu Asp Thr Ala Ile Tyr Tyr Cys
85 90 95

Ala Val Asp Arg Phe Tyr Thr Gly Arg Tyr Tyr Ser Ser Asp Glu Tyr
100 105 110

Asp Tyr Trp Gly Gln Gly Thr Gln Val Thr Val Ser Ser
115 120 125

<210> 86
<211> 125
<212> PRT
<213> Lama glama

<400> 86

Glu Val Gln Leu Val Glu Ser Gly Gly Gly Leu Val Gln Ala Gly Gly
1 5 10 15

Ser Leu Arg Leu Ser Cys Ala Ala Ser Thr Ser Ile Tyr Ser Ile Thr
20 25 30

Val Met Ala Trp Phe Arg Gln Ala Pro Gly Lys Glu Arg Glu Phe Val
35 40 45

Ala Ala Ile Thr Trp Ser Ala Pro Ser Ser Tyr Tyr Ala Asp Ser Val
50 55 60

Lys Gly Arg Phe Thr Ile Ser Arg Asp Asn Ala Lys Asn Thr Val Tyr
65 70 75 80

Leu Gln Val Asn Ser Leu Lys Pro Glu Asp Ser Ala Ile Tyr Tyr Cys
85 90 95

Ala Ala Asp Arg Phe Lys Gly Arg Ser Ile Val Thr Arg Ser Asp Tyr
100 105 110

Arg Tyr Trp Gly Gln Gly Thr Gln Val Thr Val Ser Ser
115 120 125

<210> 87
<211> 125
<212> PRT
<213> Lama glama

<400> 87

Glu Val Gln Leu Val Glu Ser Gly Gly Gly Leu Val Gln Ser Gly Gly
1 5 10 15

Ser Leu Arg Leu Ser Cys Ala Ala Ser Gly Arg Ser Phe Ser Ser Leu
20 25 30

Ala Met Gly Trp Phe Arg Gln Val Pro Gly Lys Asp Arg Glu Phe Val
35 40 45

Ala Ser Ile Ser Gln Ser Gly Ile Thr Thr Ser Tyr Ala Asp Ser Val
50 55 60

Lys Ser Arg Phe Thr Ile Ser Arg Asp Ser Ala Lys Asn Thr Val Tyr
65 70 75 80

Leu Gln Met Asn Leu Leu Lys Pro Glu Asp Thr Ala Val Tyr Tyr Cys
85 90 95

Ala Thr Ser Val Phe Tyr Ser Thr Ala Leu Thr Arg Pro Val Asp Tyr
100 105 110

Arg Tyr Trp Gly Gln Gly Thr Gln Val Thr Val Ser Ser
115 120 125

<210> 88
<211> 125
<212> PRT
<213> Lama glama

<400> 88

Glu Val Gln Leu Val Glu Ser Gly Gly Gly Leu Val Gln Ala Gly Gly

1 5 10 15

Ser Leu Arg Leu Ser Cys Ala Ala Ser Thr Ser Ile Tyr Ser Ile Thr
20 25 30

Val Met Ala Trp Phe Arg Gln Ala Pro Gly Lys Glu Arg Glu Phe Val
35 40 45

Ala Ala Ile Thr Trp Ser Ala Pro Thr Thr Tyr Ser Ala Asp Ser Val
50 55 60

Lys Gly Arg Phe Thr Ile Ser Arg Asp Asn Ala Lys Asn Thr Val Tyr
65 70 75 80

Leu Gln Met Asn Ser Leu Lys Pro Glu Asp Ser Ala Ile Tyr Tyr Cys
85 90 95

Ala Ala Asp Arg Phe Lys Gly Arg Ser Ile Val Thr Arg Ser Asp Tyr
100 105 110

Arg Tyr Trp Gly Gln Gly Thr Gln Val Thr Val Ser Ser
115 120 125

<210> 89
<211> 129
<212> PRT
<213> Lama glama

<400> 89

Glu Val Gln Leu Val Glu Ser Gly Gly Gly Leu Val Gln Ala Gly Gly
1 5 10 15

Ser Leu Arg Leu Ser Cys Ser Val Thr Gly Arg Thr Phe Asn Lys Tyr
20 25 30

Val Met Gly Trp Phe Arg Gln Ala Pro Gly Asn Asp Arg Glu Phe Val
35 40 45

Ala Ala Ile Thr Ser Arg Asp Gly Pro Thr Tyr Tyr Ala Asp Ser Val
50 55 60

Lys Gly Arg Phe Thr Ile Ser Gly Asp Asn Thr Lys Asn Lys Ile Phe
65 70 75 80

Leu Gln Met Asn Ser Leu Met Pro Glu Asp Thr Ala Val Tyr Tyr Cys
85 90 95

Ala Ile Asp Glu Asp Leu Tyr His Tyr Ser Ser Tyr His Phe Thr Arg

```
            100                 105                 110

Val Asp Leu Tyr His Tyr Trp Gly Gln Gly Thr Gln Val Thr Val Ser
        115                 120                 125

Ser


<210>  90
<211>  117
<212>  PRT
<213>  Lama glama

<400>  90

Glu Val Gln Leu Val Glu Ser Gly Gly Gly Leu Val Gln Pro Gly Gly
1               5                   10                  15

Ser Leu Arg Leu Ala Cys Ala Ala Ser Gly Phe Thr Leu Ser Ser Ser
            20                  25                  30

Trp Met Tyr Trp Val Arg Gln Ala Pro Gly Lys Gly Leu Glu Trp Val
        35                  40                  45

Ser Arg Ile Ser Pro Gly Gly Leu Phe Thr Tyr Tyr Val Asp Ser Val
    50                  55                  60

Lys Gly Arg Phe Ser Val Ser Thr Asp Asn Ala Asn Asn Thr Leu Tyr
65                  70                  75                  80

Leu Gln Met Asn Ser Leu Lys Pro Glu Asp Thr Ala Leu Tyr Ser Cys
                85                  90                  95

Ala Lys Gly Gly Ala Pro Asn Tyr Thr Pro Arg Gly Arg Gly Thr Gln
            100                 105                 110

Val Thr Val Ser Ser
        115


<210>  91
<211>  115
<212>  PRT
<213>  Lama glama

<400>  91

Glu Val Gln Leu Val Glu Ser Gly Gly Gly Leu Val Gln Pro Gly Gly
1               5                   10                  15

Ser Leu Arg Leu Ser Cys Ala Ala Ser Gly Ser Ile Val Arg Ser Asp
            20                  25                  30
```

```
Val Met Gly Trp Tyr Arg Gln Ala Pro Gly Lys Gln Arg Glu Leu Val
        35                  40                  45

Ala Phe Ile Arg Ser Leu Gly Ser Thr Tyr Tyr Ala Gly Ser Val Lys
    50                  55                  60

Gly Arg Phe Thr Ile Ser Arg Asp Asn Ala Ala Asn Thr Val Tyr Leu
65                  70                  75                  80

Gln Met Asn Asn Leu Lys Pro Glu Asp Thr Ala Val Tyr Tyr Cys Asn
                85                  90                  95

Ala Arg Phe Ser Gly Glu Ser Tyr Trp Gly Gln Gly Thr Pro Val Thr
            100                 105                 110

Val Ser Ser
        115


<210>  92
<211>  129
<212>  PRT
<213>  Lama glama

<400>  92

Glu Val Gln Leu Val Glu Ser Gly Gly Gly Leu Ala Gln Ala Gly Gly
1               5                   10                  15

Ser Leu Arg Leu Ser Cys Thr Ala Ser Gly Arg Thr Phe Asn Asn Tyr
            20                  25                  30

Val Met Gly Trp Phe Arg Gln Ala Pro Gly Asn Glu Arg Glu Phe Val
        35                  40                  45

Ala Ala Ile Thr Ser Thr Asn Gly Pro Thr Tyr Tyr Ala Asp Ser Val
    50                  55                  60

Lys Gly Arg Phe Thr Ile Ser Gly Asp Asn Thr Lys Asn Lys Val Phe
65                  70                  75                  80

Leu Gln Met Asp Ser Leu Arg Pro Glu Asp Thr Ala Val Tyr Tyr Cys
                85                  90                  95

Ala Ile Asp Glu Asp Leu Tyr His Tyr Ser Ser Tyr His Tyr Thr Arg
            100                 105                 110

Val Ala Leu Tyr His Tyr Trp Gly Gln Gly Thr Gln Val Thr Val Ser
        115                 120                 125
```

Ser

<210> 93
<211> 124
<212> PRT
<213> Lama glama

<400> 93

```
Glu Val Gln Leu Val Glu Ser Gly Gly Gly Leu Val Gln Ser Gly Asp
1               5                   10                  15

Ser Leu Arg Leu Ser Cys Ala Val Ser Gly Asn Thr Phe Gly Leu Tyr
            20                  25                  30

Ala Met Gly Trp Phe Arg Gln Ala Pro Gly Arg Glu Arg Glu Phe Val
        35                  40                  45

Ser Ala Ile Thr Trp Ser Ala Gly Asp Thr Gln Tyr Ala Asp Ser Val
    50                  55                  60

Lys Gly Arg Phe Thr Ile Ser Arg Asp Asn Ala Arg Asn Thr Val Asn
65                  70                  75                  80

Leu Gln Met Asn Gly Leu Lys Pro Glu Asp Thr Ala Val Tyr Tyr Cys
                85                  90                  95

Ala Gly Arg Gln Trp Gly Gly Thr Tyr Tyr Tyr His Gly Ser Tyr Ala
            100                 105                 110

Trp Trp Gly Gln Gly Thr Gln Val Thr Val Ser Ser
        115                 120
```

<210> 94
<211> 128
<212> PRT
<213> Lama glama

<400> 94

```
Glu Val Gln Leu Val Glu Ser Glu Gly Gly Leu Val Gln Ala Gly Gly
1               5                   10                  15

Ser Leu Arg Leu Ser Cys Ala Ala Ser Gly Arg Thr Phe Ser Thr Asp
            20                  25                  30

Asp Val Gly Trp Phe Arg Gln Ala Pro Gly Lys Glu Arg Glu Phe Val
        35                  40                  45
```

```
Ala Val Ile Arg Trp Ser Thr Gly Gly Thr Tyr Thr Ser Asp Ser Val
    50                  55                  60

Lys Gly Arg Phe Thr Leu Ser Arg Asp Asn Ala Lys Asn Thr Met Tyr
65                  70                  75                  80

Leu Gln Met Asn Ser Leu Lys Pro Glu Asp Thr Ala Val Tyr Tyr Cys
                85                  90                  95

Ala Ala Arg Ser Arg Pro Leu Gly Ala Gly Ala Trp Tyr Thr Gly Glu
            100                 105                 110

Asn Tyr Tyr Asn Tyr Trp Gly Gln Gly Thr Gln Val Thr Val Ser Ser
        115                 120                 125


<210>  95
<211>  126
<212>  PRT
<213>  Lama glama

<400>  95

Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Ser Ser Gly Tyr
            20                  25                  30

Asp Met Gly Trp Phe Arg Gln Ala Pro Gly Lys Glu Arg Glu Phe Val
        35                  40                  45

Thr Ala Ile Thr Trp Ser Gly Gly Ser Thr Tyr Ser Pro Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Asn Leu Thr Pro Glu Asp Thr Ala Val Tyr Tyr Cys
                85                  90                  95

Ala Ser Gly Arg Ile Trp Arg Ser Arg Asp Tyr Asp Ser Glu Lys Tyr
            100                 105                 110

Tyr Asp Ile Trp Gly His Gly Thr Gln Val Thr Val Ser Ser
        115                 120                 125


<210>  96
<211>  129
<212>  PRT
```

<213> Lama glama

<400> 96

```
Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Phe Ser Ala Tyr
            20                  25                  30

Asp Met Gly Trp Phe Arg Gln Ala Pro Gly Lys Glu Arg Glu Phe Val
        35                  40                  45

Ala Val Ile Ser Trp Thr Asn Ser Met Thr Tyr Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Val Asp Arg Arg Arg Thr Tyr Ser Arg Trp Arg Phe Tyr Thr Gly
            100                 105                 110

Val Asn Asp Tyr Asp Tyr Trp Gly Gln Gly Thr Gln Val Thr Val Ser
        115                 120                 125

Ser
```

<210> 97
<211> 129
<212> PRT
<213> Lama glama

<400> 97

```
Glu Val Gln Leu Val Glu Ser Gly Gly Gly Leu Val Gln Thr Gly Gly
1               5                   10                  15

Ser Leu Arg Leu Ser Cys Ala Ala Ser Gly Arg Thr Phe Ser Ala Tyr
            20                  25                  30

Asp Met Gly Trp Phe Arg Gln Ala Pro Gly Lys Glu Arg Glu Phe Val
        35                  40                  45

Ala Val Ile Ser Trp Ser Gly Gly Met Thr Tyr Tyr Ala Asp Ser Val
    50                  55                  60
```

Gln Gly Arg Phe Thr Ile Ser Arg Asp Asn Ala Lys Ser Thr Val Tyr
65                  70                  75                  80

Leu Gln Met Asn Ser Pro Lys Pro Glu Asp Thr Ala Val Tyr Tyr Cys
                85                  90                  95

Ala Val Asp Arg Arg Arg Ala Tyr Ser Arg Trp Arg Tyr Tyr Thr Gly
            100                 105                 110

Val Asn Asp Tyr Glu Phe Trp Gly Gln Gly Thr Gln Val Thr Val Ser
        115                 120                 125

Ser

<210> 98
<211> 129
<212> PRT
<213> Lama glama

<400> 98

Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Phe Ser Ala Tyr
            20                  25                  30

Asp Met Gly Trp Phe Arg Gln Ala Pro Gly Lys Glu Arg Glu Phe Val
        35                  40                  45

Ala Val Ile Ser Trp Ser Gly Gly Met Thr Tyr Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Val Asp Arg Arg Arg Leu Tyr Ser Arg Trp Arg Tyr Tyr Thr Gly
            100                 105                 110

Val Asn Asp Tyr Asp Tyr Trp Gly Gln Gly Thr Gln Val Thr Val Ser
        115                 120                 125

Ser

<210> 99
<211> 129
<212> PRT
<213> Lama glama

<400> 99

```
Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Phe Ser Ala Tyr
            20                  25                  30

Asp Met Gly Trp Phe Arg Gln Ala Pro Gly Lys Glu Arg Glu Phe Val
        35                  40                  45

Ala Val Ile Ser Trp Thr Gly Gly Met Thr Tyr Tyr Ala Asp Ser Val
    50                  55                  60

Lys Gly Arg Phe Thr Ile Ser Arg Asp Lys Ala Lys Asn Thr Val Ser
65                  70                  75                  80

Leu Gln Met Asn Ser Leu Lys Pro Glu Asp Thr Ala Val Tyr Tyr Cys
                85                  90                  95

Ala Val Asp Arg Arg Arg Thr Tyr Ser Arg Trp Arg Tyr Tyr Thr Gly
            100                 105                 110

Val Asn Glu Tyr Glu Tyr Trp Gly Gln Gly Thr Gln Val Thr Val Ser
        115                 120                 125

Ser
```

<210> 100
<211> 129
<212> PRT
<213> Lama glama

<400> 100

```
Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Phe Ser Ala Tyr
            20                  25                  30

Asp Met Gly Trp Phe Arg Gln Ala Pro Gly Lys Glu Arg Glu Phe Val
        35                  40                  45

Ala Val Ile Ser Trp Thr Gly Asp Met Thr Tyr Tyr Ala Asp Ser Val
```

50 55 60

Lys Gly Arg Phe Thr Ile Ser Arg Asp Lys Ala Lys Asn Thr Val Ser
65 70 75 80

Leu Gln Met Asn Ser Leu Lys Pro Glu Asp Thr Ala Val Tyr Tyr Cys
85 90 95

Ala Ala Asp Arg Arg Arg Thr Tyr Ser Arg Trp Arg Tyr Tyr Thr Gly
100 105 110

Val Asn Glu Tyr Glu Tyr Trp Gly Gln Gly Thr Gln Val Thr Val Ser
115 120 125

Ser

<210> 101
<211> 123
<212> PRT
<213> Lama glama

<400> 101

Glu Val Gln Leu Val Glu Ser Gly Gly Gly Leu Val Gln Ala Gly Gly
1 5 10 15

Ser Leu Arg Leu Ser Cys Ala Ala Ser Gly Arg Thr Phe Ser Val Tyr
20 25 30

Thr Met Gly Trp Phe Arg Gln Ala Pro Gly Lys Glu Arg Glu Phe Val
35 40 45

Ala Thr Ile Ser Arg Thr Gly Asp Arg Thr Ser Tyr Ala Asn Ser Val
50 55 60

Lys Gly Arg Phe Thr Ile Ser Arg Glu Asn Ala Lys Asn Thr Val Tyr
65 70 75 80

Leu Gln Met Asn Ser Leu Lys Pro Glu Asp Thr Ala Val Tyr Ser Cys
85 90 95

Ala Ala Gly Pro Ile Ala Pro Ser Pro Arg Pro Arg Glu Tyr Tyr Tyr
100 105 110

Trp Gly Gln Gly Thr Gln Val Thr Val Ser Ser
115 120

<210> 102

<211> 129
<212> PRT
<213> Lama glama

<400> 102

Glu Val Gln Leu Met Glu Ser Gly Gly Gly Leu Val Gln Ala Gly Gly
1 5 10 15

Ser Leu Arg Leu Ser Cys Ala Ala Ser Gly Arg Thr Phe Ser Ala Tyr
20 25 30

Asp Met Gly Trp Phe Arg Gln Ala Pro Gly Lys Glu Arg Glu Phe Val
35 40 45

Ala Val Ile Ser Trp Thr Gly Gly Met Thr Tyr Tyr Ala Asp Ser Val
50 55 60

Lys Gly Arg Phe Thr Ile Ser Arg Asp Lys Ala Lys Asn Thr Val Ser
65 70 75 80

Leu Gln Met Asn Ser Leu Lys Pro Glu Asp Thr Ala Val Tyr Tyr Cys
85 90 95

Ala Val Asp Arg Arg Arg Thr Tyr Ser Arg Trp Arg Tyr Tyr Thr Gly
100 105 110

Val Asn Glu Tyr Glu Tyr Trp Gly Gln Gly Thr Gln Val Thr Val Ser
115 120 125

Ser

<210> 103
<211> 129
<212> PRT
<213> Lama glama

<400> 103

Glu Val Gln Leu Val Glu Ser Gly Gly Gly Leu Val Gln Ala Gly Gly
1 5 10 15

Ser Leu Arg Leu Ser Cys Ala Ala Ser Gly Arg Thr Phe Ser Ala Tyr
20 25 30

Asp Met Gly Trp Phe Arg Gln Ala Pro Gly Lys Glu Arg Glu Phe Val
35 40 45

Ala Val Ile Ser Trp Ser Gly Gly Met Thr Asp Tyr Ala Asp Ser Val
50 55 60

```
Lys Gly Arg Phe Thr Ile Ser Arg Glu Asn Ala Lys Asn Thr Gln Phe
65                  70                  75                  80

Leu Gln Met Asn Ser Leu Lys Pro Glu Asp Thr Ala Val Tyr Tyr Cys
                85                  90                  95

Ala Val Gly Arg Arg Arg Ala Tyr Ser Arg Trp Arg Tyr Tyr Thr Gly
            100                 105                 110

Val Asn Glu Tyr Asp Tyr Trp Gly Gln Gly Thr Gln Val Thr Val Ser
        115                 120                 125

Ser


<210>  104
<211>  125
<212>  PRT
<213>  Lama glama

<400>  104

Glu Val Gln Leu Val Glu Ser Gly Gly Gly Leu Val Gln Ala Gly Asp
1               5                   10                  15

Ser Leu Arg Leu Ser Cys Thr Ala Ser Gly Arg Thr Phe Asn Ser Tyr
            20                  25                  30

Ala Met Gly Trp Phe Arg Gln Ala Pro Gly Lys Glu Arg Glu Ser Val
        35                  40                  45

Ala His Ile Asn Arg Ser Gly Ser Ser Thr Tyr Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Leu Asn Ser Leu Lys Pro Glu Asp Thr Ala Val Tyr Tyr Cys
                85                  90                  95

Ala Ala Gly Arg Tyr Tyr Ser Ser Asp Gly Val Pro Ser Ala Ser Phe
            100                 105                 110

Asn Tyr Trp Gly Gln Gly Thr Gln Val Thr Val Ser Ser
        115                 120                 125


<210>  105
<211>  128
```

<212> PRT
<213> Lama glama

<400> 105

Glu Val Gln Leu Val Glu Ser Gly Gly Gly Leu Val Gln Ala Gly Asp
1 5 10 15

Ser Leu Arg Leu Ser Cys Phe Thr Ser Ala Arg Thr Phe Asp Thr Trp
20 25 30

Ala Met Ala Trp Phe Arg Gln Ala Pro Gly Lys Glu Arg Glu Phe Ile
35 40 45

Ser Ala Ile Ser Trp Ser Gly Ser Met Thr Tyr Tyr Thr Asp Ser Val
50 55 60

Lys Gly Arg Phe Ile Ile Ser Arg Asp Asn Ala Gln Asn Thr Leu Phe
65 70 75 80

Leu Gln Met Asn Asn Thr Ala Pro Glu Asp Thr Ala Val Tyr Tyr Cys
85 90 95

Ala Ala Lys Thr Val Asp Tyr Cys Ser Ala Tyr Glu Cys Tyr Ala Arg
100 105 110

Leu Glu Tyr Asp Tyr Trp Gly Arg Gly Ala Gln Val Thr Val Ser Ser
115 120 125

<210> 106
<211> 126
<212> PRT
<213> Lama glama

<400> 106

Glu Val Gln Leu Val Glu Ser Gly Gly Gly Leu Met Gln Thr Gly Asp
1 5 10 15

Ser Leu Arg Leu Ser Cys Ala Ala Ser Gly Leu Arg Phe Thr Ser Thr
20 25 30

Asn Met Gly Trp Phe Arg Gln Gly Pro Gly Lys Glu Arg Glu Phe Val
35 40 45

Ala Ala Ile Thr Leu Ser Gly Thr Thr Tyr Tyr Ala Glu Ala Val Lys
50 55 60

Gly Arg Phe Thr Ile Ser Arg Asp Asn Asp Lys Asn Thr Val Ala Leu
65 70 75 80

Gln Met Asn Ser Leu Lys Pro Glu Asp Thr Ala Val Tyr Tyr Cys Gly
85 90 95

Ala Asp Pro Ser Tyr Tyr Ser Thr Ser Arg Tyr Thr Lys Ala Thr Glu
100 105 110

Tyr Asp Tyr Trp Gly Gln Gly Thr Gln Val Thr Val Ser Ser
115 120 125

<210> 107
<211> 126
<212> PRT
<213> Lama glama

<400> 107

Glu Val Gln Leu Val Glu Ser Gly Gly Gly Leu Val Gln Ala Gly Gly
1 5 10 15

Ser Leu Arg Leu Ser Cys Ala Ala Ser Gly Arg Thr Phe Asn Thr Tyr
20 25 30

Thr Met Gly Trp Phe Arg Gln Thr Pro Gly Thr Glu Arg Glu Phe Val
35 40 45

Ala Ala Ile Arg Trp Thr Val Asn Ile Thr Tyr Tyr Ala Asp Ser Val
50 55 60

Lys Gly Arg Phe Thr Ile Ser Arg Asp Ile Val Lys Asn Thr Val Tyr
65 70 75 80

Leu Gln Met Asn Ser Leu Lys Pro Glu Asp Thr Ala Val Tyr Tyr Cys
85 90 95

Ala Ala Gln Thr Ser Ala Pro Arg Ser Leu Ile Arg Met Ser Asn Glu
100 105 110

Tyr Pro Tyr Trp Gly Gln Gly Thr Gln Val Thr Val Ser Ser
115 120 125

<210> 108
<211> 123
<212> PRT
<213> Lama glama

<400> 108

Glu Val Gln Leu Val Glu Ser Gly Gly Gly Leu Val Gln Ala Gly Gly
1 5 10 15

Ser Leu Arg Leu Ser Cys Ala Ala Ser Gly Leu Thr Phe Ser Leu Tyr
20 25 30

Thr Val Gly Trp Phe Arg Gln Ala Pro Gly Lys Glu Arg Glu Phe Val
35 40 45

Ala Tyr Ile Ser Arg Ser Gly Ser Asn Arg Tyr Tyr Val Asp Ser Val
50 55 60

Lys Gly Arg Phe Thr Leu Ser Arg Asp Asn Ala Lys Asn Thr Val Asp
65 70 75 80

Leu Gln Met Asn Ser Leu Lys Thr Glu Asp Thr Ala Val Tyr Tyr Cys
85 90 95

Ala Ala Thr Ser Arg Gly Leu Ser Ser Leu Ala Gly Glu Tyr Asn Tyr
100 105 110

Trp Gly Arg Gly Thr Gln Val Thr Val Ser Ser
115 120

<210> 109
<211> 123
<212> PRT
<213> Lama glama

<400> 109

Glu Val Gln Leu Val Glu Ser Gly Gly Gly Leu Val Gln Ala Gly Gly
1 5 10 15

Ser Leu Arg Leu Ser Cys Thr Ala Ser Gly Ser Ala Phe Lys Ser Tyr
20 25 30

Arg Met Gly Trp Phe Arg Arg Thr Pro Gly Lys Glu Asp Glu Phe Val
35 40 45

Ala Ser Ile Ser Trp Thr Tyr Gly Ser Thr Phe Tyr Ala Asp Ser Val
50 55 60

Lys Gly Arg Phe Thr Met Ser Arg Asp Lys Ala Lys Asn Ala Gly Tyr
65 70 75 80

Leu Gln Met Asn Ser Leu Lys Pro Glu Asp Thr Ala Leu Tyr Tyr Cys
85 90 95

Ala Ala Gly Ala Gln Ser Asp Arg Tyr Asn Ile Arg Ser Tyr Asp Tyr
100 105 110

Trp Gly Gln Gly Thr Gln Val Thr Val Ser Ser
115 120

<210> 110
<211> 115
<212> PRT
<213> Lama glama

<400> 110

Glu Val Gln Leu Val Glu Ser Gly Gly Gly Leu Val Gln Pro Gly Gly
1 5 10 15

Ser Leu Lys Leu Ser Cys Thr Ala Ser Gly Phe Thr Phe Ser Thr Ser
20 25 30

Trp Met His Trp Val Arg Gln Ala Pro Gly Lys Gly Leu Glu Trp Val
35 40 45

Ser Ser Ile Pro Pro Val Gly His Phe Ala Asn Tyr Ala Pro Ser Val
50 55 60

Lys Gly Arg Phe Thr Ile Ser Arg Asp Asn Ala Lys Asn Thr Leu Phe
65 70 75 80

Leu Gln Met Asn Ser Leu Lys Ser Glu Asp Thr Ala Val Tyr Tyr Cys
85 90 95

Ala Lys Asp Ser Ala Gly Arg Thr Lys Gly Gln Gly Thr Gln Val Thr
100 105 110

Val Ser Ser
115

<210> 111
<211> 124
<212> PRT
<213> Lama glama

<400> 111

Lys Val Gln Leu Val Glu Ser Gly Gly Gly Leu Val Gln Ala Gly Gly
1 5 10 15

Ser Leu Arg Leu Ser Cys Ala Ala Ser Glu Arg Thr Phe Ser Asn Tyr
20 25 30

Ala Met Asp Trp Phe Arg Gln Ala Pro Gly Lys Glu Arg Glu Phe Val
35 40 45

Ala Ala Ile Thr Arg Ser Gly Gly Gly Thr Tyr Tyr Ala Asp Ser Val

50 55 60

Lys Gly Arg Phe Thr Ile Ser Arg Asp Asn Ala Lys Asn Thr Val Tyr
65 70 75 80

Leu Gln Met Asn Ser Leu Lys Pro Glu Asp Thr Ala Val Tyr Tyr Cys
85 90 95

Ala Ala Thr Arg Ser Ser Thr Ile Val Val Gly Val Gly Gly Met Glu
100 105 110

Tyr Trp Gly Lys Gly Thr Leu Val Thr Val Ser Ser
115 120

<210> 112
<211> 128
<212> PRT
<213> Lama glama

<400> 112

Glu Val Gln Leu Val Glu Ser Gly Gly Gly Leu Val Gln Ala Gly Gly
1 5 10 15

Ser Leu Arg Leu Ser Cys Ala Ala Ser Gly Phe Thr Phe Gly Asp Tyr
20 25 30

Asp Ile Gly Trp Phe Arg Gln Ala Pro Gly Asn Glu Arg Glu Gly Val
35 40 45

Ser Cys Ile Thr Thr Asp Val Gly Thr Thr Tyr Tyr Ala Asp Ser Val
50 55 60

Lys Gly Arg Phe Thr Ile Ser Ser Asp Asn Ala Lys Asn Thr Val Tyr
65 70 75 80

Leu Gln Ile Asn Asp Leu Lys Pro Glu Asp Thr Ala Ile Tyr Tyr Cys
85 90 95

Ala Val Asp Thr Gln Asp Leu Gly Leu Asp Ile Phe Cys Arg Gly Asn
100 105 110

Gly Pro Phe Asp Gly Trp Gly Gln Gly Thr Gln Val Thr Val Ser Ser
115 120 125

<210> 113
<211> 134
<212> PRT
<213> Lama glama

<400> 113

Glu Val Gln Leu Val Glu Ser Gly Gly Gly Leu Val Gln Pro Gly Gly
1               5                   10                  15

Ser Leu Arg Leu Ser Cys Thr Ala Ser Gly Leu Asn Leu Asp Asp Tyr
            20                  25                  30

Ala Ile Gly Trp Phe Arg Gln Ala Pro Gly Lys Glu Arg Glu Gly Val
        35                  40                  45

Ser Cys Ile Ser Ser Tyr Asp Ser Val Thr Tyr Tyr Ala Asp His Val
    50                  55                  60

Lys Gly Arg Phe Thr Ile Ser Arg Asp Ser Ala Lys Asn Thr Leu Tyr
65                  70                  75                  80

Leu Gln Met Asn Ser Leu Ser Ile Glu Asp Thr Gly Val Tyr Tyr Cys
                85                  90                  95

Ala Ala Glu Arg Glu Gln Leu Arg Arg Arg Glu Ser Pro His Asp Glu
            100                 105                 110

Leu Leu Arg Leu Cys Phe Tyr Gly Met Arg Tyr Ser Gly Lys Gly Thr
        115                 120                 125

Leu Val Thr Val Ser Ser
    130

<210> 114
<211> 123
<212> PRT
<213> Lama glama

<400> 114

Glu Val Gln Leu Val Glu Ser Gly Gly Gly Leu Val Gln Pro Gly Gly
1               5                   10                  15

Ser Leu Arg Leu Ser Cys Val Ala Ser Gly Phe Arg Leu Asp Asp Tyr
            20                  25                  30

Ala Ile Gly Trp Phe Arg Gln Ala Pro Gly Lys Glu Arg Glu Ala Val
        35                  40                  45

Ser Cys Ile Ser Ser Ser Asp Thr Ser Ile Asp Tyr Thr Asn Ser Val
    50                  55                  60

Lys Gly Arg Phe Thr Phe Ser Arg Asp Asn Ala Lys Asn Thr Val Tyr
65                  70                  75                  80

```
Leu Gln Met Asn Ser Leu Lys Pro Glu Asp Thr Ala Val Tyr Tyr Cys
                85                  90                  95

Ala Ala Ala Phe Arg Cys Ser Gly Tyr Glu Leu Arg Gly Phe Pro Thr
            100                 105                 110

Trp Gly Gln Gly Thr Gln Val Thr Val Ser Ser
        115                 120
```

<210> 115
<211> 126
<212> PRT
<213> Lama glama

<400> 115

```
Glu Val Gln Leu Val Glu Ser Gly Gly Gly Leu Val Gln Ala Gly Gly
1               5                   10                  15

Ser Leu Arg Leu Ser Cys Ala Ala Ser Gly Gly Thr Phe Ser Ser Leu
            20                  25                  30

Ala Val Gly Trp Phe Arg Gln Ala Pro Gly Lys Glu Arg Glu Phe Val
        35                  40                  45

Ala Arg Ile Thr Trp Ser Gly Ala Thr Thr Tyr Tyr Ala Asp Ala Val
    50                  55                  60

Lys Asp Arg Phe Thr Ile Ser Arg Asp Asn Ala Lys Asn Thr Met Tyr
65                  70                  75                  80

Leu Gln Met Asn Ser Leu Lys Pro Glu Asp Thr Ala Val Tyr Tyr Cys
                85                  90                  95

Ala Ala Asp Arg Ser Pro Asn Ile Ile Asn Val Val Thr Ala Tyr Glu
            100                 105                 110

Tyr Asp Tyr Trp Gly Gln Gly Thr Gln Val Thr Val Ser Ser
        115                 120                 125
```

<210> 116
<211> 126
<212> PRT
<213> Lama glama

<400> 116

```
Glu Val Gln Leu Val Glu Ser Gly Gly Gly Leu Val Gln Pro Gly Ala
1               5                   10                  15
```

```
Ser Leu Arg Leu Ser Cys Ala Ala Ser Gly Asp Gly Phe Thr Leu Tyr
            20                  25                  30

Asn Met Gly Trp Phe Arg Gln Ala Pro Gly Lys Glu Arg Glu Phe Val
        35                  40                  45

Ala Ala Ile Thr Ser Ser Pro Met Ser Thr Tyr Tyr Ala Asp Ser Val
    50                  55                  60

Lys Gly Arg Phe Ser Ile Ser Ile Asn Asn Asp Lys Thr Thr Gly Phe
65                  70                  75                  80

Leu Gln Met Asn Val Leu Lys Pro Glu Asp Thr Gly Val Tyr Phe Cys
                85                  90                  95

Ala Ala Pro Glu Gly Ser Phe Arg Arg Gln Tyr Ala Asp Arg Ala Met
            100                 105                 110

Tyr Asp Tyr Trp Gly Gln Gly Thr Gln Val Thr Val Ser Ser
        115                 120                 125


<210>  117
<211>  127
<212>  PRT
<213>  Lama glama

<400>  117

Glu Val Gln Leu Val Glu Ser Gly Gly Gly Leu Ala Gln Ala Gly Gly
1               5                   10                  15

Ser Leu Arg Leu Ser Cys Ala Ala Ser Gly Arg Thr Phe Ser Gly Ser
            20                  25                  30

Asp Met Gly Trp Phe Arg Gln Ser Pro Gly Lys Glu Arg Glu Ile Val
        35                  40                  45

Ala Ala Ile Arg Leu Ser Gly Ser Ile Thr Tyr Tyr Pro Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Arg Ser Thr Tyr Ser Tyr Tyr Leu Ala Leu Ala Asp Arg Gly
            100                 105                 110
```

Gly Tyr Asp Tyr Trp Gly Gln Gly Thr Gln Val Thr Val Ser Ser
115 120 125

<210> 118
<211> 125
<212> PRT
<213> Lama glama

<400> 118

Glu Val Gln Leu Val Glu Ser Gly Gly Gly Leu Val Gln Ala Gly Gly
1 5 10 15

Ser Leu Arg Leu Ser Cys Val Ala Ser Gly Phe Thr Leu Gly Thr Tyr
20 25 30

Ala Ile Gly Trp Phe Arg Gln Ala Pro Gly Lys Glu Arg Glu Ala Val
35 40 45

Ser Cys Met Ser Ala Gly Asp Ser Ile Pro Trp Tyr Thr Ala Ser Val
50 55 60

Lys Gly Arg Phe Thr Thr Ser Thr Asp Asn Ala Arg Asn Thr Val Tyr
65 70 75 80

Leu Gln Met Asn Ser Leu Lys Pro Glu Asp Thr Ala His Tyr Tyr Cys
85 90 95

Ala Ala Ala Arg Tyr His Gly Asp Tyr Cys Tyr Tyr Glu Gly Tyr Tyr
100 105 110

Pro Phe Trp Gly Gln Gly Thr Gln Val Thr Val Ser Ser
115 120 125

<210> 119
<211> 117
<212> PRT
<213> Lama glama

<400> 119

Glu Val Gln Leu Val Glu Ser Gly Gly Gly Leu Val Gln Ala Gly Gly
1 5 10 15

Ser Leu Arg Leu Ser Cys Ala Ala Ser Thr Ser Ile Ser Ser Thr Asn
20 25 30

Phe Met Gly Trp Tyr Arg Gln Ala Pro Gly Lys Gln Arg Glu Leu Val
35 40 45

Ala Thr Ile Thr Ser Ser Ser Ile Thr Asn Tyr Val Asp Ser Val Lys
    50                  55                  60

Gly Arg Phe Thr Ile Ser Arg Asp Asn Ala Lys Asn Thr Val Tyr Leu
65                  70                  75                  80

Gln Met Thr Ser Leu Lys Pro Glu Asp Thr Ala Val Tyr Tyr Cys His
                85                  90                  95

Ala Arg Trp Arg Trp Ser Asp Val Glu Tyr Trp Gly Lys Gly Thr Leu
            100                 105                 110

Val Thr Val Ser Ser
        115

<210> 120
<211> 121
<212> PRT
<213> Lama glama

<400> 120

Glu Val Gln Leu Val Glu Ser Gly Gly Gly Leu Val Gln Pro Gly Gly
1               5                   10                  15

Ser Leu Arg Leu Ser Cys Ala Ala Ser Gly Thr Thr Ser Ser Ile Phe
            20                  25                  30

Ala Met Arg Trp Tyr Arg Gln Ala Pro Gly Lys Gln Arg Glu Leu Val
        35                  40                  45

Ala Ser Ile Thr Arg Ser Ser Ile Thr Thr Tyr Ala Asp Ser Val Lys
    50                  55                  60

Gly Arg Phe Thr Pro Ser Arg Asp Asn Ala Lys Asn Thr Val Ser Leu
65                  70                  75                  80

Gln Met Asn Ser Leu Lys Pro Glu Asp Thr Ala Val Tyr Tyr Cys Asn
                85                  90                  95

Ala Ala Ile Arg Pro Glu Leu Tyr Ser Val Val Asn Asp Tyr Trp Gly
            100                 105                 110

Gln Gly Thr Gln Val Thr Val Ser Ser
        115                 120

<210> 121
<211> 127
<212> PRT
<213> Lama glama

<400> 121

Glu Val Gln Leu Val Glu Ser Gly Gly Gly Leu Val Gln Pro Gly Gly
1               5                   10                  15

Ser Leu Arg Leu Ser Cys Ala Thr Ser Gly Leu Thr Phe Ser Asp Tyr
            20                  25                  30

Asn Leu Gly Trp Phe Arg Gln Ala Pro Gly Lys Glu Arg Gln Phe Val
        35                  40                  45

Ala Val Ile Ser Trp Arg Asp Ser Phe Ala Tyr Tyr Ala Glu Pro Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Asp Arg Val Ser Ser Arg Leu Val Leu Pro Asn Thr Ser Pro
            100                 105                 110

Asp Phe Gly Ser Trp Gly Gln Gly Thr Gln Val Thr Val Ser Ser
        115                 120                 125

<210> 122
<211> 129
<212> PRT
<213> Lama glama

<400> 122

Glu Val Gln Leu Val Glu Ser Gly Gly Gly Leu Val Gln Ala Gly Asp
1               5                   10                  15

Ser Leu Arg Leu Ser Cys Ala Ala Ser Gly Arg Thr Phe Asn Asn Ala
            20                  25                  30

Ile Met Gly Trp Phe Arg Gln Ala Pro Gly Gln Glu Arg Glu Phe Val
        35                  40                  45

Ala Ala Met Asn Trp Arg Gly Gly Pro Thr Tyr Tyr Ala Asp Ser Val
    50                  55                  60

Lys Gly Arg Phe Thr Ile Ser Gly Asp Asn Thr Lys Asn Thr Val Phe
65                  70                  75                  80

Leu Gln Met Asn Phe Leu Lys Pro Glu Asp Thr Ala Val Tyr Tyr Cys

85 90 95

Ala Ala Asp Glu Asp Leu Tyr His Tyr Ser Ser Tyr His Tyr Ser Arg
100 105 110

Val Asp Leu Tyr His Tyr Trp Gly Gln Gly Thr Gln Val Thr Val Ser
115 120 125

Ser

<210> 123
<211> 121
<212> PRT
<213> Lama glama

<400> 123

Glu Val Gln Leu Val Glu Ser Gly Gly Gly Leu Val Gln Pro Gly Gly
1 5 10 15

Ser Leu Arg Leu Ser Cys Ala Ala Ser Gly Thr Thr Ser Ser Ile Phe
20 25 30

Ala Met Arg Trp Tyr Arg Gln Ala Pro Gly Lys Gln Arg Glu Leu Val
35 40 45

Ala Ser Ile Thr Arg Ser Ser Ile Thr Thr Tyr Ala Asp Ser Val Lys
50 55 60

Gly Arg Phe Thr Leu Ser Arg Asp Asn Ala Lys Asn Thr Val Ser Leu
65 70 75 80

Gln Met Asn Ser Leu Lys Pro Glu Asp Thr Ala Val Tyr Tyr Cys Asn
85 90 95

Ala Ala Ile Arg Pro Glu Leu Tyr Ser Val Val Asn Asp Tyr Trp Gly
100 105 110

Gln Gly Thr Gln Val Thr Val Ser Ser
115 120

<210> 124
<211> 123
<212> PRT
<213> Lama glama

<400> 124

Glu Val Gln Leu Val Glu Ser Gly Gly Gly Leu Val Gln Ala Gly Gly
1 5 10 15

Ser Leu Arg Leu Ser Cys Ala Ala Ser Gly Gly Ser Phe Ser Ser Tyr
20 25 30

Ala Pro Gly Trp Phe Arg Gln Ala Pro Gly Lys Glu Arg Glu Phe Val
35 40 45

Ala Ala Phe Thr Arg Ser Ser Asn Ile Pro Tyr Tyr Lys Asp Ser Val
50 55 60

Lys Gly Arg Phe Thr Ile Ser Arg Asp Asn Ala His Thr Val Tyr Leu
65 70 75 80

Gln Met Asn Ser Leu Lys Pro Glu Asp Thr Ala Ile Tyr Tyr Cys Ala
85 90 95

Val Asn Leu Gly Ser Thr Trp Ser Arg Asp Gln Arg Thr Tyr Asp Tyr
100 105 110

Trp Gly Gln Gly Thr Gln Val Thr Val Ser Ser
115 120

<210> 125
<211> 113
<212> PRT
<213> Artificial

<220>
<223> Mutated lama glama sequence

<400> 125

Glu Val Gln Leu Val Glu Ser Gly Gly Gly Leu Val Gln Pro Gly Gly
1 5 10 15

Ser Leu Arg Leu Ser Cys Ala Ala Ser Gly Ile Arg Phe Met Ser Met
20 25 30

Ala Trp Tyr Arg Gln Ala Pro Gly Lys Gln Arg Glu Leu Val Ala Arg
35 40 45

Ile Ser Ser Gly Gly Thr Thr Ala Tyr Ala Asp Ser Val Lys Gly Arg
50 55 60

Phe Thr Ile Ser Arg Asp Asn Ser Lys Asn Thr Val Tyr Leu Gln Met
65 70 75 80

Asn Ser Leu Arg Ala Glu Asp Thr Ala Val Tyr Tyr Cys Asn Thr Phe
85 90 95

Ser Ser Arg Pro Asn Pro Trp Gly Gln Gly Thr Leu Val Thr Val Ser
100 105 110

Ser

<210> 126
<211> 113
<212> PRT
<213> Artificial

<220>
<223> Mutated lama glama sequence

<400> 126

Glu Val Gln Leu Val Glu Ser Gly Gly Gly Leu Val Gln Pro Gly Gly
1 5 10 15

Ser Leu Arg Leu Ser Cys Val Ala Ser Gly Ile Arg Phe Ile Ser Met
20 25 30

Ala Trp Tyr Arg Gln Ala Pro Gly Lys His Arg Glu Leu Val Ala Arg
35 40 45

Ile Ser Ser Gly Gly Thr Thr Ala Tyr Val Asp Ser Val Lys Gly Arg
50 55 60

Phe Thr Ile Ser Arg Asp Asn Ser Lys Asn Thr Val Tyr Leu Gln Met
65 70 75 80

Asn Ser Leu Lys Ala Glu Asp Thr Ala Val Tyr Tyr Cys Asn Thr Phe
85 90 95

Ser Ser Arg Pro Asn Pro Trp Gly Ala Gly Thr Gln Val Thr Val Ser
100 105 110

Ser

<210> 127
<211> 113
<212> PRT
<213> Artificial

<220>
<223> Mutated lama glama sequence

<400> 127

Glu Val Gln Leu Val Glu Ser Gly Gly Gly Leu Val Gln Pro Gly Gly
1 5 10 15

Ser Leu Arg Leu Ser Cys Ala Ala Ser Gly Ile Arg Phe Ile Ser Met
20 25 30

Ala Trp Tyr Arg Gln Ala Pro Gly Lys Gln Arg Glu Leu Val Ala Arg
35 40 45

Ile Ser Ser Gly Gly Thr Thr Ala Tyr Ala Asp Ser Val Lys Gly Arg
50 55 60

Phe Thr Ile Ser Arg Asp Asn Ser Lys Asn Thr Val Tyr Leu Gln Met
65 70 75 80

Asn Ser Leu Arg Ala Glu Asp Thr Ala Val Tyr Tyr Cys Asn Thr Phe
85 90 95

Ser Ser Arg Pro Asn Pro Trp Gly Gln Gly Thr Leu Val Thr Val Ser
100 105 110

Ser

<210> 128
<211> 285
<212> PRT
<213> Artificial

<220>
<223> Artificial polypeptide comprising lama glama sequences

<400> 128

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Lys Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Gln Val Thr Val Ser Ser Gly Gly Gly
115 120 125

Gly Ser Gly Gly Gly Gly Ser Gly Gly Gly Gly Ser Gly Gly Gly Gly
130 135 140

Ser Gly Gly Gly Gly Ser Gly Gly Gly Gly Ser Gly Gly Gly Gly Ser
145 150 155 160

Glu Val Gln Leu Val Glu Ser Gly Gly Gly Leu Val Gln Thr Gly Asp
165 170 175

Ser Leu Arg Leu Ser Cys Glu Val Ser Gly Arg Thr Phe Ser Ser Tyr
180 185 190

Ser Met Gly Trp Phe Arg Gln Ala Gln Gly Lys Glu Arg Glu Phe Val
195 200 205

Val Ala Ile Ser Lys Gly Gly Tyr Lys Tyr Asp Ser Val Ser Leu Glu
210 215 220

Gly Arg Phe Thr Ile Ser Lys Asp Asn Ala Lys Asn Thr Val Tyr Leu
225 230 235 240

Gln Ile Asn Ser Leu Lys Pro Glu Asp Thr Ala Val Tyr Tyr Cys Ala
245 250 255

Ser Ser Arg Ala Tyr Gly Ser Ser Arg Leu Arg Leu Ala Asp Thr Tyr
260 265 270

Glu Tyr Trp Gly Gln Gly Thr Gln Val Thr Val Ser Ser
275 280 285

<210> 129
<211> 245
<212> PRT
<213> Artificial

<220>
<223> Artificial polypeptide comprising lama glama sequences

<400> 129

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Lys Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Gln Val Thr Val Ser Ser Gly Gly Gly
115 120 125

Gly Ser Gly Gly Gly Ser Glu Val Gln Leu Val Glu Ser Gly Gly Gly
130 135 140

Leu Val Gln Pro Gly Gly Ser Leu Arg Leu Ser Cys Ala Ala Ser Gly
145 150 155 160

Ser Ala Val Gly Asp Ile Thr Val Ala Trp Tyr Arg Gln Ala Pro Gly
165 170 175

Ile Gln Arg Gln Leu Val Ala Thr Ile Thr Pro Ser Gly Tyr Thr Tyr
180 185 190

Tyr Trp Asp Phe Val Lys Gly Arg Phe Thr Ile Ser Arg Asp Asn Ser
195 200 205

Lys Asn Ile Val Tyr Leu Gln Met Asn Ser Leu Lys Pro Glu Asp Thr
210 215 220

Ala Ala Tyr Tyr Cys Asn Thr Gln Phe Tyr Trp Gly Gln Gly Thr Gln
225 230 235 240

Val Thr Val Ser Ser
245

<210> 130
<211> 255
<212> PRT

<213> Artificial

<220>
<223> Artificial polypeptide comprising lama glama sequences

<400> 130

```
Glu Val Gln Leu Val Glu Ser Gly Gly Gly Leu Val Gln Thr Gly Asp
1               5                   10                  15

Ser Leu Arg Leu Ser Cys Glu Val Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly
        115                 120                 125

Gly Ser Gly Gly Gly Ser Glu Val Gln Leu Val Glu Ser Gly Gly Gly
    130                 135                 140

Leu Ala Gln Ala Gly Asp Ser Leu Arg Leu Ser Cys Ala Ala Ser Gly
145                 150                 155                 160

Arg Ser Phe Ser His Tyr Asn Met Gly Trp Phe Arg Gln Ala Pro Gly
                165                 170                 175

Lys Glu Arg Glu Phe Val Ala Ser Ile Arg Gly Gly Gly Gly Ser Thr
            180                 185                 190

Thr Tyr Ala Asn Ser Val Lys Asp Arg Phe Thr Ile Ser Arg Glu Asn
        195                 200                 205

Ala Lys Asn Thr Val Tyr Leu Gln Met Asn Ser Leu Lys Pro Glu Asp
    210                 215                 220
```

```
Thr Ala Val Tyr Tyr Cys Ala Ala Thr Ala Phe Tyr Arg Gly Pro Tyr
225                 230                 235                 240

Asp Tyr Asp Tyr Trp Gly Gln Gly Thr Gln Val Thr Val Ser Ser
                245                 250                 255


<210>  131
<211>  247
<212>  PRT
<213>  Artificial

<220>
<223>  Artificial polypeptide comprising lama glama sequences

<400>  131

Glu Val Gln Leu Val Glu Ser Gly Gly Gly Leu Val Gln Thr Gly Asp
1               5                   10                  15

Ser Leu Arg Leu Ser Cys Glu Val Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly
        115                 120                 125

Gly Ser Gly Gly Gly Ser Glu Val Gln Leu Val Glu Ser Gly Gly Gly
    130                 135                 140

Leu Val Gln Pro Gly Gly Ser Leu Arg Leu Ser Cys Val Ala Ser Gly
145                 150                 155                 160

Ile Arg Phe Met Ser Met Ala Trp Tyr Arg Gln Ala Pro Gly Lys His
                165                 170                 175

Arg Glu Leu Val Ala Arg Ile Ser Ser Gly Gly Thr Thr Ala Tyr Val
```

180 185 190

Asp Ser Val Lys Gly Arg Phe Thr Ile Ser Arg Asp Asn Ser Lys Asn
195 200 205

Thr Val Tyr Leu Gln Met Asn Ser Leu Lys Ala Glu Asp Thr Ala Val
210 215 220

Tyr Tyr Cys Asn Thr Phe Ser Ser Arg Pro Asn Pro Trp Gly Ala Gly
225 230 235 240

Thr Gln Val Thr Val Ser Ser
245

<210> 132
<211> 250
<212> PRT
<213> Artificial

<220>
<223> Artificial polypeptide comprising lama glama sequences

<400> 132

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Lys Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Gln Val Thr Val Ser Ser Gly Gly Gly
115 120 125

Gly Ser Gly Gly Gly Ser Glu Val Gln Leu Val Glu Ser Gly Gly Gly
130 135 140

Leu Val Gln Pro Gly Gly Ser Leu Arg Leu Ser Cys Ala Ala Ser Gly
145 150 155 160

Asn Ile Phe Ser Asn Asn Ala Met Ala Trp Tyr Arg Gln Ala Pro Gly
165 170 175

Lys Gln Arg Glu Leu Val Ala Arg Ile Ser Ser Gly Gly Gly Phe Thr
180 185 190

Tyr Tyr Leu Asp Ser Val Lys Gly Arg Phe Thr Val Ser Arg Asp Asn
195 200 205

Ala Lys Asn Thr Val Tyr Leu Gln Met Asn Ser Leu Lys Pro Glu Asp
210 215 220

Thr Ala Val Tyr Tyr Cys Asn Ala Ala Tyr Arg Thr Tyr Asn Tyr Trp
225 230 235 240

Gly Gln Gly Thr Gln Val Thr Val Ser Ser
245 250

<210> 133
<211> 258
<212> PRT
<213> Artificial

<220>
<223> Artificial polypeptide comprising lama glama sequences

<400> 133

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Lys Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Gln Val Thr Val Ser Ser Gly Gly Gly
115 120 125

Gly Ser Gly Gly Gly Ser Glu Val Gln Leu Val Glu Ser Gly Gly Gly
130 135 140

Leu Val Gln Ala Gly Gly Ser Leu Arg Leu Ser Cys Ala Ala Ser Gly
145 150 155 160

Arg Thr Phe Ser Asn Tyr Ala Met Gly Trp Phe Arg Gln Ala Pro Gly
165 170 175

Lys Glu Arg Val Leu Val Ala Asp Ile Ser Ser Ser Gly Ile Asn Thr
180 185 190

Tyr Val Ala Asp Ala Val Lys Gly Arg Phe Thr Ile Ser Arg Asp Asn
195 200 205

Ala Lys Asn Thr Val Tyr Leu Gln Met Asn Ser Leu Lys Pro Glu Asp
210 215 220

Thr Ala Val Tyr Tyr Cys Ala Ala Ser Ala Trp Trp Tyr Ser Gln Met
225 230 235 240

Ala Arg Asp Asn Tyr Arg Tyr Trp Gly Gln Gly Thr Gln Val Thr Val
245 250 255

Ser Ser

<210> 134
<211> 251
<212> PRT
<213> Artificial

<220>
<223> Artificial polypeptide comprising lama glama sequences

<400> 134

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

```
Ser Met Gly Trp Phe Arg Gln Ala Gln Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly
        115                 120                 125

Gly Ser Gly Gly Gly Ser Glu Val Gln Leu Val Glu Ser Gly Gly Gly
    130                 135                 140

Leu Val Gln Pro Gly Gly Ser Leu Arg Leu Ala Cys Ala Ala Ser Gly
145                 150                 155                 160

Phe Thr Leu Ser Ser Ser Trp Met Tyr Trp Val Arg Gln Ala Pro Gly
                165                 170                 175

Lys Gly Leu Glu Trp Val Ser Arg Ile Ser Pro Gly Gly Leu Phe Thr
            180                 185                 190

Tyr Tyr Val Asp Ser Val Lys Gly Arg Phe Ser Val Ser Thr Asp Asn
        195                 200                 205

Ala Asn Asn Thr Leu Tyr Leu Gln Met Asn Ser Leu Lys Pro Glu Asp
    210                 215                 220

Thr Ala Leu Tyr Ser Cys Ala Lys Gly Gly Ala Pro Asn Tyr Thr Pro
225                 230                 235                 240

Arg Gly Arg Gly Thr Gln Val Thr Val Ser Ser
                245                 250


<210>  135
<211>  259
<212>  PRT
<213>  Artificial

<220>
<223>  Artificial polypeptide comprising lama glama sequences
```

<400> 135

Glu Val Gln Leu Val Glu Ser Gly Gly Gly Leu Val Gln Thr Gly Asp
1               5                   10                  15

Ser Leu Arg Leu Ser Cys Glu Val Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly
        115                 120                 125

Gly Ser Gly Gly Gly Ser Glu Val Gln Leu Val Glu Ser Gly Gly Gly
    130                 135                 140

Leu Val Gln Ala Gly Asp Ser Leu Arg Leu Ser Cys Thr Ala Ser Gly
145                 150                 155                 160

Arg Thr Phe Asn Ser Tyr Ala Met Gly Trp Phe Arg Gln Ala Pro Gly
                165                 170                 175

Lys Glu Arg Glu Ser Val Ala His Ile Asn Arg Ser Gly Ser Ser Thr
            180                 185                 190

Tyr Tyr Ala Asp Ser Val Lys Gly Arg Phe Thr Ile Ser Arg Asp Asn
        195                 200                 205

Ala Lys Asn Thr Val Tyr Leu Gln Leu Asn Ser Leu Lys Pro Glu Asp
    210                 215                 220

Thr Ala Val Tyr Tyr Cys Ala Ala Gly Arg Tyr Tyr Ser Ser Asp Gly
225                 230                 235                 240

Val Pro Ser Ala Ser Phe Asn Tyr Trp Gly Gln Gly Thr Gln Val Thr
245 250 255

Val Ser Ser

<210> 136
<211> 257
<212> PRT
<213> Artificial

<220>
<223> Artificial polypeptide comprising lama glama sequences

<400> 136

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Lys Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Gln Val Thr Val Ser Ser Gly Gly Gly
115 120 125

Gly Ser Gly Gly Gly Ser Glu Val Gln Leu Val Glu Ser Gly Gly Gly
130 135 140

Leu Val Gln Ala Gly Gly Ser Leu Arg Leu Ser Cys Thr Ala Ser Gly
145 150 155 160

Ser Ala Phe Lys Ser Tyr Arg Met Gly Trp Phe Arg Arg Thr Pro Gly
165 170 175

Lys Glu Asp Glu Phe Val Ala Ser Ile Ser Trp Thr Tyr Gly Ser Thr

180 185 190

Phe Tyr Ala Asp Ser Val Lys Gly Arg Phe Thr Met Ser Arg Asp Lys
195 200 205

Ala Lys Asn Ala Gly Tyr Leu Gln Met Asn Ser Leu Lys Pro Glu Asp
210 215 220

Thr Ala Leu Tyr Tyr Cys Ala Ala Gly Ala Gln Ser Asp Arg Tyr Asn
225 230 235 240

Ile Arg Ser Tyr Asp Tyr Trp Gly Gln Gly Thr Gln Val Thr Val Ser
245 250 255

Ser

<210> 137
<211> 249
<212> PRT
<213> Artificial

<220>
<223> Artificial polypeptide comprising lama glama sequences

<400> 137

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Lys Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Gln Val Thr Val Ser Ser Gly Gly Gly
115 120 125

Gly Ser Gly Gly Gly Ser Glu Val Gln Leu Val Glu Ser Gly Gly Gly
    130                 135                 140

Leu Val Gln Pro Gly Gly Ser Leu Lys Leu Ser Cys Thr Ala Ser Gly
145                 150                 155                 160

Phe Thr Phe Ser Thr Ser Trp Met His Trp Val Arg Gln Ala Pro Gly
                165                 170                 175

Lys Gly Leu Glu Trp Val Ser Ser Ile Pro Pro Val Gly His Phe Ala
            180                 185                 190

Asn Tyr Ala Pro Ser Val Lys Gly Arg Phe Thr Ile Ser Arg Asp Asn
        195                 200                 205

Ala Lys Asn Thr Leu Phe Leu Gln Met Asn Ser Leu Lys Ser Glu Asp
    210                 215                 220

Thr Ala Val Tyr Tyr Cys Ala Lys Asp Ser Ala Gly Arg Thr Lys Gly
225                 230                 235                 240

Gln Gly Thr Gln Val Thr Val Ser Ser
                245

<210> 138
<211> 258
<212> PRT
<213> Artificial

<220>
<223> Artificial polypeptide comprising lama glama sequences

<400> 138

Glu Val Gln Leu Val Glu Ser Gly Gly Gly Leu Val Gln Thr Gly Asp
1               5                   10                  15

Ser Leu Arg Leu Ser Cys Glu Val Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly
        115                 120                 125

Gly Ser Gly Gly Gly Ser Glu Val Gln Leu Val Glu Ser Gly Gly Gly
    130                 135                 140

Leu Val Gln Ala Gly Gly Ser Leu Arg Leu Ser Cys Ala Ala Ser Glu
145                 150                 155                 160

Arg Thr Phe Ser Asn Tyr Ala Met Asp Trp Phe Arg Gln Ala Pro Gly
                165                 170                 175

Lys Glu Arg Glu Phe Val Ala Ala Ile Thr Arg Ser Gly Gly Gly Thr
            180                 185                 190

Tyr Tyr Ala Asp Ser Val Lys Gly Arg Phe Thr Ile Ser Arg Asp Asn
        195                 200                 205

Ala Lys Asn Thr Val Tyr Leu Gln Met Asn Ser Leu Lys Pro Glu Asp
    210                 215                 220

Thr Ala Val Tyr Tyr Cys Ala Ala Thr Arg Ser Ser Thr Ile Val Val
225                 230                 235                 240

Gly Val Gly Gly Met Glu Tyr Trp Gly Lys Gly Thr Gln Val Thr Val
                245                 250                 255

Ser Ser

<210> 139
<211> 276
<212> PRT
<213> Artificial

<220>
<223> Artificial polypeptide comprising lama glama sequences

<400> 139

Glu Val Gln Leu Val Glu Ser Gly Gly Gly Leu Val Gln Thr Gly Asp
1               5                   10                  15

```
Ser Leu Arg Leu Ser Cys Glu Val Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly
        115                 120                 125

Gly Ser Gly Gly Gly Gly Ser Gly Gly Gly Gly Ser Gly Gly Gly Gly
    130                 135                 140

Ser Gly Gly Gly Gly Ser Gly Gly Gly Gly Ser Gly Gly Gly Gly Ser
145                 150                 155                 160

Gly Gly Gly Gly Ser Glu Val Gln Leu Val Glu Ser Gly Gly Gly Leu
                165                 170                 175

Val Gln Pro Gly Gly Ser Leu Arg Leu Ser Cys Ala Ala Ser Gly Ser
            180                 185                 190

Ala Val Gly Asp Ile Thr Val Ala Trp Tyr Arg Gln Ala Pro Gly Ile
        195                 200                 205

Gln Arg Gln Leu Val Ala Thr Ile Thr Pro Ser Gly Tyr Thr Tyr Tyr
    210                 215                 220

Trp Asp Phe Val Lys Gly Arg Phe Thr Ile Ser Arg Asp Asn Ser Lys
225                 230                 235                 240

Asn Ile Val Tyr Leu Gln Met Asn Ser Leu Lys Pro Glu Asp Thr Ala
                245                 250                 255

Ala Tyr Tyr Cys Asn Thr Gln Phe Tyr Trp Gly Gln Gly Thr Gln Val
            260                 265                 270
```

Thr Val Ser Ser
275

<210> 140
<211> 286
<212> PRT
<213> Artificial

<220>
<223> Artificial polypeptide comprising lama glama sequences

<400> 140

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Lys Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Gln Val Thr Val Ser Ser Gly Gly Gly
115 120 125

Gly Ser Gly Gly Gly Gly Ser Gly Gly Gly Gly Ser Gly Gly Gly Gly
130 135 140

Ser Gly Gly Gly Gly Ser Gly Gly Gly Gly Ser Gly Gly Gly Gly Ser
145 150 155 160

Gly Gly Gly Gly Ser Glu Val Gln Leu Val Glu Ser Gly Gly Gly Leu
165 170 175

Ala Gln Ala Gly Asp Ser Leu Arg Leu Ser Cys Ala Ala Ser Gly Arg
180 185 190

Ser Phe Ser His Tyr Asn Met Gly Trp Phe Arg Gln Ala Pro Gly Lys
        195                 200                 205

Glu Arg Glu Phe Val Ala Ser Ile Arg Gly Gly Gly Gly Ser Thr Thr
    210                 215                 220

Tyr Ala Asn Ser Val Lys Asp Arg Phe Thr Ile Ser Arg Glu Asn Ala
225                 230                 235                 240

Lys Asn Thr Val Tyr Leu Gln Met Asn Ser Leu Lys Pro Glu Asp Thr
                245                 250                 255

Ala Val Tyr Tyr Cys Ala Ala Thr Ala Phe Tyr Arg Gly Pro Tyr Asp
            260                 265                 270

Tyr Asp Tyr Trp Gly Gln Gly Thr Gln Val Thr Val Ser Ser
        275                 280                 285

<210> 141
<211> 278
<212> PRT
<213> Artificial

<220>
<223> Artificial polypeptide comprising lama glama sequences

<400> 141

Glu Val Gln Leu Val Glu Ser Gly Gly Gly Leu Val Gln Thr Gly Asp
1               5                   10                  15

Ser Leu Arg Leu Ser Cys Glu Val Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly

115 120 125

Gly Ser Gly Gly Gly Gly Ser Gly Gly Gly Gly Ser Gly Gly Gly Gly
130 135 140

Ser Gly Gly Gly Gly Ser Gly Gly Gly Gly Ser Gly Gly Gly Gly Ser
145 150 155 160

Gly Gly Gly Gly Ser Glu Val Gln Leu Val Glu Ser Gly Gly Gly Leu
165 170 175

Val Gln Pro Gly Gly Ser Leu Arg Leu Ser Cys Val Ala Ser Gly Ile
180 185 190

Arg Phe Met Ser Met Ala Trp Tyr Arg Gln Ala Pro Gly Lys His Arg
195 200 205

Glu Leu Val Ala Arg Ile Ser Ser Gly Gly Thr Thr Ala Tyr Val Asp
210 215 220

Ser Val Lys Gly Arg Phe Thr Ile Ser Arg Asp Asn Ser Lys Asn Thr
225 230 235 240

Val Tyr Leu Gln Met Asn Ser Leu Lys Ala Glu Asp Thr Ala Val Tyr
245 250 255

Tyr Cys Asn Thr Phe Ser Ser Arg Pro Asn Pro Trp Gly Ala Gly Thr
260 265 270

Gln Val Thr Val Ser Ser
275

<210> 142
<211> 281
<212> PRT
<213> Artificial

<220>
<223> Artificial polypeptide comprising lama glama sequences

<400> 142

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

```
Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly
        115                 120                 125

Gly Ser Gly Gly Gly Gly Ser Gly Gly Gly Gly Ser Gly Gly Gly Gly
    130                 135                 140

Ser Gly Gly Gly Gly Ser Gly Gly Gly Gly Ser Gly Gly Gly Gly Ser
145                 150                 155                 160

Gly Gly Gly Gly Ser Glu Val Gln Leu Val Glu Ser Gly Gly Gly Leu
                165                 170                 175

Val Gln Pro Gly Gly Ser Leu Arg Leu Ser Cys Ala Ala Ser Gly Asn
            180                 185                 190

Ile Phe Ser Asn Asn Ala Met Ala Trp Tyr Arg Gln Ala Pro Gly Lys
        195                 200                 205

Gln Arg Glu Leu Val Ala Arg Ile Ser Ser Gly Gly Gly Phe Thr Tyr
    210                 215                 220

Tyr Leu Asp Ser Val Lys Gly Arg Phe Thr Val Ser Arg Asp Asn Ala
225                 230                 235                 240

Lys Asn Thr Val Tyr Leu Gln Met Asn Ser Leu Lys Pro Glu Asp Thr
                245                 250                 255

Ala Val Tyr Tyr Cys Asn Ala Ala Tyr Arg Thr Tyr Asn Tyr Trp Gly
            260                 265                 270

Gln Gly Thr Gln Val Thr Val Ser Ser
        275                 280
```

<210> 143

<211> 289
<212> PRT
<213> Artificial

<220>
<223> Artificial polypeptide comprising lama glama sequences

<400> 143

```
Glu Val Gln Leu Val Glu Ser Gly Gly Gly Leu Val Gln Thr Gly Asp
1               5                   10                  15

Ser Leu Arg Leu Ser Cys Glu Val Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly
        115                 120                 125

Gly Ser Gly Gly Gly Gly Ser Gly Gly Gly Gly Ser Gly Gly Gly Gly
    130                 135                 140

Ser Gly Gly Gly Gly Ser Gly Gly Gly Gly Ser Gly Gly Gly Gly Ser
145                 150                 155                 160

Gly Gly Gly Gly Ser Glu Val Gln Leu Val Glu Ser Gly Gly Gly Leu
                165                 170                 175

Val Gln Ala Gly Gly Ser Leu Arg Leu Ser Cys Ala Ala Ser Gly Arg
            180                 185                 190

Thr Phe Ser Asn Tyr Ala Met Gly Trp Phe Arg Gln Ala Pro Gly Lys
        195                 200                 205

Glu Arg Val Leu Val Ala Asp Ile Ser Ser Ser Gly Ile Asn Thr Tyr
    210                 215                 220
```

Val Ala Asp Ala Val Lys Gly Arg Phe Thr Ile Ser Arg Asp Asn Ala
225                 230                 235                 240

Lys Asn Thr Val Tyr Leu Gln Met Asn Ser Leu Lys Pro Glu Asp Thr
                245                 250                 255

Ala Val Tyr Tyr Cys Ala Ala Ser Ala Trp Trp Tyr Ser Gln Met Ala
            260                 265                 270

Arg Asp Asn Tyr Arg Tyr Trp Gly Gln Gly Thr Gln Val Thr Val Ser
        275                 280                 285

Ser

<210>  144
<211>  282
<212>  PRT
<213>  Artificial

<220>
<223>  Artificial polypeptide comprising lama glama sequences

<400>  144

Glu Val Gln Leu Val Glu Ser Gly Gly Gly Leu Val Gln Thr Gly Asp
1               5                   10                  15

Ser Leu Arg Leu Ser Cys Glu Val Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly
        115                 120                 125

Gly Ser Gly Gly Gly Gly Ser Gly Gly Gly Gly Ser Gly Gly Gly Gly
130 135 140

Ser Gly Gly Gly Gly Ser Gly Gly Gly Gly Ser Gly Gly Gly Gly Ser
145 150 155 160

Gly Gly Gly Gly Ser Glu Val Gln Leu Val Glu Ser Gly Gly Gly Leu
165 170 175

Val Gln Pro Gly Gly Ser Leu Arg Leu Ala Cys Ala Ala Ser Gly Phe
180 185 190

Thr Leu Ser Ser Ser Trp Met Tyr Trp Val Arg Gln Ala Pro Gly Lys
195 200 205

Gly Leu Glu Trp Val Ser Arg Ile Ser Pro Gly Gly Leu Phe Thr Tyr
210 215 220

Tyr Val Asp Ser Val Lys Gly Arg Phe Ser Val Ser Thr Asp Asn Ala
225 230 235 240

Asn Asn Thr Leu Tyr Leu Gln Met Asn Ser Leu Lys Pro Glu Asp Thr
245 250 255

Ala Leu Tyr Ser Cys Ala Lys Gly Gly Ala Pro Asn Tyr Thr Pro Arg
260 265 270

Gly Arg Gly Thr Gln Val Thr Val Ser Ser
275 280

<210> 145
<211> 290
<212> PRT
<213> Artificial

<220>
<223> Artificial polypeptide comprising lama glama sequences

<400> 145

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Lys Gly Gly Tyr Lys Tyr Asp Ser Val Ser Leu Glu
    50              55              60

Gly Arg Phe Thr Ile Ser Lys Asp Asn Ala Lys Asn Thr Val Tyr Leu
65              70              75              80

Gln Ile Asn Ser Leu Lys Pro Glu Asp Thr Ala Val Tyr Tyr Cys Ala
                85              90              95

Ser Ser Arg Ala Tyr Gly Ser Ser Arg Leu Arg Leu Ala Asp Thr Tyr
            100             105             110

Glu Tyr Trp Gly Gln Gly Thr Gln Val Thr Val Ser Ser Gly Gly Gly
        115             120             125

Gly Ser Gly Gly Gly Gly Ser Gly Gly Gly Gly Ser Gly Gly Gly Gly
    130             135             140

Ser Gly Gly Gly Gly Ser Gly Gly Gly Gly Ser Gly Gly Gly Gly Ser
145             150             155             160

Gly Gly Gly Gly Ser Glu Val Gln Leu Val Glu Ser Gly Gly Gly Leu
                165             170             175

Val Gln Ala Gly Asp Ser Leu Arg Leu Ser Cys Thr Ala Ser Gly Arg
            180             185             190

Thr Phe Asn Ser Tyr Ala Met Gly Trp Phe Arg Gln Ala Pro Gly Lys
        195             200             205

Glu Arg Glu Ser Val Ala His Ile Asn Arg Ser Gly Ser Ser Thr Tyr
    210             215             220

Tyr Ala Asp Ser Val Lys Gly Arg Phe Thr Ile Ser Arg Asp Asn Ala
225             230             235             240

Lys Asn Thr Val Tyr Leu Gln Leu Asn Ser Leu Lys Pro Glu Asp Thr
                245             250             255

Ala Val Tyr Tyr Cys Ala Ala Gly Arg Tyr Tyr Ser Ser Asp Gly Val
            260             265             270

Pro Ser Ala Ser Phe Asn Tyr Trp Gly Gln Gly Thr Gln Val Thr Val
        275             280             285

Ser Ser
    290

<210> 146
<211> 288
<212> PRT
<213> Artificial

<220>
<223> Artificial polypeptide comprising lama glama sequences

<400> 146

```
Glu Val Gln Leu Val Glu Ser Gly Gly Gly Leu Val Gln Thr Gly Asp
1               5                   10                  15

Ser Leu Arg Leu Ser Cys Glu Val Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly
        115                 120                 125

Gly Ser Gly Gly Gly Gly Ser Gly Gly Gly Gly Ser Gly Gly Gly Gly
    130                 135                 140

Ser Gly Gly Gly Gly Ser Gly Gly Gly Gly Ser Gly Gly Gly Gly Ser
145                 150                 155                 160

Gly Gly Gly Gly Ser Glu Val Gln Leu Val Glu Ser Gly Gly Gly Leu
                165                 170                 175

Val Gln Ala Gly Gly Ser Leu Arg Leu Ser Cys Thr Ala Ser Gly Ser
            180                 185                 190

Ala Phe Lys Ser Tyr Arg Met Gly Trp Phe Arg Arg Thr Pro Gly Lys
        195                 200                 205
```

Glu Asp Glu Phe Val Ala Ser Ile Ser Trp Thr Tyr Gly Ser Thr Phe
    210                 215                 220

Tyr Ala Asp Ser Val Lys Gly Arg Phe Thr Met Ser Arg Asp Lys Ala
225                 230                 235                 240

Lys Asn Ala Gly Tyr Leu Gln Met Asn Ser Leu Lys Pro Glu Asp Thr
                245                 250                 255

Ala Leu Tyr Tyr Cys Ala Ala Gly Ala Gln Ser Asp Arg Tyr Asn Ile
            260                 265                 270

Arg Ser Tyr Asp Tyr Trp Gly Gln Gly Thr Gln Val Thr Val Ser Ser
        275                 280                 285

<210> 147
<211> 280
<212> PRT
<213> Artificial

<220>
<223> Artificial polypeptide comprising lama glama sequences

<400> 147

Glu Val Gln Leu Val Glu Ser Gly Gly Gly Leu Val Gln Thr Gly Asp
1               5                   10                  15

Ser Leu Arg Leu Ser Cys Glu Val Ser Gly Arg Thr Phe Ser Ser Tyr
            20                  25                  30

Ser Met Gly Trp Phe Arg Gln Ala Gln Gly Lys Glu Arg Glu Phe Val
        35                  40                  45

Val Ala Ile Ser Lys Gly Gly Tyr Lys Tyr Asp Ser Val Ser Leu Glu
    50                  55                  60

Gly Arg Phe Thr Ile Ser Lys Asp Asn Ala Lys Asn Thr Val Tyr Leu
65                  70                  75                  80

Gln Ile Asn Ser Leu Lys Pro Glu Asp Thr Ala Val Tyr Tyr Cys Ala
                85                  90                  95

Ser Ser Arg Ala Tyr Gly Ser Ser Arg Leu Arg Leu Ala Asp Thr Tyr
            100                 105                 110

Glu Tyr Trp Gly Gln Gly Thr Gln Val Thr Val Ser Ser Gly Gly Gly
        115                 120                 125

Gly Ser Gly Gly Gly Gly Ser Gly Gly Gly Gly Ser Gly Gly Gly Gly

130 135 140

Ser Gly Gly Gly Gly Ser Gly Gly Gly Gly Ser Gly Gly Gly Gly Ser
145 150 155 160

Gly Gly Gly Gly Ser Glu Val Gln Leu Val Glu Ser Gly Gly Gly Leu
165 170 175

Val Gln Pro Gly Gly Ser Leu Lys Leu Ser Cys Thr Ala Ser Gly Phe
180 185 190

Thr Phe Ser Thr Ser Trp Met His Trp Val Arg Gln Ala Pro Gly Lys
195 200 205

Gly Leu Glu Trp Val Ser Ser Ile Pro Pro Val Gly His Phe Ala Asn
210 215 220

Tyr Ala Pro Ser Val Lys Gly Arg Phe Thr Ile Ser Arg Asp Asn Ala
225 230 235 240

Lys Asn Thr Leu Phe Leu Gln Met Asn Ser Leu Lys Ser Glu Asp Thr
245 250 255

Ala Val Tyr Tyr Cys Ala Lys Asp Ser Ala Gly Arg Thr Lys Gly Gln
260 265 270

Gly Thr Gln Val Thr Val Ser Ser
275 280

<210> 148
<211> 289
<212> PRT
<213> Artificial

<220>
<223> Artificial polypeptide comprising lama glama sequences

<400> 148

Glu Val Gln Leu Val Glu Ser Gly Gly Gly Leu Val Gln Thr Gly Asp
1 5 10 15

Ser Leu Arg Leu Ser Cys Glu Val Ser Gly Arg Thr Phe Ser Ser Tyr
20 25 30

Ser Met Gly Trp Phe Arg Gln Ala Gln Gly Lys Glu Arg Glu Phe Val
35 40 45

Val Ala Ile Ser Lys Gly Gly Tyr Lys Tyr Asp Ser Val Ser Leu Glu
50 55 60

Gly Arg Phe Thr Ile Ser Lys Asp Asn Ala Lys Asn Thr Val Tyr Leu
65 70 75 80

Gln Ile Asn Ser Leu Lys Pro Glu Asp Thr Ala Val Tyr Tyr Cys Ala
85 90 95

Ser Ser Arg Ala Tyr Gly Ser Ser Arg Leu Arg Leu Ala Asp Thr Tyr
100 105 110

Glu Tyr Trp Gly Gln Gly Thr Gln Val Thr Val Ser Ser Gly Gly Gly
115 120 125

Gly Ser Gly Gly Gly Gly Ser Gly Gly Gly Gly Ser Gly Gly Gly Gly
130 135 140

Ser Gly Gly Gly Gly Ser Gly Gly Gly Gly Ser Gly Gly Gly Gly Ser
145 150 155 160

Gly Gly Gly Gly Ser Glu Val Gln Leu Val Glu Ser Gly Gly Gly Leu
165 170 175

Val Gln Ala Gly Gly Ser Leu Arg Leu Ser Cys Ala Ala Ser Glu Arg
180 185 190

Thr Phe Ser Asn Tyr Ala Met Asp Trp Phe Arg Gln Ala Pro Gly Lys
195 200 205

Glu Arg Glu Phe Val Ala Ala Ile Thr Arg Ser Gly Gly Gly Thr Tyr
210 215 220

Tyr Ala Asp Ser Val Lys Gly Arg Phe Thr Ile Ser Arg Asp Asn Ala
225 230 235 240

Lys Asn Thr Val Tyr Leu Gln Met Asn Ser Leu Lys Pro Glu Asp Thr
245 250 255

Ala Val Tyr Tyr Cys Ala Ala Thr Arg Ser Ser Thr Ile Val Val Gly
260 265 270

Val Gly Gly Met Glu Tyr Trp Gly Lys Gly Thr Gln Val Thr Val Ser
275 280 285

Ser

<210> 149

<211> 245
<212> PRT
<213> Artificial

<220>
<223> Artificial polypeptide comprising lama glama sequences

<400> 149

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Ser Ala Val Gly Asp Ile
            20                  25                  30

Thr Val Ala Trp Tyr Arg Gln Ala Pro Gly Ile Gln Arg Gln Leu Val
        35                  40                  45

Ala Thr Ile Thr Pro Ser Gly Tyr Thr Tyr Tyr Trp Asp Phe Val Lys
    50                  55                  60

Gly Arg Phe Thr Ile Ser Arg Asp Asn Ser Lys Asn Ile Val Tyr Leu
65                  70                  75                  80

Gln Met Asn Ser Leu Lys Pro Glu Asp Thr Ala Ala Tyr Tyr Cys Asn
                85                  90                  95

Thr Gln Phe Tyr Trp Gly Gln Gly Thr Gln Val Thr Val Ser Ser Gly
            100                 105                 110

Gly Gly Gly Ser Gly Gly Gly Ser Glu Val Gln Leu Val Glu Ser Gly
        115                 120                 125

Gly Gly Leu Val Gln Thr Gly Asp Ser Leu Arg Leu Ser Cys Glu Val
    130                 135                 140

Ser Gly Arg Thr Phe Ser Ser Tyr Ser Met Gly Trp Phe Arg Gln Ala
145                 150                 155                 160

Gln Gly Lys Glu Arg Glu Phe Val Val Ala Ile Ser Lys Gly Gly Tyr
                165                 170                 175

Lys Tyr Asp Ser Val Ser Leu Glu Gly Arg Phe Thr Ile Ser Lys Asp
            180                 185                 190

Asn Ala Lys Asn Thr Val Tyr Leu Gln Ile Asn Ser Leu Lys Pro Glu
        195                 200                 205

Asp Thr Ala Val Tyr Tyr Cys Ala Ser Ser Arg Ala Tyr Gly Ser Ser
    210                 215                 220
```

```
Arg Leu Arg Leu Ala Asp Thr Tyr Glu Tyr Trp Gly Gln Gly Thr Gln
225                 230                 235                 240

Val Thr Val Ser Ser
                245

<210>  150
<211>  255
<212>  PRT
<213>  Artificial

<220>
<223>  Artificial polypeptide comprising lama glama sequences

<400>  150

Glu Val Gln Leu Val Glu Ser Gly Gly Gly Leu Ala Gln Ala Gly Asp
1               5                   10                  15

Ser Leu Arg Leu Ser Cys Ala Ala Ser Gly Arg Ser Phe Ser His Tyr
            20                  25                  30

Asn Met Gly Trp Phe Arg Gln Ala Pro Gly Lys Glu Arg Glu Phe Val
        35                  40                  45

Ala Ser Ile Arg Gly Gly Gly Gly Ser Thr Thr Tyr Ala Asn Ser Val
    50                  55                  60

Lys Asp Arg Phe Thr Ile Ser Arg Glu Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Thr Ala Phe Tyr Arg Gly Pro Tyr Asp Tyr Asp Tyr Trp Gly
            100                 105                 110

Gln Gly Thr Gln Val Thr Val Ser Ser Gly Gly Gly Gly Ser Gly Gly
        115                 120                 125

Gly Ser Glu Val Gln Leu Val Glu Ser Gly Gly Gly Leu Val Gln Thr
    130                 135                 140

Gly Asp Ser Leu Arg Leu Ser Cys Glu Val Ser Gly Arg Thr Phe Ser
145                 150                 155                 160

Ser Tyr Ser Met Gly Trp Phe Arg Gln Ala Gln Gly Lys Glu Arg Glu
                165                 170                 175
```

Phe Val Val Ala Ile Ser Lys Gly Gly Tyr Lys Tyr Asp Ser Val Ser
180 185 190

Leu Glu Gly Arg Phe Thr Ile Ser Lys Asp Asn Ala Lys Asn Thr Val
195 200 205

Tyr Leu Gln Ile Asn Ser Leu Lys Pro Glu Asp Thr Ala Val Tyr Tyr
210 215 220

Cys Ala Ser Ser Arg Ala Tyr Gly Ser Ser Arg Leu Arg Leu Ala Asp
225 230 235 240

Thr Tyr Glu Tyr Trp Gly Gln Gly Thr Gln Val Thr Val Ser Ser
245 250 255

<210> 151
<211> 247
<212> PRT
<213> Artificial

<220>
<223> Artificial polypeptide comprising lama glama sequences

<400> 151

Glu Val Gln Leu Val Glu Ser Gly Gly Gly Leu Val Gln Pro Gly Gly
1 5 10 15

Ser Leu Arg Leu Ser Cys Val Ala Ser Gly Ile Arg Phe Met Ser Met
20 25 30

Ala Trp Tyr Arg Gln Ala Pro Gly Lys His Arg Glu Leu Val Ala Arg
35 40 45

Ile Ser Ser Gly Gly Thr Thr Ala Tyr Val Asp Ser Val Lys Gly Arg
50 55 60

Phe Thr Ile Ser Arg Asp Asn Ser Lys Asn Thr Val Tyr Leu Gln Met
65 70 75 80

Asn Ser Leu Lys Ala Glu Asp Thr Ala Val Tyr Tyr Cys Asn Thr Phe
85 90 95

Ser Ser Arg Pro Asn Pro Trp Gly Ala Gly Thr Gln Val Thr Val Ser
100 105 110

Ser Gly Gly Gly Gly Ser Gly Gly Gly Ser Glu Val Gln Leu Val Glu
115 120 125

Ser Gly Gly Gly Leu Val Gln Thr Gly Asp Ser Leu Arg Leu Ser Cys
    130                 135                 140

Glu Val Ser Gly Arg Thr Phe Ser Ser Tyr Ser Met Gly Trp Phe Arg
145                 150                 155                 160

Gln Ala Gln Gly Lys Glu Arg Glu Phe Val Val Ala Ile Ser Lys Gly
                165                 170                 175

Gly Tyr Lys Tyr Asp Ser Val Ser Leu Glu Gly Arg Phe Thr Ile Ser
            180                 185                 190

Lys Asp Asn Ala Lys Asn Thr Val Tyr Leu Gln Ile Asn Ser Leu Lys
        195                 200                 205

Pro Glu Asp Thr Ala Val Tyr Tyr Cys Ala Ser Ser Arg Ala Tyr Gly
    210                 215                 220

Ser Ser Arg Leu Arg Leu Ala Asp Thr Tyr Glu Tyr Trp Gly Gln Gly
225                 230                 235                 240

Thr Gln Val Thr Val Ser Ser
                245

<210> 152
<211> 250
<212> PRT
<213> Artificial

<220>
<223> Artificial polypeptide comprising lama glama sequences

<400> 152

Glu Val Gln Leu Val Glu Ser Gly Gly Gly Leu Val Gln Pro Gly Gly
1               5                   10                  15

Ser Leu Arg Leu Ser Cys Ala Ala Ser Gly Asn Ile Phe Ser Asn Asn
            20                  25                  30

Ala Met Ala Trp Tyr Arg Gln Ala Pro Gly Lys Gln Arg Glu Leu Val
        35                  40                  45

Ala Arg Ile Ser Ser Gly Gly Gly Phe Thr Tyr Tyr Leu Asp Ser Val
    50                  55                  60

Lys Gly Arg Phe Thr Val Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys

85 90 95

Asn Ala Ala Tyr Arg Thr Tyr Asn Tyr Trp Gly Gln Gly Thr Gln Val
100 105 110

Thr Val Ser Ser Gly Gly Gly Gly Ser Gly Gly Gly Ser Glu Val Gln
115 120 125

Leu Val Glu Ser Gly Gly Gly Leu Val Gln Thr Gly Asp Ser Leu Arg
130 135 140

Leu Ser Cys Glu Val Ser Gly Arg Thr Phe Ser Ser Tyr Ser Met Gly
145 150 155 160

Trp Phe Arg Gln Ala Gln Gly Lys Glu Arg Glu Phe Val Val Ala Ile
165 170 175

Ser Lys Gly Gly Tyr Lys Tyr Asp Ser Val Ser Leu Glu Gly Arg Phe
180 185 190

Thr Ile Ser Lys Asp Asn Ala Lys Asn Thr Val Tyr Leu Gln Ile Asn
195 200 205

Ser Leu Lys Pro Glu Asp Thr Ala Val Tyr Tyr Cys Ala Ser Ser Arg
210 215 220

Ala Tyr Gly Ser Ser Arg Leu Arg Leu Ala Asp Thr Tyr Glu Tyr Trp
225 230 235 240

Gly Gln Gly Thr Gln Val Thr Val Ser Ser
245 250

<210> 153
<211> 258
<212> PRT
<213> Artificial

<220>
<223> Artificial polypeptide comprising lama glama sequences

<400> 153

Glu Val Gln Leu Val Glu Ser Gly Gly Gly Leu Val Gln Ala Gly Gly
1 5 10 15

Ser Leu Arg Leu Ser Cys Ala Ala Ser Gly Arg Thr Phe Ser Asn Tyr
20 25 30

Ala Met Gly Trp Phe Arg Gln Ala Pro Gly Lys Glu Arg Val Leu Val
35 40 45

Ala Asp Ile Ser Ser Ser Gly Ile Asn Thr Tyr Val Ala Asp Ala Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Ser Ala Trp Trp Tyr Ser Gln Met Ala Arg Asp Asn Tyr Arg
            100                 105                 110

Tyr Trp Gly Gln Gly Thr Gln Val Thr Val Ser Ser Gly Gly Gly Gly
        115                 120                 125

Ser Gly Gly Gly Ser Glu Val Gln Leu Val Glu Ser Gly Gly Gly Leu
    130                 135                 140

Val Gln Thr Gly Asp Ser Leu Arg Leu Ser Cys Glu Val Ser Gly Arg
145                 150                 155                 160

Thr Phe Ser Ser Tyr Ser Met Gly Trp Phe Arg Gln Ala Gln Gly Lys
                165                 170                 175

Glu Arg Glu Phe Val Val Ala Ile Ser Lys Gly Gly Tyr Lys Tyr Asp
            180                 185                 190

Ser Val Ser Leu Glu Gly Arg Phe Thr Ile Ser Lys Asp Asn Ala Lys
        195                 200                 205

Asn Thr Val Tyr Leu Gln Ile Asn Ser Leu Lys Pro Glu Asp Thr Ala
    210                 215                 220

Val Tyr Tyr Cys Ala Ser Ser Arg Ala Tyr Gly Ser Ser Arg Leu Arg
225                 230                 235                 240

Leu Ala Asp Thr Tyr Glu Tyr Trp Gly Gln Gly Thr Gln Val Thr Val
                245                 250                 255

Ser Ser

<210> 154
<211> 251
<212> PRT
<213> Artificial

<220>
<223>  Artificial polypeptide comprising lama glama sequences

<400>  154

```
Glu Val Gln Leu Val Glu Ser Gly Gly Gly Leu Val Gln Pro Gly Gly
1               5                   10                  15

Ser Leu Arg Leu Ala Cys Ala Ala Ser Gly Phe Thr Leu Ser Ser Ser
            20                  25                  30

Trp Met Tyr Trp Val Arg Gln Ala Pro Gly Lys Gly Leu Glu Trp Val
        35                  40                  45

Ser Arg Ile Ser Pro Gly Gly Leu Phe Thr Tyr Tyr Val Asp Ser Val
    50                  55                  60

Lys Gly Arg Phe Ser Val Ser Thr Asp Asn Ala Asn Asn Thr Leu Tyr
65                  70                  75                  80

Leu Gln Met Asn Ser Leu Lys Pro Glu Asp Thr Ala Leu Tyr Ser Cys
                85                  90                  95

Ala Lys Gly Gly Ala Pro Asn Tyr Thr Pro Arg Gly Arg Gly Thr Gln
            100                 105                 110

Val Thr Val Ser Ser Gly Gly Gly Gly Ser Gly Gly Gly Ser Glu Val
        115                 120                 125

Gln Leu Val Glu Ser Gly Gly Gly Leu Val Gln Thr Gly Asp Ser Leu
    130                 135                 140

Arg Leu Ser Cys Glu Val Ser Gly Arg Thr Phe Ser Ser Tyr Ser Met
145                 150                 155                 160

Gly Trp Phe Arg Gln Ala Gln Gly Lys Glu Arg Glu Phe Val Val Ala
                165                 170                 175

Ile Ser Lys Gly Gly Tyr Lys Tyr Asp Ser Val Ser Leu Glu Gly Arg
            180                 185                 190

Phe Thr Ile Ser Lys Asp Asn Ala Lys Asn Thr Val Tyr Leu Gln Ile
        195                 200                 205

Asn Ser Leu Lys Pro Glu Asp Thr Ala Val Tyr Tyr Cys Ala Ser Ser
    210                 215                 220

Arg Ala Tyr Gly Ser Ser Arg Leu Arg Leu Ala Asp Thr Tyr Glu Tyr
225                 230                 235                 240
```

Trp Gly Gln Gly Thr Gln Val Thr Val Ser Ser
245 250

<210> 155
<211> 259
<212> PRT
<213> Artificial

<220>
<223> Artificial polypeptide comprising lama glama sequences

<400> 155

Glu Val Gln Leu Val Glu Ser Gly Gly Gly Leu Val Gln Ala Gly Asp
1 5 10 15

Ser Leu Arg Leu Ser Cys Thr Ala Ser Gly Arg Thr Phe Asn Ser Tyr
20 25 30

Ala Met Gly Trp Phe Arg Gln Ala Pro Gly Lys Glu Arg Glu Ser Val
35 40 45

Ala His Ile Asn Arg Ser Gly Ser Ser Thr Tyr Tyr Ala Asp Ser Val
50 55 60

Lys Gly Arg Phe Thr Ile Ser Arg Asp Asn Ala Lys Asn Thr Val Tyr
65 70 75 80

Leu Gln Leu Asn Ser Leu Lys Pro Glu Asp Thr Ala Val Tyr Tyr Cys
85 90 95

Ala Ala Gly Arg Tyr Tyr Ser Ser Asp Gly Val Pro Ser Ala Ser Phe
100 105 110

Asn Tyr Trp Gly Gln Gly Thr Gln Val Thr Val Ser Ser Gly Gly Gly
115 120 125

Gly Ser Gly Gly Gly Ser Glu Val Gln Leu Val Glu Ser Gly Gly Gly
130 135 140

Leu Val Gln Thr Gly Asp Ser Leu Arg Leu Ser Cys Glu Val Ser Gly
145 150 155 160

Arg Thr Phe Ser Ser Tyr Ser Met Gly Trp Phe Arg Gln Ala Gln Gly
165 170 175

Lys Glu Arg Glu Phe Val Val Ala Ile Ser Lys Gly Gly Tyr Lys Tyr
180 185 190

```
Asp Ser Val Ser Leu Glu Gly Arg Phe Thr Ile Ser Lys Asp Asn Ala
        195                 200                 205

Lys Asn Thr Val Tyr Leu Gln Ile Asn Ser Leu Lys Pro Glu Asp Thr
    210                 215                 220

Ala Val Tyr Tyr Cys Ala Ser Ser Arg Ala Tyr Gly Ser Ser Arg Leu
225                 230                 235                 240

Arg Leu Ala Asp Thr Tyr Glu Tyr Trp Gly Gln Gly Thr Gln Val Thr
                245                 250                 255

Val Ser Ser



<210>  156
<211>  257
<212>  PRT
<213>  Artificial

<220>
<223>  Artificial polypeptide comprising lama glama sequences

<400>  156

Glu Val Gln Leu Val Glu Ser Gly Gly Gly Leu Val Gln Ala Gly Gly
1               5                   10                  15

Ser Leu Arg Leu Ser Cys Thr Ala Ser Gly Ser Ala Phe Lys Ser Tyr
            20                  25                  30

Arg Met Gly Trp Phe Arg Arg Thr Pro Gly Lys Glu Asp Glu Phe Val
        35                  40                  45

Ala Ser Ile Ser Trp Thr Tyr Gly Ser Thr Phe Tyr Ala Asp Ser Val
    50                  55                  60

Lys Gly Arg Phe Thr Met Ser Arg Asp Lys Ala Lys Asn Ala Gly Tyr
65                  70                  75                  80

Leu Gln Met Asn Ser Leu Lys Pro Glu Asp Thr Ala Leu Tyr Tyr Cys
                85                  90                  95

Ala Ala Gly Ala Gln Ser Asp Arg Tyr Asn Ile Arg Ser Tyr Asp Tyr
            100                 105                 110

Trp Gly Gln Gly Thr Gln Val Thr Val Ser Ser Gly Gly Gly Gly Ser
        115                 120                 125
```

Gly Gly Gly Ser Glu Val Gln Leu Val Glu Ser Gly Gly Gly Leu Val
    130                 135                 140

Gln Thr Gly Asp Ser Leu Arg Leu Ser Cys Glu Val Ser Gly Arg Thr
145                 150                 155                 160

Phe Ser Ser Tyr Ser Met Gly Trp Phe Arg Gln Ala Gln Gly Lys Glu
                165                 170                 175

Arg Glu Phe Val Val Ala Ile Ser Lys Gly Gly Tyr Lys Tyr Asp Ser
            180                 185                 190

Val Ser Leu Glu Gly Arg Phe Thr Ile Ser Lys Asp Asn Ala Lys Asn
        195                 200                 205

Thr Val Tyr Leu Gln Ile Asn Ser Leu Lys Pro Glu Asp Thr Ala Val
    210                 215                 220

Tyr Tyr Cys Ala Ser Ser Arg Ala Tyr Gly Ser Ser Arg Leu Arg Leu
225                 230                 235                 240

Ala Asp Thr Tyr Glu Tyr Trp Gly Gln Gly Thr Gln Val Thr Val Ser
                245                 250                 255

Ser

<210> 157
<211> 249
<212> PRT
<213> Artificial

<220>
<223> Artificial polypeptide comprising lama glama sequences

<400> 157

Glu Val Gln Leu Val Glu Ser Gly Gly Gly Leu Val Gln Pro Gly Gly
1               5                   10                  15

Ser Leu Lys Leu Ser Cys Thr Ala Ser Gly Phe Thr Phe Ser Thr Ser
            20                  25                  30

Trp Met His Trp Val Arg Gln Ala Pro Gly Lys Gly Leu Glu Trp Val
        35                  40                  45

Ser Ser Ile Pro Pro Val Gly His Phe Ala Asn Tyr Ala Pro Ser Val
    50                  55                  60

Lys Gly Arg Phe Thr Ile Ser Arg Asp Asn Ala Lys Asn Thr Leu Phe

65 70 75 80

Leu Gln Met Asn Ser Leu Lys Ser Glu Asp Thr Ala Val Tyr Tyr Cys
85 90 95

Ala Lys Asp Ser Ala Gly Arg Thr Lys Gly Gln Gly Thr Gln Val Thr
100 105 110

Val Ser Ser Gly Gly Gly Gly Ser Gly Gly Gly Ser Glu Val Gln Leu
115 120 125

Val Glu Ser Gly Gly Gly Leu Val Gln Thr Gly Asp Ser Leu Arg Leu
130 135 140

Ser Cys Glu Val Ser Gly Arg Thr Phe Ser Ser Tyr Ser Met Gly Trp
145 150 155 160

Phe Arg Gln Ala Gln Gly Lys Glu Arg Glu Phe Val Val Ala Ile Ser
165 170 175

Lys Gly Gly Tyr Lys Tyr Asp Ser Val Ser Leu Glu Gly Arg Phe Thr
180 185 190

Ile Ser Lys Asp Asn Ala Lys Asn Thr Val Tyr Leu Gln Ile Asn Ser
195 200 205

Leu Lys Pro Glu Asp Thr Ala Val Tyr Tyr Cys Ala Ser Ser Arg Ala
210 215 220

Tyr Gly Ser Ser Arg Leu Arg Leu Ala Asp Thr Tyr Glu Tyr Trp Gly
225 230 235 240

Gln Gly Thr Gln Val Thr Val Ser Ser
245

<210> 158
<211> 258
<212> PRT
<213> Artificial

<220>
<223> Artificial polypeptide comprising lama glama sequences

<400> 158

Glu Val Gln Leu Val Glu Ser Gly Gly Gly Leu Val Gln Ala Gly Gly
1 5 10 15

Ser Leu Arg Leu Ser Cys Ala Ala Ser Glu Arg Thr Phe Ser Asn Tyr
20 25 30

Ala Met Asp Trp Phe Arg Gln Ala Pro Gly Lys Glu Arg Glu Phe Val
35 40 45

Ala Ala Ile Thr Arg Ser Gly Gly Gly Thr Tyr Tyr Ala Asp Ser Val
50 55 60

Lys Gly Arg Phe Thr Ile Ser Arg Asp Asn Ala Lys Asn Thr Val Tyr
65 70 75 80

Leu Gln Met Asn Ser Leu Lys Pro Glu Asp Thr Ala Val Tyr Tyr Cys
85 90 95

Ala Ala Thr Arg Ser Ser Thr Ile Val Val Gly Val Gly Gly Met Glu
100 105 110

Tyr Trp Gly Lys Gly Thr Gln Val Thr Val Ser Ser Gly Gly Gly Gly
115 120 125

Ser Gly Gly Gly Ser Glu Val Gln Leu Val Glu Ser Gly Gly Gly Leu
130 135 140

Val Gln Thr Gly Asp Ser Leu Arg Leu Ser Cys Glu Val Ser Gly Arg
145 150 155 160

Thr Phe Ser Ser Tyr Ser Met Gly Trp Phe Arg Gln Ala Gln Gly Lys
165 170 175

Glu Arg Glu Phe Val Val Ala Ile Ser Lys Gly Gly Tyr Lys Tyr Asp
180 185 190

Ser Val Ser Leu Glu Gly Arg Phe Thr Ile Ser Lys Asp Asn Ala Lys
195 200 205

Asn Thr Val Tyr Leu Gln Ile Asn Ser Leu Lys Pro Glu Asp Thr Ala
210 215 220

Val Tyr Tyr Cys Ala Ser Ser Arg Ala Tyr Gly Ser Ser Arg Leu Arg
225 230 235 240

Leu Ala Asp Thr Tyr Glu Tyr Trp Gly Gln Gly Thr Gln Val Thr Val
245 250 255

Ser Ser

<210> 159

<211> 276
<212> PRT
<213> Artificial

<220>
<223> Artificial polypeptide comprising lama glama sequences

<400> 159

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Ser Ala Val Gly Asp Ile
            20                  25                  30

Thr Val Ala Trp Tyr Arg Gln Ala Pro Gly Ile Gln Arg Gln Leu Val
        35                  40                  45

Ala Thr Ile Thr Pro Ser Gly Tyr Thr Tyr Tyr Trp Asp Phe Val Lys
    50                  55                  60

Gly Arg Phe Thr Ile Ser Arg Asp Asn Ser Lys Asn Ile Val Tyr Leu
65                  70                  75                  80

Gln Met Asn Ser Leu Lys Pro Glu Asp Thr Ala Ala Tyr Tyr Cys Asn
                85                  90                  95

Thr Gln Phe Tyr Trp Gly Gln Gly Thr Gln Val Thr Val Ser Ser Gly
            100                 105                 110

Gly Gly Gly Ser Gly Gly Gly Gly Ser Gly Gly Gly Gly Ser Gly Gly
        115                 120                 125

Gly Gly Ser Gly Gly Gly Gly Ser Gly Gly Gly Gly Ser Gly Gly Gly
    130                 135                 140

Gly Ser Gly Gly Gly Gly Ser Glu Val Gln Leu Val Glu Ser Gly Gly
145                 150                 155                 160

Gly Leu Val Gln Thr Gly Asp Ser Leu Arg Leu Ser Cys Glu Val Ser
                165                 170                 175

Gly Arg Thr Phe Ser Ser Tyr Ser Met Gly Trp Phe Arg Gln Ala Gln
            180                 185                 190

Gly Lys Glu Arg Glu Phe Val Val Ala Ile Ser Lys Gly Gly Tyr Lys
        195                 200                 205

Tyr Asp Ser Val Ser Leu Glu Gly Arg Phe Thr Ile Ser Lys Asp Asn
    210                 215                 220
```

Ala Lys Asn Thr Val Tyr Leu Gln Ile Asn Ser Leu Lys Pro Glu Asp
225                 230                 235                 240

Thr Ala Val Tyr Tyr Cys Ala Ser Ser Arg Ala Tyr Gly Ser Ser Arg
                245                 250                 255

Leu Arg Leu Ala Asp Thr Tyr Glu Tyr Trp Gly Gln Gly Thr Gln Val
            260                 265                 270

Thr Val Ser Ser
        275

<210> 160
<211> 286
<212> PRT
<213> Artificial

<220>
<223> Artificial polypeptide comprising lama glama sequences

<400> 160

Glu Val Gln Leu Val Glu Ser Gly Gly Gly Leu Ala Gln Ala Gly Asp
1               5                   10                  15

Ser Leu Arg Leu Ser Cys Ala Ala Ser Gly Arg Ser Phe Ser His Tyr
            20                  25                  30

Asn Met Gly Trp Phe Arg Gln Ala Pro Gly Lys Glu Arg Glu Phe Val
        35                  40                  45

Ala Ser Ile Arg Gly Gly Gly Gly Ser Thr Thr Tyr Ala Asn Ser Val
    50                  55                  60

Lys Asp Arg Phe Thr Ile Ser Arg Glu Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Thr Ala Phe Tyr Arg Gly Pro Tyr Asp Tyr Asp Tyr Trp Gly
            100                 105                 110

Gln Gly Thr Gln Val Thr Val Ser Ser Gly Gly Gly Gly Ser Gly Gly
        115                 120                 125

Gly Gly Ser Gly Gly Gly Gly Ser Gly Gly Gly Gly Ser Gly Gly Gly
    130                 135                 140

Gly Ser Gly Gly Gly Gly Ser Gly Gly Gly Gly Ser Gly Gly Gly Gly
145 150 155 160

Ser Glu Val Gln Leu Val Glu Ser Gly Gly Gly Leu Val Gln Thr Gly
165 170 175

Asp Ser Leu Arg Leu Ser Cys Glu Val Ser Gly Arg Thr Phe Ser Ser
180 185 190

Tyr Ser Met Gly Trp Phe Arg Gln Ala Gln Gly Lys Glu Arg Glu Phe
195 200 205

Val Val Ala Ile Ser Lys Gly Gly Tyr Lys Tyr Asp Ser Val Ser Leu
210 215 220

Glu Gly Arg Phe Thr Ile Ser Lys Asp Asn Ala Lys Asn Thr Val Tyr
225 230 235 240

Leu Gln Ile Asn Ser Leu Lys Pro Glu Asp Thr Ala Val Tyr Tyr Cys
245 250 255

Ala Ser Ser Arg Ala Tyr Gly Ser Ser Arg Leu Arg Leu Ala Asp Thr
260 265 270

Tyr Glu Tyr Trp Gly Gln Gly Thr Gln Val Thr Val Ser Ser
275 280 285

<210> 161
<211> 278
<212> PRT
<213> Artificial

<220>
<223> Artificial polypeptide comprising lama glama sequences

<400> 161

Glu Val Gln Leu Val Glu Ser Gly Gly Gly Leu Val Gln Pro Gly Gly
1 5 10 15

Ser Leu Arg Leu Ser Cys Val Ala Ser Gly Ile Arg Phe Met Ser Met
20 25 30

Ala Trp Tyr Arg Gln Ala Pro Gly Lys His Arg Glu Leu Val Ala Arg
35 40 45

Ile Ser Ser Gly Gly Thr Thr Ala Tyr Val Asp Ser Val Lys Gly Arg
50 55 60

Phe Thr Ile Ser Arg Asp Asn Ser Lys Asn Thr Val Tyr Leu Gln Met
65 70 75 80

Asn Ser Leu Lys Ala Glu Asp Thr Ala Val Tyr Tyr Cys Asn Thr Phe
85 90 95

Ser Ser Arg Pro Asn Pro Trp Gly Ala Gly Thr Gln Val Thr Val Ser
100 105 110

Ser Gly Gly Gly Gly Ser Gly Gly Gly Gly Ser Gly Gly Gly Gly Ser
115 120 125

Gly Gly Gly Gly Ser Gly Gly Gly Gly Ser Gly Gly Gly Gly Ser Gly
130 135 140

Gly Gly Gly Ser Gly Gly Gly Gly Ser Glu Val Gln Leu Val Glu Ser
145 150 155 160

Gly Gly Gly Leu Val Gln Thr Gly Asp Ser Leu Arg Leu Ser Cys Glu
165 170 175

Val Ser Gly Arg Thr Phe Ser Ser Tyr Ser Met Gly Trp Phe Arg Gln
180 185 190

Ala Gln Gly Lys Glu Arg Glu Phe Val Val Ala Ile Ser Lys Gly Gly
195 200 205

Tyr Lys Tyr Asp Ser Val Ser Leu Glu Gly Arg Phe Thr Ile Ser Lys
210 215 220

Asp Asn Ala Lys Asn Thr Val Tyr Leu Gln Ile Asn Ser Leu Lys Pro
225 230 235 240

Glu Asp Thr Ala Val Tyr Tyr Cys Ala Ser Ser Arg Ala Tyr Gly Ser
245 250 255

Ser Arg Leu Arg Leu Ala Asp Thr Tyr Glu Tyr Trp Gly Gln Gly Thr
260 265 270

Gln Val Thr Val Ser Ser
275

<210> 162
<211> 281
<212> PRT
<213> Artificial

<220>
<223> Artificial polypeptide comprising lama glama sequences

<400> 162

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Asn Ile Phe Ser Asn Asn
            20                  25                  30

Ala Met Ala Trp Tyr Arg Gln Ala Pro Gly Lys Gln Arg Glu Leu Val
        35                  40                  45

Ala Arg Ile Ser Ser Gly Gly Gly Phe Thr Tyr Tyr Leu Asp Ser Val
    50                  55                  60

Lys Gly Arg Phe Thr Val Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Asn Ala Ala Tyr Arg Thr Tyr Asn Tyr Trp Gly Gln Gly Thr Gln Val
            100                 105                 110

Thr Val Ser Ser Gly Gly Gly Gly Ser Gly Gly Gly Gly Ser Gly Gly
        115                 120                 125

Gly Gly Ser Gly Gly Gly Gly Ser Gly Gly Gly Gly Ser Gly Gly Gly
    130                 135                 140

Gly Ser Gly Gly Gly Gly Ser Gly Gly Gly Gly Ser Glu Val Gln Leu
145                 150                 155                 160

Val Glu Ser Gly Gly Gly Leu Val Gln Thr Gly Asp Ser Leu Arg Leu
                165                 170                 175

Ser Cys Glu Val Ser Gly Arg Thr Phe Ser Ser Tyr Ser Met Gly Trp
            180                 185                 190

Phe Arg Gln Ala Gln Gly Lys Glu Arg Glu Phe Val Val Ala Ile Ser
        195                 200                 205

Lys Gly Gly Tyr Lys Tyr Asp Ser Val Ser Leu Glu Gly Arg Phe Thr
    210                 215                 220

Ile Ser Lys Asp Asn Ala Lys Asn Thr Val Tyr Leu Gln Ile Asn Ser
225                 230                 235                 240
```

Leu Lys Pro Glu Asp Thr Ala Val Tyr Tyr Cys Ala Ser Ser Arg Ala
245 250 255

Tyr Gly Ser Ser Arg Leu Arg Leu Ala Asp Thr Tyr Glu Tyr Trp Gly
260 265 270

Gln Gly Thr Gln Val Thr Val Ser Ser
275 280

<210> 163
<211> 289
<212> PRT
<213> Artificial

<220>
<223> Artificial polypeptide comprising lama glama sequences

<400> 163

Glu Val Gln Leu Val Glu Ser Gly Gly Gly Leu Val Gln Ala Gly Gly
1 5 10 15

Ser Leu Arg Leu Ser Cys Ala Ala Ser Gly Arg Thr Phe Ser Asn Tyr
20 25 30

Ala Met Gly Trp Phe Arg Gln Ala Pro Gly Lys Glu Arg Val Leu Val
35 40 45

Ala Asp Ile Ser Ser Ser Gly Ile Asn Thr Tyr Val Ala Asp Ala Val
50 55 60

Lys Gly Arg Phe Thr Ile Ser Arg Asp Asn Ala Lys Asn Thr Val Tyr
65 70 75 80

Leu Gln Met Asn Ser Leu Lys Pro Glu Asp Thr Ala Val Tyr Tyr Cys
85 90 95

Ala Ala Ser Ala Trp Trp Tyr Ser Gln Met Ala Arg Asp Asn Tyr Arg
100 105 110

Tyr Trp Gly Gln Gly Thr Gln Val Thr Val Ser Ser Gly Gly Gly Gly
115 120 125

Ser Gly Gly Gly Gly Ser Gly Gly Gly Gly Ser Gly Gly Gly Gly Ser
130 135 140

Gly Gly Gly Gly Ser Gly Gly Gly Gly Ser Gly Gly Gly Gly Ser Gly
145 150 155 160

Gly Gly Gly Ser Glu Val Gln Leu Val Glu Ser Gly Gly Gly Leu Val

165 170 175

Gln Thr Gly Asp Ser Leu Arg Leu Ser Cys Glu Val Ser Gly Arg Thr
180 185 190

Phe Ser Ser Tyr Ser Met Gly Trp Phe Arg Gln Ala Gln Gly Lys Glu
195 200 205

Arg Glu Phe Val Val Ala Ile Ser Lys Gly Gly Tyr Lys Tyr Asp Ser
210 215 220

Val Ser Leu Glu Gly Arg Phe Thr Ile Ser Lys Asp Asn Ala Lys Asn
225 230 235 240

Thr Val Tyr Leu Gln Ile Asn Ser Leu Lys Pro Glu Asp Thr Ala Val
245 250 255

Tyr Tyr Cys Ala Ser Ser Arg Ala Tyr Gly Ser Ser Arg Leu Arg Leu
260 265 270

Ala Asp Thr Tyr Glu Tyr Trp Gly Gln Gly Thr Gln Val Thr Val Ser
275 280 285

Ser

<210> 164
<211> 282
<212> PRT
<213> Artificial

<220>
<223> Artificial polypeptide comprising lama glama sequences

<400> 164

Glu Val Gln Leu Val Glu Ser Gly Gly Gly Leu Val Gln Pro Gly Gly
1 5 10 15

Ser Leu Arg Leu Ala Cys Ala Ala Ser Gly Phe Thr Leu Ser Ser Ser
20 25 30

Trp Met Tyr Trp Val Arg Gln Ala Pro Gly Lys Gly Leu Glu Trp Val
35 40 45

Ser Arg Ile Ser Pro Gly Gly Leu Phe Thr Tyr Tyr Val Asp Ser Val
50 55 60

Lys Gly Arg Phe Ser Val Ser Thr Asp Asn Ala Asn Asn Thr Leu Tyr
65 70 75 80

Leu Gln Met Asn Ser Leu Lys Pro Glu Asp Thr Ala Leu Tyr Ser Cys
85 90 95

Ala Lys Gly Gly Ala Pro Asn Tyr Thr Pro Arg Gly Arg Gly Thr Gln
100 105 110

Val Thr Val Ser Ser Gly Gly Gly Gly Ser Gly Gly Gly Gly Ser Gly
115 120 125

Gly Gly Gly Ser Gly Gly Gly Gly Ser Gly Gly Gly Gly Ser Gly Gly
130 135 140

Gly Gly Ser Gly Gly Gly Gly Ser Gly Gly Gly Gly Ser Glu Val Gln
145 150 155 160

Leu Val Glu Ser Gly Gly Gly Leu Val Gln Thr Gly Asp Ser Leu Arg
165 170 175

Leu Ser Cys Glu Val Ser Gly Arg Thr Phe Ser Ser Tyr Ser Met Gly
180 185 190

Trp Phe Arg Gln Ala Gln Gly Lys Glu Arg Glu Phe Val Val Ala Ile
195 200 205

Ser Lys Gly Gly Tyr Lys Tyr Asp Ser Val Ser Leu Glu Gly Arg Phe
210 215 220

Thr Ile Ser Lys Asp Asn Ala Lys Asn Thr Val Tyr Leu Gln Ile Asn
225 230 235 240

Ser Leu Lys Pro Glu Asp Thr Ala Val Tyr Tyr Cys Ala Ser Ser Arg
245 250 255

Ala Tyr Gly Ser Ser Arg Leu Arg Leu Ala Asp Thr Tyr Glu Tyr Trp
260 265 270

Gly Gln Gly Thr Gln Val Thr Val Ser Ser
275 280

<210> 165
<211> 290
<212> PRT
<213> Artificial

<220>
<223> Artificial polypeptide comprising lama glama sequences

<400> 165

Glu Val Gln Leu Val Glu Ser Gly Gly Gly Leu Val Gln Ala Gly Asp
1               5                   10                  15

Ser Leu Arg Leu Ser Cys Thr Ala Ser Gly Arg Thr Phe Asn Ser Tyr
            20                  25                  30

Ala Met Gly Trp Phe Arg Gln Ala Pro Gly Lys Glu Arg Glu Ser Val
        35                  40                  45

Ala His Ile Asn Arg Ser Gly Ser Ser Thr Tyr Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Leu Asn Ser Leu Lys Pro Glu Asp Thr Ala Val Tyr Tyr Cys
                85                  90                  95

Ala Ala Gly Arg Tyr Tyr Ser Ser Asp Gly Val Pro Ser Ala Ser Phe
            100                 105                 110

Asn Tyr Trp Gly Gln Gly Thr Gln Val Thr Val Ser Ser Gly Gly Gly
        115                 120                 125

Gly Ser Gly Gly Gly Gly Ser Gly Gly Gly Gly Ser Gly Gly Gly Gly
    130                 135                 140

Ser Gly Gly Gly Gly Ser Gly Gly Gly Gly Ser Gly Gly Gly Gly Ser
145                 150                 155                 160

Gly Gly Gly Gly Ser Glu Val Gln Leu Val Glu Ser Gly Gly Gly Leu
                165                 170                 175

Val Gln Thr Gly Asp Ser Leu Arg Leu Ser Cys Glu Val Ser Gly Arg
            180                 185                 190

Thr Phe Ser Ser Tyr Ser Met Gly Trp Phe Arg Gln Ala Gln Gly Lys
        195                 200                 205

Glu Arg Glu Phe Val Val Ala Ile Ser Lys Gly Gly Tyr Lys Tyr Asp
    210                 215                 220

Ser Val Ser Leu Glu Gly Arg Phe Thr Ile Ser Lys Asp Asn Ala Lys
225                 230                 235                 240

Asn Thr Val Tyr Leu Gln Ile Asn Ser Leu Lys Pro Glu Asp Thr Ala
                245                 250                 255

```
Val Tyr Tyr Cys Ala Ser Ser Arg Ala Tyr Gly Ser Ser Arg Leu Arg
            260                 265                 270

Leu Ala Asp Thr Tyr Glu Tyr Trp Gly Gln Gly Thr Gln Val Thr Val
        275                 280                 285

Ser Ser
    290
```

<210> 166
<211> 288
<212> PRT
<213> Artificial

<220>
<223> Artificial polypeptide comprising lama glama sequences

<400> 166

```
Glu Val Gln Leu Val Glu Ser Gly Gly Gly Leu Val Gln Ala Gly Gly
1               5                   10                  15

Ser Leu Arg Leu Ser Cys Thr Ala Ser Gly Ser Ala Phe Lys Ser Tyr
            20                  25                  30

Arg Met Gly Trp Phe Arg Arg Thr Pro Gly Lys Glu Asp Glu Phe Val
        35                  40                  45

Ala Ser Ile Ser Trp Thr Tyr Gly Ser Thr Phe Tyr Ala Asp Ser Val
    50                  55                  60

Lys Gly Arg Phe Thr Met Ser Arg Asp Lys Ala Lys Asn Ala Gly Tyr
65                  70                  75                  80

Leu Gln Met Asn Ser Leu Lys Pro Glu Asp Thr Ala Leu Tyr Tyr Cys
                85                  90                  95

Ala Ala Gly Ala Gln Ser Asp Arg Tyr Asn Ile Arg Ser Tyr Asp Tyr
            100                 105                 110

Trp Gly Gln Gly Thr Gln Val Thr Val Ser Ser Gly Gly Gly Gly Ser
        115                 120                 125

Gly Gly Gly Gly Ser Gly Gly Gly Gly Ser Gly Gly Gly Gly Ser Gly
    130                 135                 140

Gly Gly Gly Ser Gly Gly Gly Gly Ser Gly Gly Gly Gly Ser Gly Gly
145                 150                 155                 160
```

Gly Gly Ser Glu Val Gln Leu Val Glu Ser Gly Gly Gly Leu Val Gln
165 170 175

Thr Gly Asp Ser Leu Arg Leu Ser Cys Glu Val Ser Gly Arg Thr Phe
180 185 190

Ser Ser Tyr Ser Met Gly Trp Phe Arg Gln Ala Gln Gly Lys Glu Arg
195 200 205

Glu Phe Val Val Ala Ile Ser Lys Gly Gly Tyr Lys Tyr Asp Ser Val
210 215 220

Ser Leu Glu Gly Arg Phe Thr Ile Ser Lys Asp Asn Ala Lys Asn Thr
225 230 235 240

Val Tyr Leu Gln Ile Asn Ser Leu Lys Pro Glu Asp Thr Ala Val Tyr
245 250 255

Tyr Cys Ala Ser Ser Arg Ala Tyr Gly Ser Ser Arg Leu Arg Leu Ala
260 265 270

Asp Thr Tyr Glu Tyr Trp Gly Gln Gly Thr Gln Val Thr Val Ser Ser
275 280 285

<210> 167
<211> 280
<212> PRT
<213> Artificial

<220>
<223> Artificial polypeptide comprising lama glama sequences

<400> 167

Glu Val Gln Leu Val Glu Ser Gly Gly Gly Leu Val Gln Pro Gly Gly
1 5 10 15

Ser Leu Lys Leu Ser Cys Thr Ala Ser Gly Phe Thr Phe Ser Thr Ser
20 25 30

Trp Met His Trp Val Arg Gln Ala Pro Gly Lys Gly Leu Glu Trp Val
35 40 45

Ser Ser Ile Pro Pro Val Gly His Phe Ala Asn Tyr Ala Pro Ser Val
50 55 60

Lys Gly Arg Phe Thr Ile Ser Arg Asp Asn Ala Lys Asn Thr Leu Phe
65 70 75 80

Leu Gln Met Asn Ser Leu Lys Ser Glu Asp Thr Ala Val Tyr Tyr Cys
85 90 95

Ala Lys Asp Ser Ala Gly Arg Thr Lys Gly Gln Gly Thr Gln Val Thr
100 105 110

Val Ser Ser Gly Gly Gly Gly Ser Gly Gly Gly Gly Ser Gly Gly Gly
115 120 125

Gly Ser Gly Gly Gly Gly Ser Gly Gly Gly Gly Ser Gly Gly Gly Gly
130 135 140

Ser Gly Gly Gly Gly Ser Gly Gly Gly Gly Ser Glu Val Gln Leu Val
145 150 155 160

Glu Ser Gly Gly Gly Leu Val Gln Thr Gly Asp Ser Leu Arg Leu Ser
165 170 175

Cys Glu Val Ser Gly Arg Thr Phe Ser Ser Tyr Ser Met Gly Trp Phe
180 185 190

Arg Gln Ala Gln Gly Lys Glu Arg Glu Phe Val Val Ala Ile Ser Lys
195 200 205

Gly Gly Tyr Lys Tyr Asp Ser Val Ser Leu Glu Gly Arg Phe Thr Ile
210 215 220

Ser Lys Asp Asn Ala Lys Asn Thr Val Tyr Leu Gln Ile Asn Ser Leu
225 230 235 240

Lys Pro Glu Asp Thr Ala Val Tyr Tyr Cys Ala Ser Ser Arg Ala Tyr
245 250 255

Gly Ser Ser Arg Leu Arg Leu Ala Asp Thr Tyr Glu Tyr Trp Gly Gln
260 265 270

Gly Thr Gln Val Thr Val Ser Ser
275 280

<210> 168
<211> 289
<212> PRT
<213> Artificial

<220>
<223> Artificial polypeptide comprising lama glama sequences

<400> 168

Glu Val Gln Leu Val Glu Ser Gly Gly Gly Leu Val Gln Ala Gly Gly

1 5 10 15

Ser Leu Arg Leu Ser Cys Ala Ala Ser Glu Arg Thr Phe Ser Asn Tyr
20 25 30

Ala Met Asp Trp Phe Arg Gln Ala Pro Gly Lys Glu Arg Glu Phe Val
35 40 45

Ala Ala Ile Thr Arg Ser Gly Gly Gly Thr Tyr Tyr Ala Asp Ser Val
50 55 60

Lys Gly Arg Phe Thr Ile Ser Arg Asp Asn Ala Lys Asn Thr Val Tyr
65 70 75 80

Leu Gln Met Asn Ser Leu Lys Pro Glu Asp Thr Ala Val Tyr Tyr Cys
85 90 95

Ala Ala Thr Arg Ser Ser Thr Ile Val Val Gly Val Gly Gly Met Glu
100 105 110

Tyr Trp Gly Lys Gly Thr Gln Val Thr Val Ser Ser Gly Gly Gly Gly
115 120 125

Ser Gly Gly Gly Gly Ser Gly Gly Gly Gly Ser Gly Gly Gly Gly Ser
130 135 140

Gly Gly Gly Gly Ser Gly Gly Gly Gly Ser Gly Gly Gly Gly Ser Gly
145 150 155 160

Gly Gly Gly Ser Glu Val Gln Leu Val Glu Ser Gly Gly Gly Leu Val
165 170 175

Gln Thr Gly Asp Ser Leu Arg Leu Ser Cys Glu Val Ser Gly Arg Thr
180 185 190

Phe Ser Ser Tyr Ser Met Gly Trp Phe Arg Gln Ala Gln Gly Lys Glu
195 200 205

Arg Glu Phe Val Val Ala Ile Ser Lys Gly Gly Tyr Lys Tyr Asp Ser
210 215 220

Val Ser Leu Glu Gly Arg Phe Thr Ile Ser Lys Asp Asn Ala Lys Asn
225 230 235 240

Thr Val Tyr Leu Gln Ile Asn Ser Leu Lys Pro Glu Asp Thr Ala Val
245 250 255

```
Tyr Tyr Cys Ala Ser Ser Arg Ala Tyr Gly Ser Ser Arg Leu Arg Leu
            260                 265                 270


Ala Asp Thr Tyr Glu Tyr Trp Gly Gln Gly Thr Gln Val Thr Val Ser
        275                 280                 285


Ser
```

<210> 169
<211> 35
<212> PRT
<213> Artificial

<220>
<223> Synthetic linker

<400> 169

```
Gly Gly Gly Gly Ser Gly Gly Gly Gly Ser Gly Gly Gly Gly Ser Gly
1               5                   10                  15


Gly Gly Gly Ser Gly Gly Gly Gly Ser Gly Gly Gly Gly Ser Gly Gly
            20                  25                  30


Gly Gly Ser
        35
```

<210> 170
<211> 9
<212> PRT
<213> Artificial

<220>
<223> Synthetic linker

<400> 170

```
Gly Gly Gly Gly Ser Gly Gly Gly Ser
1               5
```

<210> 171
<211> 40
<212> PRT
<213> Artificial

<220>
<223> Synthetic linker

<400> 171

```
Gly Gly Gly Gly Ser Gly Gly Gly Gly Ser Gly Gly Gly Gly Ser Gly
1               5                   10                  15


Gly Gly Gly Ser Gly Gly Gly Gly Ser Gly Gly Gly Gly Ser Gly Gly
```

20 25 30

Gly Gly Ser Gly Gly Gly Gly Ser
35 40

<210> 172
<211> 9
<212> PRT
<213> Artificial

<220>
<223> Synthetic linker

<400> 172

Gly Gly Gly Gly Cys Gly Gly Gly Ser
1 5

<210> 173
<211> 25
<212> PRT
<213> Artificial

<220>
<223> Synthetic linker

<400> 173

Gly Gly Gly Gly Cys Gly Gly Gly Gly Ser Gly Gly Gly Gly Ser Gly
1 5 10 15

Gly Gly Gly Ser Gly Gly Gly Gly Ser
20 25

<210> 174
<211> 27
<212> PRT
<213> Artificial

<220>
<223> Synthetic linker

<400> 174

Gly Gly Gly Ser Gly Gly Gly Gly Ser Gly Gly Gly Gly Cys Gly Gly
1 5 10 15

Gly Gly Ser Gly Gly Gly Gly Ser Gly Gly Gly
20 25

<210> 175
<211> 35
<212> PRT
<213> Artificial

<220>

<223> Synthetic linker

<400> 175

Gly Gly Gly Gly Ser Gly Gly Gly Gly Ser Gly Gly Gly Gly Cys Gly
1               5                   10                  15

Gly Gly Gly Ser Gly Gly Gly Gly Ser Gly Gly Gly Gly Ser Gly Gly
            20                  25                  30

Gly Gly Ser
        35

<210> 176
<211> 35
<212> PRT
<213> Artificial

<220>
<223> Synthetic linker

<400> 176

Gly Gly Gly Gly Cys Gly Gly Gly Gly Ser Gly Gly Gly Gly Ser Gly
1               5                   10                  15

Gly Gly Gly Ser Gly Gly Gly Gly Ser Gly Gly Gly Gly Ser Gly Gly
            20                  25                  30

Gly Gly Ser
        35

<210> 177
<211> 115
<212> PRT
<213> Artificial

<220>
<223> Mutated lama glama sequence

<400> 177

Glu Val Gln Leu Val Glu Ser Gly Gly Gly Leu Val Gln Pro Gly Asn
1               5                   10                  15

Ser Leu Arg Leu Ser Cys Ala Ala Ser Gly Phe Thr Phe Ser Ser Phe
            20                  25                  30

Gly Met Ser Trp Val Arg Gln Ala Pro Gly Lys Gly Leu Glu Trp Val
        35                  40                  45

Ser Ser Ile Ser Gly Ser Gly Ser Asp Thr Leu Tyr Ala Asp Ser Val
    50                  55                  60

Lys Gly Arg Phe Thr Ile Ser Arg Asp Asn Ala Lys Thr Thr Leu Tyr
65 70 75 80

Leu Gln Met Asn Ser Leu Arg Pro Glu Asp Thr Ala Val Tyr Tyr Cys
85 90 95

Thr Ile Gly Gly Ser Leu Ser Arg Ser Ser Gln Gly Thr Leu Val Thr
100 105 110

Val Ser Ser
115

<210> 178
<211> 585
<212> PRT
<213> Homo sapiens

<400> 178

Asp Ala His Lys Ser Glu Val Ala His Arg Phe Lys Asp Leu Gly Glu
1 5 10 15

Glu Asn Phe Lys Ala Leu Val Leu Ile Ala Phe Ala Gln Tyr Leu Gln
20 25 30

Gln Cys Pro Phe Glu Asp His Val Lys Leu Val Asn Glu Val Thr Glu
35 40 45

Phe Ala Lys Thr Cys Val Ala Asp Glu Ser Ala Glu Asn Cys Asp Lys
50 55 60

Ser Leu His Thr Leu Phe Gly Asp Lys Leu Cys Thr Val Ala Thr Leu
65 70 75 80

Arg Glu Thr Tyr Gly Glu Met Ala Asp Cys Cys Ala Lys Gln Glu Pro
85 90 95

Glu Arg Asn Glu Cys Phe Leu Gln His Lys Asp Asp Asn Pro Asn Leu
100 105 110

Pro Arg Leu Val Arg Pro Glu Val Asp Val Met Cys Thr Ala Phe His
115 120 125

Asp Asn Glu Glu Thr Phe Leu Lys Lys Tyr Leu Tyr Glu Ile Ala Arg
130 135 140

Arg His Pro Tyr Phe Tyr Ala Pro Glu Leu Leu Phe Phe Ala Lys Arg
145 150 155 160

Tyr Lys Ala Ala Phe Thr Glu Cys Cys Gln Ala Ala Asp Lys Ala Ala
165 170 175

Cys Leu Leu Pro Lys Leu Asp Glu Leu Arg Asp Glu Gly Lys Ala Ser
180 185 190

Ser Ala Lys Gln Arg Leu Lys Cys Ala Ser Leu Gln Lys Phe Gly Glu
195 200 205

Arg Ala Phe Lys Ala Trp Ala Val Ala Arg Leu Ser Gln Arg Phe Pro
210 215 220

Lys Ala Glu Phe Ala Glu Val Ser Lys Leu Val Thr Asp Leu Thr Lys
225 230 235 240

Val His Thr Glu Cys Cys His Gly Asp Leu Leu Glu Cys Ala Asp Asp
245 250 255

Arg Ala Asp Leu Ala Lys Tyr Ile Cys Glu Asn Gln Asp Ser Ile Ser
260 265 270

Ser Lys Leu Lys Glu Cys Cys Glu Lys Pro Leu Leu Glu Lys Ser His
275 280 285

Cys Ile Ala Glu Val Glu Asn Asp Glu Met Pro Ala Asp Leu Pro Ser
290 295 300

Leu Ala Ala Asp Phe Val Glu Ser Lys Asp Val Cys Lys Asn Tyr Ala
305 310 315 320

Glu Ala Lys Asp Val Phe Leu Gly Met Phe Leu Tyr Glu Tyr Ala Arg
325 330 335

Arg His Pro Asp Tyr Ser Val Val Leu Leu Leu Arg Leu Ala Lys Thr
340 345 350

Tyr Glu Thr Thr Leu Glu Lys Cys Cys Ala Ala Ala Asp Pro His Glu
355 360 365

Cys Tyr Ala Lys Val Phe Asp Glu Phe Lys Pro Leu Val Glu Glu Pro
370 375 380

Gln Asn Leu Ile Lys Gln Asn Cys Glu Leu Phe Glu Gln Leu Gly Glu
385 390 395 400

Tyr Lys Phe Gln Asn Ala Leu Leu Val Arg Tyr Thr Lys Lys Val Pro
405 410 415

```
Gln Val Ser Thr Pro Thr Leu Val Glu Val Ser Arg Asn Leu Gly Lys
            420                 425                 430

Val Gly Ser Lys Cys Cys Lys His Pro Glu Ala Lys Arg Met Pro Cys
        435                 440                 445

Ala Glu Asp Tyr Leu Ser Val Val Leu Asn Gln Leu Cys Val Leu His
    450                 455                 460

Glu Lys Thr Pro Val Ser Asp Arg Val Thr Lys Cys Cys Thr Glu Ser
465                 470                 475                 480

Leu Val Asn Arg Arg Pro Cys Phe Ser Ala Leu Glu Val Asp Glu Thr
                485                 490                 495

Tyr Val Pro Lys Glu Phe Asn Ala Glu Thr Phe Thr Phe His Ala Asp
            500                 505                 510

Ile Cys Thr Leu Ser Glu Lys Glu Arg Gln Ile Lys Lys Gln Thr Ala
        515                 520                 525

Leu Val Glu Leu Val Lys His Lys Pro Lys Ala Thr Lys Glu Gln Leu
    530                 535                 540

Lys Ala Val Met Asp Asp Phe Ala Ala Phe Val Glu Lys Cys Cys Lys
545                 550                 555                 560

Ala Asp Asp Lys Glu Thr Cys Phe Ala Glu Glu Gly Lys Lys Leu Val
                565                 570                 575

Ala Ala Ser Gln Ala Ala Leu Gly Leu
            580                 585


<210>  179
<211>  109
<212>  PRT
<213>  Homo sapiens

<400>  179

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45
```

```
Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Trp Gly Gln Gly Thr Leu Val Thr Val Ser Ser
            100                 105
```

## Description

FIELD OF THE INVENTION

The invention relates to the field of human therapy, in particular cancer therapy and agents and compositions useful in such therapy.

BACKGROUND OF THE INVENTION

As described in e.g. US 2008/0014196 and WO2008101985, angiogenesis is implicated in the pathogenesis of a number of disorders, including solid tumors and metastasis as well as eye diseases. One of the most important pro-angiogenic factors is vascular endothelial growth factor (VEGF), also termed VEGF-A or vascular permeability factor (VPF). VEGF belongs to a gene family that includes placenta growth factor (PIGF), VEGF-B, VEGF-C, VEGF-D, VEGF-E and VEGF-F. Alternative splicing of mRNA of a single gene of human VEGF results in at least six isoforms (VEGF121, VEGF145, VEGF165, VEGF183, VEGF189, and VEGF206), VEGF165 being the most abundant isoform.
Two VEGF tyrosine kinase receptors (VEGFR) have been identified that interact with VEGF, i.e. VEGFR-1 (also known as Flt-1) and VEGFR-2 (also known as KDR or FIK-1). VEGFR-1 has the highest affinity for VEGF, while VEGFR- 2 has a somewhat lower affinity for VEGF. Ferrara (Endocrine Rev. 2004, 25: 581-611) provide a detailed description of VEGF, the interaction with its receptors and its function in normal and pathological processes can be found in Hoeben et al. Pharmacol. Rev. 2004, 56: 549-580.
VEGF has been reported to be a pivotal regulator of both normal and abnormal angiogenesis (Ferrara and Davis-Smyth, Endocrine Rev. 1997, 18: 4-25; Ferrara J. MoL Med. 1999, 77: 527-543). Compared to other growth factors that contribute to the processes of vascular formation, VEGF is unique in its high specificity for endothelial cells within the vascular system.
VEGF mRNA is overexpressed by the majority of human tumors. In the case of tumor growth, angiogenesis appears to be crucial for the transition from hyperplasia to neoplasia, and for providing nourishment for the growth and metastasis of the tumor (Folkman et al., 1989, Nature 339 -58), which allows the tumor cells to acquire a growth advantage compared to the normal cells. Therefore, anti-angiogenesis therapies have become an important treatment option for several types of tumors. These therapies have focused on blocking the VEGF pathway (Ferrara et al., Nat Rev Drug Discov. 2004 May; 3(5): 391-400.
VEGF is also involved in eye diseases. The concentration of VEGF in eye fluids is highly correlated with the presence of active proliferation of blood vessels in patients with diabetic and other ischemia-related retinopathies. Furthermore, recent studies have demonstrated the localization of VEGF in choroidal neovascular membranes in patients affected by age-related macular degeneration (AMD). Up-regulation of VEGF has also been observed in various inflammatory disorders. VEGF has been implicated in the pathogenesis of RA, an inflammatory disease in which angiogenesis plays a significant role.
The elucidation of VEGF and its role in angiogenesis and different processes has provided a potential new target of therapeutic intervention. The function of VEGF has been inhibited by small molecules that block or prevent activation of VEGF receptor tyrosine kinases (Schlaeppi and Wood, 1999, Cancer Metastasis Rev., 18: 473-481) and consequently interfere with the VEGF receptor signal transduction pathway. Cytotoxic conjugates containing bacterial or plant toxins can inhibit the stimulating effect of VEGF on tumor angiogenesis. VEGF-DT385 toxin conjugates (diphtheria toxin domains fused or chemically conjugated to VEGF165), for example, efficiently inhibit tumor growth in vivo. Tumor growth inhibition could also be achieved by delivering a Flk-1 mutant or soluble VEGF receptors by a retrovirus.
VEGF-neutralizing antibodies, such as A4.6.I and MV833, have been developed to block VEGF from binding to its receptors and have shown preclinical antitumor activity (Kim et al. Nature 1993, 362: 841-844; Folkman Nat. Med. 1995, 1: 27-31; Presta et al. Cancer Res. 1997, 57: 4593-4599; Kanai et al. Int. J. Cancer 1998, 77: 933-936; Ferrara and Alitalo Nat. Med. 1999, 5: 1359-1364; 320, 340. For a review of therapeutic anti-VEGF approaches trials, see Campochiaro and Hackett (Oncogene 2003, 22: 6537-6548).
Most clinical experience has been obtained with A4.6.1, also called bevacizumab (Avastin®; Genentech, San Francisco, CA).
WO2008101985 describes immunoglobulin single variable domains from camelides (VHHs or "Nanobodies®, as defined herein) that bind to VEGF, and their use in the treatment of conditions and diseases characterized by excessive and/or pathological angiogenesis or neovascularization.
It has been an object of the present invention to provide novel improved VEGF-binding molecules.
It has been a further object of the invention to provide methods for the prevention, treatment, alleviation and/or diagnosis of such diseases, disorders or conditions, involving the use and/or administration of such agents and compositions. In particular, it is has been an object of the invention to provide such pharmacologically active agents, compositions and/or methods that provide advantages compared to the agents, compositions and/or methods currently used and/or

known in the art. These advantages include improved therapeutic and/or pharmacological properties and/or other advantageous properties, e.g. for manufacturing purposes, especially as compared to conventional anti-VEGF antibodies as those described above, or fragments thereof.

More in particular, it has been an object of the invention to provide novel VEGF-binding molecules, and, specifically, VEGF-binding molecules that bind to mammalian VEGF and, especially, human VEGF, wherein such molecules or polypeptides are suitable for the therapeutic and diagnostic purposes as described herein. It has been a further object of the invention to provide immunoglobulin single variable domains that specifically bind to VEGF.

## BRIEF SUMMARY OF THE INVENTION

According to a first aspect, there are provided VEGF-binding molecules, preferably VEGF-binding immunoglobulin single variable domains like VHHs and domain antibodies.

In another aspect, the invention relates to nucleic acids encoding VEGF-binding molecules as well as host cells containing such nucleic acids.

The invention further relates to a product or composition containing or comprising at least one VEGF-binding molecule of the invention and optionally one or more further components of such compositions.

The invention further relates to methods for preparing or generating the VEGF-binding molecules, nucleic acids, host cells, products and compositions described herein.

The invention further relates to applications and uses of the VEGF-binding molecules, nucleic acids, host cells, products and compositions described herein, as well as to methods for the prevention and/or treatment for diseases associated with VEGF-mediated effects on angiogenesis.

These and other aspects, embodiments, advantages and applications of the invention will become clear from the further description hereinbelow.

## DEFINITIONS

Unless indicated or defined otherwise, all terms used have their usual meaning in the art, which will be clear to the skilled person. Reference is for example made to the standard handbooks, such as Sambrook et al, "Molecular Cloning: A Laboratory Manual" (2nd Ed.), Vols. 1-3, Cold Spring Harbor Laboratory Press (1989); Lewin, "Genes IV", Oxford University Press, New York, (1990), and Roitt et al., "Immunology" (2nd Ed.), Gower Medical Publishing, London, New York (1989), as well as to the general background art cited herein; Furthermore, unless indicated otherwise, all methods, steps, techniques and manipulations that are not specifically described in detail can be performed and have been performed in a manner known *per se,* as will be clear to the skilled person. Reference is for example again made to the standard handbooks, to the general background art referred to above and to the further references cited therein.

Unless indicated otherwise, the terms "*immunoglobulin*" and "*immunoglobulin sequence*" - whether used herein to refer to a *heavy chain antibody* or to a *conventional 4-chain antibody* - are used as general terms to include both the full-size antibody, the individual chains thereof, as well as all parts, domains or fragments thereof (including but not limited to antigen-binding domains or fragments such as VHH domains or VH/VL domains, respectively). In addition, the term "sequence" as used herein (for example in terms like "immunoglobulin sequence", "antibody sequence", "(single) variable domain sequence", "VHH sequence" or "protein sequence"), should generally be understood to include both the relevant amino acid sequence as well as nucleic acid sequences or nucleotide sequences encoding the same, unless the context requires a more limited interpretation.

The term "*domain*" (of a polypeptide or protein) as used herein refers to a folded protein structure which has the ability to retain its tertiary structure independently of the rest of the protein. Generally, domains are responsible for discrete functional properties of proteins, and in many cases may be added, removed or transferred to other proteins without loss of function of the remainder of the protein and/or of the domain.

The term "*immunoglobulin domain*" as used herein refers to a globular region of an antibody chain (such as e.g. a chain of a conventional 4-chain antibody or of a heavy chain antibody), or to a polypeptide that essentially consists of such a globular region. Immunoglobulin domains are characterized in that they retain the immunoglobulin fold characteristic of antibody molecules, which consists of a 2-layer sandwich of about 7 antiparallel beta-strands arranged in two beta-sheets, optionally stabilized by a conserved disulphide bond.

The term "*immunoglobulin variable domain*" as used herein means an immunoglobulin domain essentially consisting of four "framework regions" which are referred to in the art and hereinbelow as "framework region 1" or "FR1"; as "framework region 2" or"FR2"; as "framework region 3" or "FR3"; and as "framework region 4" or "FR4", respectively; which framework regions are interrupted by three "complementarity determining regions" or "CDRs", which are referred to in the art and hereinbelow as "complementarity determining region 1 "or "CDR1 "; as "complementarity determining region 2" or "CDR2"; and as "complementarity determining region 3" or "CDR3", respectively. Thus, the general structure or sequence of an immunoglobulin variable domain can be indicated as follows: FR1 - CDR1 - FR2 - CDR2 - FR3 -

CDR3 - FR4. It is the immunoglobulin variable domain(s) that confer specificity to an antibody for the antigen by carrying the antigen-binding site.

The term "*immunoglobulin single variable domain*" as used herein means an immunoglobulin variable domain which is capable of specifically binding to an epitope of the antigen without pairing with an additional variable immunoglobulin domain. One example of immunoglobulin single variable domains in the meaning of the present invention are *"domain antibodies*", such as the immunoglobulin single variable domains VH and VL (VH domains and VL domains). Another example of immunoglobulin single variable domains are *"VHH domains"* (or simply "VHHs") from camelids, as defined hereinafter.

In view of the above definition, the antigen-binding domain of a conventional 4-chain antibody (such as an IgG, IgM, IgA, IgD or IgE molecule; known in the art) or of a Fab fragment, a F(ab')2 fragment, an Fv fragment such as a disulphide linked Fv or a scFv fragment, or a diabody (all known in the art) derived from such conventional 4-chain antibody, would normally not be regarded as an immunoglobulin single variable domain, as, in these cases, binding to the respective epitope of an antigen would normally not occur by one (single) immunoglobulin domain but by a pair of (associating) immunoglobulin domains such as light and heavy chain variable domains, i.e. by a VH-VL pair of immunoglobulin domains, which jointly bind to an epitope of the respective antigen.

"*VHH domains*", also known as VHHs, $V_HH$ domains, VHH antibody fragments, and VHH antibodies, have originally been described as the antigen binding immunoglobulin (variable) domain of "heavy chain antibodies" (i.e. of "antibodies devoid of light chains"; Hamers-Casterman C, Atarhouch T, Muyldermans S, Robinson G, Hamers C, Songa EB, Bendahman N, Hamers R.: "Naturally occurring antibodies devoid of light chains"; Nature 363, 446-448 (1993)). The term "VHH domain" has been chosen in order to distinguish these variable domains from the heavy chain variable domains that are present in conventional 4-chain antibodies (which are referred to herein as "$V_H$ domains" or "VH domains") and from the light chain variable domains that are present in conventional 4-chain antibodies (which are referred to herein as "$V_L$ domains" or "VL domains"). VHH domains can specifically bind to an epitope without an additional antigen binding domain (as opposed to VH or VL domains in a conventional 4-chain antibody, in which case the epitope is recognized by a VL domain together with a VH domain). VHH domains are small, robust and efficient antigen recognition units formed by a single immunoglobulin domain.

In the context of the present invention, the terms VHH domain, VHH, $V_HH$ domain, VHH antibody fragment, VHH antibody, as well as "Nanobody®" and "Nanobody® domain" ("Nanobody" being a trademark of the company Ablynx N.V.; Ghent; Belgium) are used interchangeably and are representatives of immunoglobulin single variable domains (having the structure FR1-CDR1-FR2-CDR2-FR3-CDR3-FR4 and specifically binding to an epitope without requiring the presence of a second immunoglobulin variable domain), and which are distinguished from VH domains by the so-called "hallmark residues", as defined in e.g. WO2009/109635, Fig. 1.

The amino acid residues of a immunoglobulin single variable domain, e.g. a VHH, are numbered according to the general numbering for $V_H$ domains given by Kabat et al. ("Sequence of proteins of immunological interest", US Public Health Services, NIH Bethesda, MD, Publication No. 91), as applied to VHH domains from Camelids, as shown e.g. in Figure 2 of Riechmann and Muyldermans, J. Immunol. Methods 231, 25-38 (1999). According to this numbering,

- FR1 comprises the amino acid residues at positions 1-30,
- CDR1 comprises the amino acid residues at positions 31-35,
- FR2 comprises the amino acids at positions 36-49,
- CDR2 comprises the amino acid residues at positions 50-65,
- FR3 comprises the amino acid residues at positions 66-94,
- CDR3 comprises the amino acid residues at positions 95-102, and
- FR4 comprises the amino acid residues at positions 103-113.

However, it should be noted that - as is well known in the art for $V_H$ domains and for VHH domains - the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence.

Alternative methods for numbering the amino acid residues of $V_H$ domains, which methods can also be applied in an analogous manner to VHH domains, are known in the art. However, in the present description, claims and figures, the numbering according to Kabat and applied to VHH domains as described above will be followed, unless indicated otherwise.

The total number of amino acid residues in a VHH domain will usually be in the range of from 110 to 120, often between 112 and 115. It should however be noted that smaller and longer sequences may also be suitable for the purposes described herein.

Methods of obtaining VHHs that bind to a specific antigen or epitope have been described earlier, e.g. in WO2006/040153 and WO2006/122786. As also described therein in detail, VHH domains derived from camelids can be "humanized" (also termed "sequence-optimized" herein, "sequence-optimizing" may, in addition to humanization, encompass an additional modification of the sequence by one or more mutations that furnish the VHH with improved properties, such as the removal of potential post translational modification sites) by replacing one or more amino acid residues in the amino acid sequence of the original VHH sequence by one or more of the amino acid residues that occur at the corresponding position(s) in a VH domain from a conventional 4-chain antibody from a human being. A humanized VHH domain can contain one or more fully human framework region sequences, and, in an even more specific embodiment, can contain human framework region sequences derived from DP-29, DP-47, DP-51, or parts thereof, optionally combined with JH sequences, such as JH5.

*Domain antibodies,* also known as "Dab"s and "dAbs" (the terms "Domain Antibodies" and "dAbs" being used as trademarks by the GlaxoSmithKline group of companies) have been described in e.g. Ward, E.S., et al.: "Binding activities of a repertoire of single immunoglobulin variable domains secreted from Escherichia coli"; Nature 341: 544-546 (1989); Holt, L.J. et al.: "Domain antibodies: proteins for therapy"; TRENDS in Biotechnology 21(11): 484-490 (2003); and WO2003/002609.

Domain antibodies essentially correspond to the VH or VL domains of antibodies from non-camelid mammals, in particular human 4-chain antibodies. In order to bind an epitope as a single antigen binding domain, i.e. without being paired with a VL or VH domain, respectively, specific selection for such antigen binding properties is required, e.g. by using libraries of human single VH or VL domain sequences.

Domain antibodies have, like VHHs, a molecular weight of approximately 13 to approximately 16 kDa and, if derived from fully human sequences, do not require humanization for e.g. therapeutical use in humans. As in the case of VHH domains, they are well expressed also in prokaryotic expression systems, providing a significant reduction in overall manufacturing cost.

Furthermore, it will also be clear to the skilled person that it is possible to "graft" one or more of the CDR's mentioned above onto other "scaffolds", including but not limited to human scaffolds or non-immunoglobulin scaffolds. Suitable scaffolds and techniques for such CDR grafting are known in the art.

The terms *"epitope"* and "*antigenic determinant*", which can be used interchangeably, refer to the part of a macromolecule, such as a polypeptide, that is recognized by antigen-binding molecules, such as conventional antibodies or the polypeptides of the invention, and more particularly by the antigen-binding site of said molecules. Epitopes define the minimum binding site for an immunoglobulin, and thus represent the target of specificity of an immunoglobulin.

A polypeptide (such as an immunoglobulin, an antibody, an immunoglobulin single variable domain of the invention, or generally an antigen-binding molecule or a fragment thereof) that can "*bind to"* or *"specifically bind to",* that "*has affinity for*" and/or that *"has specificity for*" a certain epitope, antigen or protein (or for at least one part, fragment or epitope thereof) is said to be "*against*" or *"directed against*" said epitope, antigen or protein or is a "*binding*" molecule with respect to such epitope, antigen or protein. In this context, a VEGF-binding molecule may also be referred to as "VEGF-neutralizing.

Generally, the term "*specificity*" refers to the number of different types of antigens or epitopes to which a particular antigen-binding molecule or antigen-binding protein (such as an immunoglobulin single variable domain of the invention) molecule can bind. The specificity of an antigen-binding molecule can be determined based on its affinity and/or avidity. The affinity, represented by the equilibrium constant for the dissociation of an antigen with an antigen-binding protein (KD), is a measure for the binding strength between an epitope and an antigen-binding site on the antigen-binding protein: the lesser the value of the KD, the stronger the binding strength between an epitope and the antigen-binding molecule (alternatively, the affinity can also be expressed as the affinity constant (KA), which is 1/KD). As will be clear to the skilled person (for example on the basis of the further disclosure herein), affinity can be determined in a manner known per se, depending on the specific antigen of interest. Avidity is the measure of the strength of binding between an antigen-binding molecule (such as an immunoglobulin, an antibody, an immunoglobulin single variable domain or a polypeptides containing it and the pertinent antigen. Avidity is related to both the affinity between an epitope and its antigen binding site on the antigen-binding molecule and the number of pertinent binding sites present on the antigen-binding molecule.

The part of an antigen-binding molecule that recognizes the epitope is called a *paratope.*

Unless indicated otherwise, the term "VEGF-binding molecule" includes anti-VEGF antibodies, anti-VEGF antibody fragments, "anti-VEGF antibody-like molecules" and conjugates with any of these. Antibodies include, but are not limited to, monoclonal and chimerized monoclonal antibodies. The term "antibody" encompasses complete immunoglobulins, like monoclonal antibodies produced by recombinant expression in host cells, as well as VEGF-binding antibody fragments or "antibody-like molecules", including single-chain antibodies and linear antibodies, so-called "SMIPs" ("Small Modular Immunopharmaceuticals"), as e.g described in WO02/056910. Anti-VEGF antibody-like molecules include immunoglobulin single variable domains, as defined herein. Other examples for antibody-like molecules are immunoglobulin super family antibodies (IgSF), or CDR-grafted molecules.

"*VEGF-binding molecule"* refers to both monovalent VEGF-binding molecules (i.e. molecules that bind to one epitope of VEGF) as well as to bi- or multivalent binding molecules (i.e. binding molecules that bind to more than one epitope, e.g. "biparatopic" molecules as defined hereinbelow).

VEGF-binding molecules containing more than one VEGF-binding immunoglobulin single variable domain are also termed "formatted" VEGF-binding molecules, they may, in addition to the VEGF-binding immunoglobulin single variable domains, comprise linkers and/or moieties with effector functions, e.g. half-life-extending moieties like albumin-binding immunoglobulin single variable domains, and/or a fusion partner like serum albumin and/or an attached polymer like PEG.

The term "*biparatopic VEGF-binding molecule"* or "*biparatopic immunoglobulin single variable domain"* as used herein shall mean a VEGF-binding molecule comprising a first immunoglobulin single variable domain and a second immunoglobulin single variable domain as herein defined, wherein the two molecules bind to two different, i.e. non-overlapping epitopes of the VEGF antigen. The biparatopic polypeptides according to the invention are composed of immunoglobulin single variable domains which have different specificities with respect to the epitope. The part of an antigen-binding molecule (such as an antibody or an immunoglobulin single variable domain of the invention) that recognizes the epitope is called a *paratope.*

A formatted VEGF-binding molecule may, albeit less preferred, also comprise two identical VEGF-binding immunoglobulin single variable domains or two different immunoglobulin single variable domains that recognize the same or overlapping epitopes. In this case, the two immunoglobulin single variable domains may bind to the same or an overlapping epitope in each of the two monomers that form the VEGF dimer.

Typically, the VEGF-binding molecules of the invention will bind with a dissociation constant ($K_D$) of 10E-5 to 10E-14 moles/liter (M) or less, and preferably 10E-7 to 10E-14 moles/liter (M) or less, more preferably 10E-8 to 10E-14 moles/liter, and even more preferably 10E-11 to 10E-13 (as measured in a Biacore or in a KinExA assay), and/or with an association constant ($K_A$) of at least 10E7 ME-1 , preferably at least 10E8 ME-1, more preferably at least 10E9 ME-1, such as at least 10E11 ME-1. Any $K_D$ value greater than 10E-4 M is generally considered to indicate non-specific binding. Preferably, a polypeptide of the invention will bind to the desired antigen, i.e. VEGF, with a $K_D$ less than 500 nM, preferably less than 200 nM, more preferably less than 10 nM, such as less than 500 pM. Specific binding of an antigen-binding protein to an antigen or epitope can be determined in any suitable manner known per se, including, for example, the assays described herein, Scatchard analysis and/or competitive binding assays, such as radioimmunoassays (RIA), enzyme immunoassays (EIA) and sandwich competition assays, and the different variants thereof known per se in the art.

Amino acid residues will be indicated according to the standard three-letter or one-letter amino acid code, as generally known and agreed upon in the art. When comparing two amino acid sequences, the term "*amino acid difference*" refers to insertions, deletions or substitutions of the indicated number of amino acid residues at a position of the reference sequence, compared to a second sequence. In case of substitution(s), such substitution(s) will preferably be conservative amino acid substitution(s), which means that an amino acid residue is replaced with another amino acid residue of similar chemical structure and which has little or essentially no influence on the function, activity or other biological properties of the polypeptide. Such conservative amino acid substitutions are well known in the art, for example from WO98/49185, wherein conservative amino acid substitutions preferably are substitutions in which one amino acid within the following groups (i) - (v) is substituted by another amino acid residue within the same group: (i) small aliphatic, nonpolar or slightly polar residues: Ala, Ser, Thr, Pro and Gly; (ii) polar, negatively charged residues and their (uncharged) amides: Asp, Asn, Glu and Gln; (iii) polar, positively charged residues: His, Arg and Lys; (iv) large aliphatic, nonpolar residues: Met, Leu, Ile, Val and Cys; and (v) aromatic residues: Phe, Tyr and Trp. Particularly preferred conservative amino acid substitutions are as follows: Ala into Gly or into Ser; Arg into Lys; Asn into Gln or into His; Asp into Glu; Cys into Ser; Gln into Asn; Glu into Asp; Gly into Ala or into Pro; His into Asn or into Gln; Ile into Leu or into Val; Leu into Ile or into Val; Lys into Arg, into Gln or into Glu; Met into Leu, into Tyr or into Ile; Phe into Met, into Leu or into Tyr; Ser into Thr; Thr into Ser;Trp into Tyr; Tyr into Trp or into Phe; Val into Ile or into Leu.

A polypeptide or nucleic acid molecule is considered to be "*(in) essentially isolated (form)*" - for example, when compared to its native biological source and/or the reaction medium or cultivation medium from which it has been obtained - when it has been separated from at least one other component with which it is usually associated in said source or medium, such as another protein/polypeptide, another nucleic acid, another biological component or macromolecule or at least one contaminant, impurity or minor component. In particular, a polypeptide or nucleic acid molecule is considered "essentially isolated" when it has been purified at least 2-fold, in particular at least 10- fold, more in particular at least 100-fold, and up to 1000-fold or more. A polypeptide or nucleic acid molecule that is "in essentially isolated form" is preferably essentially homogeneous, as determined using a suitable technique, such as a suitable chromatographical technique, such as polyacrylamide gel electrophoresis.

*"Sequence identity*" between two VEGF-binding molecule sequences indicates the percentage of amino acids that are identical between the sequences. It may be calculated or determined as described in paragraph f) on pages 49 and 50 of WO08/020079. "Sequence similarity" indicates the percentage of amino acids that either are identical or that

represent conservative amino acid substitutions.

Alternative methods for numbering the amino acid residues of $V_H$ domains, which methods can also be applied in an analogous manner to VHH domains, are known in the art. However, in the present description, claims and figures, the numbering according to Kabat and applied to VHH domains as described above will be followed, unless indicated otherwise.

An "affinity-matured" VEGF-binding molecule, in particular a VHH or a domain antibody, has one or more alterations in one or more CDRs which result in an improved affinity for VEGF, as compared to the respective parent VEGF-binding molecule. Afffinity-matured VEGF-binding molecules of the invention may be prepared by methods known in the art, for example, as described by Marks et al., 1992, Biotechnology 10:779-783, or Barbas, et al., 1994, Proc. Nat. Acad. Sci, USA 91: 3809-3813.; Shier et al., 1995, Gene 169:147-155; Yelton et al., 1995, Immunol. 155: 1994-2004; Jackson et al., 1995, J. Immunol. 154(7):3310-9; and Hawkins et al., 1992, J. Mol. Biol. 226(3): 889 896; KS Johnson and RE Hawkins, "Affinity maturation of antibodies using phage display", Oxford University Press 1996.

For the present invention, an "amino acid sequences of SEQ ID NO: x": includes, if not otherwise stated, an amino acid sequence that is 100% identical with the sequence shown in the respective SEQ ID NO: x;

a) amino acid sequences that have at least 80% amino acid identity with the sequence shown in the respective SEQ ID NO: x;

b) amino acid sequences that have 3, 2, or 1 amino acid differences with the sequence shown in the respective SEQ ID NO: x.

The terms "cancer" and "cancerous" refer to or describe the physiological condition in mammals that is typically characterized by unregulated cell growth/proliferation. Examples of cancer to be treated with a VEGF-binding molecule of the invention, include but are not limited to carcinoma, lymphoma, blastoma, sarcoma, and leukemia. More particular examples of such cancers, as suggested for treatment with VEGF antagonists in US 2008/0014196, include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, gastric cancer, melanoma, and various types of head and neck cancer. Dysregulation of angiogenesis can lead to many disorders that can be treated by compositions and methods of the invention. These disorders include both non-neoplastic and neoplastic conditions. Neoplasties include but are not limited those described above.

Non-neoplastic disorders include, but are not limited to, as suggested for treatment with VEGF antagonists in US 2008/0014196, undesired or aberrant hypertrophy, arthritis, rheumatoid arthritis (RA), psoriasis, psoriatic plaques, sarcoidosis, atherosclerosis, atherosclerotic plaques, diabetic and other proliferative retinopathies including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, chronic inflammation, lung inflammation, acute lung injury/ ARDS, sepsis, primary pulmonary hypertension, malignant pulmonary effusions, cerebral edema (e.g., associated with acute stroke/ closed head injury/ trauma), synovial inflammation, pannus formation in RA, myositis ossificans, hypertropic bone formation, osteoarthritis (OA), refractory ascites, polycystic ovarian disease, endometriosis, 3rd spacing of fluid diseases (pancreatitis, compartment syndrome, burns, bowel disease), uterine fibroids, premature labor, chronic inflammation such as IBD (Crohn's disease and ulcerative colitis), renal allograft rejection, inflammatory bowel disease, nephrotic syndrome, undesired or aberrant tissue mass growth (non-cancer), hemophilic joints, hypertrophic scars, inhibition of hair growth, Osier-Weber syndrome, pyogenic granuloma retrolental fibroplasias, scleroderma, trachoma, vascular adhesions, synovitis, dermatitis, preeclampsia, ascites, pericardial effusion (such as that associated with pericarditis), and pleural effusion.

## DETAILED DESCRIPTION OF THE INVENTION

In a first aspect, the present invention relates to a VEGF-binding molecule comprising at least a variable domain with four framework regions and three complementarity determining regions CDR1, CDR2 and CDR3, respectively, wherein said CDR3 has the amino acid sequence Ser Arg Ala Tyr Xaa Ser Xaa Arg Leu Arg Leu Xaa Xaa Thr Tyr Xaa Tyr as shown in SEQ ID NO: 1, wherein

Xaa at position 5 is Gly or Ala;

Xaa at position 7 is Ser or Gly;
Xaa at position 12 is Gly, Ala or Pro;
Xaa at position 13 is Asp or Gly;
Xaa at position 16 is Asp or Glu; and

wherein said VEGF-binding molecule is capable of blocking the interaction of human recombinant VEGF165 with the human recombinant VEGFR-2 with an inhibition rate of ≥60%.

According to preferred embodiments, Xaa at position 5 is Gly, Xaa at position 7 is Ser, Xaa at position 12 is Ala, and Xaa at position 13 is Asp.

In particular, said CDR3 has a sequence selected from

SEQ ID NO: 2 SRAYGSSRLRLGDTYDY,
SEQ ID NO: 3 SRAYGSSRLRLADTYDY;
SEQ ID NO: 4 SRAYGSSRLRLADTYEY;
SEQ ID NO: 5 SRAYGSGRLRLADTYDY;
SEQ ID NO: 6 SRAYASSRLRLADTYDY;
SEQ ID NO: 7 SRAYGSSRLRLPDTYDY;
SEQ ID NO: 8 SRAYGSSRLRLPGTYDY.

According to certain embodiments, a VEGF-binding molecule comprises one or more immunoglobulin single variable domains each containing

a. a CDR3 with an amino acid sequence selected from a first group of sequences shown in SEQ ID NO: 2 to 8;

b. a CDR1 and a CDR2 with an amino acid sequences that is contained, as indicated in Table 3, in a sequence selected from a second group of amino acid sequences shown in SEQ ID NOs: 9 to 46, wherein said second sequence contains the respective CDR3 selected according to a).
According to preferred embodiments, the immunoglobulin single variable domains are VHHs.
According to specific embodiments, the VHHs have amino acid sequences selected from sequences shown in SEQ ID NOs: 9 - 46.
According to another specific embodiment, the VHHs have amino acid sequences selected from SEQ ID NOs: 15, SEQ ID NO: 18 and SEQ ID NO: 25.

The invention also relates to VEGF-binding molecules that have been obtained by affinity maturation and/or sequence optimization of an above-defined VHH, e.g. to a VHH that has been obtained by sequence optimization of a VHH having an amino acid sequence shown in SEQ ID NO: 18. Examples are VHHs having amino acid sequences selected from sequences shown in SEQ ID NOs: 47 - 57.

According to certain embodiments, a VEGF-binding molecule of the invention may be formatted, as herein defined, e.g. it may be biparatopic or comprise two identical immunoglobulin single variable domains. Such VEGF-binding molecules may comprise two or more VHHs, which are

a) identical VHHs that are capable of blocking the interaction between recombinant human VEGF and the recombinant human VEGFR-2 with an inhibition rate of ≥ 60% or

b) different VHHs that bind to non-overlapping epitopes of VEGF, wherein at least one VHH is capable of blocking the interaction between recombinant human VEGF and the recombinant human VEGFR-2 with an inhibition rate of ≥ 60% and wherein at least one VHH binds is capable of blocking said interaction with an inhibition rate of ≤ 60 %.

The percentage of blocking said interaction at an inhibition rate of ≥ 60% or ≤ 60 %, respectively, refers to an inhibition rate as determined by an Amplified Luminescent Proximity Homogeneous Assay (AlphaScreen®), a competition ELISA, a plasmon resonance (SPR) based assay (Biacore®) as used in the Examples.

In the following, the ability of VHHs according to a) is also termed "receptor-blocking", while the ability of VHHs according to b) is also termed "non-receptor-blocking".

Preferably, the receptor-blocking VHHs have an inhibition rate of ≥ 80%, more preferably ≥ 90%; the most preferred VHHs being complete receptor blockers, i.e. have an inhibition rate of 100 %.

A VEGF-binding may contain two or more identical VHHs a) selected from VHHs having amino acid sequences shown in SEQ ID NOs: 9 - 46 or VHHs that have been obtained by affinity maturation and/or sequence optimization of such VHH. The VHH may be selected from VHHs having the amino acid shown in SEQ ID NO: 18 or SEQ ID NO: 47 - 57.

According to preferred embodiments, a formatted VEGF-binding molecule comprises two VHHs each having the amino acid sequence shown in SEQ ID NO: 57.

In formatted VEGF-binding molecules comprising two different VHHs

a) said one or more VHHs with an inhibition rate of ≥ 60% are selected from

i. VHHs having an amino acid sequence selected from amino acid sequences shown in SEQ ID NOs: 9 - 46 or

ii. VHHs that have been obtained by affinity maturation and/or sequence optimization of such VHHs, and wherein

b) said one or more VHHs with an inhibition rate of ≥ 60 % are selected from

i. SEQ ID NOs: 58 - 124 or

ii. VHHs that have been obtained by affinity maturation and/or sequence optimization of such VHH.

According to preferred embodiments, two VHHs are contained in polypeptides with amino acid sequences shown in SEQ ID NOs: 128 - 168, separated by linker sequences as indicated in Table 15.

In a preferred VEGF-binding molecule VHH a) i. has an amino acid sequence shown in SEQ ID NO: 18 and VHH b) i. has an amino acid sequence shown in SEQ ID NO: 64.

In other preferred VEGF-binding molecules VHHs according to a) ii. are selected from VHHs having an amino acid sequence shown in SEQ ID NOs: 47 - 57 and VHHs according to b) ii. are selected from VHHs having an amino acid sequence shown in SEQ ID NOs: 125 - 127.

Particularly preferred is a biparatopic VEGF-binding molecule comprising two VHHs, one of them having the amino acid shown in SEQ ID NO: 57 and one of them having the amino acid shown in SEQ ID NO: 127.

The VEGF-binding molecules with improved properties in view of therapeutic application, e.g. enhanced affinity or decreased immunogenicity, may be obtained from individual VEGF-binding molecules of the invention by techniques known in the art, such as affinity maturation (for example, starting from synthetic, random or naturally occurring immunoglobulin sequences), CDR grafting, humanizing, combining fragments derived from different immunoglobulin sequences, PCR assembly using overlapping primers, and similar techniques for engineering immunoglobulin sequences well known to the skilled person; or any suitable combination of any of the foregoing, also termed "sequence optimization", as described herein. Reference is, for example, made to standard handbooks, as well as to the further description and Examples.

If appropriate, a VEGF-binding molecule of the invention with increased affinity may be obtained by affinity-maturation of another VEGF-binding molecule, the latter representing, with respect to the affinity-matured molecule, the "parent" VEGF-binding molecule.

Immunoglobulin single variable domains, e.g. VHHs and domain antibodies, according to the preferred embodiments of the invention, have a number of unique structural characteristics and functional properties which makes them highly advantageous for use in therapy as functional antigen-binding molecules. In particular, and without being limited thereto, VHH domains (which have been "designed" by nature to functionally bind to an antigen without pairing with a light chain variable domain) can function as single, relatively small, functional antigen-binding structural units.

Due to their unique properties, immunoglobulin single variable domains, as defined herein, like VHHs or VHs (or VLs) - either alone or as part of a larger polypeptide, e.g. a biparatopic molecule - offer a number of significant advantages:

- only a single domain is required to bind an antigen with high affinity and with high selectivity, so that there is no need to have two separate domains present, nor to assure that these two domains are present in the right spacial conformation and configuration (i.e. through the use of especially designed linkers, as with scFv's);
- immunoglobulin single variable domains can be expressed from a single nucleic acid molecule and do not require any post-translational modification (like glycosylation;
- immunoglobulin single variable domains can easily be engineered into multivalent and multispecific formats (as further discussed herein);
- immunoglobulin single variable domains have high specificity and affinity for their target, low inherent toxicity and can be administered via alternative routes than infusion or injection;

- immunoglobulin single variable domains are highly stable to heat, pH, proteases and other denaturing agents or conditions and, thus, may be prepared, stored or transported without the use of refrigeration equipments;

- immunoglobulin single variable domains are easy and relatively inexpensive to prepare, both on small scale and on a manufacturing scale. For example, immunoglobulin single variable domains can be produced using microbial fermentation (e.g. as further described below) and do not require the use of mammalian expression systems, as with for example conventional antibodies;

- immunoglobulin single variable domains are relatively small (approximately 15 kDa, or 10 times smaller than a conventional IgG) compared to conventional 4-chain antibodies and antigen-binding fragments thereof, and therefore show high(er) penetration into tissues (including but not limited to solid tumors and other dense tissues) and can be administered in higher doses than such conventional 4-chain antibodies and antigen-binding fragments thereof;

- VHHs have specific so-called "cavity-binding properties" (inter alia due to their extended CDR3 loop, compared to VH domains from 4-chain antibodies) and can therefore also access targets and epitopes not accessible to conventional 4-chain antibodies and antigen-binding fragments thereof;

- VHHs have the particular advantage that they are highly soluble and very stable and do not have a tendency to aggregate (as with the mouse-derived antigen-binding domains described by Ward et al., Nature 341: 544-546 (1989)).

The immunoglobulin single variable domains of the invention are not limited with respect to a specific biological source from which they have been obtained or to a specific method of preparation. For example, obtaining VHHs may include the following steps:

(1) isolating the VHH domain of a naturally occurring heavy chain antibody; or screening a library comprising heavy chain antibodies or VHHs and isolating VHHs therefrom;

(2) expressing a nucleic acid molecule encoding a VHH with the naturally occurring sequence;

(3) "humanizing" (as described herein) a VHH, optionally after affinity maturation, with a naturally occurring sequence or expressing a nucleic acid encoding such humanized VHH;

(4) "camelizing" (as described below) a immunoglobulin single variable heavy domain from a naturally occurring antibody from an animal species, in particular a species of mammal, such as from a human being, or expressing a nucleic acid molecule encoding such camelized domain;

(5) "camelizing" a VH, or expressing a nucleic acid molecule encoding such a camelized VH;

(6) using techniques for preparing synthetically or semi-synthetically proteins, polypeptides or other amino acid sequences;

(7) preparing a nucleic acid molecule encoding a VHH domain using techniques for nucleic acid synthesis, followed by expression of the nucleic acid thus obtained;

(8) subjecting heavy chain antibodies or VHHs to affinity maturation, to mutagenesis (e.g. random mutagenesis or site-directed mutagenesis) and/or any other technique(s) in order to increase the affinity and/or specificity of the VHH; and/or

(9) combinations or selections of the foregoing steps.

Suitable methods and techniques for performing the above-described steps are known in the art and will be clear to the skilled person. By way of example, methods of obtaining VHH domains binding to a specific antigen or epitope have been described in WO2006/040153 and WO2006/122786.

According to specific embodiments, the immunoglobulin single variable domains of the invention or present in the polypeptides of the invention are VHH domains with an amino acid sequence that essentially corresponds to the amino acid sequence of a naturally occurring VHH domain, but that has been "humanized" or "sequence-optimized" (optionally after affinity-maturation), i.e. by replacing one or more amino acid residues in the amino acid sequence of

said naturally occurring VHH sequence by one or more of the amino acid residues that occur at the corresponding position(s) in a variable heavy domain of a conventional 4-chain antibody from a human being. This can be performed using methods known in the art, which can by routinely used by the skilled person.

A humanized VHH domain may contain one or more fully human framework region sequences, and, in an even more specific embodiment, may contain human framework region sequences derived from the human germline Vh3 sequences DP-29, DP-47, DP-51, or parts thereof, or be highly homologous thereto, optionally combined with JH sequences, such as JH5. Thus, a humanization protocol may comprise the replacement of any of the VHH residues with the corresponding framework 1, 2 and 3 (FRI, FR2 and FR3) residues of germline VH genes such as DP 47, DP 29 and DP 51) either alone or in combination. Suitable framework regions (FR) of the immunoglobulin single variable domains of the invention can be selected from those as set out e.g. in WO 2006/004678 and specifically, include the so-called "KERE" and "GLEW" classes. Examples are immunoglobulin single variable domains having the amino acid sequence G-L-E-W at about positions 44 to 47, and their respective humanized counterparts. A humanized VHH domain may contain one or more fully human framework region sequences.

In VHHs of the invention that start with EVQ, the N-terminal E may be replaced by a D (which is often a result of sequence-optimization) or it may be missing (as for expression of the VHH in *E.coli).* For formatted VEGF-binding molecules, this usually applies only to the VHH that is situated N-terminally.

A preferred, but non-limiting humanizing substitution for VHH domains belonging to the 103 P,R,S-group and/or the GLEW-group (as defined below) is 108Q to 108L. Methods for humanizing immunoglobulin single variable domains are known in the art.

According to another embodiment, the immunoglobulin single variable domain is a domain antibody, as defined herein.

In yet another embodiment, the representatives of the class of VEGF-binding immunoglobulin single variable domains of the invention have amino acid sequences that correspond to the amino acid sequence of a naturally occurring VH domain that has been "camelized", i.e. by replacing one or more amino acid residues in the amino acid sequence of a naturally occurring variable heavy chain from a conventional 4-chain antibody by one or more amino acid residues that occur at the corresponding position(s) in a VHH domain of a heavy chain antibody. This can be performed in a manner known per se, which will be clear to the skilled person, and reference is additionally be made to WO 94/04678. Such camelization may preferentially occur at amino acid positions which are present at the VH-VL interface and at the so-called Camelidae Hallmark residues (see for example also WO 94/04678). A detailled description of such "humanization" and "camelization" techniques and preferred framework region sequences consistent therewith can additionally be taken from e.g. pp. 46 and pp. 98 of WO 2006/040153 and pp. 107 of WO 2006/122786.

The VEGF-binding molecules of the invention, e.g. immunoglobulin single variable domains, have specificity for VEGF in that they comprise one or more immunoglobulin single variable domains specifically binding to one or more epitopes within the VEGF molecule.

Specific binding of an VEGF-binding molecule to its antigen VEGF can be determined in any suitable manner known per se, including, for example, the assays described herein, Scatchard analysis and/or competitive binding assays, such as radioimmunoassays (RIA), enzyme immunoassays (EIA and ELISA) and sandwich competition assays, and the different variants thereof known per se in the art.

With regard to the antigen VEGF, a VEGF-binding molecule of the invention, e.g. an immunoglobulin single variable domain, is not limited with regard to the species. Thus, the immunoglobulin single variable domains of the invention preferably bind to human VEGF, if intended for therapeutic purposes in humans. However, immunoglobulin single variable domains that bind to VEGF from another mammalian species are also within the scope of the invention. An immunoglobulin single variable domain of the invention binding to one species form of VEGF may cross-react with VEGF, which has a different sequence than the human one, from one or more other species. For example, immunoglobulin single variable domains of the invention binding to human VEGF may exhibit cross reactivity with VEGF from one or more other species of primates and/or with VEGF from one or more species of animals that are used in animal models for diseases, for example monkey, mouse, rat, rabbit, pig, dog, and in particular in animal models for diseases and disorders associated with VEGF-mediated effects on angiogenesis (such as the species and animal models mentioned herein). Immunoglobulin single variable domains of the invention that show such cross-reactivity are advantageous in a research and/or drug development, since it allows the immunoglobulin single variable domains of the invention to be tested in acknowledged disease models such as monkeys, in particular Cynomolgus or Rhesus, or mice and rats.

Preferably, in view of cross-reactivity with one or more VEGF molecules from species other than human that is/are intended for use as an animal model during development of a therapeutic VEGF antagonist, a VEGF-binding molecule recognizes an epitope in a region of the VEGF of interest that has a high degree of identity with human VEGF.

An immunoglobulin single variable domain of the invention recognizes an epitope which is, totally or in part, located in a region of VEGF that is relevant for binding to its receptor, in particular to VEGFR-2, which has been shown to be the receptor whose activation is causally involved in the neovascularisation of tumors. According to preferred aspects, immunoglobulin single variable domains of the invention block VEGF receptor activation, in particular VEGFR-2 activation, at least partially, preferably substantially and most preferably totally.

As described above, the ability of a VEGF-binding molecule to block the interaction between VEGF and its receptors, in particular the VEGFR-2, can be determined by an Amplified Luminescent Proximity Homogeneous Assay (AlphaScreen®), a competition ELISA, or a plasmon resonance (SPR) based assay (Biacore®), as described in the Examples.

Preferably, an immunoglobulin single variable domain of the invention binds to VEGF with an affinity less than 500 nM, preferably less than 200 nM, more preferably less than 10 nM, such as less than 500 pM (as determined by Surface Plasmon Resonance analysis, as described in Example 5.7).

Preferably, the immunoglobulin single variable domains of the invention have $IC_{50}$ values, as measured in a competition ELISA assay as described in Example 5.1. in the range of $10^{-6}$ to $10^{-10}$ moles/litre or less, more preferably in the range of $10^{-8}$ to $10^{-10}$ moles/litre or less and even more preferably in the range of $10^{-9}$ to $10^{-10}$ moles/litre or less.

According to a non-limiting but preferred embodiment of the invention, VEGF-binding immunoglobulin single variable domains of the invention bind to VEGF with an dissociation constant ($K_D$) of $10^{-5}$ to $10^{-12}$ moles/liter (M) or less, and preferably $10^{-7}$ to $10^{-12}$ moles/liter (M) or less and more preferably $10^{-8}$ to $10^{-12}$ moles/liter (M), and/or with an association constant ($K_A$) of at least $10^7$ $M^{-1}$, preferably at least $10^8$ $M^{-1}$, more preferably at least $10^9$ $M^{-1}$, such as at least $10^{12}$ $M^{-1}$; and in particular with a $K_D$ less than 500 nM, preferably less than 200 nM, more preferably less than 10 nM, such as less than 500 pM. The $K_D$ and $K_A$ values of the immunoglobulin single variable domain of the invention against VEGF can be determined.

Biparatopic VEGF-binding molecules comprising two or more immunoglobulin single variable domains essentially consist of or comprise (i) a first immunoglobulin single variable domain specifically binding to a first epitope of VEGF and (ii) a second immunoglobulin single variable domain specifically binding to a second epitope of VEGF, wherein the first epitope of VEGF and the second epitope of VEGF are not identical epitopes. In other words, such polypeptide of the invention comprises or essentially consist of two or more immunoglobulin single variable domains that are directed against at least two non-overlapping epitopes present in VEGF, wherein said immunoglobulin single variable domains are linked to each other in such a way that they are capable of simultaneously binding VEGF. In this sense, the polypeptide of the invention can also be regarded as a "bivalent" or "multivalent" immunoglobulin construct, and especially as a "multivalent immunoglobulin single variable domain construct", in that the polypeptide contains at least two binding sites for VEGF. (Such constructs are also termed "formatted" VEGF binding molecules, e.g. "formatted" VHHs).

Such VEGF-binding molecule of the invention includes (at least) two anti-VEGF immunoglobulin single variable domains, wherein (the) two immunoglobulin single variable domains are preferably directed against non-overlapping epitopes within the VEGF molecule. Thus, these two immunoglobulin single variable domains will have a different antigen specificity and therefore different CDR sequences. For this reason, such polypeptides of the invention will herein also be named "biparatopic polypeptides", or "biparatopic domain antibody constructs" (if the immunoglobulin single variable domains consist or essentially consist of domain antibodies), or "biparatopic VHH constructs" (if the immunoglobulin single variable domains consist or essentially consist of VHHs), respectively, as the two immunoglobulin single variable domains will include two different paratopes.

If a polypeptide of the invention is a biparatopic molecule as defined herein, at least one of the immunoglobulin single variable domain components binds to an epitope such that the interaction between recombinant human VEGF and recombinant humen VEGFR-2 is blocked at an inhibition rate of ≥80%. As has been shown in experiments of the invention, certain formatted molecules contain two VHHs that both block the VEGFR2 receptor at an inhibition rate of ≥80%. Certain VHHs of the invention block the VEGFR-2 at an inhibition rate of 100%, i.e. they are complete blockers.

In both cases, additional sequences and moieties may be present within the VEGF-binding molecules of the invention, e.g. N-terminally, C-terminally, or located between the two immunoglobulin single variable domains, e.g. linker sequences and sequences providing for effector functions, as set out in more detail herein.

According to another, albeit less preferred embodiment, a VEGF-binding molecule of the invention may include more than two anti-VEGF immunoglobulin single variable domains, i.e. three, four or even more anti-VEGF VHHs. In this case, at least two of the anti-VEGF immunoglobulin single variable domains are directed against non-overlapping epitopes within the VEGF molecule, wherein any further immunoglobulin single variable domain may bind to any of the two non-overlapping epitopes and/or a further epitope present in the VEGF molecule.

According to the invention, the two or more immunoglobulin single variable domains can be, independently of each other, VHHs or domain antibodies, and/or any other sort of immunoglobulin single variable domains, such as VL domains, as defined herein, provided that these immunoglobulin single variable domains will bind the antigen, i.e. VEGF.

According to a preferred embodiment, the first and the second immunoglobulin single variable domains essentially consist of either VHH sequences or domain antibody sequences, as defined herein. According to a particularly preferred embodiment, the first and the second immunoglobulin single variable domains essentially consist of VHH sequences.

According to certain embodiments of the invention, the at least two immunoglobulin single variable domains present in a VEGF-binding molecule of the invention can be connected with each other directly (i.e. without use of a

linker) or via a linker. The linker is preferably a linker peptide and will be selected so as to allow binding of the at least two different immunoglobulin single variable domains to each of their at least two non-overlapping epitopes of VEGF, either within one and the same VEGF molecule, or within two different molecules.

Suitable linkers will *inter alia* depend on the epitopes and, specifically, the distance between the epitopes on VEGF to which the immunoglobulin single variable domains bind, and will be clear to the skilled person based on the disclosure herein, optionally after some limited degree of routine experimentation.

Also, when the two or more immunoglobulin single variable domains that bind to VEGF are VHHs or domain antibodies, they may be linked to each other via a third VHH or antibody, respectively (in such VEGF-binding molecules, the two or more immunoglobulin single variable domains may be linked directly to said third immunoglobulin single variable domain or via suitable linkers). Such a third VHH or domain antibody may for example be a VHH or domain antibody that provides for an increased half-life. For example, the latter VHH or domain antibody may be a domain antibody or VHH that is capable of binding to a (human) serum protein such as (human) serum albumin or (human) transferrin.

Alternatively, the two or more immunoglobulin single variable domains that bind to VEGF may be linked in series (either directly or via a suitable linker) and the third VHH or domain antibody (which may provide for increased half-life) may be connected directly or via a linker to one of these two or more aforementioned immunoglobulin sequences.

Suitable linkers are described herein in connection with specific polypeptides of the invention and may - for example and without limitation - comprise an amino acid sequence, which amino acid sequence preferably has a length of 9 or more amino acids, more preferably at least 17 amino acids, such as about 20 to 40 amino acids. However, the upper limit is not critical but is chosen for reasons of convenience regarding e.g. biopharmaceutical production of such polypeptides.

The linker sequence may be a naturally occurring sequence or a non-naturally occurring sequence. If used for therapeutic purposes, the linker is preferably non-immunogenic in the subject to which the VEGF-binding molecule of the invention is administered.

One useful group of linker sequences are linkers derived from the hinge region of heavy chain antibodies as described in WO96/34103 and WO94/04678.

Other examples are poly-alanine linker sequences such as Ala- Ala- Ala.

Further preferred examples of linker sequences are Gly/Ser linkers of different length such as $(gly_xser_y)_z$ linkers, including $(gly_4ser)_3$, $(gly_4ser)_4$, $(gly_4ser)$, $(gly_3ser)$, $gly_3$, and $(gly_3ser_2)_3$.

Some non-limiting examples of linkers are contained in VEGF-binding molecules of the invention shown in Table 15 (SEQ ID NOs 128 - 168), e.g. the linkers

GGGGSGGGGSGGGGSGGGGSGGGGSGGGGSGGGGS (35GS; SEQ ID NO: 169);

GGGGSGGGS (9GS; SEQ ID NO: 170);

GGGGSGGGGSGGGGSGGGGSGGGGSGGGGSGGGGSGGGGS(40GS; SEQ ID NO: 171).

If a formatted VEGF-binding molecule of the invention is modified by the attachment of a polymer, for example of a polyethylene glycol PEG (polyethylene glycol) moiety, the linker sequence preferably includes an amino acid residue, such as a cysteine or a lysine, allowing such modification, e.g. PEGylation, in the linker region.

Examples of linkers useful for for PEGylation are:

GGGGCGGGS ("GS9,C5", SEQ ID NO: 172);

GGGGCGGGGSGGGGSGGGGSGGGGS ("GS25,C5, SEQ ID NO:173)

GGGSGGGGSGGGGCGGGGSGGGGSGGG ("GS27,C14", SEQ ID NO:174),

GGGGSGGGGSGGGGCGGGGSGGGGSGGGGSGGGGS ("GS35,C15", SEQ ID NO:175), and

GGGGCGGGGSGGGGSGGGGSGGGGSGGGGSGGGGS ("GS35,C5", SEQ ID NO:176).

Furthermore, the linker may also be a poly(ethylene glycol) moiety, as shown in e.g. W02004/081026.

In another embodiment, the at least two VEGF-binding immunoglobulin single variable domains are linked to each other via another moiety (optionally via one or two linkers), such as another polypeptide which, in a preferred but non-limiting embodiment, may be a further immunoglobulin single variable domain as described above. Such moiety may either be essentially inactive or may have a biological effect such as improving the desired properties of the polypeptide or may confer one or more additional desired properties to the polypeptide. For example, and without limitation, the moiety may improve the half-life of the protein or polypeptide, and/or may reduce its immunogenicity or improve any other desired property.

According to a preferred embodiment, a VEGF-binding molecule of the invention includes, especially when intended for use or used as a therapeutic agent, a moiety which extends the half-life of the polypeptide of the invention in serum or other body fluids of a patient. The term "half-life" is defined as the time it takes for the serum concentration of the (modified) polypeptide to reduce by 50%, *in vivo,* for example due to degradation of the polypeptide and/or clearance and/or sequestration by natural mechanisms.

More specifically, such half-life extending moiety can be covalently linked to or fused to an immunoglobulin single variable domain and may be, without limitation, an Fc portion, an albumin moiety, a fragment of an albumin moiety, an albumin binding moiety, such as an anti-albumin immunoglobulin single variable domain, a transferrin binding moiety, such as an anti-transferrin immunoglobulin single variable domain, a polyoxyalkylene molecule, such as a polyethylene glycol molecule, an albumin binding peptide or a hydroxyethyl starch (HES) derivative.

In another embodiment, the VEGF-binding molecule of the invention comprises a moiety which binds to an antigen found in blood, such as serum albumin, serum immunoglobulins, thyroxine-binding protein, fibrinogen or transferrin, thereby conferring an increased half-life *in vivo* to the resulting polypeptide of the invention. According to a specifically preferred embodiment, such moiety is an albumin-binding immunoglobulin and, especially preferred, an albumin-binding immunoglobulin single variable domain such as an albumin-binding VHH domain.

If intended for use in humans, such albumin-binding immunoglobulin single variable domain preferably binds to human serum albumin and preferably is a humanized albumin-binding VHH domain.

Immunoglobulin single variable domains binding to human serum albumin are known in the art and are described in further detail in e.g. WO2006/122786. Specifically, useful albumin binding VHHs are ALB 1 and its humanized counterpart, ALB 8 (WO2009/095489). Other albumin binding VHH domains mentioned in the above patent publication may, however, be used as well.

A specifically useful albumin binding VHH domain is ALB8 which consists of or contains the amino acid sequence shown in SEQ ID NO: 177.

According to a further embodiment of the invention, the two immunoglobulin single variable domains, in preferably VHHs, may be fused to a serum albumin molecule, such as described e.g. in WO01/79271 and WO03/59934. As e.g. described in WO01/79271, the fusion protein may be obtained by conventional recombinant technology: a DNA molecule coding for serum albumin, or a fragment thereof, is joined to the DNA coding for the VEGF-binding molecule, the obtained construct is inserted into a plasmid suitable for expression in the selected host cell, e.g. a yeast cell like Pichia pastoris or a bacterial cell, and the host cell is then transfected with the fused nucleotide sequence and grown under suitable conditions. The sequence of a useful HSA is shown in SEQ ID NO: 178:

According to another embodiment, a half-life extending modification of a polypeptide of the invention (such modification also reducing immunogenicity of the polypeptide) comprises attachment of a suitable pharmacologically acceptable polymer, such as straight or branched chain poly(ethylene glycol) (PEG) or derivatives thereof (such as methoxypoly(ethylene glycol) or mPEG). Generally, any suitable form of PEGylation can be used, such as the PEGylation used in the art for antibodies and antibody fragments (including but not limited to domain antibodies and scFv's); reference is made, for example, to: Chapman, Nat. Biotechnol., 54, 531-545 (2002); Veronese and Harris, Adv. Drug Deliv. Rev. 54, 453-456 (2003); Harris and Chess, Nat. Rev. Drug. Discov. 2 (2003); and WO2004/060965.

Various reagents for PEGylation of polypeptides are also commercially available, for example from Nektar Therapeutics, USA, or NOF Corporation, Japan, such as the Sunbright® EA Series, SH Series, MA Series, CA Series, and ME Series, such as Sunbright® ME-100MA, Sunbright® ME-200MA, and Sunbright® ME-400MA.

Preferably, site-directed PEGylation is used, in particular via a cysteine-residue (see for example Yang et al., Protein Engineering 16, 761-770 (2003)). For example, for this purpose, PEG may be attached to a cysteine residue that naturally occurs in a polypeptide of the invention, a polypeptide of the invention may be modified so as to suitably introduce one or more cysteine residues for attachment of PEG, or an amino acid sequence comprising one or more cysteine residues for attachment of PEG may be fused to the N- and/or C-terminus of a polypeptide of the invention, all using techniques of protein engineering known per se to the skilled person.

Preferably, for the polypeptides of the invention, a PEG is used with a molecular weight of more than 5 kDa, such as more than 10 kDa and less than 200 kDa, such as less than 100 kDa; for example in the range of 20 kDa to 80 kDa.

With regard to PEGylation, its should be noted that generally, the invention also encompasses any biparatopic VEGF-binding molecule that has been PEGylated at one or more amino acid positions, preferably in such a way that said PEGylation either (1) increases the half-life in vivo; (2) reduces immunogenicity; (3) provides one or more further beneficial properties known per se for PEGylation; (4) does not essentially affect the affinity of the polypeptide for VEGF

(e.g. does not reduce said affinity by more than 50 %, and more preferably not by more than 10%, as determined by a suitable assay described in the art); and/or (4) does not affect any of the other desired properties of the VEGF-binding molecules of the invention. Suitable PEG-groups and methods for attaching them, either specifically or non-specifically, will be clear to the skilled person. Various reagents for PEGylation of polypeptides are also commercially available, for example from Nektar Therapeutics, USA, or NOF Corporation, Japan, such as the Sunbright® EA Series, SH Series, MA Series, CA Series, and ME Series, such as Sunbright® ME-100MA, Sunbright® ME-200MA, and Sunbright® ME-400MA.

According to an especially preferred embodiment of the invention, a PEGylated polypeptide of the invention includes one PEG moiety of linear PEG having a molecular weight of 40 kDa or 60 kDa, wherein the PEG moiety is attached to the polypeptide in a linker region and, specifically, at a Cys residue at position 5 of a GS9-linker peptide as shown in SEQ ID NO: 172, at position 14 of a GS27-linker peptide as shown in SEQ ID NO:174, or at position 15 of a GS35-linker peptide as shown in SEQ ID NO:175, or at position 5 of a 35GS-linker peptide as shown in SEQ ID NO:176.

A VEGF-binding molecule of the invention may be PEGylated with one of the PEG reagents as mentioned above, such as "Sunbright® ME-400MA", as shown in the following chemical formula:

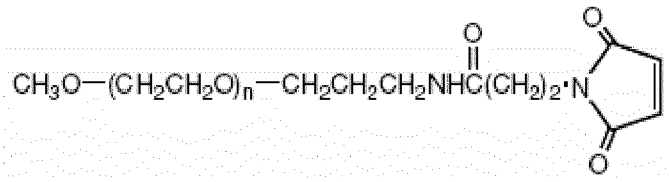

In another aspect, the invention relates to nucleic acid molecules that encode VEGF-binding molecules of the invention. Such nucleic acid molecules will also be referred to herein as "nucleic acids of the invention" and may also be in the form of a genetic construct, as defined herein. A nucleic acid of the invention may be genomic DNA, cDNA or synthetic DNA (such as DNA with a codon usage that has been specifically adapted for expression in the intended host cell or host organism). According to one embodiment of the invention, the nucleic acid of the invention is in essentially isolated form, as defined hereabove.

The nucleic acid of the invention may also be in the form of, may be present in and/or may be part of a vector, such as for example a plasmid, cosmid or YAC. The vector may especially be an expression vector, i.e. a vector that can provide for expression of the VEGF-binding molecule *in vitro* and/or *in vivo* (i.e. in a suitable host cell, host organism and/or expression system). Such expression vector generally comprises at least one nucleic acid of the invention that is operably linked to one or more suitable regulatory elements, such as promoter(s), enhancer(s), terminator(s), and the like. Such elements and their selection in view of expression of a specific sequence in a specific host are common knowledge of the skilled person. Specific examples of regulatory elements and other elements useful or necessary for expressing VEGF-binding molecules of the invention, such as promoters, enhancers, terminators, integration factors, selection markers, leader sequences, reporter genes, and the like, are disclosed e.g. on pp. 131 to 133 of W02006/040153.

The nucleic acids of the invention may be prepared or obtained in a manner known *per se* (e.g. by automated DNA synthesis and/or recombinant DNA technology), based on the information on the amino acid sequences for the polypeptides of the invention given herein, and/or can be isolated from a suitable natural source.

In another aspect, the invention relates to host cells that express or that are capable of expressing one or more a VEGF-binding molecule of the invention; and/or that contain a nucleic acid of the invention. According to a particularly preferred embodiment, said host cells are bacterial cells; other useful cells are yeast cells, fungal cells or mammalian cells.

Suitable bacterial cells include cells from gram-negative bacterial strains such as strains of *Escherichia coli, Proteus,* and *Pseudomonas,* and gram-positive bacterial strains such as strains of *Bacillus, Streptomyces, Staphylococcus,* and *Lactococcus.* Suitable fungal cell include cells from species of *Trichoderma, Neurospora,* and *Aspergillus.* Suitable yeast cells include cells from species of *Saccharomyces* (for example *Saccharomyces cerevisiae), Schizosaccharomyces* (for example *Schizosaccharomyces pombe), Pichia* (for example *Pichia pastoris* and *Pichia methanolica*), and *Hansenula.*

Suitable mammalian cells include for example CHO cells, BHK cells, HeLa cells, COS cells, and the like. However, amphibian cells, insect cells, plant cells, and any other cells used in the art for the expression of heterologous proteins can be used as well.

The invention further provides methods of manufacturing a VEGF-binding molecule of the invention, such methods generally comprising the steps of:

- culturing host cells comprising a nucleic acid capable of encoding a VEGF-binding molecule under conditions that allow expression of the VEGF-binding molecule of the invention; and
- recovering or isolating the polypeptide expressed by the host cells from the culture; and

- optionally further purifying and/or modifying and/or formulating the VEGF-binding molecule of the invention.

For production on an industrial scale, preferred host organisms include strains of *E. coli, Pichia pastoris,* and *S. cerevisiae* that are suitable for large scale expression, production and fermentation, and in particular for large scale pharmaceutical expression, production and fermentation.

The choice of the specific expression system depends in part on the requirement for certain post-translational modifications, more specifically glycosylation. The production of a VEGF-binding molecule of the invention for which glycosylation is desired or required would necessitate the use of mammalian expression hosts that have the ability to glycosylate the expressed protein. In this respect, it will be clear to the skilled person that the glycosylation pattern obtained (i.e. the kind, number and position of residues attached) will depend on the cell or cell line that is used for the expression.

VEGF-binding molecules of the invention may be produced in a cell as set out above either intracellullarly (e.g. in the cytosol, in the periplasma or in inclusion bodies) and then isolated from the host cells and optionally further purified; or they can be produced extracellularly (e.g. in the medium in which the host cells are cultured) and then isolated from the culture medium and optionally further purified.

Methods and reagents used for the recombinant production of polypeptides, such as specific suitable expression vectors, transformation or transfection methods, selection markers, methods of induction of protein expression, culture conditions, and the like, are known in the art. Similarly, protein isolation and purification techniques useful in a method of manufacture of a polypeptide of the invention are well known to the skilled person.

In a further aspect, the invention relates to a peptide having an amino acid sequence of a CDR3 contained in an anti-VEGF-VHH having an amino acid sequence selected from sequences shown in SEQ ID NOs: 9 to 57 or SEQ ID NOs: 58 - 127, respectively, and a nucleic acid molecule encoding same.

These peptides correspond to CDR3s derived from the VHHs of the invention. They, in particular the nucleic acid molecules encoding them, are useful for CDR grafting in order to replace a CDR3 in an immunoglobulin chain, or for insertion into a non-immunoglobulin scaffold, e.g. a protease inhibitor, DNA-binding protein, cytochrome b562, a helix-bundle protein, a disulfide-bridged peptide, a lipocalin or an anticalin, thus conferring target-binding properties to such scaffold. The method of CDR-grafting is well known in the art and has been widely used, e.g. for humanizing antibodies (which usually comprises grafting the CDRs from a rodent antibody onto the Fv frameworks of a human antibody).

In order to obtain an immunoglobulin or a non-immunoglobulin scaffold containing a CDR3 of the invention, the DNA encoding such molecule may be obtained according to standard methods of molecular biology, e.g. by gene synthesis, by oligonucleotide annealing or by means of overlapping PCR fragments, as e.g. described by Daugherty et al., 1991, Nucleic Acids Research, Vol. 19, 9, 2471 - 2476. A method for inserting a VHH CDR3 into a non-immunoglobulin scaffold has been described by Nicaise et al., 2004, Protein Science, 13, 1882 - 1891.

The invention further relates to a product or composition containing or comprising at least one VEGF-binding molecule of the invention and optionally one or more further components of such compositions known *per se*, i.e. depending on the intended use of the composition.

For pharmaceutical use, a VEGF-binding molecule of the invention may be formulated as a pharmaceutical preparation or composition comprising at least one VEGF-binding molecule of the invention and at least one pharmaceutically acceptable carrier, diluent or excipient and/or adjuvant, and optionally one or more further pharmaceutically active polypeptides and/or compounds. By means of non-limiting examples, such a formulation may be in a form suitable for oral administration, for parenteral administration (such as by intravenous, intramuscular or subcutaneous injection or intravenous infusion), for topical administration, for administration by inhalation, by a skin patch, by an implant, by a suppository, etc. Such suitable administration forms - which may be solid, semi-solid or liquid, depending on the manner of administration - as well as methods and carriers for use in the preparation thereof, will be clear to the skilled person, and are further described herein.

Thus, in a further aspect, the invention relates to a pharmaceutical composition that contains at least one VEGF-binding molecule, in particular one immunoglobulin single variable domain, of the invention and at least one suitable carrier, diluent or excipient (i.e. suitable for pharmaceutical use), and optionally one or more further active substances.

The VEGF-binding molecules of the invention may be formulated and administered in any suitable manner known per se: Reference, in particular for the immunoglobulin single variable domains, is for example made to WO04/041862, WO04/041863, WO04/041865, WO04/041867 and WO08/020079, as well as to the standard handbooks, such as Remington's Pharmaceutical Sciences, 18th Ed., Mack Publishing Company, USA (1990), Remington, the Science and Practice of Pharmacy, 21th Edition, Lippincott Williams and Wilkins (2005); or the Handbook of Therapeutic Antibodies (S. Dubel, Ed.), Wiley, Weinheim, 2007 (see for example pages 252-255).

For example, an immunoglobulin single variable domain of the invention may be formulated and administered in any manner known per se for conventional antibodies and antibody fragments (including ScFv's and diabodies) and other pharmaceutically active proteins. Such formulations and methods for preparing the same will be clear to the skilled

person, and for example include preparations suitable for parenteral administration (for example intravenous, intraperitoneal, subcutaneous, intramuscular, intraluminal, intra-arterial or intrathecal administration) or for topical (i.e. transdermal or intradermal) administration.

Preparations for parenteral administration may for example be sterile solutions, suspensions, dispersions or emulsions that are suitable for infusion or injection. Suitable carriers or diluents for such preparations for example include, without limitation, sterile water and pharmaceutically acceptable aqueous buffers and solutions such as physiological phosphate-buffered saline, Ringer's solutions, dextrose solution, and Hank's solution; water oils; glycerol; ethanol; glycols such as propylene glycol or as well as mineral oils, animal oils and vegetable oils, for example peanut oil, soybean oil, as well as suitable mixtures thereof. Usually, aqueous solutions or suspensions will be preferred.

Thus, the VEGF-binding molecule of the invention may be systemically administered, e.g., orally, in combination with a pharmaceutically acceptable vehicle such as an inert diluent or an assimilable edible carrier. For oral therapeutic administration, the VEGF-binding molecule of the invention may be combined with one or more excipients and used in the form of ingestible tablets, buccal tablets, troches, capsules, elixirs, suspensions, syrups, wafers, and the like. Such compositions and preparations should contain at least 0.1 % of the VEGF-binding molecule of the invention. Their percentage in the compositions and preparations may, of course, be varied and may conveniently be between about 2 to about 60% of the weight of a given unit dosage form. The amount of the VEGF-binding molecule of the invention in such therapeutically useful compositions is such that an effective dosage level will be obtained.

The tablets, pills, capsules, and the like may also contain binders, excipients, disintegrating agents, lubricants and sweetening or flavouring agents, for example those mentioned on pages 143-144 of WO08/020079. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the VEGF-binding molecules of the invention, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the VEGF-binding molecules of the invention may be incorporated into sustained-release preparations and devices.

Preparations and formulations for oral administration may also be provided with an enteric coating that will allow the constructs of the invention to resist the gastric environment and pass into the intestines. More generally, preparations and formulations for oral administration may be suitably formulated for delivery into any desired part of the gastrointestinal tract. In addition, suitable suppositories may be used for delivery into the gastrointestinal tract.

The VEGF-binding molecules of the invention may also be administered intravenously or intraperitoneally by infusion or injection, as further described on pages 144 and 145 of WO08/020079.

For topical administration of the VEGF-binding molecules of the invention, it will generally be desirable to administer them to the skin as compositions or formulations, in combination with a dermatologically acceptable carrier, which may be a solid or a liquid, as further described on page 145 of WO08/020079.

Generally, the concentration of the VEGF-binding molecules of the invention in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The amount of the VEGF-binding molecules of the invention required for use in treatment will vary not only with the particular VEGF-binding molecule selected, but also with the route of administration, the nature of the condition being treated and the age and condition of the patient and will be ultimately at the discretion of the attendant physician or clinician. Also, the dosage of the VEGF-binding molecules of the invention varies depending on the target cell, tumor, tissue, graft, or organ.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

An administration regimen may include long-term, daily treatment. By "long-term" is meant at least two weeks and preferably, several weeks, months, or years of duration. Necessary modifications in this dosage range may be determined by one of ordinary skill in the art using only routine experimentation given the teachings herein. See Remington's Pharmaceutical Sciences (Martin, E.W., ed. 4), Mack Publishing Co., Easton, PA. The dosage can also be adjusted by the individual physician in the event of any complication.

According to a further embodiment, the invention relates to the use of VEGF-binding molecules, e.g. immunoglobulin single variable domains, for therapeutic purposes, such as

- for the prevention, treatment and/or alleviation of a disorder, disease or condition, especially in a human being, that is associated with VEGF-mediated effects on angiogenesis or that can be prevented, treated or alleviated by modulating the Notch signaling pathway with a VEGF-binding molecule,

- in a method of treatment of a patient in need of such therapy, such method comprising administering, to a subject in need thereof, a pharmaceutically active amount of at least one VEGF-binding molecule of the invention, e.g. an immunoglobulin single variable domain, or a pharmaceutical composition containing same;
- for the preparation of a medicament for the prevention, treatment or alleviation of disorders, diseases or conditions associated with VEGF-mediated effects on angiogenesis;
- as an active ingredient in a pharmaceutical composition or medicament used for the above purposes.

According to a specific aspect, said disorder disorder, disease or condition is a cancer or cancerous disease, as defined herein.

According to another aspect, the disease is an eye disease associated with VEGF-mediated effects on angiogenesis or which can be treated or alleviated by modulating the Notch signaling pathway with a VEGF-binding molecule.

Depending on the cancerous disease to be treated, a VEGF-binding molecule of the invention may be used on its own or in combination with one or more additional therapeutic agents, in particular selected from chemotherapeutic agents like DNA damaging agents or therapeutically active compounds that inhibit angiogenesis, signal transduction pathways or mitotic checkpoints in cancer cells.

The additional therapeutic agent may be administered simultaneously with, optionally as a component of the same pharmaceutical preparation, or before or after administration of the VEGF-binding molecule.

In certain embodiments, the additional therapeutic agent may be, without limitation (and in the case of the receptors, including the respective ligands), one or more inhibitors selected from the group of inhibitors of EGFR, VEGFR, HER2-neu, Her3, AuroraA, AuroraB, PLK and PI3 kinase, FGFR, PDGFR, Raf, KSP, PDK1, PTK2, IGF-R or IR.

Further examples of additional therapeutic agents are inhibitors of CDK, Akt, src/bcr abl, cKit, cMet/HGF, c-Myc, Flt3, HSP90, hedgehog antagonists, inhibitors of JAK/STAT, Mek, mTor, NFkappaB, the proteasome, Rho, an inhibitor of wnt signaling or an inhibitor of the ubiquitination pathway or another inhibitor of the Notch signaling pathway.

Examples for Aurora inhibitors are, without limitation, PHA-739358, AZD-1152, AT 9283, CYC-116, R-763, VX-680, VX-667, MLN-8045, PF-3814735.

An example for a PLK inhibitor is GSK-461364.

Examples for raf inhibitors are BAY-73-4506 (also a VEGFR inhibitor), PLX 4032, RAF-265 (also in addition a VEGFR inhibitor), sorafenib (also in addition a VEGFR inhibitor), and XL 281.

Examples for KSP inhibitors are ispinesib, ARRY-520, AZD-4877, CK-1122697, GSK 246053A, GSK-923295, MK-0731, and SB-743921.

Examples for a src and/or bcr-abl inhibitors are dasatinib, AZD-0530, bosutinib, XL 228 (also an IGF-1R inhibitor), nilotinib (also a PDGFR and cKit inhibitor), imatinib (also a cKit inhibitor), and NS-187.

An example for a PDK1 inhibitor is BX-517.

An example for a Rho inhibitor is BA-210.

Examples for PI3 kinase inhibitors are PX-866, BEZ-235 (also an mTor inhibitor), XL 418 (also an Akt inhibitor), XL-147, and XL 765 (also an mTor inhibitor).

Examples for inhibitors of cMet or HGF are XL-184 (also an inhibitor of VEGFR, cKit, Flt3), PF-2341066, MK-2461, XL-880 (also an inhibitor of VEGFR), MGCD-265 (also an inhibitor of VEGFR, Ron, Tie2), SU-11274, PHA-665752, AMG-102, and AV-299.

An example for a c-Myc inhibitor is CX-3543.

Examples for Flt3 inhibitors are AC-220 (also an inhibitor of cKit and PDGFR), KW 2449, lestaurtinib (also an inhibitor of VEGFR, PDGFR, PKC), TG-101348 (also an inhibitor of JAK2), XL-999 (also an inhibitor of cKit, FGFR, PDGFR and VEGFR), sunitinib (also an inhibitor of PDGFR, VEGFR and cKit), and tandutinib (also an inhibitor of PDGFR, and cKit).

Examples for HSP90 inhibitors are tanespimycin, alvespimycin, IPI-504 and CNF 2024.

Examples for JAK/STAT inhibitors are CYT-997 (also interacting with tubulin), TG 101348 (also an inhibitor of Flt3), and XL-019.

Examples for Mek inhibitors are ARRY-142886, PD-325901, AZD-8330, and XL 518.

Examples for mTor inhibitors are temsirolimus, AP-23573 (which also acts as a VEGF inhibitor), everolimus (a VEGF inhibitor in addition). XL-765 (also a PI3 kinase inhibitor), and BEZ-235 (also a PI3 kinase inhibitor).

Examples for Akt inhibitors are perifosine, GSK-690693, RX-0201, and triciribine.

Examples for cKit inhibitors are AB-1010, OSI-930 (also acts as a VEGFR inhibitor), AC-220 (also an inhibitor of Flt3 and PDGFR), tandutinib (also an inhibitor of Flt3 and PDGFR), axitinib (also an inhibitor of VEGFR and PDGFR), XL-999 (also an inhibitor of Flt3, PDGFR, VEGFR, FGFR), sunitinib (also an inhibitor of Flt3, PDGFR, VEGFR), and XL-820 (also acts as a VEGFR- and PDGFR inhibitor), imatinib (also a bcr-abl inhibitor), nilotinib (also an inhibitor of bcr-abl and PDGFR).

Examples for hedgehog antagonists are IPI-609 and CUR-61414.

Examples for CDK inhibitors are seliciclib, AT-7519, P-276, ZK-CDK (also inhibiting VEGFR2 and PDGFR), PD-332991, R-547, SNS-032, PHA-690509, and AG 024322.

Examples for proteasome inhibitors are bortezomib, carfilzomib, and NPI-0052 (also an inhibitor of NFkappaB).

An example for an NFkappaB pathway inhibitor is NPI-0052.

An example for an ubiquitination pathway inhibitor is HBX-41108.

In preferred embodiments, the additional therapeutic agent is an anti-angiogenic agent.

Examples for anti-angiogenic agents are inhibitors of the FGFR, PDGFR and VEGFR or the respective ligands (e.g VEGF inhibitors like pegaptanib or the anti-VEGF antibody bevacizumab), EGFL7 inhibitors, such as anti-EGFL7 MAb , angiopoietin1/2 inhibitors such as AMG386, and thalidomides, such agents being selected from, without limitation, bevacizumab, motesanib, CDP-791, SU-14813, telatinib, KRN-951, ZK-CDK (also an inhibitor of CDK), ABT-869, BMS-690514, RAF-265, IMC-KDR, IMC-18F1, IMiDs (immunomodulatory drugs), thalidomide derivative CC-4047, lenalidomide, ENMD 0995, IMC-D11, Ki 23057, brivanib, cediranib, XL-999 (also an inhibitor of cKit and Flt3), 1 B3, CP 868596, IMC 3G3, R-1530 (also an inhibitor of Flt3), sunitinib (also an inhibitor of cKit and Flt3), axitinib (also an inhibitor of cKit), lestaurtinib (also an inhibitor of Flt3 and PKC), vatalanib, tandutinib (also an inhibitor of Flt3 and cKit), pazopanib, GW 786034, PF-337210, IMC-1121B, AVE-0005, AG-13736, E-7080, CHIR 258, sorafenib tosylate (also an inhibitor of Raf), RAF-265 (also an inhibitor of Raf), vandetanib, CP-547632, OSI-930, AEE-788 (also an inhibitor of EGFR and Her2), BAY-57-9352 (also an inhibitor of Raf), BAY-73-4506 (also an inhibitor of Raf), XL 880 (also an inhibitor of cMet), XL-647 (also an inhibitor of EGFR and EphB4), XL 820 (also an inhibitor of cKit), and nilotinib (also an inhibitor of cKit and brc-abl).

The additional therapeutic agent may also be selected from EGFR inhibitors, it may be a small molecule EGFR inhibitor or an anti-EGFR antibody. Examples for anti-EGFR antibodies, without limitation, are cetuximab, panitumumab, matuzumab; an example for a small molecule EGFR inhibitor is gefitinib. Another example for an EGFR modulator is the EGF fusion toxin.

Among the EGFR and Her2 inhibitors useful for combination with the VEGF-binding molecule of the invention are lapatinib, gefitinib, erlotinib, cetuximab, trastuzumab, nimotuzumab, zalutumumab, vandetanib (also an inhibitor of VEGFR), pertuzumab, XL-647, HKI-272, BMS-599626 ARRY-334543, AV 412, mAB-806, BMS-690514, JNJ-26483327, AEE-788 (also an inhibitor of VEGFR), ARRY-333786, IMC-11F8, Zemab.

Other agents that may be advantageously combined in a therapy with the VEGF-binding molecule of the invention are tositumumab and ibritumomab tiuxetan (two radiolabelled anti-CD20 antibodies), alemtuzumab (an anti-CD52 antibody), denosumab, (an osteoclast differentiation factor ligand inhibitor), galiximab (a CD80 antagonist), ofatumumab (a CD20 inhibitor), zanolimumab (a CD4 antagonist), SGN40 (a CD40 ligand receptor modulator), rituximab (a CD20 inhibitor), mapatumumab (a TRAIL-1 receptor agonist), REGN421 (SAR153192) or OMP-21 M18 (Dll4 inhibitors).

Other chemotherapeutic drugs that may be used in combination with the VEGF-binding molecules of the present invention are selected from, but not limited to hormones, hormonal analogues and antihormonals (e.g. tamoxifen, toremifene, raloxifene, fulvestrant, megestrol acetate, flutamide, nilutamide, bicalutamide, cyproterone acetate, finasteride, buserelin acetate, fludrocortisone, fluoxymesterone, medroxyprogesterone, octreotide, arzoxifene, pasireotide, vapreotide), aromatase inhibitors (e.g. anastrozole, letrozole, liarozole, exemestane, atamestane, formestane), LHRH agonists and antagonists (e.g. goserelin acetate, leuprolide, abarelix, cetrorelix, deslorelin, histrelin, triptorelin), antimetabolites (e.g. antifolates like methotrexate, pemetrexed, pyrimidine analogues like 5 fluorouracil, capecitabine, decitabine, nelarabine, and gemcitabine, purine and adenosine analogues such as mercaptopurine thioguanine, cladribine and pentostatin, cytarabine, fludarabine); antitumor antibiotics (e.g. anthracyclines like doxorubicin, daunorubicin, epirubicin and idarubicin, mitomycin-C, bleomycin dactinomycin, plicamycin, mitoxantrone, pixantrone, streptozocin); platinum derivatives (e.g. cisplatin, oxaliplatin, carboplatin, lobaplatin, satraplatin); alkylating agents (e.g. estramustine, meclorethamine, melphalan, chlorambucil, busulphan, dacarbazine, cyclophosphamide, ifosfamide, hydroxyurea, temozolomide, nitrosoureas such as carmustine and lomustine, thiotepa); antimitotic agents (e.g. vinca alkaloids like vinblastine, vindesine, vinorelbine, vinflunine and vincristine; and taxanes like paclitaxel, docetaxel and their formulations, larotaxel; simotaxel, and epothilones like ixabepilone, patupilone, ZK-EPO); topoisomerase inhibitors (e.g. epipodophyllotoxins like etoposide and etopophos, teniposide, amsacrine, topotecan, irinotecan) and miscellaneous chemotherapeutics such as amifostine, anagrelide, interferone alpha, procarbazine, mitotane, and porfimer, bexarotene, celecoxib.

The efficacy of VEGF-binding molecules of the invention or polypeptides, and of compositions comprising the same, can be tested using any suitable *in vitro* assay, cell- based assay, *in vivo* assay and/or animal model known per se, or any combination thereof, depending on the specific disease or disorder of interest. Suitable assays and animal models will be clear to the skilled person, and for example include the assays described herein and used in the Examples below, e.g. a proliferation assay.

The data obtained in the experiments of the invention confirm that VEGF-binding molecules of the invention have properties that are superior to those of VEGF-binding molecules of the prior art. Among such properties are complete inhibition of the VEGF165-VEGFR2 interaction and a low IC50, as can e.g. be taken from the ELISA data of Figure 1 and Table 5 as well as the $IC_{50}$ (nM) values for VHHs in the AlphaScreen assay as shown in Figures 3, 17, 18 and Table 7; and the affinity $K_D$ (nM) of purified VHHs on recombinant human VEGF and mouse VEGF in Table 9, 10 and Figures 5-1 and 5-2. Also, as shown in Table 13, VEGF binders of the invention have high potency, i.e. in the subnanomolar range, in the HUVEC proliferation assay. This indicates that VEGF-binding molecules of the invention are promising candidates to have therapeutic efficacy in diseases and disorders associated with VEGF-mediated effects on angiogenesis, such as cancer.

According to another embodiment of the invention, there is provided a method of diagnosing a disease by

a) contacting a sample with a VEGF-binding molecule of the invention as defined above, and

b) detecting binding of said VEGF-binding molecule to said sample, and

c) comparing the binding detected in step (b) with a standard, wherein a difference in binding relative to said sample is diagnostic of a disease or disorder associated with VEGF-mediated effects on angiogenesis.

For this and other uses, it may be useful to further modify a VEGF-binding molecule of the invention, such as by introduction of a functional group that is one part of a specific binding pair, such as the biotin-(strept)avidin binding pair. Such a functional group may be used to link the VEGF-binding molecule of the invention to another protein, polypeptide or chemical compound that is bound to the other half of the binding pair, i.e. through formation of the binding pair. For example, a VEGF-binding molecule of the invention may be conjugated to biotin, and linked to another protein, polypeptide, compound or carrier conjugated to avidin or streptavidin. For example, such a conjugated VEGF-binding molecule of the invention may be used as a reporter, for example in a diagnostic system where a detectable signal-producing agent is conjugated to avidin or streptavidin.

**Brief description of the Figures:**

| | |
|---|---|
| **Figure 1:** | Purified monovalent VHHs block the hVEGF165/hVEGFR2-Fc interaction (ELISA) |
| **Figure 2:** | Purified monovalent VHHs block the hVEGF165/hVEGFR1-Fc interaction (ELISA) |
| **Figure 3:** | Purified monovalent VHHs block the hVEGF165/hVEGFR2-Fc interaction (AlphaScreen) |
| **Figure 4:** | Purified monovalent VHHs block the hVEGF165/hVEGFR1-Fc interaction (AlphaScreen) |
| **Figures 5-1 and 5-2:** | Binding of monovalent VHHs to recombinant human and mouse VEGF (ELISA) |
| **Figure 6:** | Binding of monovalent VHHs to human VEGF121 |
| **Figure 7-1 through 7-4:** | Purified VHHs do not bind to VEGFB, VEGFC, VEGFD and PIGF |
| **Figure 8-1 and 8-2:** | Formatted VHHs block hVEGF165/hVEGFR2-Fc interaction (ELISA) |
| **Figure 9-1 and 9-2:** | Formatted VHHs block hVEGF165/hVEGFR1-Fc interaction (ELISA) |
| **Figure 10:** | Formatted VHHs block hVEGF165/hVEGFR2-Fc interaction (AlphaScreen) |
| **Figure 11**: | Formatted VHHs block hVEGF165/hVEGFR1-Fc interaction (AlphaScreen) |
| **Figure 12:** | Formatted VHHs block mVEGF164/mVEGFR2-Fc interaction (AlphaScreen) |
| **Figure 13-1 and 13-2:** | Formatted VHHs bind to mouse and human VEGF |
| **Figure 14-1 through 14-8**: | Formatted VHHs do not bind to VEGFB, VEGFC, VEGFD and PIGF |
| **Figure 15:** | Formatted VHHs bind to VEGF121 |
| **Figure 16:** | Sequence alignment of VHH VEGFBII23B04 with human VH3/JH germline consensus sequence |
| **Figure 17:** | VHH variants of VEGFBII23B04 block hVEGF165/hVEGFR2-Fc interaction (AlphaScreen) |
| **Figure 18:** | Sequence-optimized clones of VEGFBII23B04 block the hVEGF165/hVEGFR2-Fc interaction (AlphaScreen) |
| **Figure 19:** | Sequence alignment of VHH VEGFBII5B05 with human VH3/JH germline consensus sequence |

**Materials and methods:**

**a) Production and functionality testing of VEGF109**

A cDNA encoding the receptor binding domain of human vascular endothelial growth factor isoform VEGF165 (GenBank: AAM03108.1; AA residues 27 - 135) is cloned into pET28a vector (Novagen, Madison, WI) and overexpressed in E.coli (BL21 Star DE3) as a His-tagged insoluble protein. Expression is induced by addition of 1 mM IPTG and allowed to continue for 4 hours at 37°C. Cells are harvested by centrifugation and lysed by sonication of the cell pellet. Inclusion bodies are isolated by centrifugation. After a washing step with 1% Triton X 100 (Sigma-Aldrich), proteins are solubilized using 7.5M guanidine hydrochloride and refolded by consecutive rounds of overnight dialysis using buffers with decreasing urea concentrations from 6M till 0M. The refolded protein is purified by ion exchange chromatography using a MonoQ5/50GL (Amersham BioSciences) column followed by gel filtration with a Superdex75 10/300 GL column (Amersheim BioSciences). The purity and homogeneity of the protein is confirmed by SDS-PAGE and Westen blot. In addition, binding activity to VEGFR1, VEGFR2 and Bevacizumab is monitored by ELISA. To this end, 1 μg/mL of recombinant human VEGF109 is immobilized overnight at 4°C in a 96-well MaxiSorp plate (Nunc, Wiesbaden, Germany). Wells are blocked with a casein solution (1%). Serial dilutions of VEGFR1, VEGFR2 or Bevacizumab are added to the VEGF109 coated plate and binding is detected using alkaline phosphatase (AP) conjugated goat anti-human IgG, Fc specific (Jackson Immuno Research Laboratories Inc., West Grove, PA, USA) and a subsequent enzymatic reaction in the presence of the substrate PNPP (p-nitrophenylphosphate) (Sigma-Aldrich). VEGF109 could bind to VEGFR1, VEGFR2 and Bevacizumab, indicating that the produced VEGF109 is active. **b) KLH conjugation of VEGF165 and functionality testing of KLH-conjugated VEGF165**

Recombinant human VEGF165 (R&D Systems, Minneapolis, MN, USA) is conjugated to mariculture keyhole limpet hemocyanin (mcKLH) using the Imject Immunogen EDC kit with mcKLH (Pierce, Rockford, IL, USA) according to the manufacturer's instructions. Efficient conjugation of the polypeptide to mcKLH is confirmed by SDS-PAGE. Functionality of the conjugated protein is checked by ELISA: 2 μg/mL of KLH conjugated VEGF165 is immobilized overnight at 4°C in a 96-well MaxiSorp plate (Nunc, Wiesbaden, Germany). Wells are blocked with a casein solution (1%). Serial dilutions of VEGFR1 or VEGFR2 are added and binding is detected using a horseradish peroxidase (HRP)-conjugated goat anti-human IgG, Fc specific (Jackson Immuno Research Laboratories Inc., West Grove, PA, USA) and a subsequent enzymatic reaction in the presence of the substrate TMB (3,3',5,5'-tetramentylbenzidine) (Pierce, Rockford, IL, USA). The KLH conjugated protein could still interact with VEGFR1, VEGFR2 and Bevacizumab, confirming that the relevant epitopes onVEGF165 are still accessible.

**Example 1**

**Immunization with different VEGF formats induces a humoral immune response in llama**

*1.1 Immunizations*

After approval of the Ethical Committee of the faculty of Veterinary Medicine (University Ghent, Belgium), 4 llamas (designated No. 264, 265, 266, 267) are immunized according to standard protocols with 6 intramuscular injections (100 or 50 μg/dose at weekly intervals) of recombinant human VEGF109. The first injection at day 0 is formulated in Complete Freund's Adjuvant (Difco, Detroit, MI, USA), while the subsequent injections are formulated in Incomplete Freund's Adjuvant (Difco, Detroit, MI, USA). In addition, four llamas (designated No. 234, 235, 280 and 281) are immunized according to the following protocol: 5 intramuscular injections with KLH-conjugated human VEGH165 (100 or 50 μg/dose at biweekly intervals) followed by 4 intramuscular injections of human VEGF109 (first dose of 100 μg followed 2 weeks later with three 50 μg/dose at weekly interval).

*1.2 Evaluation of VEGF-induced immune responses in llama*

To monitor VEGF specific serum titers, an ELISA assay is set up in which 2 μg/mL of recombinant human VEGF165 or VEGF109 is immobilized overnight at 4°C in a 96-well MaxiSorp plate (Nunc, Wiesbaden, Germany). Wells are blocked with a casein solution (1%). After addition of serum dilutions, bound total IgG is detected using horseradish peroxidase (HRP)-conjugated goat anti-llama immunoglobulin (Bethyl Laboratories Inc., Montgomery, TX, USA) and a subsequent enzymatic reaction in the presence of the substrate TMB (3,3',5,5'-tetramentylbenzidine) (Pierce, Rockford, IL, USA). For llamas 264, 265, 266 and 267, an additional ELISA is performed in which the isotype-specific responses against VEGF165 and VEGF109 are evaluated. Isotype specific responses are detected using mouse mAbs specifically recognizing conventional llama IgG1 and the heavy-chain only llama IgG2 and IgG3 [Daley et al. (2005). Clin. Diagn. Lab. Imm. 12:380-386] followed by a rabbit anti-mouse-HRP conjugate (DAKO). ELISAs are developed using TMB as

chromogenic substrate and absorbance is measured at 450nm. The serum titers for each llama are depicted in Table 1.

**Table 1:** Antibody-mediated specific serum response against VEGF165 and VEGF109

| ELISA (recombinant protein solid phase coated) | | | | | | | | | |
|---|---|---|---|---|---|---|---|---|---|
| | | **Recombinant human EGF165** | | | | **Recombinant human VEGF109** | | | |
| **Llama** | **Immunogen** | **Total IgG** | **IgG1** | **IgG2** | **IgG3** | **Total IgG** | **IgG1** | **IgG2** | **IgG3** |
| | | | | | | | | | |
| 234 | VEGF165-KLH + VEGF109 | ++ | n/d | n/d | n/d | ++ | n/d | n/d | n/d |
| 235 | VEGF165-KLH + VEGF109 | ++ | n/d | n/d | n/d | ++ | n/d | n/d | n/d |
| 280 | VEGF165-KLH + VEGF109 | + | n/d | n/d | n/d | + | n/d | n/d | n/d |
| 281 | VEGF165-KLH + VEGF109 | + | n/d | n/d | n/d | + | n/d | n/d | n/d |
| | | | | | | | | | |
| 264 | VEGF109 | n/d | ++ | + | + | ++ | ++ | + | + |
| 265 | VEGF109 | n/d | ++ | + | + | + | ++ | + | + |
| 266 | VEGF109 | n/d | ++ | + | +/- | ++ | ++ | + | +/- |
| 267 | VEGF109 | n/d | +/- | - | - | +/- | +/- | - | - |
| n/d, not determined | | | | | | | | | |

**Example 2**

**Cloning of the heavy-chain only antibody fragment repertoires and preparation of phage**

Following the final immunogen injection, immune tissues as the source of B-cells that produce the heavy-chain antibodies are collected from the immunized llamas. Typically, two 150-ml blood samples, collected 4 and 8 days after the last antigen injection, and one lymph node biopsy, collected 4 days after the last antigen injection are collected per animal. From the blood samples, peripheral blood mononuclear cells (PBMCs) are prepared using Ficoll-Hypaque according to the manufacturer's instructions (Amersham Biosciences, Piscataway, NJ, USA). From the PBMCs and the lymph node biopsy, total RNA is extracted, which is used as starting material for RT-PCR to amplify the VHH encoding DNA segments, as described in WO05/044858. For each immunized llama, a library is constructed by pooling the total RNA isolated from all collected immune tissues of that animal. In short, the PCR-amplified VHH repertoire is cloned via specific restriction sites into a vector designed to facilitate phage display of the VHH library. The vector is derived from pUC119 and contains the LacZ promoter, a M13 phage gIII protein coding sequence, a resistance gene for ampicillin or carbenicillin, a multiple cloning site and a hybrid gIII-pelB leader sequence (pAX050). In frame with the VHH coding sequence, the vector encodes a C-terminal c-myc tag and a His6 tag. Phage are prepared according to standard protocols and stored after filter sterilization at 4°C for further use.

**Example 3**

**Selection of VEGF-specific VHHs via phage display**

VHH phage libraries are used in different selection strategies applying a multiplicity of selection conditions. Variables include i) the VEGF protein format (rhVEGF165, rhVEGF109 or rmVEGF164), ii) the antigen presentation method (solid phase: directly coated or via a biotin-tag onto Neutravidin-coated plates; solution phase: incubation in solution followed by capturing on Neutravidin-coated plates), iii) the antigen concentration and iv) the elution method (trypsin or competitive elution using VEGFR2). All selections are carried out in Maxisorp 96-well plates (Nunc, Wiesbaden, Germany).

Selections are performed as follows: Phage libraries are incubated at RT with variable concentrations of VEGF antigen, either in solution or immobilized on a solid support. After 2hrs of incubation and extensive washing, bound phage are eluted. In case trypsin is used for phage elution, the protease activity is immediately neutralized by addition of 0.8 mM protease inhibitor AEBSF. Phage outputs that show enrichment over background are used to infect *E. coli.* Infected *E. coli* cells are either used to prepare phage for the next selection round (phage rescue) or plated on agar

plates (LB+amp+glucose$^{2\%}$) for analysis of individual VHH clones. In order to screen a selection output for specific binders, single colonies are picked from the agar plates and grown in 1 mL 96-deep-well plates. The lacZ-controlled VHH expression is induced by adding IPTG (0.1-1mM final). Periplasmic extracts (in a volume of ~ 80 μL) are prepared according to standard methods.

**Example 4**

**Identification of VEGF-binding and VEGF receptor-blocking VHHs**

Periplasmic extracts are tested for binding to human VEGF165 by ELISA. In brief, 2 μg/mL of recombinant human VEGF165 is immobilized overnight at 4°C in a 96-well MaxiSorp plate (Nunc, Wiesbaden, Germany). Wells are blocked with a casein solution (1%). After addition of typically a 10-fold dilution of the periplasmic extracts, VHH binding is detected using a mouse anti-myc (Roche) and an anti-mouse-HRP conjugate (DAKO). Clones showing ELISA signals of >3-fold above background are considered as VEGF binding VHHs.

In addition, periplasmic extracts are screened in a human VEGF165/human VEGFR2 AlphaScreen assay (Amplified Luminescent Proximity Homogeneous Assay) to assess the blocking capacity of the VHHs. Human VEGF165 is biotinylated using Sulfo-NHS-LC-Biotin (Pierce, Rockford, IL, USA). Human VEGFR2/Fc chimera (R&D Systems, Minneapolis, MN, USA) is captured using an anti-humanFc VHH which is coupled to acceptor beads according to the manufacturer's instructions (Perkin Elmer, Waltham, MA, US). To evaluate the neutralizing capacity of the VHHs, periplasmic extracts are diluted 1/25 in PBS buffer containing 0.03 % Tween 20 (Sigma-Aldrich) and preincubated with 0.4 nM biotinylated human VEGF165 for 15 minutes at room temperature (RT). To this mixture the acceptor beads (10μg/ml) and 0.4 nM VEGFR2-huFc are added and further incubated for 1 hour at RT in the dark. Subsequently donor beads (10μg/ml) are added followed by incubation of 1 hour at RT in the dark. Fluorescence is measured by reading plates on the Envision Multi label Plate reader (Perkin Elmer, Waltham, MA, USA) using an excitation wavelength of 680 nm and an emission wavelength between 520 nm and 620nm. Periplasmic extract containing irrelevant VHH is used as negative control. Periplasmic extracts containing anti-VEGF165 VHHs which are able to decrease the fluorescence signal with more than 60 % relative to the signal of the negative control are identified as a hit. All hits identified in the AlphaScreen are confirmed in a competition ELISA. To this end, 1 μg/mL of human VEGFR2 chimera (R&D Systems, Minneapolis, MN, USA) is coated in a 96-well MaxiSorp plate (Nunc, Wiesbaden, Germany). Fivefold dilutions of the periplasmic extracts are incubated in the presence of a fixed concentration (4nM) of biotinylated human VEGF165 in PBS buffer containing 0.1 % casein and 0.05 % Tween 20 (Sigma-Aldrich). Binding of these VHH/bio-VEGF165 complexes to the human VEGFR2 chimera coated plate is detected using horseradish peroxidase (HRP) conjugated extravidin (Sigma, St Louis, MO, USA). VHH sequence IDs and the corresponding AA sequences of VEGF-binding (non-receptor-blocking) VHHs and inhibitory (receptor-blocking) VHHs are listed in Table 2 and Table 3, respectively.

**Table 2:** Sequence IDs and AA sequences of monovalent "non-receptor-blocking" anti-VEGF VHHs (FR, framework; CDR, complementary determining region)

| VHH ID/ SEQ ID NO: | FR1 | CDR1 | FR2 | CDR2 | FR3 | CDR3 | FR4 |
|---|---|---|---|---|---|---|---|
| VEGFBII 01C02/**58** | EVQLVESGGG LVQAGGSLRL SCTASGGSFS | SYGMG | WFRQSPG KEREFVS | AISEYSNTY CSDSVRG | RFTISRDNTKNTV YLQMNSLTPDDTA IYYCAA | SPTILLTTEQWYK Y | WGQGTQ VTVSS |
| VEGFBII 01E07/**59** | EVQLVESGGG LVQAGDSLRL SCVATGRTFR | ASDMG | WFRQAPG KEREFVA | AINWSGLST FYTDSVKG | RFTISRDNDNGAL YLQMNTLKPEDTA VYSCAA | GRIPSSSRFSSPA AYAS | WGQGTQ VTVSS |
| VEGFBII 03D12/**60** | EVQLVESGGG LVQAGGSLRL SCTASTSIYT | ITVMA | WFRQAPG KEREFVA | AITWSAPTT YYADSVKG | RFTISRDNAKNTV YLRMNSLKPEDSA IYYCAA | DRFKGRSIVTPSD YRY | WGQGTQ VTVSS |
| VEGFBII 04B08/**61** | EVQLVESGGG LVQPGGSLRL SCAASGSAVG | DITVA | WYRQAPG IQRQLVA | TITPSGYTY YWDFVKG | RFTISRDNSKNIV YLQMNSLKPEDTA AYYCNT | QFY | WGQGTQ VTVSS |
| VEGFBII 05B02/**62** | EVQLVESGGG LVQAGGSLRL SCAASGRTFS | TDDVG | WFRQAPG KEREFVA | VIRWSTGGT YTSDSVKG | RFTLSRDNAKNTM YLQMNSLKPEDTA VYYCAA | RSRPLGAGAWYSG EKHYNY | WGQGTQ VTVSS |
| VEGFBII 05B03/**63** | EVQLVESGGG LAQAGDSLRL SCAASGRSFS | HYNMG | WFRQAPG KEREFVA | SIRGGGGST TYANSVKD | RFTISRENAKNTV YLQMNSLKPEDTA VYYCAA | TAFYRGPYDYDY | WGQGTQ VTVSS |
| VEGFBII 05B05/**64** | EVQLVESGGG LVQPGGSLRL SCVASGIRFM | SMA | WYRQAPG KHRELVA | RISSGGTTA YVDSVKG | RFTISRDNSKNTV YLQMNSLKAEDTA VYYCNT | FSSRPNP | WGAGTQ VTVSS |
| VEGFBII 06G02/**65** | EVQLVESGGG LVQPGGSLRL SCAASGNIFS | NNAMA | WYRQAPG KQRELVA | RISSGGGFT YYLDSVKG | RFTVSRDNAKNTV YLQMNSLKPEDTA VYYCNA | AYRTYNY | WGQGTQ VTVSS |
| VEGFBII 07A03/**66** | EVQLVESGGG LVQAGGSLRL SCAASTSIYS | ITVMA | WFRQAPG KESEFVA | AITWSAPSS YYADSVKG | RFTISRDNAKNTV YLQMNSLKPEDSA IYYCAA | DRFKGRSIVTRSD YKY | WGQGTQ VTVSS |

(continued)

| VHH ID/ SEQ ID NO: | FR1 | CDR1 | FR2 | CDR2 | FR3 | CDR3 | FR4 |
|---|---|---|---|---|---|---|---|
| VEGFBII 07A06/**67** | EVQLVESGGG LVQAGGSLRL SCAVSTSIYS | ISVMA | WFRQAPG KERAFVA | AITWSAPTT YYADSVKG | RFTISRDNAKNTV YLQTNSLKPEDSA IYYCAA | DRFKGRSIVTRSD YRY | WGQGTQ VTVSS |
| VEGFBII 07D08/**68** | EVQLVESGGG LVQTGGSLRL SCAASGRTFS | NYAMA | WFRQAPG KEREFVS | AINQRGSNT NYADSVKG | RFTISRDSAKNSV FLQMNSLKPEDTA VYYCAA | STWYGYSTYARRE EYRY | WGQGTQ VTVSS |
| VEGFBII 08D09/**69** | EVQLVESGGG LVQAGGSLRL SCAASGRSFS | DNVMG | WFRQAAG KEREFVA | HISRGGSRT EYAESVKG | RFTISRDNTKKTM YLQMNSLKPEDTA VYYCAA | SRSVALATARPYD Y | WGQGTQ VTVSS |
| VEGFBII 08E07/**70** | EVQLVESGGG LAQAGGSLRL SCTTSGLTFS | SYYMG | WFRQAPG KEREFVA | TISWNKIST IYTDSVKG | RFTVSRDNNKNTV YLQMNSLKPEDTA VYYCAA | DASRPTLRIPQY | WGQGTQ VTVSS |
| VEGFBII 08F06/**71** | EVQLVESGGG LVQPGGSLRL SCAASGSIVR | SDVMG | WYRQAPG KQRELVA | FIRSLGSTY YAGSVKG | RFTISRDDAANTV YLQMNNLKPEDTA VYYCNA | RFSGESY | WGQGTP VTVSS |
| VEGFBII 08F07/**72** | EVQLVESGGG LVQAGGSLRL SCAVSGSTFG | LYAMG | WFRQAPG REREFLS | AITWSAGDT QYADSVKG | RFTISRDNARNTV NLQMNGLKPEDTA VYYCAG | RQWGGTYYYHGSY AY | WGQGTQ VTVSS |
| VEGFBII 09A09/**73** | EVQLVESGGG LVQPGGSLRL SCVASGIRFM | SMA | WYRQAPG KHRELVA | RISSEGTTA YVDSVKG | RFTISRDNSKNTV YLQMNSLKAEDTA VYYCNT | FSSRPNP | WGAGTT VTVSS |
| VEGFBII 09A12/**74** | EVQLVESGGG LVQAGGSLRL SCAASGRTFS | TDDVG | WFRQAPG KEREFVA | VIRWSTGGT YTSDSVAG | RFTLSRDNAKNTM YLQMNSLKPEDTA VYYCAA | RSRPLGAGAWYTG ETRYDS | WGQGTQ VTVSS |

(continued)

| VHH ID/ SEQ ID NO: | FR1 | CDR1 | FR2 | CDR2 | FR3 | CDR3 | FR4 |
|---|---|---|---|---|---|---|---|
| VEGFBII 09D05/**75** | EVQLVESGGGLVQPGDSLRLSCAASGLSFS | RYGMG | WFRQAPGKEREFVI | AISEYDNVYTADSVRG | RFTISRDNSKSTVYLQMNSLKSEDTAVYYCAA | SPTILLSTDEWYKY | WGRGTQVTVSS |
| VEGFBII 09F05/**76** | EVQLVESGGGLVQAGGSLRLSCAASGRTFS | TDDVG | WFRQAPGKEREFVA | VIRWSTGGTYTSDSVKG | RFTLSRDNAKNTMYLQMNSLKPEDTAVYYCAA | RSRPLGAGAWYTGETRYNY | WGQGTQVTVSS |
| VEGFBII 10C07/**77** | EVQLVESGGGLVQAGGSLSLSCAASARAFS | NYAMG | WFRQVPGREREFVA | VITRSPSNTYYTDSVKG | RFTISRDNAKNIVYLQMNSLKPEDTAVYYCAA | HYWNSDSYTYTDSRWYNY | WGQGTQVTVSS |
| VEGFBII 10E07/**78** | EVQLVESGGGLVQAGGSLRLSCAASGRTFS | NYAMG | WFRQAPGKERVLVA | DISSSGINTYVADAVKG | RFTISRDNAKNTVYLQMNSLKPEDTAVYYCAA | SAWWYSQMARDNYRY | WGQGTQVTVSS |
| VEGFBII 10G04/**79** | EVQLVESGGGLVQAGGSLRLSCAASGDTLS | RYAMG | WFRQAPGKEREFVA | SINTSGKRTSYADSMKG | RFAVSRDNAKNTGYLQMNSLKLEDTATYYCAA | DRFFGSDSNEPRAYRY | WGQGTQVTVSS |
| VEGFBII 10G05/**80** | EVQLVESGGGLVQAGESLRLSCVASGITFS | NYNMG | WFRQAPGKEREFVA | TIRHHGYDTYYAESVKG | RFTISRDNAKNTVYLQMNSLKPEDTALYSCAK | KLFWDMDPKTGFSS | WGQGTQVTVSS |
| VEGFBII 11C08/**81** | EVQLVESGGGLVQAGGSLRLSCAASGRTLS | SYGLG | WFRQAPGKEREFVA | AIGWSGSSTYYADSVKG | RFTVSVDNAKNTVYLKMNSLEPEDTAVYYCAA | KVRNFNSDWDLLTSYNY | WGQGTQVTVSS |
| VEGFBII 11C11/**82** | EVQLVESGGGLVQAGGSLMLSCAASGRALS | SYAIG | WFRQAPGREREFVA | RISWSGANTYYADSVKG | RFTISRGNAKNTVYLQMNSLKPEDTAAYYCAA | QTTSKYDNYDARAYGY | WGQGTQVTVSS |

(continued)

| VHH ID/ SEQ ID NO: | FR1 | CDR1 | FR2 | CDR2 | FR3 | CDR3 | FR4 |
|---|---|---|---|---|---|---|---|
| VEGFBII 11D09/**83** | EEQLVESGGGLVQAGGSLMLSCAASGRALS | SYAIG | WFRQAPGREREFVA | RISWSGANTYYADSVKG | RFTISRGNAKNTVYLQMNSLKPEDTAAYYCAA | QTTSKYDNYDARAYGY | WGQGTQVTVSS |
| VEGFBII 11E04/**84** | EVQLVESGGGLVQAGGSLRLSCAASGRTFS | SYAMG | WFRQAPGKEREFVA | TISQSGYSTYYADSVKG | RFTISRDNAKNTVNLQMNSLKPEDTAVYYCAA | DPFYSYGSPSPYRY | WGQGTQVTVSS |
| VEGFBII 11E05/**85** | EVQLVESGGGLVQPGGSLRLSCASSGRLFS | FSAMG | WFRQAPGKEREFVA | AFKWSGSTTYYADYVKG | RFTISTDNAKNILFLQMNSLKPEDTAIYYCAV | DRFYTGRYYSSDEYDY | WGQGTQVTVSS |
| VEGFBII 11F10/**86** | EVQLVESGGGLVQAGGSLRLSCAASTSIYS | ITVMA | WFRQAPGKEREFVA | AITWSAPSSYYADSVKG | RFTISRDNAKNTVYLQVNSLKPEDSAIYYCAA | DRFKGRSIVTRSDYRY | WGQGTQVTVSS |
| VEGFBII 11F12/**87** | EVQLVESGGGLVQSGGSLRLSCAASGRSFS | SLAMG | WFRQVPGKDREFVA | SISQSGITTSYADSVKS | RFTISRDSAKNTVYLQMNLLKPEDTAVYYCAT | SVFYSTALTRPVDYRY | WGQGTQVTVSS |
| VEGFBII 11G09/**88** | EVQLVESGGGLVQAGGSLRLSCAASTSIYS | ITVMA | WFRQAPGKEREFVA | AITWSAPTTYSADSVKG | RFTISRDNAKNTVYLQMNSLKPEDSAIYYCAA | DRFKGRSIVTRSDYRY | WGQGTQVTVSS |
| VEGFBII 12A07/**89** | EVQLVESGGGLVQAGGSLRLSCSVTGRTFN | KYVMG | WFRQAPGNDREFVA | AITSRDGPTYYADSVKG | RFTISGDNTKNKIFLQMNSLMPEDTAVYYCAI | DEDLYHYSSYHFTRVDLYHY | WGQGTQVTVSS |
| VEGFBII 12B01/**90** | EVQLVESGGGLVQPGGSLRLACAASGFTLS | SSWMY | WVRQAPGKGLEWVS | RISPGGLFTYYVDSVKG | RFSVSTDNANNTLYLQMNSLKPEDTALYSCAK | GGAPNYTP | RGRGTQVTVSS |

(continued)

| VHH ID/ SEQ ID NO: | FR1 | CDR1 | FR2 | CDR2 | FR3 | CDR3 | FR4 |
|---|---|---|---|---|---|---|---|
| VEGFBII 12C04/**91** | EVQLVESGGGLVQPGGSLRLSCAASGSIVR | SDVMG | WYRQAPGKQRELVA | FIRSLGSTYYAGSVKG | RFTISRDNAANTVYLQMNNLKPEDTAVYYCNA | RFSGESY | WGQGTPVTVSS |
| VEGFBII 12E10/**92** | EVQLVESGGGLAQAGGSLRLSCTASGRTFN | NYVMG | WFRQAPGNEREFVA | AITSTNGPTYYADSVKG | RFTISGDNTKNKVFLQMDSLRPEDTAVYYCAI | DEDLYHYSSYHYTRVALYHY | WGQGTQVTVSS |
| VEGFBII 12G04/**93** | EVQLVESGGGLVQSGDSLRLSCAVSGNTFG | LYAMG | WFRQAPGREREFVS | AITWSAGDTQYADSVKG | RFTISRDNARNTVNLQMNGLKPEDTAVYYCAG | RQWGGTYYYHGSYAW | WGQGTQVTVSS |
| VEGFBII 16C03/**94** | EVQLVESEGGLVQAGGSLRLSCAASGRTFS | TDDVG | WFRQAPGKEREFVA | VIRWSTGGTYTSDSVKG | RFTLSRDNAKNTMYLQMNSLKPEDTAVYYCAA | RSRPLGAGAWYTGENYYNY | WGQGTQVTVSS |
| VEGFBII 16F11/**95** | EVQLVESGGGLVQAGGSLRLSCAASGRTSS | GYDMG | WFRQAPGKEREFVT | AITWSGGSTYSPDSVKG | RFTISRDNAKNTVYLQMNNLTPEDTAVYYCAS | GRIWRSRDYDSEKYYDI | WGHGTQVTVSS |
| VEGFBII 36C08/**96** | EVQLVESGGGLVQAGGSLRLSCAASGRTFS | AYDMG | WFRQAPGKEREFVA | VISWTNSMTYYADSVKG | RFTISRDNAKNTVYLQMNSLKPEDTAVYYCAV | DRRRTYSRWRFYTGVNDYDY | WGQGTQVTVSS |
| VEGFBII 37F09/**97** | EVQLVESGGGLVQTGGSLRLSCAASGRTFS | AYDMG | WFRQAPGKEREFVA | VISWSGGMTYYADSVQG | RFTISRDNAKSTVYLQMNSPKPEDTAVYYCAV | DRRRAYSRWRYYTGVNDYEF | WGQGTQVTVSS |
| VEGFBII 38A06/**98** | EVQLVESGGGLVQAGGSLRLSCAASGRTFS | AYDMG | WFRQAPGKEREFVA | VISWSGGMTYYADSVKG | RFTISRDNAKNTVYLQMNSLKPEDTAVYYCAV | DRRRLYSRWRYYTGVNDYDY | WGQGTQVTVSS |

(continued)

| VHH ID/ SEQ ID NO: | FR1 | CDR1 | FR2 | CDR2 | FR3 | CDR3 | FR4 |
|---|---|---|---|---|---|---|---|
| VEGFBII 39H11/**99** | EVQLVESGGG LVQAGGSLRL SCAASGRTFS | AYDMG | WFRQAPG KEREFVA | VISWTGGMT YYADSVKG | RFTISRDKAKNTV SLQMNSLKPEDTA VYYCAV | DRRRTYSRWRYYT GVNEYEY | WGQGTQ VTVSS |
| VEGFBII 41B06/ **100** | EVQLVESGGG LVQAGGSLRL SCAASGRTFS | AYDMG | WFRQAPG KEREFVA | VISWTGDMT YYADSVKG | RFTISRDKAKNTV SLQMNSLKPEDTA VYYCAA | DRRRTYSRWRYYT GVNEYEY | WGQGTQ VTVSS |
| VEGFBII 41C05/ **101** | EVQLVESGGG LVQAGGSLRL SCAASGRTFS | VYTMG | WFRQAPG KEREFVA | TISRTGDRT SYANSVKG | RFTISRENAKNTV YLQMNSLKPEDTA VYSCAA | GPIAPSPRPREYY Y | WGQGTQ VTVSS |
| VEGFBII 41D11/ **102** | EVQLMESGGG LVQAGGSLRL SCAASGRTFS | AYDMG | WFRQAPG KEREFVA | VISWTGGMT YYADSVKG | RFTISRDKAKNTV SLQMNSLKPEDTA VYYCAV | DRRRTYSRWRYYT GVNEYEY | WGQGTQ VTVSS |
| VEGFBII 42F10/ **103** | EVQLVESGGG LVQAGGSLRL SCAASGRTFS | AYDMG | WFRQAPG KEREFVA | VISWSGGMT DYADSVKG | RFTISRENAKNTQ FLQMNSLKPEDTA VYYCAV | GRRRAYSRWRYYT GVNEYDY | WGQGTQ VTVSS |
| VEGFBII 86C11/ **104** | EVQLVESGGG LVQAGDSLRL SCTASGRTFN | SYAMG | WFRQAPG KERESVA | HINRSGSST YYADSVKG | RFTISRDNAKNTV YLQLNSLKPEDTA VYYCAA | GRYYSSDGVPSAS FNY | WGQGTQ VTVSS |
| VEGFBII 86F11/ **105** | EVQLVESGGG LVQAGDSLRL SCFTSARTFD | TWAMA | WFRQAPG KEREFIS | AISWSGSMT YYTDSVKG | RFIISRDNAQNTL FLQMNNTAPEDTA VYYCAA | KTVDYCSAYECYA RLEYDY | WGRGAQ VTVSS |
| VEGFBII 86G08/ **106** | EVQLVESGGG LMQTGDSLRL SCAASGLRFT | STNMG | WFRQGPG KEREFVA | AITLSGTTY YAEAVKG | RFTISRDNDKNTV ALQMNSLKPEDTA VYYCGA | DPSYYSTSRYTKA TEYDY | WGQGTQ VTVSS |

(continued)

| VHH ID/ SEQ ID NO: | FR1 | CDR1 | FR2 | CDR2 | FR3 | CDR3 | FR4 |
|---|---|---|---|---|---|---|---|
| VEGFBII 86G10/ **107** | EVQLVESGGGLVQAGGSLRLSCAASGRTFN | TYTMG | WFRQTPGTEREFVA | AIRWTVNITYYADSVKG | RFTISRDIVKNTVYLQMNSLKPEDTAVYYCAA | QTSAPRSLIRMSNEYPY | WGQGTQVTVSS |
| VEGFBII 86G11/ **108** | EVQLVESGGGLVQAGGSLRLSCAASGLTFS | LYTVG | WFRQAPGKEREFVA | YISRSGSNRYYVDSVKG | RFTLSRDNAKNTVDLQMNSLKTEDTAVYYCAA | TSRGLSSLAGEYNY | WGRGTQVTVSS |
| VEGFBII 86H09/ **109** | EVQLVESGGGLVQAGGSLRLSCTASGSAFK | SYRMG | WFRRTPGKEDEFVA | SISWTYGSTFYADSVKG | RFTMSRDKAKNAGYLQMNSLKPEDTALYYCAA | GAQSDRYNIRSYDY | WGQGTQVTVSS |
| VEGFBII 87B07/ **110** | EVQLVESGGGLVQPGGSLKLSCTASGFTFS | TSWMH | WVRQAPGKGLEWVS | SIPPVGHFANYAPSVKG | RFTISRDNAKNTLFLQMNSLKSEDTAVYYCAK | DSAGRT | KGQGTQVTVSS |
| VEGFBII 88A01/ **111** | KVQLVESGGGLVQAGGSLRLSCAASERTFS | NYAMD | WFRQAPGKEREFVA | AITRSGGGTYYADSVKG | RFTISRDNAKNTVYLQMNSLKPEDTAVYYCAA | TRSSTIVVGVGGMEY | WGKGTLVTVSS |
| VEGFBII 88A02/ **112** | EVQLVESGGGLVQAGGSLRLSCAASGFTFG | DYDIG | WFRQAPGNEREGVS | CITTDVGTTYYADSVKG | RFTISSDNAKNTVYLQINDLKPEDTAIYYCAV | DTQDLGLDIFCRGNGPFDG | WGQGTQVTVSS |
| VEGFBII 88B02/ **113** | EVQLVESGGGLVQPGGSLRLSCTASGLNLD | DYAIG | WFRQAPGKEREGVS | CISSYDSVTYYADHVKG | RFTISRDSAKNTLYLQMNSLSIEDTGVYYCAA | EREQLRRRESPHDELLRLCFYGMRY | SGKGTLVTVSS |
| VEGFBII 88E02/ **114** | EVQLVESGGGLVQPGGSLRLSCVASGFRLD | DYAIG | WFRQAPGKEREAVS | CISSSDTSIDYTNSVKG | RFTFSRDNAKNTVYLQMNSLKPEDTAVYYCAA | AFRCSGYELRGFPT | WGQGTQVTVSS |

(continued)

| VHH ID/ SEQ ID NO: | FR1 | CDR1 | FR2 | CDR2 | FR3 | CDR3 | FR4 |
|---|---|---|---|---|---|---|---|
| VEGFBII 88G03/ **115** | EVQLVESGGG LVQAGGSLRL SCAASGGTFS | SLAVG | WFRQAPG KEREFVA | RITWSGATT YYADAVKD | RFTISRDNAKNTM YLQMNSLKPEDTA VYYCAA | DRSPNIINVVTAY EYDY | WGQGTQ VTVSS |
| VEGFBII 88G05/ **116** | EVQLVESGGG LVQPGASLRL SCAASGDGFT | LYNMG | WFRQAPG KEREFVA | AITSSPMST YYADSVKG | RFSISINNDKTTG FLQMNVLKPEDTG VYFCAA | PEGSFRRQYADRA MYDY | WGQGTQ VTVSS |
| VEGFBII 88G11/ **117** | EVQLVESGGG LAQAGGSLRL SCAASGRTFS | GSDMG | WFRQSPG KEREIVA | AIRLSGSIT YYPDSVKG | RFTISRDNAKNTV YLQMNSLKPEDTA VYYCAA | RSTYSYYLALADR GGYDY | WGQGTQ VTVSS |
| VEGFBII 88H01/ **118** | EVQLVESGGG LVQAGGSLRL SCVASGFTLG | TYAIG | WFRQAPG KEREAVS | CMSAGDSIP WYTASVKG | RFTTSTDNARNTV YLQMNSLKPEDTA HYYCAA | ARYHGDYCYYEGY YPF | WGQGTQ VTVSS |
| VEGFBII 89B04/ **119** | EVQLVESGGG LVQAGGSLRL SCAASTSISS | TNFMG | WYRQAPG KQRELVA | TITSSSITN YVDSVKG | RFTISRDNAKNTV YLQMTSLKPEDTA VYYCHA | RWRWSDVEY | WGKGTL VTVSS |
| VEGFBII 89B08/ **120** | EVQLVESGGG LVQPGGSLRL SCAASGTTSS | IFAMR | WYRQAPG KQRELVA | SITRSSITT YADSVKG | RFTPSRDNAKNTV SLQMNSLKPEDTA VYYCNA | AIRPELYSVVNDY | WGQGTQ VTVSS |
| VEGFBII 89D04/ **121** | EVQLVESGGG LVQPGGSLRL SCATSGLTFS | DYNLG | WFRQAPG KERQFVA | VISWRDSFA YYAEPVKG | RFTISRDNAKNTV YLQMNSLKPEDTA VYYCAA | DRVSSRLVLPNTS PDFGS | WGQGTQ VTVSS |
| VEGFBII 89F09/ **122** | EVQLVESGGG LVQAGDSLRL SCAASGRTFN | NAIMG | WFRQAPG QEREFVA | AMNWRGGPT YYADSVKG | RFTISGDNTKNTV FLQMNFLKPEDTA VYYCAA | DEDLYHYSSYHYS RVDLYHY | WGQGTQ VTVSS |

(continued)

| VHH ID/ SEQ ID NO: | FR1 | CDR1 | FR2 | CDR2 | FR3 | CDR3 | FR4 |
|---|---|---|---|---|---|---|---|
| VEGFBII 89G09/ **123** | EVQLVESGGG LVQPGGSLRL SCAASGTTSS | IFAMR | WYRQAPG KQRELVA | SITRSSITT YADSVKG | RFTLSRDNAKNTV SLQMNSLKPEDTA VYYCNA | AIRPELYSVVNDY | WGQGTQ VTVSS |
| VEGFBII 89H08/ **124** | EVQLVESGGG LVQAGGSLRL SCAASGGSFS | SYAPG | WFRQAPG KEREFVA | AFTRSSNIP YYKDSVKG | RFTISRDNAHTVY LQMNSLKPEDTAI YYCAV | NLGSTWSRDQRTY DY | WGQGTQ VTVSS |

**Table 3:** Sequence IDs and AA sequences of monovalent receptor-blocking anti-VEGF VHHs (FR, framework; CDR, complementary determining region) SEQ ID NO: 9 - 46

| VHH ID/ **SEQ ID NO:** | **FR1** | **CDR1** | **FR2** | **CDR2** | **FR3** | **CDR3** | **FR4** |
|---|---|---|---|---|---|---|---|
| VEGFBII 22A10/**9** | EVQLVESGGGLVQPGDSLKLSCAFSGRTFS | SYSMG | WFRQAQGKEREFVV | AISSSGGYIYDSVSLEG | RFTISRDNTKNTVYLQTPSLKPEDTADYYCAA | SRAYGSSRLRLADTYDY | WGQGTQVTVSS |
| VEGFBII 22A11/**10** | EVQLVESGGGLVQPGDSLKLSCAFSGRTFS | SYSMA | WFRQAQGKEREFVV | AISSGGFIYDAVSLEG | RFTISRDNTKNTVYLQTPSLKPEDTAVYYCAA | SRAYGSSRLRLADTYDY | WGQGTQVTVSS |
| VEGFBII 22B06/**11** | EVQLVESGGGLVQPGDSLKLSCAASGRTFS | SYSMG | WFRQAQGKEREFVV | AISSSGGYIYDSVSLEG | RFTISRDNTKNTVYLQTPSLKPEDTAVYYCAA | SRAYGSSRLRLADTYDY | WGQGTQVTVSS |
| VEGFBII 22B07/**12** | EVQLVESGGGLVQAGDSLRLSCAASGRTFS | SYSMG | WFRQAQGKEREFVV | AISSSGNYKYDSVSLEG | RFTISRDNTKNTVYLQINSLKPEDTAVYYCAA | SRAYGSSRLRLGDTYDY | WGQGTQVTVSS |
| VEGFBII 22E04/**13** | EVQLVESGGGLVQPGDSLKLSCVASGRTSS | SYSMG | WFRQAQGKEREFVV | AISSGGSIYDSVSLQG | RFTISRDNTKNTVYLQTPSLKPEDTAVYYCAA | SRAYASSRLRLADTYDY | WGQGTQVTVSS |
| VEGFBII 23A03/**14** | EVQLVESGGGLVQPGDSLKLSCVASGRTFS | SYSMG | WFRQAQGKEREFVV | AISSGGYIYDSVSLQG | RFTISRDNTKNTVYLQTPSLKPEDTAVYYCAA | SRAYGSSRLRLADTYDY | WGQGTQVTVSS |

(continued)

| VHH ID/ **SEQ ID NO:** | **FR1** | **CDR1** | **FR2** | **CDR2** | **FR3** | **CDR3** | **FR4** |
|---|---|---|---|---|---|---|---|
| VEGFBII 23A06/**15** | EVQLVESGGGLVQPGDSLKLSCAFSGRTFS | SYSMG | WFRQAQGKEREFVV | AISSGGFIYDAVSLEG | RFTISRDNTKNTVYLQTPSLKPEDTAVYYCAA | SRAYGSSRLRLADTYDY | WGQGTQVTVSS |
| VEGFBII 23A08/**16** | EVQLVESGGGLVQTGDSLRLSCVASGRTFS | SYSMG | WFRQAQGKEREFVV | AISNGGYKYDSVSLEG | RFTISRDNTKNTVYLQINSLKPEDTAVYYCAA | SRAYGSSRLRLADTYDY | WGQGTQVTVSS |
| VEGFBII 23A09/**17** | EVQLVESGGGLVQPGDSLKLSCAFSGRTFG | SYSMG | WFRQAQGKEREFVV | AISSSGGYIYDSVSLEG | RFTISRDNSKNTVYLQTPSLKPEDTAVYYCAA | SRAYGSSRLRLPDTYDY | WGQGTQVTVSS |
| VEGFBII 23B04/**18** | EVQLVESGGGLVQTGDSLRLSCEVSGRTFS | SYSMG | WFRQAQGKEREFVV | AISKGGYKYDSVSLEG | RFTISKDNAKNTVYLQINSLKPEDTAVYYCAS | SRAYGSSRLRLADTYEY | WGQGTQVTVSS |
| VEGFBII 23D11/**19** | EVQLVESGGGLVQPGDSLRLSCAFSGRTFS | SYSMA | WFRQAQGKEREFVV | AISSGGFIYDAVSLEG | RFTISRDNTKNTVYLQTPSLKPEDTAVYYCAA | SRAYGSSRLRLADTYDY | WGQGTQVTVSS |
| VEGFBII 23E05/**20** | EVQLVESEGGLVQPGDSLKLSCVASGRTSS | SYSMG | WFRQAQGKEREFVV | AISSGGYIYDSVSLQG | RFTISRDNTKNTVYLQTPSLKPEDTAVYYCAA | SRAYGSSRLRLADTYDY | WGQGTQVTVSS |

(continued)

| VHH ID/ **SEQ ID NO:** | **FR1** | **CDR1** | **FR2** | **CDR2** | **FR3** | **CDR3** | **FR4** |
|---|---|---|---|---|---|---|---|
| VEGFBII 23F02/**21** | EMQLVESGG GLVQPGDSL KLSCAFSGR TFS | SYSM G | WFRQAQGKE REFVV | AISSSGGYI YDSVSLEG | RFTISRDNTKNT VYLQTPSLKPED TADYYCAA | SRAYGSSRLRL ADTYDY | WGQGTQV TVSS |
| VEGFBII 23F05/**22** | EVQLVESGG GLVQAGDSL RLSCAASGR TFS | SYSM G | WFRQAQGKE REFVV | AISSSGNYK YDSVSLEG | RFTISRDNTKNT VYLQINSLKPKD TAVYYCAA | SRAYGSSRLRL GDTYDY | WGQGTQV TVSS |
| VEGFBII 23F11/**23** | EVQLVESGG GLVQPGDSL KLSCAFSGR TFS | SYSM G | WFRQAQGKE REFVV | AISSGGGYI YDSVSLEG | RFTISRDNTKNT VYLQTPSLKPED TADYYCAA | SRAYGSSRLRL ADTYDY | WGQGTQV TVSS |
| VEGFBII 23G03/**24** | EVQLVESGG GLVQPGDSL KLSCAFSGR TFG | SYSM G | WFRQAQGKE REFVV | AISSSGGYI YDSVSLEG | RFTISRDNSKNT VYLQTPSLKPED TAVYYCAA | SRAYGSSRLRL PGTYDY | WGQGTQV TVSS |
| VEGFBII 24C04/**25** | EVQLVESGG GLVQPGDSL KLSCVASGR TSS | SYSM G | WFRQAQGKE REFVV | AISSGGYIY DSVSLQG | RFTISRDNTKNT VYLQTPSLKPED TAVYYCAA | SRAYGSSRLRL ADTYDY | WGQGTQV TVSS |
| VEGFBII 27D08/**26** | EVQLVESGG GLVQTGDSL RLSCAASGR TFS | SYSM G | WFRQAQGKE REFVV | AISSGGYKY DSVSLEG | RFTISRDNTQNT VYLQINSLKPED TAVYYCAA | SRAYGSGRLRL ADTYDY | WGQGTQV TVSS |

(continued)

| VHH ID/ **SEQ ID NO:** | **FR1** | **CDR1** | **FR2** | **CDR2** | **FR3** | **CDR3** | **FR4** |
|---|---|---|---|---|---|---|---|
| VEGFBII 27G07/**27** | EVQLVESGGGLVQPGDSLKLSCVASGRTSS | SYSMG | WFRQAQGQEREFVV | AISSGGYIYDSVSLQG | RFTISRDNTKNTVYLQTPSLKPEDTAVYYCAA | SRAYGSSRLRLADTYDY | WGQGTQVTVSS |
| VEGFBII 30C09/**28** | EVQLVESGGGLVQPGDSLKLSCIASGRTSS | SYSMG | WFRQAQGQEREFVV | AISSGGYIYDSVSLQG | RFTISRDNTKNTVYLQTPSLKPEDTAVYYCAA | SRAYGSSRLRLADTYDY | WGQGTQVTVSS |
| VEGFBII 30E07/**29** | EVQLVESGGGLVQAGDSLRLSCAASGRTFS | SYSMG | WFRQAQGKEREFVV | AISSSGNYKYDSVSLEG | RFTISRDNTKNTVYLQINSLKPEDTAVYYCAA | SRAYGSSRLRLGDTYDY | WGQGTRVTVSS |
| VEGFBII 31C07/**30** | EVQLVESGGGLVQTGDSLRLSCAASGGTFS | SYSMG | WFRQAQGKEREFVV | AISSSGGYIYDSVSLEG | RFTISRDNTKNTVYLQTPSLKPEDTADYYCAA | SRAYGSSRLRLADTYDY | WGQGTQVTVSS |
| VEGFBII 39E02/**31** | EVQLVESGGGLVQPGDPLKLSCAFSGRTFS | SYSMG | WFRQAQGKEREFVV | AISSSGGYIYDSVSLEG | RFTISRDNTKNTVYLQTPSLKPEDTADYYCAA | SRAYGSSRLRLADTYDY | WGQGTQVTVSS |
| VEGFBII 39G04/**32** | EVPLVESGGGLVQAGDSLRLSCAASGRTFS | SYSMG | WFRQAQGKEREFVV | AISSSGNYKYDSASLEG | RFTISRDNTKNTVYLQINSLKPEDTAVYYCAA | SRAYGSSRLRLGDTYDY | WGQGTQVTVSS |

(continued)

| VHH ID/ **SEQ ID NO:** | **FR1** | **CDR1** | **FR2** | **CDR2** | **FR3** | **CDR3** | **FR4** |
|---|---|---|---|---|---|---|---|
| VEGFBII 40F02/**33** | EVQLVESGGGLVQPGDSLKLSCAFSGRTFS | SYSMA | WFRQAQGKEREFVV | AISSGGFIYDAVSLEG | RFTISRDNTKNTVYLQTPSLKPEGTAVYYCAA | SRAYGSSRLRLADTYDY | WGQGTQVTVSS |
| VEGFBII 40G07/**34** | EVQLVESGGGLVQPGDSLKLSCAFSGRTFS | SYSMG | WFRQAQGKEREFVV | AISSSGGYIYDSVSLEG | RFTISRDNTKNAVYLQTPSLKPEDTADYYCAA | SRAYGSSRLRLADTYDY | WGQGTQVTVSS |
| VEGFBII 40H10/**35** | EVQLMESGGGLVQPGDSLKLSCAFSGRTFS | SYSMG | WFRQAQGKEREFVV | AISSSGGYIYDSVSLEG | RFTISRDNTKNTVYLQTPSLKPEDTADYYCAA | SRAYGSSRLRLADTYDY | WGQGTQVTVSS |
| VEGFBII 41B05/**36** | EVQLVESGGGLVQPGGSLRLSCAFSGRTFS | SYSMG | WFRQAQGKEREFVV | AISSGGFIYDAVSLEG | RFTISRDNTKNTVYLQTPSLKPEDTAVYYCAA | SRAYGSSRLRLADTYDY | WGQGTQVTVSS |
| VEGFBII 41G03/**37** | EVQLVESGGGLVQPGDSLKLSCAFSGRTFS | SYSMA | WFRQAQGKEREFVV | AISSGGFIYDAVSLEG | RFTISRENTKNTVYLQTPSLKPEDTAVYYCAA | SRAYGSSRLRLADTYDY | WGQGTQVTVSS |
| VEGFBII 42A05/**38** | EVQLVESGGGLVQPGDSLKLSCAFSGRTFS | SYSMG | WFRQAQGKEREFVV | AISSSGGYIYDSVSLEG | RFTISRDNTKNTVYLQMPSLKPEDTADYYCAA | SRAYGSSRLRLADTYDY | WGQGTQVTVSS |

(continued)

| VHH ID/ **SEQ ID NO:** | **FR1** | **CDR1** | **FR2** | **CDR2** | **FR3** | **CDR3** | **FR4** |
|---|---|---|---|---|---|---|---|
| VEGFBII 42D05/**39** | EVQLVESGG GLVQPGDSL KLSCAFSGR TFS | SYSM G | WFRQAQGKE REFVV | AISSSGGYI YDSVSLEG | RFTISRDNTKNT VYLQTPSLKPED TAVYYCAA | SRAYGSSRLRL ADTYDY | WGQGTQV TVSS |
| VEGFBII 42F11/**40** | EVQLVESGG GLVQPGDSL KLSCVASGR TSS | SYSV G | WFRQAQGKE REFVV | AISSGGYIY DSVSLQG | RFTISRDNTKNT VYLQTPSLKPED TAVYYCAA | SRAYGSSRLRL ADTYDY | WGQGTQV TVSS |
| VEGFBII 56E11/**41** | EVQLVESGG GLVQPGDSL KLSCAFSGR TFS | SYSM G | WFRQAQGKE REFVV | AISSSGGYI YDSVSLEG | RFTISRDNTKNT VYLQTPSLKPED AADYYCAA | SRAYGSSRLRL ADTYDY | WGQGTQV TVSS |
| VEGFBII 60A09/**42** | EVQLVESGG GLVQPGDSL KLSCAFSGR TFS | SYSM G | WFRQAQGKE REFVV | AISSSGGYI YDSVSLEG | RFTISRDNTRNT VYLQTPSLKPED TADYYCAA | SRAYGSSRLRL ADTYDY | WGQGTQV TVSS |
| VEGFBII 61A01/**43** | EVQLVESGG GLVQAGGSL RLSCAFSGR TFS | SYSM G | WFRQAQGKE REFVV | AISSGGYKY DAVSLEG | RFTISRDNTKNT VYLQTPSLKPED TAVYYCAA | SRAYASSRLRL ADTYDY | WGQGTQV TVSS |
| VEGFBII 62A09/**44** | EVQLVESGG DLVQPGDSL KLSCAASGR TFS | SYSM G | WFRQAQGKE REFVV | AISSSGGYI YDSVSLEG | RFTISRDNTKNT VYLQTPSLKPED TAVYYCAA | SRAYGSSRLRL ADTYDY | WGQGTQV TVSS |

(continued)

| VHH ID/ **SEQ ID NO:** | **FR1** | **CDR1** | **FR2** | **CDR2** | **FR3** | **CDR3** | **FR4** |
|---|---|---|---|---|---|---|---|
| VEGFBII 62D10/**45** | EVQLVESEG GLVQAGDSL RLSCAASGR TFS | SYSM G | WFRQAQGKE REFVV | AISSSGNYK YDSVSLEG | RFTISRDNTKNT VYLQINSLKPED TAVYYCAA | SRAYGSSRLRL GDTYDY | WGQGTQV TVSS |
| VEGFBII 62F02/**46** | EVQLVESGG GLVQPGDSL KLSCAFSGR TFS | SYSM G | WFRQAQGKE REFVV | AIASGGYIY DAVSLEG | RFTISRDNTKDT VYLQTPSLKPED TAVYYCAA | SRAYGSSRLRL ADTYDY | WGQGTQV TVSS |

Dissociation rates of inhibitory VHHs are analyzed on Biacore (Biacore T100 instrument, GE Healthcare). HBS-EP+ buffer is used as running buffer and experiments are performed at 25°C. Recombinant human VEGF165 is irreversibly captured on a CM5 sensor chip via amine coupling (using EDC and NHS) up to a target level of +/- 1500RU. After immobilization, surfaces are deactivated with 10 min injection of 1 M ethanolamine pH8.5. A reference surface is activated and deactivated with respectively EDC/NHS and ethanolamine. Periplasmic extracts of VHHs are injected at a 10-fold dilution in running buffer for 2 min at 45μl/min and allowed to dissociate for 10 or 15 min. Between different samples, the surfaces are regenerated with regeneration buffer. Data are double referenced by subtraction of the curves on the reference channel and of a blank running buffer injection. The of the processed curves is evaluated by fitting a two phase decay model in the Biacore T100 Evaluation software v2.0.1. Values for $k_d$-fast, $k_d$-slow and % fast are listed in Table 4.

**Table 4:** Off-rate determination of receptor-blocking VHHs with Biacore

| B-cell lineage | Unique sequence variant | Representative VHH ID | $k_d$(fast) | $k_d$(slow) | % fast | Binding level (RU) |
|---|---|---|---|---|---|---|
| 1 | 1 | VEGFBII22B07 | 1.50E-02 | 7.80E-05 | 31 | 328 |
| 1 | 2 | VEGFBII23A08 | 1.30E-02 | 5.00E-05 | 19 | 502 |
| 1 | 3 | VEGFBII23B04 | 8.80E-03 | 4.00E-05 | 12 | 768 |
| 1 | 4 | VEGFBII27D08 | 2.40E-02 | 8.10E-05 | 13 | 225 |
| 1 | 5 | VEGFBII24C04 | 1.30E-02 | 3.40E-05 | 17 | 456 |
| 1 | 6 | VEGFBII27G07 | 1.30E-02 | 3.80E-05 | 18 | 471 |
| 1 | 7 | VEGFBII22E04 | 1.80E-02 | 1.10E-04 | 14 | 520 |
| 1 | 8 | VEGFBII23A03 | 1.50E-02 | 3.20E-05 | 15 | 487 |
| 1 | 9 | VEGFBII22B06 | 3.80E-02 | 9.00E-05 | 23 | 168 |
| 1 | 10 | VEGFBII23A09 | 2.70E-02 | 4.60E-05 | 20 | 247 |
| 1 | 11 | VEGFBII23G03 | 2.80E-02 | 8.60E-05 | 28 | 141 |
| 1 | 12 | VEGFBII22A11 | 2.20E-02 | 4.70E-05 | 12 | 461 |
| 1 | 13 | VEGFBII23A06 | 1.70E-02 | 3.70E-05 | 13 | 547 |
| 1 | 14 | VEGFBII23F11 | 2.70E-02 | 1.30E-04 | 22 | 134 |
| 1 | 15 | VEGFBII22A10 | 3.70E-02 | 4.00E-05 | 19 | 229 |
| 1 | 16 | VEGFBII23F05 | 1.60E-02 | 1.30E-04 | 29 | 198 |
| 1 | 17 | VEGFBII23D11 | 1.90E-02 | 5.80E-05 | 13 | 510 |
| 1 | 18 | VEGFBII23F02 | n/d | n/d | n/d | n/d |
| 1 | 19 | VEGFBII23E05 | 1.50E-02 | 6.90E-05 | 18 | 275 |
| 1 | 20 | VEGFBII31C07 | 3.70E-02 | 1.50E-04 | 25 | 77 |
| 1 | 21 | VEGFBII30C09 | 1.50E-02 | 7.60E-05 | 19 | 264 |
| 1 | 22 | VEGFBII30E07 | 1.70E-02 | 1.30E-04 | 29 | 226 |
| 1 | 23 | VEGFBII39G04 | 1.40E-02 | 7.40E-04 | 40 | 210 |
| 1 | 24 | VEGFBII41G03 | 1.20E-02 | 2.70E-04 | 20 | 332 |
| 1 | 25 | VEGFBII41B05 | 1.90E-02 | 1.20E-04 | 16 | 324 |
| 1 | 26 | VEGFBII40F02 | 1.20E-02 | 9.80E-05 | 20 | 258 |
| 1 | 27 | VEGFBII39E02 | 1.90E-02 | 2.40E-04 | 13 | 181 |
| 1 | 28 | VEGFBII42D05 | 3.30E-02 | 1.50E-04 | 26 | 77 |
| 1 | 29 | VEGFBII40G07 | 1.80E-02 | 3.20E-04 | 19 | 139 |
| 1 | 30 | VEGFBII42A05 | 1.60E-02 | 3.40E-04 | 25 | 118 |

(continued)

| B-cell lineage | Unique sequence variant | Representative VHH ID | $k_d$(fast) | $k_d$(slow) | % fast | Binding level (RU) |
|---|---|---|---|---|---|---|
| 1 | 31 | VEGFBII42F11 | 9.10E-03 | 5.00E-04 | 46 | 100 |
| 1 | 32 | VEGFBII40H10 | 1.40E-02 | 2.90E-04 | 17 | 200 |
| 1 | 33 | VEGFBII62A09 | 4.10E-02 | 1.10E-04 | 23 | 84 |
| 1 | 34 | VEGFBII60A09 | 3.70E-02 | 9.30E-05 | 20 | 106 |
| 1 | 35 | VEGFBII62F02 | 1.40E-02 | 8.50E-05 | 21 | 205 |
| 1 | 36 | VEGFBII62D10 | 1.90E-02 | 1.60E-04 | 40 | 94 |
| 1 | 37 | VEGFBII61A01 | 7.40E-03 | 1.70E-04 | 21 | 275 |
| 1 | 38 | VEGFBII56E11 | 3.30E-02 | 1.40E-04 | 24 | 76 |
| n/d, not determined | | | | | | |

**Example 5**

**Characterization of purified VHHs**

Three inhibitory anti-VEGF VHHs are selected for further characterization as purified protein: VEGFBII23B04, VEGFBII24C4 and VEGFBII23A6. These VHHs are expressed in *E. coli* TG1 as c-myc, His6-tagged proteins. Expression is induced by addition of 1 mM IPTG and allowed to continue for 4 hours at 37°C. After spinning the cell cultures, periplasmic extracts are prepared by freeze-thawing the pellets. These extracts are used as starting material for VHH purification via IMAC and size exclusion chromatography (SEC). Final VHH preparations show 95% purity as assessed via SDS-PAGE.

*5.1 Evaluation of human VEGF165/VEGFR2 blocking VHHs in human VEGF165/human VEGFR2-Fc blocking ELISA*

The blocking capacity of the VHHs is evaluated in a human VEGF165/human VEGFR2-Fc blocking ELISA. In brief, 1 μg/mL of VEGFR2-Fc chimera (R&D Systems, Minneapolis, MN, USA) is coated in a 96-well MaxiSorp plate (Nunc, Wiesbaden, Germany). Dilution series (concentration range 1 mM - 64pM) of the purified VHHs in PBS buffer containing 0.1 % casein and 0.05% Tween 20 (Sigma) are incubated in the presence of 4 nM biotinlyated VEGF165. Residual binding of bio-VEGF165 to VEGFR2 is detected using horseradish peroxidase (HRP) conjugated extravidin (Sigma, St Louis, MO, USA) and TMB as substrate. As controls Bevacizumab (Avastin®) and Ranibizumab (Lucentis®) are taken along. Dose inhibition curves are shown in Figure 1; the corresponding $IC_{50}$ values and % inhibition are summarized in Table 5.

**Table 5:** $IC_{50}$ (nM) values and % inhibition for monovalent VHHs in hVEGF165/hVEGFR2-Fc competition ELISA

| VHH ID | $IC_{50}$ (nM) | % inhibition |
|---|---|---|
| VEGFBII23B04 | 2.1 | 100 |
| VEGFBII23A06 | 3.0 | 100 |
| VEGFBII24C04 | 2.5 | 100 |
| Ranibizumab | 1.6 | 100 |
| Bevacizumab | 1.7 | 100 |

*5.2 Evaluation of human VEGF165/VEGFR2 blocking VHHs in human VEGF165/human VEGFR1-Fc blocking ELISA*

VHHs are also evaluated in a human VEGF165/human VEGFR1-Fc blocking ELISA. In brief, 2 μg/mL of VEGFR1-Fc chimera (R&D Systems, Minneapolis, MN, USA) is coated in a 96-well MaxiSorp plate (Nunc, Wiesbaden, Germany). Dilution series (concentration range 1 mM - 64pM) of the purified VHHs in PBS buffer containing 0.1 % casein and 0.05% Tween 20 (Sigma) are incubated in the presence of 0.5nM biotinlyated VEGF165. Residual binding of bio-VEGF165 to VEGFR1 is detected using horseradish peroxidase (HRP) conjugated extravidin (Sigma, St Louis, MO, USA) and TMB as substrate. As controls Bevacizumab, Ranibizumab and an irrelevant VHH (2E6) are taken along. Dose inhibition curves are shown in Figure 2; the corresponding $IC_{50}$ values and % inhibition are summarized in Table 6.

**Table 6:** $IC_{50}$ (nM) values and % inhibition of monovalent VHHs in hVEGF165/hVEGFR1-Fc competition ELISA

| VHH ID | $IC_{50}$ (nM) | % inhibition |
|---|---|---|
| VEGFBII23B04 | 0.5 | 64 |
| VEGFBII23A06 | 0.9 | 55 |
| VEGFBII24C04 | 0.8 | 71 |
| Ranibizumab | 1.2 | 91 |
| Bevacizumab | 1.5 | 96 |

*5.3 Evaluation of the anti-VEGF165 VHHs in the human VEGF165/human VEGFR2-Fc blocking AlphaScreen*

The blocking capacity of the VHHs is also evaluated in a human VEGF165/human VEGFR2-Fc blocking AlphaScreen. Briefly, serial dilutions of purified VHHs (concentration range: 200 nM - 0.7 pM) in PBS buffer containing 0.03 % Tween 20 (Sigma) are added to 4pM bio-VEGF165 and incubated for 15 min. Subsequently VEGFR2-Fc (0.4 nM) and anti-Fc VHH-coated acceptor beads (20 μg/ml) are added and this mixture is incubated for 1 hour in the dark. Finally, streptavidin donor beads (20 μg/ml) are added and after 1 hour of incubation in the dark, fluorescence is measured on the Envision microplate reader. Dose-response curves are shown in the Figure 3. The $IC_{50}$ values for VHHs blocking the human VEGF165 - human VEGFR2-Fc interaction are summarized in Table 7.

**Table 7:** $IC_{50}$ (pM) values and % inhibition for VHHs in hVEGF165/hVEGFR2-Fc competition AlphaScreen

| VHH ID | $IC_{50}$ (pM) | % inhibition |
|---|---|---|
| VEGFBII23B04 | 160 | 100 |
| VEGFBII23A06 | 250 | 100 |
| VEGFBII24C04 | 250 | 100 |
| Ranibizumab | 860 | 100 |

*5.4 Evaluation of the anti-VEGF165 VHHs in the human VEGF165/human VEGFR1-Fc blocking AlphaScreen*

The blocking capacity of the VHHs is also evaluated in a human VEGF165/human VEGFR1-Fc blocking AlphaScreen. Briefly, serial dilutions of purified VHHs (concentration range: 500 nM - 1.8 pM)) in PBS buffer containing 0.03 % Tween 20 (Sigma) are added to 0.4 nM bio-VEGF165 and incubated for 15 min. Subsequently VEGFR1-Fc (1 nM) and anti-Fc VHH-coated acceptor beads (20 μg/ml) are added and this mixture is incubated for 1 hour in the dark. Finally, streptavidin donor beads (20 μg/ml) are added and after 1 hour of incubation in the dark, fluorescence is measured on the Envision microplate reader. Dose-response curves are shown in the Figure 4. The $IC_{50}$ values and % inhibition for VHHs blocking the human VEGF165 - human VEGFR1-Fc interaction are summarized in Table 8.

**Table 8:** $IC_{50}$ (nM) values for VHHs in hVEGF165/hVEGFR1-Fc competition AlphaScreen

| VHH ID | $IC_{50}$ (nM) | % inhibition |
|---|---|---|
| VEGFBII23B04 | 0.9 | 41 |
| VEGFBII23A06 | 0.4 | 46 |
| VEGFBII24C04 | 0.2 | 53 |
| Ranibizumab | 3.3 | 79 |

*5.5 Determination of the affinity of the human VEGF165 -VHH interaction*

Binding kinetics of VHH VEGFBII23B04 with hVEGF165 is analyzed by SPR on a Biacore T100 instrument.

Recombinant human VEGF165 is immobilized directly on a CM5 chip via amine coupling (using EDC and NHS). VHHs are analyzed at different concentrations between 10 and 360nM. Samples are injected for 2 min and allowed to dissociate up to 20 min at a flow rate of 45 μl/min. In between sample injections, the chip surface is regenerated with 100 mM HCl. HBS-EP+ (Hepes buffer pH7.4 + EDTA) is used as running buffer. Binding curves are fitted using a Two State Reaction model by Biacore T100 Evaluation Software v2.0.1. The calculated affinities of the anti-VEGF VHHs are listed in Table 9.

**Table 9:** Affinity $K_D$ (nM) of purified VHHs for recombinant human VEGF165

| | **VEGF165** | | | | | | |
|---|---|---|---|---|---|---|---|
| **VHH ID** | **$k_a$ ($M^{-1}.s^{-1}$)** | **$k_{a1}$ ($M^{-1}.s^{-1}$)** | **$k_{a2}$ ($M^{-1}.s^{-1}$)** | **$k_d$ ($s^{-1}$)** | **$k_{d1}$ ($s^{-1}$)** | **$k_{d2}$ ($s^{-1}$)** | **$K_D$ (nM)** |
| VEGFBII23B04[(a)] | - | 2.1E+05 | 1.4E-02 | - | 8.6E-03 | 2.4E-04 | 0.7 |
| VEGFBII23A06[(a)] | - | 4.2E+05 | 2.0E-02 | - | 5.7E-02 | 1.0E-04 | 0.7 |
| VEGFBII24C04[(a)] | - | 3.2E+05 | 1.8E-02 | - | 2.6E-02 | 9.6E-05 | 0.4 |
| [(a)] Heterogeneous binding curve resulting in no 1:1 fit, curves are fitted using a Two State Reaction model by Biacore T100 Evaluation Software v2.0.1 | | | | | | | |

*5.6 Binding to mouse VEGF164*

Cross-reactivity to mouse VEGF164 is determined using a binding ELISA. In brief, recombinant mouse VEGF164 (R&D Systems, Minneapolis, MS, USA) is coated overnight at 4°C at 1 μg/mL in a 96-well MaxiSorp plate (Nunc, Wiesbaden, Germany). Wells are blocked with a casein solution (1% in PBS). VHHs are applied as dilution series (concentration range: 500nM - 32pM) in PBS buffer containing 0.1 % casein and 0.05% Tween 20 (Sigma) and binding is detected using a mouse anti-myc (Roche) and an anti-mouse-HRP conjugate (DAKO) and a subsequent enzymatic reaction in the presence of the substrate TMB (3,3',5,5'-tetramentylbenzidine) (Pierce, Rockford, IL, USA) (Figures 5-1 and 5-2). A mouse VEGF164 reactive mAb is included as positive control. As reference, binding to human VEGF165 is also measured. $EC_{50}$ values are summarized in Table 10.

**Table 10:** $EC_{50}$ (pM) values for VHHs in a recombinant human VEGF165 and mouse VEGF164 binding ELISA

| | **rhVEGF165** | **rmVEGF164** |
|---|---|---|
| **VHH ID** | **$EC_{50}$ (pM)** | **$EC_{50}$ (pM)** |
| VEGFBII23B04 | 297 | NB |
| VEGFBII24C04 | 453 | NB |
| VEGFBII23A06 | 531 | NB |
| NB, no binding | | |

*5.7 Binding to VEGF121*

Binding to recombinant human VEGF121 is assessed via a solid phase binding ELISA. Briefly, recombinant human VEGF121 (R&D Systems, Minneapolis, MS, USA) is coated overnight at 4°C at 1 μg/mL in a 96-well MaxiSorp plate (Nunc, Wiesbaden, Germany). Wells are blocked with a casein solution (1% in PBS). VHHs are applied as dilution series (concentration range: 500nM - 32pM) in PBS buffer containing 0.1 % casein and 0.05% Tween 20 (Sigma) and binding is detected using a mouse anti-myc (Roche) and an anti-mouse-HRP conjugate (DAKO) and a subsequent enzymatic reaction in the presence of the substrate TMB (3,3',5,5'-tetramentylbenzidine) (Pierce, Rockford, IL, USA) (Figure 6). As positive control serial dilutions of the VEGFR2 is taken along. $EC_{50}$ values are summarized in Table 11.

**Table 11:** $EC_{50}$ (pM) values for monovalent VHHs in a recombinant human VEGF121 binding ELISA

| **VHH ID** | **$EC_{50}$ (pM)** |
|---|---|
| VEGFBII23B04 | 510 |
| VEGFBII24C04 | 792 |
| VEGFBII23A06 | 928 |

*5.8 Binding to VEGF family members VEGFB, VEGFC, VEGFD and PIGF*

Binding to VEGFB, VEGFC, VEGFD and PIGF is assessed via a solid phase binding ELISA. In brief, VEGFB, VEGFC, VEGFD and PIGF (R&D Systems, Minneapolis, MS, USA) are coated overnight at 4°C at 1 μg/mL in a 96-well MaxiSorp plate (Nunc, Wiesbaden, Germany). Wells are blocked with a casein solution (1% in PBS). VHHs are applied as dilution series (concentration range: 500nM - 32pM) and binding is detected using a mouse anti-myc (Roche) and an anti-mouse-AP conjugate (Sigma, St Louis, MO, USA). As positive controls serial dilutions of the appropriate receptors are taken along and detected with horseradish peroxidase (HRP)-conjugated goat anti-human IgG, Fc specific antibody (Jackson Immuno Research Laboratories Inc., West Grove, PA, USA) and a subsequent enzymatic reaction in the presence of the substrate TMB (3,3',5,5'-tetramentylbenzidine) (Pierce, Rockford, IL, USA). Dose-response curves of VHHs and controls are shown in Figures 7-1 through 7-4. The results show that there was no detectable binding of the selected VHHs to VEGFB, VEGFC, VEGFD or PIGF.

*5.9 Epitope binning*

Biacore-based epitope binning experiments are performed to investigate which VEGF binders bind to a similar or overlapping epitope as VEGFBII23B04. To this end, VEGFBII23B04 is immobilized on a CM5 sensor chip. For each sample, human VEGF165 is passed over the chip surface and reversibly captured by VEGFBII23B4. Purified VHHs (100 nM) or periplasmic extracts (1/10 diluted) are then injected with a surface contact time of 240 seconds and a flow rate of 10 uL/minute. Between different samples, the surface is regenerated with regeneration buffer (100 mM HCl). Processed curves are evaluated with Biacore T100 Evaluation software. VHHs could be divided within two groups: group one which gave additional binding to VEGFBII23B04 captured VEGF165 and a second group which is not able to simultaneously bind to VEGFBII23B04 captured VEGF165. Table 12-A summarizes the binding epitopes of the tested VHHs.

The same assay set-up is used to assess whether VEGFR1, VEGFR2, Ranibizumab and Bevacizumab are able to bind to human VEGF-165 simultaneously with VEGFBII23B04. Table 12-B presents the additional binding responses to VEGFBII23B04-captured VEGF165. Only VEGFR2 is not able to bind to VEGFBII23B04-captured VEGF165, underscoring the blocking capacity of VEGFBII23B04 for the VEGF-VEGFR2 interaction. In addition, these data show that the VEGFBII23B04 epitope is different from the Bevacizumab and Ranibizumab epitope.

**Table 12-A:** Epitope binning of anti-VEGF VHHs - simultaneous binding with VEGFBII23B04

| | | | | | | | | | | |
|---|---|---|---|---|---|---|---|---|---|---|
| No or low additional binding to 23B04-captured VEGF165* | 1C02 | 1E07 | 4B08 | 8E07 | 8F07 | 12A07 | 12B01 | 86C11 | 86F11 | 86G08 |
| | 86G10 | 86G11 | 87B07 | 88A01 | 88A02 | 88B02 | 88E02 | 88G03 | 88G05 | 88G11 |
| | 88H01 | 89B04 | 89D04 | 89F09 | 89G09 | 89H08 | 24C04 | 23A6 | 27G07 | 23B04 |
| Additional binding to 23B04-captured VEGF165 | 3D12 | 5B02 | 5B03 | 5B05 | 6G02 | 7D08 | 8D09 | 8F06 | 10C07 | 10E07 |
| | 10G04 | 10G05 | 11C08 | 11D09 | 11E04 | 11E05 | 11F12 | 86H09 | 41C05 | |
| * indicating same or overlapping epitopes | | | | | | | | | | |

**Table 12-B:** Epitope binning of VEGFBII23B04 - binding of benchmark inhibitors or cognate receptors on VEGFBII23B04 captured VEGF165

| Injection step | Binding | [sample] | Binding level (RU) |
|---|---|---|---|
| 1 | VEGF165 | 100 nM | 1727 |
| 2 | VEGFBII23B04 | 100 nM | - |
| 3 | Ranibizumab | 100 nM | 763 |
| 4 | Bevacizumab | 100 nM | 1349 |
| 5 | VEGFR1 | 100 nM | 1011 |
| 6 | VEGFR2 | 100 nM | - |

*5.10 Characterization of the anti-VEGF VHHs in the HUVEC proliferation assay*

The potency of the selected VHHs is evaluated in a proliferation assay. In brief, primary HUVEC cells (Technoclone) are supplement-starved over night and then 4000 cells/well are seeded in quadruplicate in 96-well tissue culture plates. Cells are stimulated in the absence or presence of VHHs with 33ng/mL VEGF. The proliferation rates are measured by [$^3$H] Thymidine incorporation on day 4. The results of the HUVEC proliferation assay are shown in Table.

**Table 13:** $IC_{50}$ (nM) values and % inhibition of monovalent VEGFBII23B04, VEGFBII23A06 and VEGFBII24C04 in VEGF HUVEC proliferation assay

| VHH ID | $IC_{50}$ (nM) | % inhibition |
|---|---|---|
| VEGFBII23B04 | 0.36 | 91 |
| Bevacizumab | 0.21 | 92 |
| VEGFBII23A06 | 4.29 | 73 |
| VEGFBII24C04 | 3.8 | 79 |
| Bevacizumab | 0.78 | 78 |

*5.11 Characterization of the anti-VEGF VHHs in the HUVEC Erk phosphorylation assay*

The potency of the selected VHHs is assessed in the HUVEC Erk phosphorylation assay. In brief, primary HUVE cells are serum-starved over night and then stimulated in the absence or presence of VHHs with 10ng/mL VEGF for 5 min. Cells are fixed with 4% Formaldehyde in PBS and ERK phosphorylation levels are measured by ELISA using phosphoERK-specific antibodies (anti-phosphoMAP Kinase pERK1&2, M8159, Sigma) and polyclonal Rabbit Anti-Mouse-Immunoglobulin-HRP conjugate (PO161, Dako). As shown in Table 14, VEGFBII23B04 and Bevacizumab inhibit the VEGF induced Erk phosphoryaltion by at least 90%, with $IC_{50}$s <1 nM.

**Table 14:** $IC_{50}$ (nM) values and % inhibition of monovalent VEGFBII23B04 in VEGF HUVEC Erk phosphorylation assay

| VHH ID | $IC_{50}$ (nM) | % inhibition |
|---|---|---|
| VEGFBII23B04 | 0.37 | 90 |
| Bevacizumab | 0.63 | 98 |

**Example 6**

**Generation of multivalent anti-VEGF blocking VHHs**

VHH VEGFBII23B04 is genetically fused to either VEGFBII23B04 resulting in a homodimeric VHH (AA sequence see Table 15) or different VEGF binding VHHs resulting in heterodimeric VHHs. To generate the heterodimeric VHHs, a panel of 10 unique VEGF binding VHHs are linked via a 9 or 40 Gly-Ser flexible linker in two different orientations to VEGFBII23B04 (AA sequences see Table 15). Homodimeric VEGFBII23B04 (VEGFB11010) and the 40 heterodimeric bivalent' VHHs are expressed in *E. coli* TG1 as c-myc, His6-tagged proteins. Expression is induced by addition of 1 mM IPTG and allowed to continue for 4 hours at 37°C. After spinning the cell cultures, periplasmic extracts are prepared by freeze-thawing the pellets. These extracts are used as starting material and VHHs are purified via IMAC and desalting

resulting in 90% purity as assessed via SDS-PAGE.

**Table 15:** Sequence ID, VHH ID and AA sequence of bivalent anti-VEGF VHHs (each of the used linkers is highlighted in one relevant sequence)

| Sequence ID/ SEQ ID NO: | VHH ID | AA sequence |
|---|---|---|
| VEGFBII23B04-35GS-23B04/**128** | VEGFBII010 | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGGSGGGGSGGGGSGGGGSGGGGSGGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII23B04-9GS-4B08/**129** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGSEVQLVESGGGLVQPGGSLRLSCAASGSAVGDITVAWYRQAPGIQRQLVATITPSGYTYYWDFVKGRFTISRDNSKNIVYLQMNSLKPEDTAAYYCNTQFYWGQGTQVTVSS |
| VEGFBII23B04-9GS-5B03/**130** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGSEVQLVESGGGLAQAGDSLRLSCAASGRSFSHYNMGWFRQAPGKEREFVASIRGGGGSTTYANSVKDRFTISRENAKNTVYLQMNSLKPEDTAVYYCAATAFYRGPYDYDYWGQGTQVTVSS |
| VEGFBII23B04-9GS-5B05/**131** | VEGFBII022 | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGSEVQLVESGGGLVQPGGSLRLSCVASGIRFMSMAWYRQAPGKHRELVARISSGGTTAYVDSVKGRFTISRDNSKNTVYLQMNSLKAEDTAVYYCNTFSSRPNPWGAGTQVTVSS |
| VEGFBII23B04-9GS-6G02/**132** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGSEVQLVESGGGLVQPGGSLRLSCAASGNIFSNNAMAWYRQAPGKQRELVARISSGGGFTYYLDSVKGRFTVSRDNAKNTVYLQMNSLKPEDTAVYYCNAAYRTYNYWGQGTQVTVSS |

(continued)

| Sequence ID/ SEQ ID NO: | VHH ID | AA sequence |
|---|---|---|
| VEGFBII23B04-9GS-10E07/**133** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGSEVQLVESGGGLVQAGGSLRLSCAASGRTFSNYAMGWFRQAPGKERVLVADISSSGINTYVADAVKGRFTISRDNAKNTVYLQMNSLKPEDTAVYYCAASAWWYSQMARDNYRYWGQGTQVTVSS |
| VEGFBII23B04-9GS-12B01/**134** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGSEVQLVESGGGLVQPGGSLRLACAASGFTLSSSWMYWVRQAPGKGLEWVSRISPGGLFTYYVDSVKGRFSVSTDNANNTLYLQMNSLKPEDTALYSCAKGGAPNYTPRGRGTQVTVSS |
| VEGFBII23B04-9GS-86C11/**135** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGSEVQLVESGGGLVQAGDSLRLSCTASGRTFNSYAMGWFRQAPGKERESVAHINRSGSSTYYADSVKGRFTISRDNAKNTVYLQLNSLKPEDTAVYYCAAGRYYSSDGVPSASFNYWGQGTQVTVSS |
| VEGFBII23B04-9GS-86H09/**136** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGSEVQLVESGGGLVQAGGSLRLSCTASGSAFKSYRMGWFRRTPGKEDEFVASISWTYGSTFYADSVKGRFTMSRDKAKNAGYLQMNSLKPEDTALYYCAAGAQSDRYNIRSYDYWGQGTQVTVSS |
| VEGFBII23B04-9GS-87B07/**137** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGSEVQLVESGGGLVQPGGSLKLSCTASGFTFSTSWMHWVRQAPGKGLEWVSSIPPVGHFANYAPSVKGRFTISRDNAKNTLFLQMNSLKSEDTAVYYCAKDSAGRTKGQGTQVTVSS |

(continued)

| Sequence ID/ SEQ ID NO: | VHH ID | AA sequence |
|---|---|---|
| VEGFBII23B04-9GS-88A01/**138** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGSEVQLVESGGGLVQAGGSLRLSCAASERTFSNYAMDWFRQAPGKEREFVAAITRSGGGTYYADSVKGRFTISRDNAKNTVYLQMNSLKPEDTAVYYCAATRSSTIVVGVGGMEYWGKGTQVTVSS |
| VEGFBII23B04-40GS-4B08/**139** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGGSGGGGSGGGGSGGGGSGGGGSGGGGSGGGGSEVQLVESGGGLVQPGGSLRLSCAASGSAVGDITVAWYRQAPGIQRQLVATITPSGYTYYWDFVKGRFTISRDNSKNIVYLQMNSLKPEDTAAYYCNTQFYWGQGTQVTVSS |
| VEGFBII23B04-40GS-5B03/**140** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGGSGGGGSGGGGSGGGGSGGGGSGGGGSGGGGSEVQLVESGGGLAQAGDSLRLSCAASGRSFSHYNMGWFRQAPGKEREFVASIRGGGGSTTYANSVKDRFTISRENAKNTVYLQMNSLKPEDTAVYYCAATAFYRGPYDYDYWGQGTQVTVSS |
| VEGFBII23B04-40GS-5B05/**141** | VEGFBII021 | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGGSGGGGSGGGGSGGGGSGGGGSGGGGSGGGGSEVQLVESGGGLVQPGGSLRLSCVASGIRFMSMAWYRQAPGKHRELVARISSGGTTAYVDSVKGRFTISRDNSKNTVYLQMNSLKAEDTAVYYCNTFSSRPNPWGAGTQVTVSS |
| VEGFBII23B04-40GS-6G02/**142** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGGSGGGGSGGGGSGGGGSGGGGSGGGGSGGGGSEVQLVESGGGLVQPGGSLRLSCAASGNIFSNNAMAWYRQAPGKQRELVARISSGGGFTYYLDSVKGRFTVSRDNAKNTVYLQMNSLKPEDTAVYYCNAAYRTYNYWGQGTQVTVSS |

(continued)

| Sequence ID/ SEQ ID NO: | VHH ID | AA sequence |
|---|---|---|
| VEGFBII23B04-40GS-10E07/**143** | VEGFBII023 | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGGSGGGGSGGGGSGGGGSGGGGSGGGGSGGGGSEVQLVESGGGLVQAGGSLRLSCAASGRTFSNYAMGWFRQAPGKERVLVADISSSGINTYVADAVKGRFTISRDNAKNTVYLQMNSLKPEDTAVYYCAASAWWYSQMARDNYRYWGQGTQVTVSS |
| VEGFBII23B04-40GS-12B01/**144** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGGSGGGGSGGGGSGGGGSGGGGSGGGGSGGGGSEVQLVESGGGLVQPGGSLRLACAASGFTLSSSWMYWVRQAPGKGLEWVSRISPGGLFTYYVDSVKGRFSVSTDNANNTLYLQMNSLKPEDTALYSCAKGGAPNYTPRGRGTQVTVSS |
| VEGFBII23B04-40GS-86C11/**145** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGGSGGGGSGGGGSGGGGSGGGGSGGGGSGGGGSEVQLVESGGGLVQAGDSLRLSCTASGRTFNSYAMGWFRQAPGKERESVAHINRSGSSTYYADSVKGRFTISRDNAKNTVYLQLNSLKPEDTAVYYCAAGRYYSSDGVPSASFNYWGQGTQVTVSS |
| VEGFBII23B04-40GS-86H09/**146** | VEGFBII024 | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGGSGGGGSGGGGSGGGGSGGGGSGGGGSGGGGSEVQLVESGGGLVQAGGSLRLSCTASGSAFKSYRMGWFRRTPGKEDEFVASISWTYGSTFYADSVKGRFTMSRDKAKNAGYLQMNSLKPEDTALYYCAAGAQSDRYNIRSYDYWGQGTQVTVSS |

(continued)

| Sequence ID/ SEQ ID NO: | VHH ID | AA sequence |
|---|---|---|
| VEGFBII23B04-40GS-87B07/**147** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGGSGGGGSGGGGSGGGGSGGGGSGGGGSGGGGSEVQLVESGGGLVQPGGSLKLSCTASGFTFSTSWMHWVRQAPGKGLEWVSSIPPVGHFANYAPSVKGRFTISRDNAKNTLFLQMNSLKSEDTAVYYCAKDSAGRTKGQGTQVTVSS |
| VEGFBII23B04-40GS-88A01/**148** | | EVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSSGGGGSGGGGSGGGGSGGGGSGGGGSGGGGSGGGGSGGGGSEVQLVESGGGLVQAGGSLRLSCAASERTFSNYAMDWFRQAPGKEREFVAAITRSGGGTYYADSVKGRFTISRDNAKNTVYLQMNSLKPEDTAVYYCAATRSSTIVVGVGGMEYWGKGTQVTVSS |
| VEGFBII4B08-9GS-23B04/**149** | | EVQLVESGGGLVQPGGSLRLSCAASGSAVGDITVAWYRQAPGIQRQLVATITPSGYTYYWDFVKGRFTISRDNSKNIVYLQMNSLKPEDTAAYYCNTQFYWGQGTQVTVSSGGGGSGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII5B03-9GS-23B04/**150** | | EVQLVESGGGLAQAGDSLRLSCAASGRSFSHYNMGWFRQAPGKEREFVASIRGGGGSTTYANSVKDRFTISRENAKNTVYLQMNSLKPEDTAVYYCAATAFYRGPYDYDYWGQGTQVTVSSGGGGSGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII5B05-9GS-23B04/**151** | | EVQLVESGGGLVQPGGSLRLSCVASGIRFMSMAWYRQAPGKHRELVARISSGGTTAYVDSVKGRFTISRDNSKNTVYLQMNSLKAEDTAVYYCNTFSSRPNPWGAGTQVTVSSGGGGSGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |

(continued)

| Sequence ID/ SEQ ID NO: | VHH ID | AA sequence |
|---|---|---|
| VEGFBII6G02-9GS-23B04/**152** | | EVQLVESGGGLVQPGGSLRLSCAASGNIFSNNAMAWYRQAPGKQRELVARISSGGGFTYYLDSVKGRFTVSRDNAKNTVYLQMNSLKPEDTAVYYCNAAYRTYNYWGQGTQVTVSSGGGGSGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII10E07-9GS-23B04/**153** | | EVQLVESGGGLVQAGGSLRLSCAASGRTFSNYAMGWFRQAPGKERVLVADISSSGINTYVADAVKGRFTISRDNAKNTVYLQMNSLKPEDTAVYYCAASAWWYSQMARDNYRYWGQGTQVTVSSGGGGSGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII12B01-9GS-23B04/**154** | | EVQLVESGGGLVQPGGSLRLACAASGFTLSSSWMYWVRQAPGKGLEWVSRISPGGLFTYYVDSVKGRFSVSTDNANNTLYLQMNSLKPEDTALYSCAKGGAPNYTPRGRGTQVTVSSGGGGSGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII86C11-9GS-23B04/**155** | | EVQLVESGGGLVQAGDSLRLSCTASGRTFNSYAMGWFRQAPGKERESVAHINRSGSSTYYADSVKGRFTISRDNAKNTVYLQLNSLKPEDTAVYYCAAGRYYSSDGVPSASFNYWGQGTQVTVSSGGGGSGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII86H09-9GS-23B04/**156** | | EVQLVESGGGLVQAGGSLRLSCTASGSAFKSYRMGWFRRTPGKEDEFVASISWTYGSTFYADSVKGRFTMSRDKAKNAGYLQMNSLKPEDTALYYCAAGAQSDRYNIRSYDYWGQGTQVTVSSGGGGSGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |

(continued)

| Sequence ID/ SEQ ID NO: | VHH ID | AA sequence |
|---|---|---|
| VEGFBII87B07-9GS-23B04/**157** | | EVQLVESGGGLVQPGGSLKLSCTASGFTFSTSWMHWVRQAPGKGLEWVSSIPPVGHFANYAPSVKGRFTISRDNAKNTLFLQMNSLKSEDTAVYYCAKDSAGRTKGQGTQVTVSSGGGGSGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII88A01-9GS-23B04/**158** | | EVQLVESGGGLVQAGGSLRLSCAASERTFSNYAMDWFRQAPGKEREFVAAITRSGGGTYYADSVKGRFTISRDNAKNTVYLQMNSLKPEDTAVYYCAATRSSTIVVGVGGMEYWGKGTQVTVSSGGGGSGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII4B08-40GS-23B04/**159** | | EVQLVESGGGLVQPGGSLRLSCAASGSAVGDITVAWYRQAPGIQRQLVATITPSGYTYYWDFVKGRFTISRDNSKNIVYLQMNSLKPEDTAAYYCNTQFYWGQGTQVTVSSGGGGSGGGGSGGGGSGGGGSGGGGSGGGGSGGGGSGGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII5B03-40GS-23B04/**160** | | EVQLVESGGGLAQAGDSLRLSCAASGRSFSHYNMGWFRQAPGKEREFVASIRGGGGSTTYANSVKDRFTISRENAKNTVYLQMNSLKPEDTAVYYCAATAFYRGPYDYDYWGQGTQVTVSSGGGGSGGGGSGGGGSGGGGSGGGGSGGGGSGGGGSGGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII5B05-40GS-23B04/**161** | | EVQLVESGGGLVQPGGSLRLSCVASGIRFMSMAWYRQAPGKHRELVARISSGGTTAYVDSVKGRFTISRDNSKNTVYLQMNSLKAEDTAVYYCNTFSSRPNPWGAGTQVTVSSGGGGSGGGGSGGGGSGGGGSGGGGSGGGGSGGGGSGGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |

(continued)

| Sequence ID/ SEQ ID NO: | VHH ID | AA sequence |
|---|---|---|
| VEGFBII6G02-40GS-23B04/**162** | | EVQLVESGGGLVQPGGSLRLSCAASGNIFSNNAMAWYRQAPGKQRELVARISSGGGFTYYLDSVKGRFTVSRDNAKNTVYLQMNSLKPEDTAVYYCNAAYRTYNYWGQGTQVTVSSGGGGSGGGGSGGGGSGGGGSGGGGSGGGGSGGGGSGGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII10E07-40GS-23B04/**163** | VEGFBII025 | EVQLVESGGGLVQAGGSLRLSCAASGRTFSNYAMGWFRQAPGKERVLVADISSSGINTYVADAVKGRFTISRDNAKNTVYLQMNSLKPEDTAVYYCAASAWWYSQMARDNYRYWGQGTQVTVSSGGGGSGGGGSGGGGSGGGGSGGGGSGGGGSGGGGSGGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII12B01-40GS-23B04/**164** | | EVQLVESGGGLVQPGGSLRLACAASGFTLSSSWMYWVRQAPGKGLEWVSRISPGGLFTYYVDSVKGRFSVSTDNANNTLYLQMNSLKPEDTALYSCAKGGAPNYTPRGRGTQVTVSSGGGGSGGGGSGGGGSGGGGSGGGGSGGGGSGGGGSGGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |
| VEGFBII86C11-40GS-23B04/**165** | | EVQLVESGGGLVQAGDSLRLSCTASGRTFNSYAMGWFRQAPGKERESVAHINRSGSSTYYADSVKGRFTISRDNAKNTVYLQLNSLKPEDTAVYYCAAGRYYSSDGVPSASFNYWGQGTQVTVSSGGGGSGGGGSGGGGSGGGGSGGGGSGGGGSGGGGSGGGGSEVQLVESGGGLVQTGDSLRLSCEVSGRTFSSYSMGWFRQAQGKEREFVVAISKGGYKYDSVSLEGRFTISKDNAKNTVYLQINSLKPEDTAVYYCASSRAYGSSRLRLADTYEYWGQGTQVTVSS |

(continued)

| Sequence ID/ SEQ ID NO: | VHH ID | AA sequence |
|---|---|---|
| VEGFBII86H09-40GS-23B04/**166** | | EVQLVESGGGLVQAGGSLRLSCTASGSAFKSYRMGWFRRTPGKEDEFVASISWTYGS TFYADSVKGRFTMSRDKAKNAGYLQMNSLKPEDTALYYCAAGAQSDRYNIRSYDYWG QGTQVTVSSGGGGSGGGGSGGGGSGGGGSGGGGSGGGGSGGGGSGGGGSEVQL VESGGGLVQTGDSLRLSCEVSGRTFSSYSMGWFRQAQGKEREFVVAISKGGYKYDSV SLEGRFTISKDNAKNTVYLQINSLKPEDTAVYYCASSRAYGSSRLRLADTYEYWGQGT QVTVSS |
| VEGFBII87B07-40GS-23B04/**167** | | EVQLVESGGGLVQPGGSLKLSCTASGFTFSTSWMHWVRQAPGKGLEWVSSIPPVGH FANYAPSVKGRFTISRDNAKNTLFLQMNSLKSEDTAVYYCAKDSAGRTKGQGTQVTVS SGGGGSGGGGSGGGGSGGGGSGGGGSGGGGSGGGGSGGGGSEVQLVESGGGLV QTGDSLRLSCEVSGRTFSSYSMGWFRQAQGKEREFVVAISKGGYKYDSVSLEGRFTIS KDNAKNTVYLQINSLKPEDTAVYYCASSRAYGSSRLRLADTYEYWGQGTQVTVSS |
| VEGFBII88A01-40GS-23B04/**168** | | EVQLVESGGGLVQAGGSLRLSCAASERTFSNYAMDWFRQAPGKEREFVAAITRSGGG TYYADSVKGRFTISRDNAKNTVYLQMNSLKPEDTAVYYCAATRSSTIVVGVGGMEYWG KGTQVTVSSGGGGSGGGGSGGGGSGGGGSGGGGSGGGGSGGGGSGGGGSEVQL VESGGGLVQTGDSLRLSCEVSGRTFSSYSMGWFRQAQGKEREFVVAISKGGYKYDSV SLEGRFTISKDNAKNTVYLQINSLKPEDTAVYYCASSRAYGSSRLRLADTYEYWGQGT QVTVSS |

The panel of 40 bivalent VHHs is tested in the VEGFR2 and VEGFR1 blocking AlphaScreen assay, as described in Example 5.3 and 5.4, respectively. Based on potency and maximum level of inhibition, the 5 best bivalent VHHs (VEGFBII021, VEGFBII022, VEGFBI023, VEGFBI024 and VEGFBII025) are chosen for further characterization. An overview of the screening results for the 5 selected bivalent VHHs in the competitive VEGFR2 and VEGFR1 AlphaScreen is shown in Table 16.

**Table 16:** Potency and efficacy of 5 best bivalent VHHs in the VEGF/VEGFR1 and VEGF/VEGFR2 competition AlphaScreen assay

| VHH ID | VEGFR2 | VEGFR1 | |
|---|---|---|---|
| | $IC_{50}$ (pM) | $IC_{50}$ (pM) | % inhibition |
| VEGFBII021 | 9 | 16 | 100 |
| VEGFBII022 | 7 | 8 | 100 |
| VEGFBII023 | 38 | 44 | 91 |
| VEGFBII024 | 12 | 46 | 100 |
| VEGFBII025 | 51 | 39 | 82 |

**Example 7**

**Characterization of formatted VHHs**

VHHs VEGFBII010, VEGFBII021, VEGFBII022, VEGFBII023, VEGFBII024 and VEGFBII025 are compared side-by-side in the VEGFR2 and VEGFR1 blocking ELISA (Figures 8-1 and 8-2 and 9, Table 17 and Table 18 respectively) and AlphaScreen assay (Figure 10 and 11, Table 19 and 20) as described in Examples 5.1, 5.2, 5.3 and 5.4, respectively.

**Table 17:** $IC_{50}$ (pM) values and % inhibition for formatted VHHs in hVEGF165/hVEGFR2-Fc competition ELISA

| VHH ID | $IC_{50}$ (pM) | % inhibition |
|---|---|---|
| VEGFBII010 | 49 | 100 |
| VEGFBII021 | 204 | 100 |
| VEGFBII022 | 164 | 100 |
| VEGFBII023 | 213 | 100 |
| VEGFBII024 | 292 | 100 |
| VEGFBII025 | 577 | 100 |
| Bevacizumab | 315 | 100 |
| Ranibizumab | 349 | 100 |

**Table 18:** $IC_{50}$ (pM) values and % inhibition of formatted VHHs in VEGF165/hVEGFR1-Fc competition ELISA

| VHH ID | $IC_{50}$ (pM) | % inhibition |
|---|---|---|
| VEGFBII010 | 73.5 | 67 |
| VEGFBII021 | 254 | 97 |
| VEGFBII022 | 225 | 89 |
| VEGFBII023 | 279 | 91 |
| VEGFBII024 | 326 | 92 |
| VEGFBII025 | 735 | 91 |
| Bevacizumab | 484 | 91 |
| Ranibizumab | 594 | 96 |

**Table 19:** $IC_{50}$ (pM) values and % inhibition for formatted VHHs in hVEGF165/hVEGFR2-Fc competition AlphaScreen

| VHH ID | $IC_{50}$ (pM) | % inhibition |
|---|---|---|
| VEGFBII010 | 16 | 100 |
| VEGFBII021 | 7 | 100 |
| VEGFBII022 | 7 | 100 |
| VEGFBII023 | 46 | 100 |
| VEGFBII024 | 50 | 100 |
| VEGFBII025 | 51 | 100 |
| Ranibizumab | 600 | 100 |

**Table 20:** $IC_{50}$ (pM) values and % inhibition of formatted VHHs in VEGF165/hVEGFR1-Fc competition AlphaScreen

| VHH ID | $IC_{50}$ (pM) | % inhibition |
|---|---|---|
| VEGFBII010 | 21 | 70 |
| VEGFBII021 | 12 | 100 |
| VEGFBII022 | 9 | 98 |
| VEGFBII023 | 48 | 87 |
| VEGFBII024 | 69 | 98 |
| VEGFBII025 | 71 | 82 |
| Ranibizumab | 1300 | 87 |

In addition, formatted VHHs are also tested for their capacity to block the mVEGF164/mVEGFR2-huFc interaction. In brief, serial dilutions of purified VHHs (concentration range: 4μM - 14.5 pM) in PBS buffer containing 0.03 % Tween 20 (Sigma) are added to 0.1 nM biotinylated mVEGF164 and incubated for 15 min. Subsequently mouse VEGFR2-huFc (0.1 nM) and anti-huFc VHH-coated acceptor beads (20 μg/ml) are added and this mixture is incubated for 1 hour. Finally, streptavidin donor beads (20 μg/ml) are added and after 1 hour of incubation fluorescence is measured on the Envision microplate reader. Dose-response curves are shown in Figure 12. The $IC_{50}$ values for VHHs blocking the mouse VEGF164/VEGFR2-hFC interaction are summarized in Table 21.

**Table 21:** $IC_{50}$ (pM) values and % inhibition for formatted VHHs in mVEGF164/mVEGFR2-hFc competition AlphaScreen

| VHH ID | $IC_{50}$ (nM) | % inhibition |
|---|---|---|
| VEGFBII022 | 108 | 100 |
| VEGFBII024 | - | - |
| mVEGF164 | 0.05 | 100 |
| Ranibizumab | - | - |

The formatted VHHs are also tested in ELISA for their ability to bind mVEGF164 and human VEGF165 (Example 5.6; Figures 13-1 and 13-2; Table 22); VEGF121 (Example 5.7; Figure 15; Table 23) and the VEGF family members VEGFB, VEGFC, VEGFD and PIGF (Example 5.8; Figures 14-1 through 14-8). Binding kinetics for human VEGF165 are analyzed as described in Example 5.5. The $K_D$ values are listed in Table 24.

**Table 22** $EC_{50}$ (pM) values for formatted VHHs in a recombinant human VEGF165 and mouse VEGF164 binding ELISA

| | **rhVEGF165** | **rmVEGF164** |
|---|---|---|
| **VHH ID** | **$EC_{50}$ (pM)** | **$EC_{50}$ (pM)** |
| VEGFBII010 | 428 | - |
| VEGFBII021 | 334 | 502 |
| VEGFBII022 | 224 | 464 |
| VEGFBII023 | 221 | - |
| VEGFBII024 | 320 | - |
| VEGFBII025 | 668 | - |

**Table 23:** $EC_{50}$ (pM) values for formatted VHHs in a recombinant human VEGF121 binding ELISA

| | **rhVEGF121** |
|---|---|
| **VHH ID** | **$EC_{50}$ (pM)** |
| VEGFBII010 | 920 |
| VEGFBII022 | 540 |
| VEGFBII024 | 325 |
| VEGFBII025 | 475 |

**Table 24:** Affinity $K_D$ (nM) of purified formatted VHHs for recombinant human VEGF165

| **VHH ID** | **$k_{a1}$ (1/MS)** | **$k_{d1}$ (1/s)** | **$k_{a2}$ (1/S)** | **$k_{d2}$ (1/s)** | **$K_D$ (nM)[(a)]** |
|---|---|---|---|---|---|
| VEGFBII010 [(b)] | 4.5E+05 | 1.7E-02 | 2.9E-02 | 1.3E-04 | 0.16 |
| VEGFBII021[(b)] | 1.2E+06 | 1.1E-02 | 2.3E-02 | 1.9E-04 | 0.07 |
| VEGFBII022[(b)] | 1.2E+06 | 9.1E-03 | 1.4E-02 | 2.6E-04 | 0.14 |
| VEGFBII023[(b)] | 3.0E+05 | 1.8E-02 | 2.4E-02 | 2.7E-04 | 0.69 |
| VEGFBII024[(b)] | 3.0E+05 | 1.3E-02 | 2.6E-02 | 2.8E-04 | 0.47 |
| VEGFBII025[(b)] | 3.3E+05 | 1.7E-02 | 1.8E-02 | 3.7E-04 | 1.1 |

[(a)] $K_D = k_{d1}/k_{a1} * (k_{d2}/(k_{d2}+k_{a2}))$ [(b)] Curves are fitted using a Two State Reaction model by Biacore T100 Evaluation Software v2.0.1

VHHs VEGFBII010, VEGFBII022, VEGFBII024 and VEGFBII025 are also tested in the VEGF-mediated HUVEC proliferation and Erk phosphorylation assay.

The potency of the selected formatted VHHs is evaluated in a proliferation assay. In brief, primary HUVEC cells (Technoclone) are supplement-starved over night and then 4000 cells/well are seeded in quadruplicate in 96-well tissue culture plates. Cells are stimulated in the absence or presence of VHHs with 33ng/mL VEGF. The proliferation rates are measured by [$^3$H] Thymidine incorporation on day 4. The results shown in Table 25 demonstrate that the formatted VHHs and Bevacizumab inhibit the VEGF-induced HUVEC proliferation by more than 90%, with $IC_{50}$s <1 nM.

**Table 25:** $IC_{50}$ (nM) values and % inhibition of formatted VHHs in VEGF HUVEC proliferation assay

| **VHH ID** | **$IC_{50}$ (nM)** | **% inhibition** |
|---|---|---|
| VEGFBII010 | 0.22 | 95 |
| VEGFBII021 | 0.40 | 98 |

(continued)

| VHH ID | $IC_{50}$ (nM) | % inhibition |
|---|---|---|
| VEGFBII022 | 0.34 | 100 |
| VEGFBII023 | 0.52 | 98 |
| VEGFBII024 | 0.38 | 96 |
| VEGFBII025 | 0.41 | 104 |
| Bevacizumab | 0.21 | 92 |

The potency of the selected formatted VHHs is assessed in the HUVEC Erk phosphorylation assay. In brief, primary HUVE cells are serum-starved over night and then stimulated in the absence or presence of VHHs with 10ng/mL VEGF for 5 min. Cells are fixed with 4% Formaldehyde in PBS and ERK phosphorylation levels are measured by ELISA using phosphoERK-specific antibodies (anti-phosphoMAP Kinase pERK1&2, M8159, Sigma) and polyclonal Rabbit Anti-Mouse-Immunoglobulin-HRP conjugate (PO161, Dako). As shown in Table 26, the formatted VHHs and Bevacizumab inhibit the VEGF induced Erk phosphoryaltion by more than 90%, with $IC_{50}$s <1 nM.

**Table 26:** $IC_{50}$ (nM) values and % inhibition of formatted VHHs in VEGF HUVEC Erk phosphorylation assay

| VHH ID | $IC_{50}$ (nM) | % inhibition |
|---|---|---|
| VEGFBII010 | 0.19 | 92 |
| VEGFBII021 | 0.21 | 103 |
| VEGFBII022 | 0.18 | 94 |
| VEGFBII023 | 0.25 | 100 |
| VEGFBII024 | 0.23 | 94 |
| VEGFBII025 | 0.23 | 99 |
| Bevacizumab | 0.63 | 98 |

**Example 8**

**Sequence optimization**

*8.1 Sequence optimization of VEGFBII23B04*

The amino acid sequence of VEGFBII23B04 is aligned to the human germline sequence VH3-23/JH5, see Figure 16 (SEQ ID NO: 179)

The alignment shows that VEGFBII23B04 contains 19 framework mutations relative to the reference germline sequence. Non-human residues at positions 14, 16, 23, 24, 41, 71, 82, 83 and 108 are selected for substitution with their human germline counterparts. A set of 8 VEGFBII23B04 variants is generated carrying different combinations of human residues at these positions (AA sequences are listed in Table 27). One additional variant is constructed in which the potential isomerization site at position D59S60 (CDR2 region, see Figure 16, indicated as bold italic residues) is removed by introduction of a S60A mutation.

**Table 27:** AA sequence of sequence-optimized variants of VHH VEGFBII23B04 (FR, framework; CDR, complementary determining region)

| VHH ID/ SEQ ID NO: | FR1 | CDR1 | FR2 | CDR2 | FR3 | CDR3 | FR4 |
|---|---|---|---|---|---|---|---|
| VEGFBII 111D05/**47** | EVQLVESGG GLVQTGGSL RLSCEASGR TFS | SYSM G | WFRQAPGKER EFVV | AISKGGY KYDSVS LEG | RFTISRDNAKNTVYL QINSLRPEDTAVYYC AS | SRAYGS SRLRLA DTYEY | WGQGTLVT VSS |
| VEGFBII 111G06/**48** | EVQLVESGG GLVQPGGSL RLSCAASGR TFS | SYSM G | WFRQAPGKER EFVV | AISKGGY KYDSVS LEG | RFTISRDNAKNTVYL QMNSLRPEDTAVYYC AS | SRAYGS SRLRLA DTYEY | WGQGTLVT VSS |
| VEGFBII 112D11/**49** | EVQLVESGG GLVQPGGSL RLSCEASGR TFS | SYSM G | WFRQAPGKER EFVV | AISKGGY KYDSVS LEG | RFTISRDNAKNTVYL QINSLRPEDTAVYYC AS | SRAYGS SRLRLA DTYEY | WGQGTLVT VSS |
| VEGFBII 113A08/**50** | EVQLVESGG GLVQTGGSL RLSCEVSGR TFS | SYSM G | WFRQAPGKER EFVV | AISKGGY KYDSVS LEG | RFTISKDNAKNTVYLQ INSLRPEDTAVYYCAS | SRAYGS SRLRLA DTYEY | WGQGTLVT VSS |
| VEGFBII 113E03/**51** | EVQLVESGG GLVQTGDSL RLSCEVSGR TFS | SYSM G | WFRQAQGKER EFVV | AISKGGY KYDSVS LEG | RFTISKDNAKNTVYLQ MNSLRPEDTAVYYCA S | SRAYGS SRLRLA DTYEY | WGQGTLVT VSS |
| VEGFBII 114C09/**52** | EVQLVESGG GLVQPGDSL RLSCEVSGR TFS | SYSM G | WFRQAPGKER EFVV | AISKGGY KYDSVS LEG | RFTISKDNAKNTVYLQ INSLRPEDTAVYYCAS | SRAYGS SRLRLA DTYEY | WGQGTLVT VSS |

(continued)

| VHH ID/ SEQ ID NO: | FR1 | CDR1 | FR2 | CDR2 | FR3 | CDR3 | FR4 |
|---|---|---|---|---|---|---|---|
| VEGFBII 114D02/**53** | EVQLVESGG GLVQTGGSL RLSCEVSGR TFS | SYSM G | WFRQAPGKER EFVV | AISKGGY KYDSVS LEG | RFTISRDNAKNTVYL QINSLRPEDTAVYYC AS | SRAYGS SRLRLA DTYEY | WGQGTLVT VSS |
| VEGFBII 114D03/**54** | EVQLVESGG GLVQTGDSL RLSCAVSGR TFS | SYSM G | WFRQAQGKER EFVV | AISKGGY KYDSVS LEG | RFTISKDNAKNTVYLQ INSLRPEDTAVYYCAS | SRAYGS SRLRLA DTYEY | WGQGTLVT VSS |
| VEGFBII 118E10/**55** | EVQLVESGG GLVQTGDSL RLSCEVSGR TFS | SYSM G | WFRQAQGKER EFVV | AISKGGY KYDAVS LEG | RFTISKDNAKNTVYLQ INSLKPEDTAVYYCAS | SRAYGS SRLRLA DTYEY | WGQGTQVT VSS |

These variants are characterized as purified proteins in the VEGF165/VEGFR2 AlphaScreen (Example 5.3, Figure 17). The melting temperature ($T_m$) of each clone is determined in a thermal shift assay, which is based on the increase in fluorescence signal upon incorporation of Sypro Orange (Invitrogen) (Ericsson et al, Anal. Biochem. 357 (2006), pp289-298). All variants displayed comparable $IC_{50}$ when compared to VEGFBII23B04 and $T_m$ values which are similar or higher when compared to the parental VEGFBII23B04. Table 28 summarizes the $IC_{50}$ values and $T_m$ values at pH 7 for the 9 clones tested.

**Table 28:** $IC_{50}$ (pM) values, % inhibition and melting temperature (@pH 7) of sequence-optimized variants of VEGFBII23B04

| **VHH ID** | **$IC_{50}$ (pM)** | **% inhibition** | **$T_m$ @ pH 7 (°C)** |
|---|---|---|---|
| *VEGFBII23B04 (wt)* | *169* | *100* | *63* |
| VEGFBII111D05 | 209 | 100 | 68 |
| VEGFBII111G06 | 366 | 100 | 71 |
| VEGFBII112D11 | 221 | 100 | 70 |
| VEGFBII113A08 | 253 | 100 | 69 |
| VEGFBII113E03 | 290 | 100 | 68 |
| VEGFBII114C09 | 215 | 100 | 71 |
| VEGFBII114D02 | 199 | 100 | 74 |
| VEGFBII114D03 | 227 | 100 | 64 |
| VEGFBII118E10 | 189 | 100 | 62 |

In a second cycle, tolerated mutations from the humanization effort (VEGFBII111G06) and mutations to avoid potential posttranslational modification at selected sites (the D16G, the S60A substitution and an E1 D mutation) are combined resulting in a sequence-optimized clone derived from VEGFBII23B04: VEGFBII0037. One extra sequence-optimized variant (VEGFBII038) is anticipated which contains the same substitutions as VEGFBII0037, with the exception of the 182M mutation, as this mutation may be associated with a minor drop in potency. The sequences from both sequence-optimized clones are listed in Table 29. VEGFBII0037 and VEGFBII0038 are characterized in the VEGF165/VEGFR2 blocking AlphaScreen (Example 5.3, Figure 18), the melting temperature is determined in the thermal shift assay as described above and the affinity for binding on VEGF165 is determined in Biacore (Example 5.5). An overview of the characteristics of the 2 sequence-optimized VHHs is presented in Table 30.

**Table 29:** AA sequences of sequence-optimized variants of VHH VEGFBII23B04

| VHH ID/ SEQ ID NO: | FR 1 | CDR 1 | FR2 | CDR 2 | FR3 | CDR 3 | FR 4 |
|---|---|---|---|---|---|---|---|
| VEGFBII037 **56** | DVQLV ESGG GLVQP GGSL RLSCA ASGRT FS | SYSMG | WFRQ APGKE REFVV | AISKGG YKYDAV SLEG | RFTISRD NAKNTVY LQMNSL RPEDTAV YYCAS | SRAYGS SRLRLA DTYEY | WGQGT LVTVSS |
| VEGFBII038 **57** | DVQLV ESGG GLVQP GGSL RLSCA ASGRT FS | SYSMG | WFRQ APGKE REFVV | AISKGG YKYDAV SLEG | RFTISRD NAKNTVY LQINSLR PEDTAVY YCAS | SRAYGS SRLRLA DTYEY | WGQGT LVTVSS |

**Table 30:** $IC_{50}$ (pM) values, % inhibition, melting temperature (@pH 7) and affinity (pM) of sequence-optimized clones VEGFBII037 and VEGFBII038

| VHH ID | $IC_{50}$ (pM) | % inhibition | $T_m$ (°C) @ pH 7 | $K_D$ (pM) |
|---|---|---|---|---|
| *VEGFBII23B04* | *152* | *100* | *63* | *560* |
| VEGFBII037 | 300 | 100 | 72 | 270 |
| VEGFBII038 | 143 | 100 | 71 | 360 |

*8.2 Sequence optimization of VEGFBII5B05*

The amino acid sequence of VEGFBII5B05 is aligned to the human germline sequence VH3-23/JH5, see Figure 19 (SEQ ID:NO: 179 The alignment shows that VEGFB115B05 contains 15 framework mutations relative to the reference germline sequence. Non-human residues at positions 23, 60, 83, 105, 108 are selected for substitution with their human germline counterparts while the histidine at position 44 is selected for substitution by glutamine. One humanization variant is constructed carrying the 6 described mutations (AA sequence is listed in Table 31).

**Table 31:** AA sequences of sequence-optimized variants of VHH VEGFBII5B05 (FR, framework; CDR, complementary determining region)

| VHH ID/ SEQ ID NO: | FR1 | CDR1 | FR2 | CDR2 | FR3 | CDR3 | FR4 |
|---|---|---|---|---|---|---|---|
| VEGFBII119G11/ **125** | EVQLVES GGGLVQ PGGSLRL SCAASGI RFM | SMA | WYRQA PGKQR ELVA | RISSG GTTAY ADSVK G | RFTISRD NSKNTVY LQMNSL RAEDTAV YYCNT | FSSRP NP | WGQ GTLV TVSS |
| VEGFBII120E10/ **126** | EVQLVES GGGLVQ PGGSLRL SCVASGI RFI | SMA | WYRQA PGKHR ELVA | RISSG GTTAY VDSVK G | RFTISRD NSKNTVY LQMNSLK AEDTAVY YCNT | FSSRP NP | WGA GTQV TVSS |

One additional variant is constructed in which the potential oxidation site at position M30 (CDR1 region, see Figure 19 indicated as bold italic residue) is removed by introduction of a M301 mutation. Both variants are tested for their ability to bind hVEGF165 using the ProteOn. In brief, a GLC ProteOn Sensor chip is coated with human VEGF165. Periplasmic extracts of the variants are diluted 1/10 and injected across the chip coated with human VEGF165. Off-rates are calculated and compared to the off-rates of the parental VEGFBII5B05. Off-rates from the 2 variants are in the same range as the off-rates from the parental VEGFBII5B05 indicating that all mutations are tolerated (Table 32).

**Table 32:** Off-rates sequence-optimized variants VEGFBII5B05

| **VHH ID** | **binding level (RU)** | **$k_d$ (1/s)** |
|---|---|---|
| *VEGFBII5B05* | *242* | *6.15E-02* |
| VEGFBII119G11 | 234 | 7.75E-02 |
| VEGFBII120E10 | 257 | 4.68E-02 |

In a second cycle, mutations from the humanization effort and the M301 substitution are combined resulting in a sequence-optimized clone of VEGFBII5B05, designated VEGFBII032. The sequence is listed in Table 33. Affinity of VEGFBII032 is determined by Biacore (see Example 5.5) and the melting temperature is determined in the thermal shift assay as described above. An overview of the characteristics of the sequence-optimized VHH VEGFBII032 is presented in Table 34.

**Table 33:** AA sequence of sequence-optimized clone VEGFBII032 (FR, framework; CDR, complementary determining region)

| VHH ID/ SEQ ID NO: | FR1 | CDR1 | FR2 | CDR2 | FR3 | CDR3 | FR4 |
|---|---|---|---|---|---|---|---|
| VEGFBII032/ **127** | EVQLVE SGGGLV QPGGSL RLSCAA SGIRFI | SMA | WYRQA PGKQR ELVA | RISSG GTTA YADS VKG | RFTISRDNSK NTVYLQMNS LRAEDTAVY YCNT | FSSR PNP | WGQGTL VTVSS |

**Table 34:** Melting temperature (@pH 7) and affinity (nM) of sequence-optimized clone VEGFBII032

| **VHH ID** | **$T_m$ (°C) @pH7** | **$K_D$ (nM)** |
|---|---|---|
| *VEGFBII5B05(wt)* | *69* | *32* |
| VEGFBII0032 | 71 | 44 |

The potency of the sequence-optimized clones VEGFBII037 and VEGFBII038 is evaluated in a proliferation assay. In brief, primary HUVEC cells (Technoclone) are supplement-starved over night and then 4000 cells/well are seeded in quadruplicate in 96-well tissue culture plates. Cells are stimulated in the absence or presence of VHHs with 33ng/mL VEGF. The proliferation rates are measured by [$^3$H] Thymidine incorporation on day 4. The results shown in Table 35, demonstrate that the activity (potency and degree of inhibition) of the parental VHH VEGFBII23B04 is conserved in the sequence optimized clone VEGFBII038.

**Table 35:** $IC_{50}$ (nM) values and % inhibition of the sequence optimized clones VEGFBII037 and VEGFBII038 in VEGF HUVEC proliferation assay

| **VHH ID** | **$IC_{50}$ (nM)** | **% inhibition** |
|---|---|---|
| VEGFBII23B04 | 0.68 | 92 |
| VEGFBII037 | 1.54 | 78 |
| VEGFBII038 | 0.60 | 92 |
| Bevacizumab | 0.29 | 94 |

## Claims

**1.** A VEGF-binding molecule comprising at least one VHH having a sequence selected from SEQ ID NO: 10-46.

**2.** A VEGF-binding molecule according to claim 1 comprising at least one VHH having a sequence of SEQ ID NO: 18.

**3.** A nucleic acid molecule encoding a VEGF-binding molecule of claims 1 or 2.

**4.** A vector containing the nucleic acid of claim 3.

**5.** A host cell containing the vector of claim 4.

**6.** A pharmaceutical composition containing at least one VEGF-binding molecule of claims 1 or 2 as the active ingredient.

**7.** Use of a pharmaceutical composition according to claim 6 as a medicament.

**8.** Use of a pharmaceutical composition according to claim 6 for treating cancer.

**9.** Use according to claim 8, wherein the cancer is selected from squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, gastric cancer, melanoma, and various types of head and neck cancer.

**10.** Use of a pharmaceutical composition according to claim 6 for treating non-neoplastic disorders selected from undesired or aberrant hypertrophy, arthritis, rheumatoid arthritis (RA), psoriasis, psoriatic plaques, sarcoidosis, atherosclerosis, atherosclerotic plaques, diabetic and other proliferative retinopathies including retinopathy of prematurity, retrolental fibroplasia, vascular restenosis, arteriovenous malformations (AVM), meningioma, hemangioma, angiofibroma, thyroid hyperplasias (including Grave's disease), corneal and other tissue transplantation, chronic inflammation, lung inflammation, acute lung injury/ ARDS, sepsis, primary pulmonary hypertension, malignant pulmonary effusions, cerebral edema (e.g., associated with acute stroke/ closed head injury/ trauma), synovial inflammation, pannus formation in RA, myositis ossificans, hypertropic bone formation, osteoarthritis (OA), refractory ascites, polycystic ovarian disease, endometriosis, 3rd spacing of fluid diseases (pancreatitis, compartment syndrome, burns, bowel disease), uterine fibroids, premature labor, chronic inflammation such as IBD (Crohn's disease and ulcerative colitis), renal allograft rejection, inflammatory bowel disease, nephrotic syndrome, undesired or aberrant tissue mass growth (non-cancer), hemophilic joints, hypertrophic scars, inhibition of hair growth, Osier-Weber syndrome, pyogenic granuloma retrolental fibroplasias, scleroderma, trachoma, vascular adhesions, synovitis, dermatitis, preeclampsia, ascites, pericardial effusion (such as that associated with pericarditis), and pleural effusion.

**11.** Use of a pharmaceutical composition according to claim 6 for treating eye diseases.

**12.** Use according to claim 11, wherein the eye disease is selected from neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease.

**Fig. 1**

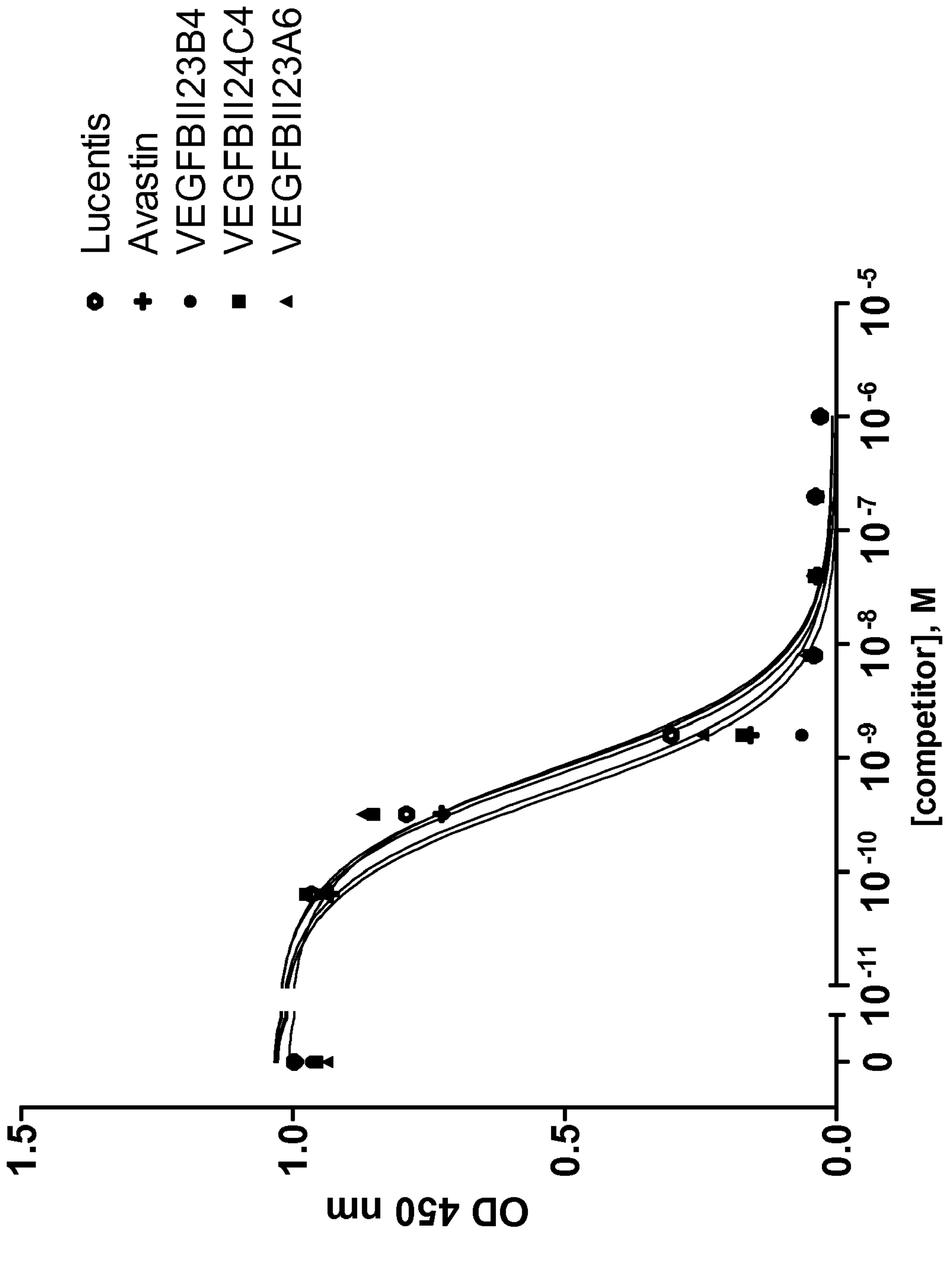
Lucentis
Avastin
VEGFBII23B4
VEGFBII24C4
VEGFBII23A6
OD 450 nm
[competitor], M
1.5
1.0
0.5
0.0
0
$10^{-11}$
$10^{-10}$
$10^{-9}$
$10^{-8}$
$10^{-7}$
$10^{-6}$
$10^{-5}$

Fig. 2

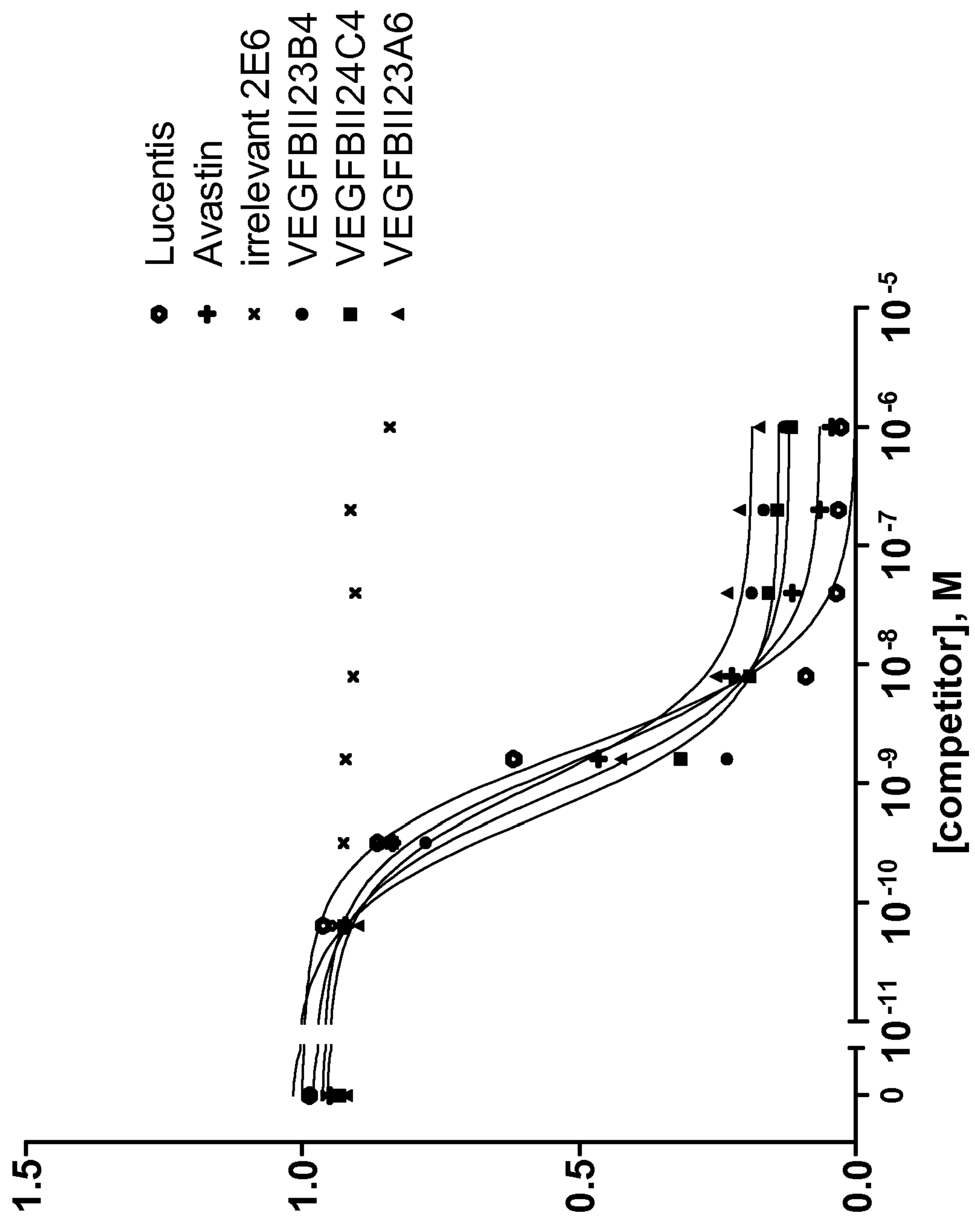
Lucentis
Avastin
irrelevant 2E6
VEGFBII23B4
VEGFBII24C4
VEGFBII23A6
OD 450 nm
1.5
1.0
0.5
0.0
0
10^-11
10^-10
10^-9
10^-8
10^-7
10^-6
10^-5
[competitor], M

**Fig. 3**

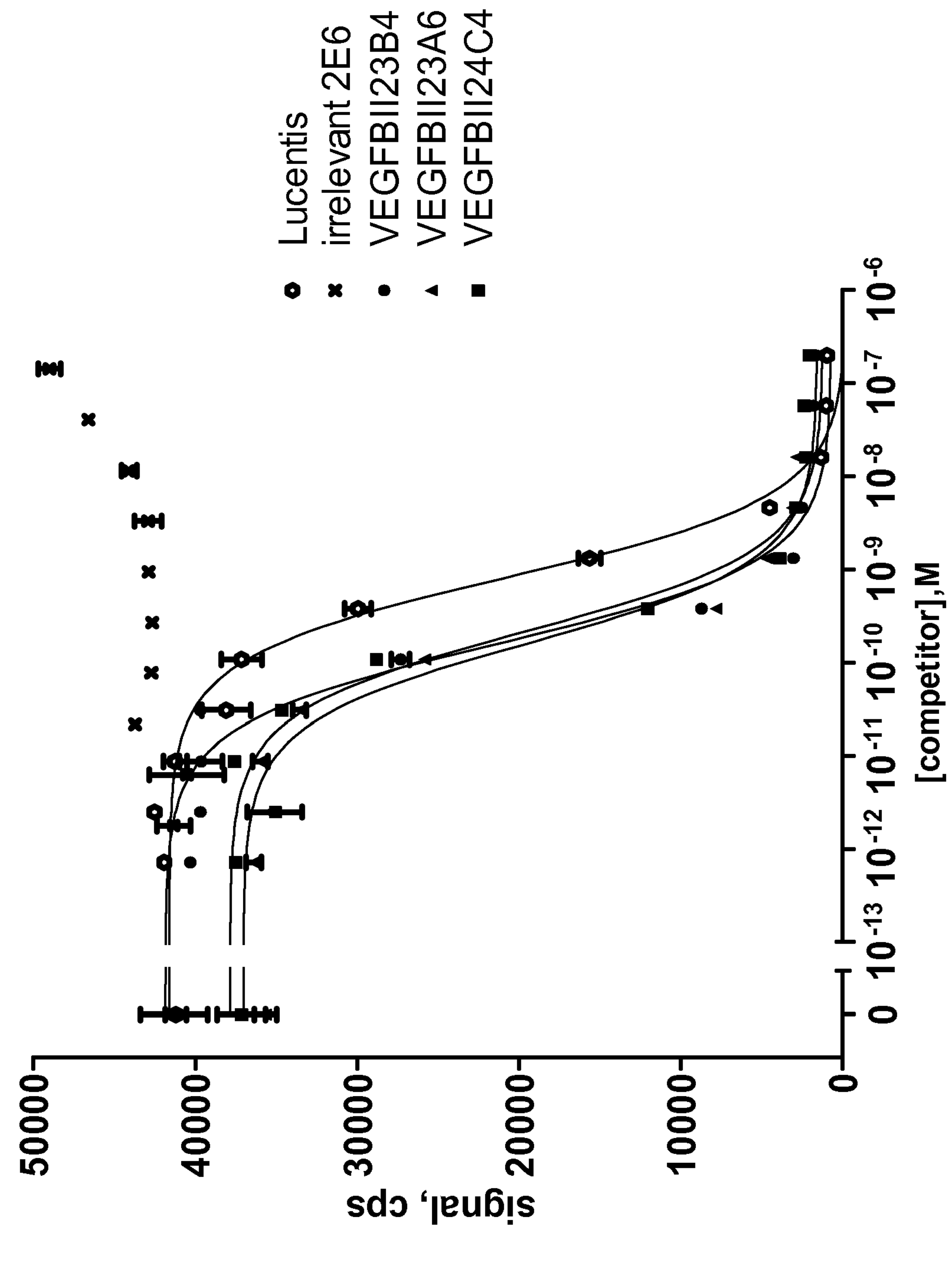
signal, cps
50000
40000
30000
20000
10000
0
0
$10^{-13}$
$10^{-12}$
$10^{-11}$
$10^{-10}$
$10^{-9}$
$10^{-8}$
$10^{-7}$
$10^{-6}$
[competitor],M
Lucentis
irrelevant 2E6
VEGFBII23B4
VEGFBII23A6
VEGFBII24C4

**Fig. 4**

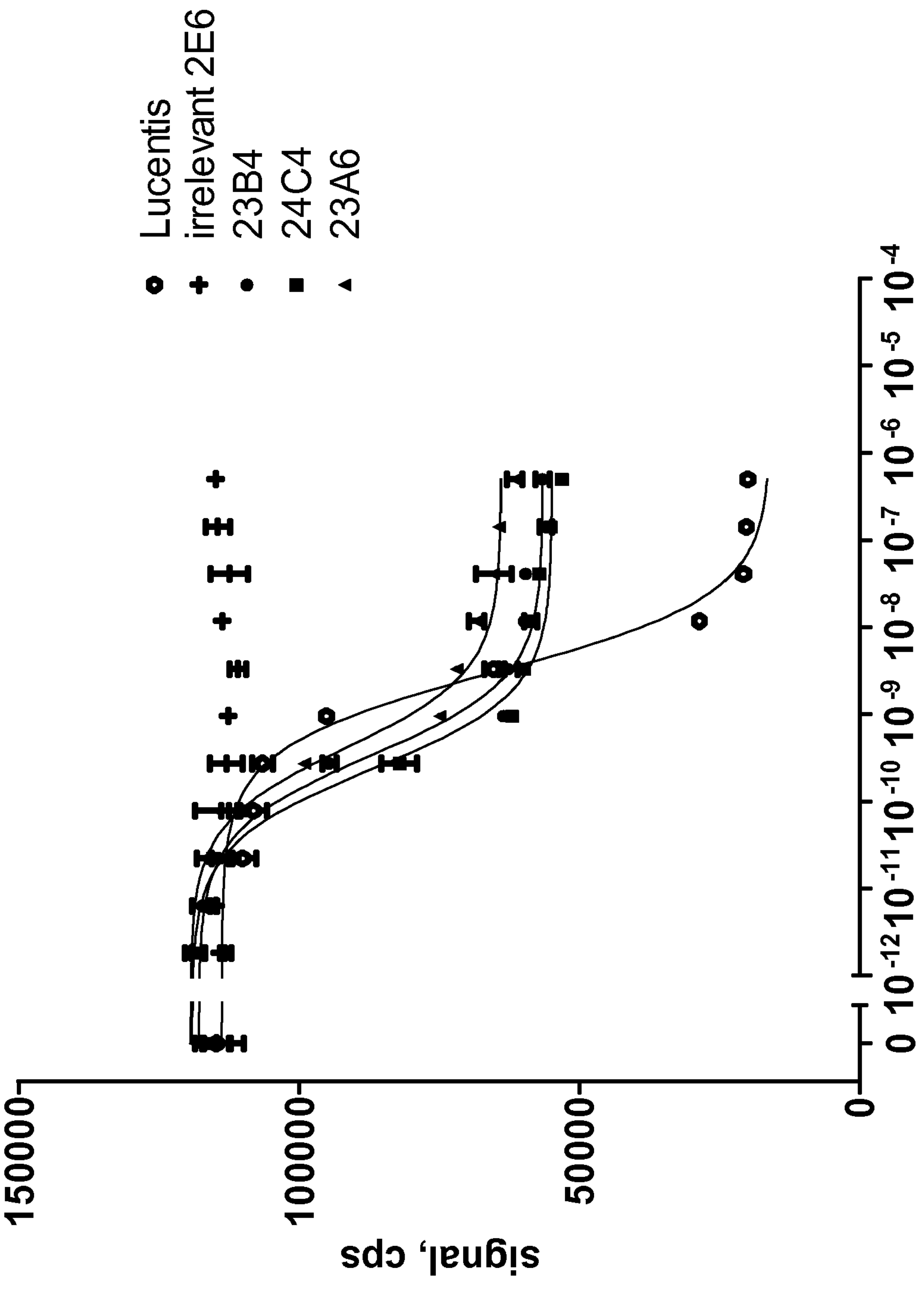
Lucentis
irrelevant 2E6
23B4
24C4
23A6
signal, cps
150000
100000
50000
0
[competitor],M
0
$10^{-12}$
$10^{-11}$
$10^{-10}$
$10^{-9}$
$10^{-8}$
$10^{-7}$
$10^{-6}$
$10^{-5}$
$10^{-4}$

**Fig. 5-1**

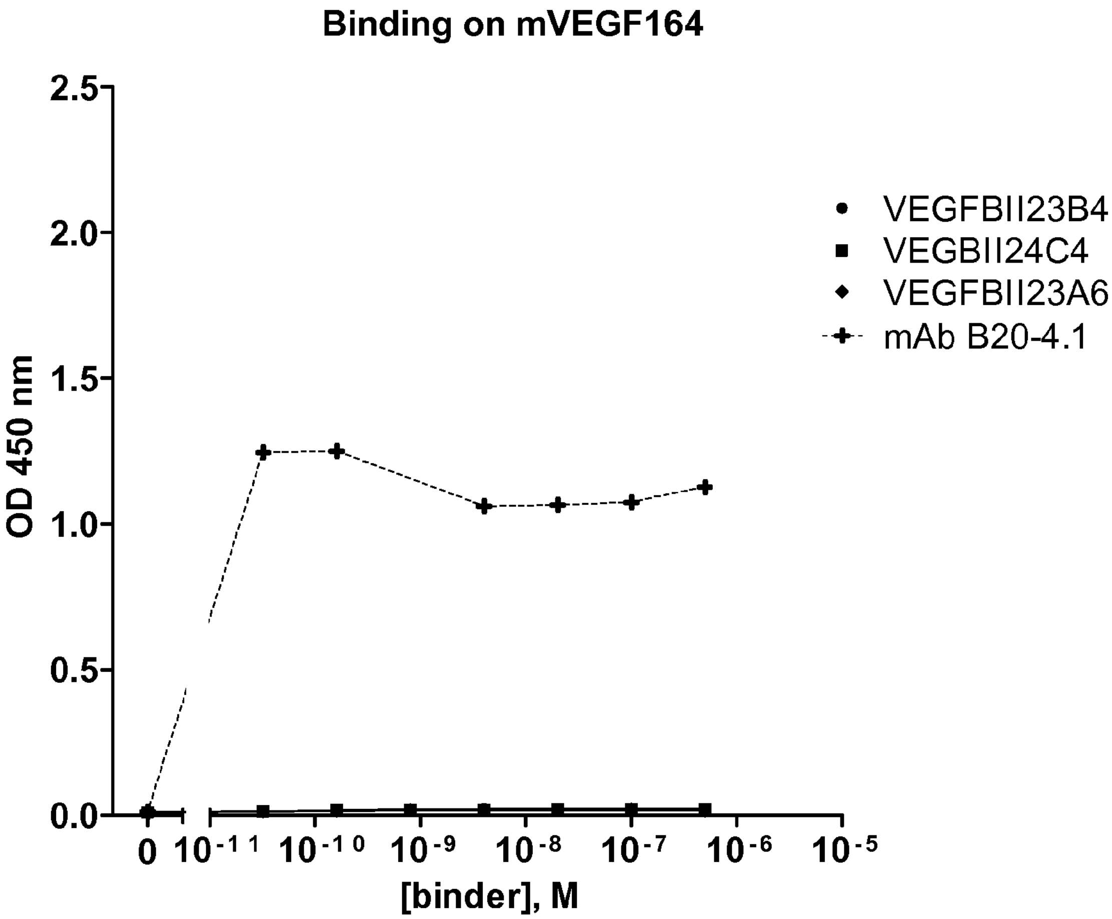
Binding on mVEGF164
2.5
2.0
1.5
1.0
0.5
0.0
OD 450 nm
VEGFBII23B4
VEGBII24C4
VEGFBII23A6
mAb B20-4.1
0
10-11
10-10
10-9
10-8
10-7
10-6
10-5
[binder], M

**Fig. 5-2**

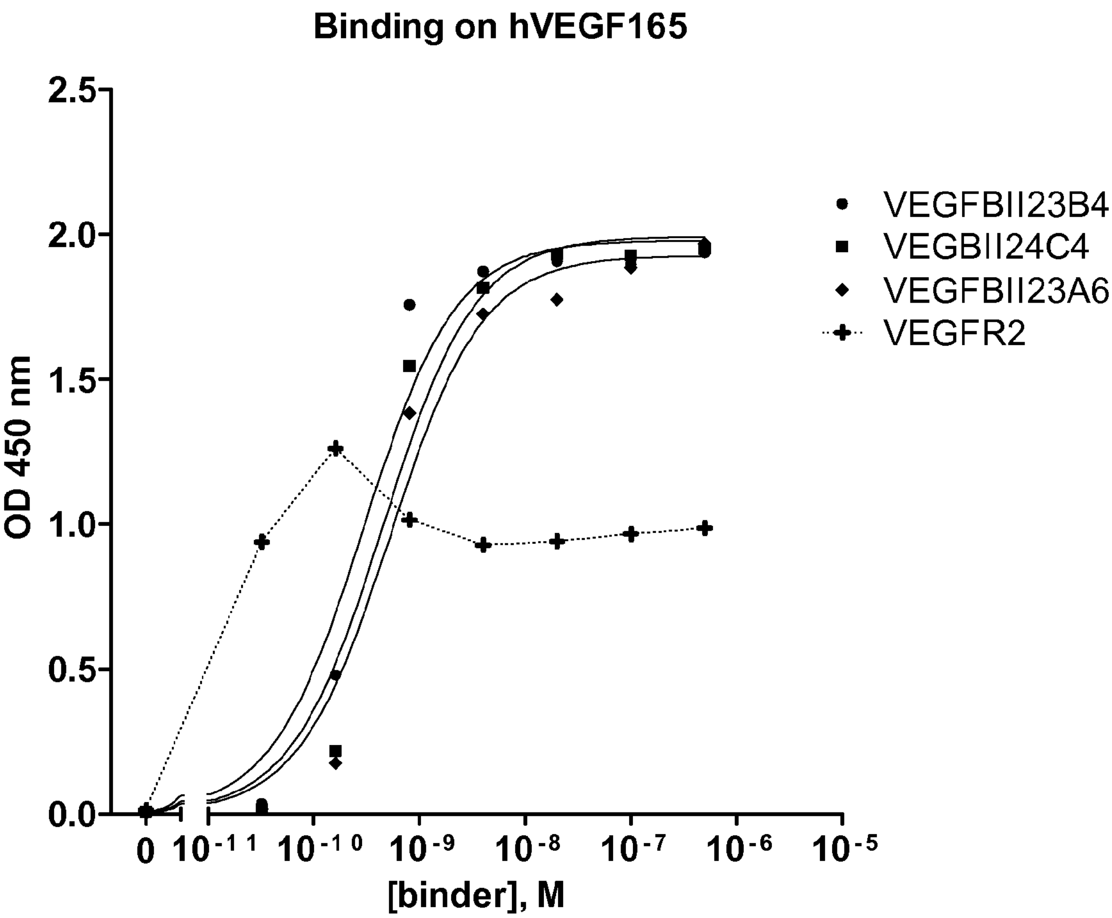
Binding on hVEGF165
OD 450 nm
2.5
2.0
1.5
1.0
0.5
0.0
0
10-11
10-10
10-9
10-8
10-7
10-6
10-5
[binder], M
VEGFBII23B4
VEGBII24C4
VEGFBII23A6
VEGFR2

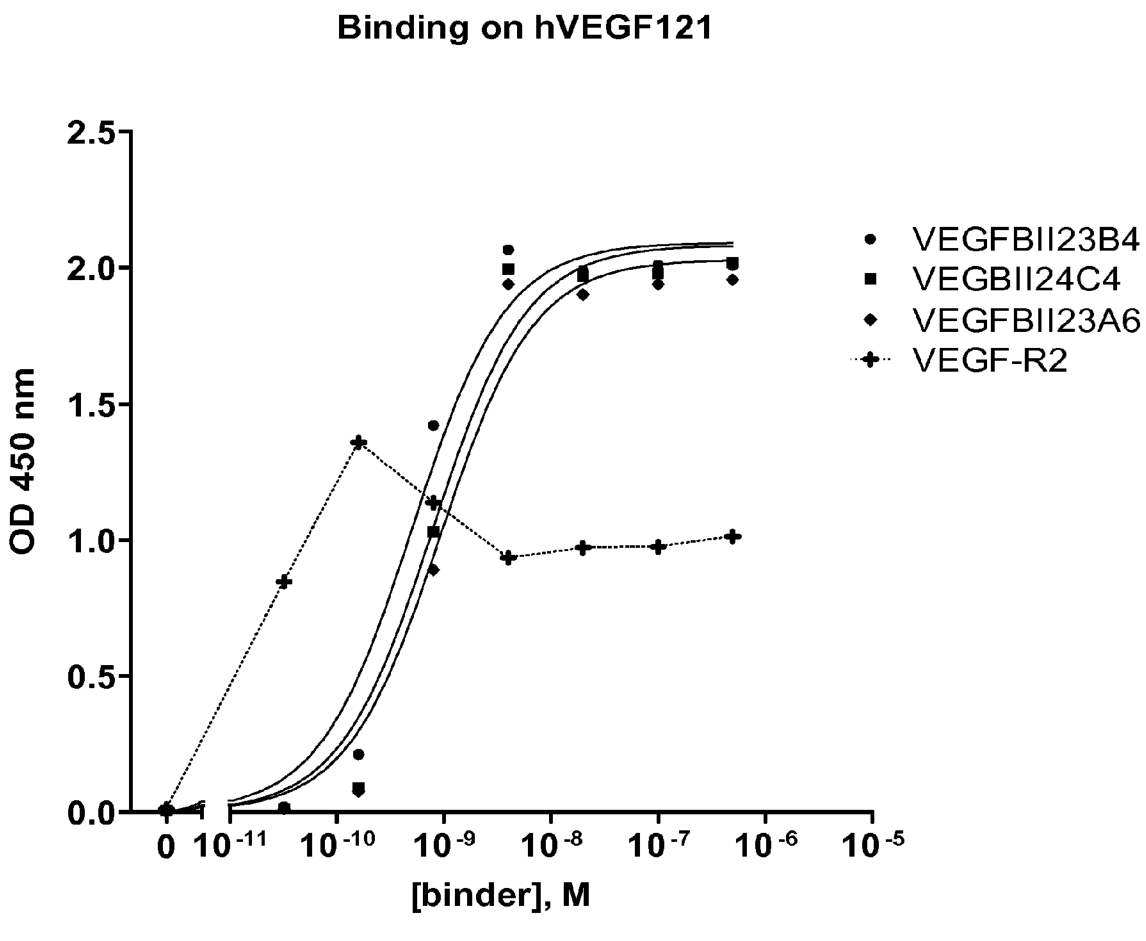

Binding on hVEGF121
OD 450 nm
2.5
2.0
1.5
1.0
0.5
0.0
0
$10^{-11}$
$10^{-10}$
$10^{-9}$
$10^{-8}$
$10^{-7}$
$10^{-6}$
$10^{-5}$
[binder], M
VEGFBII23B4
VEGBII24C4
VEGFBII23A6
VEGF-R2

Fig. 7-1

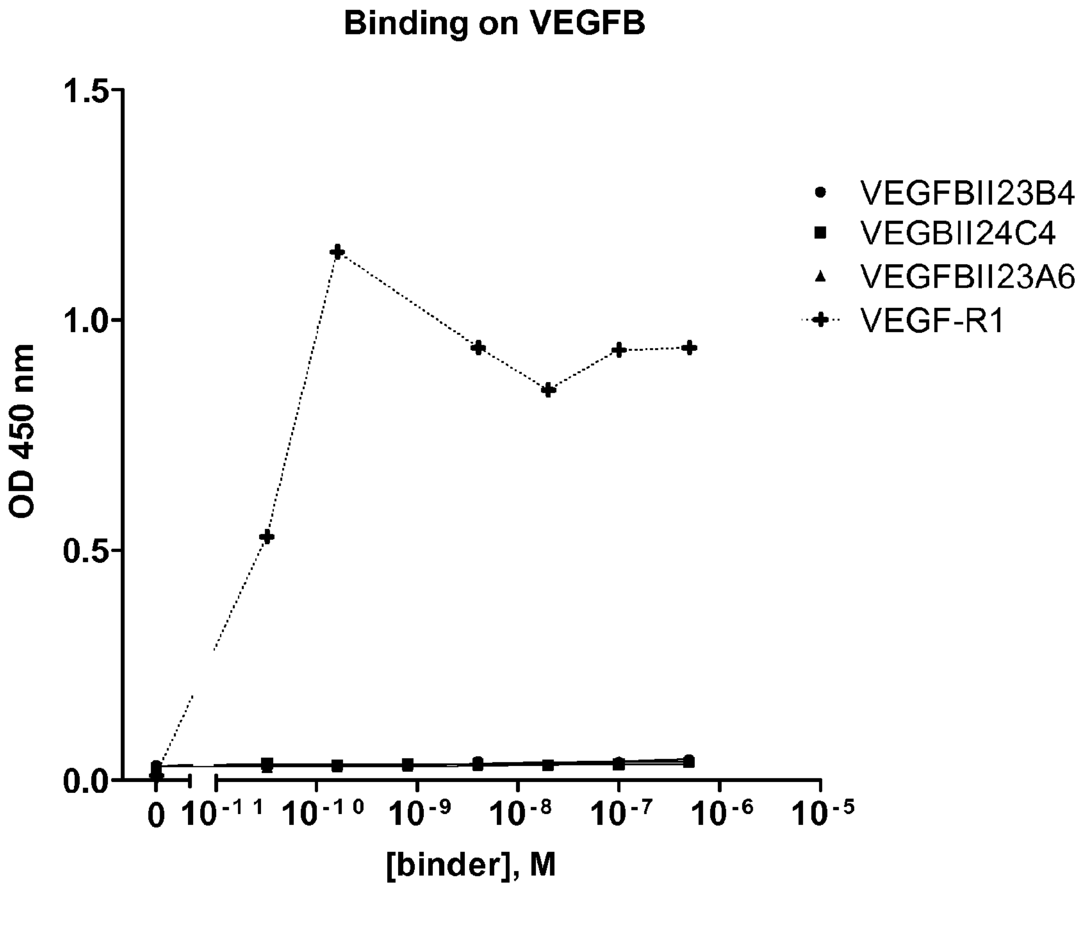
Binding on VEGFB
1.5
1.0
0.5
0.0
OD 450 nm
0
10-11
10-10
10-9
10-8
10-7
10-6
10-5
[binder], M
VEGFBII23B4
VEGBII24C4
VEGFBII23A6
VEGF-R1

Fig. 7-2

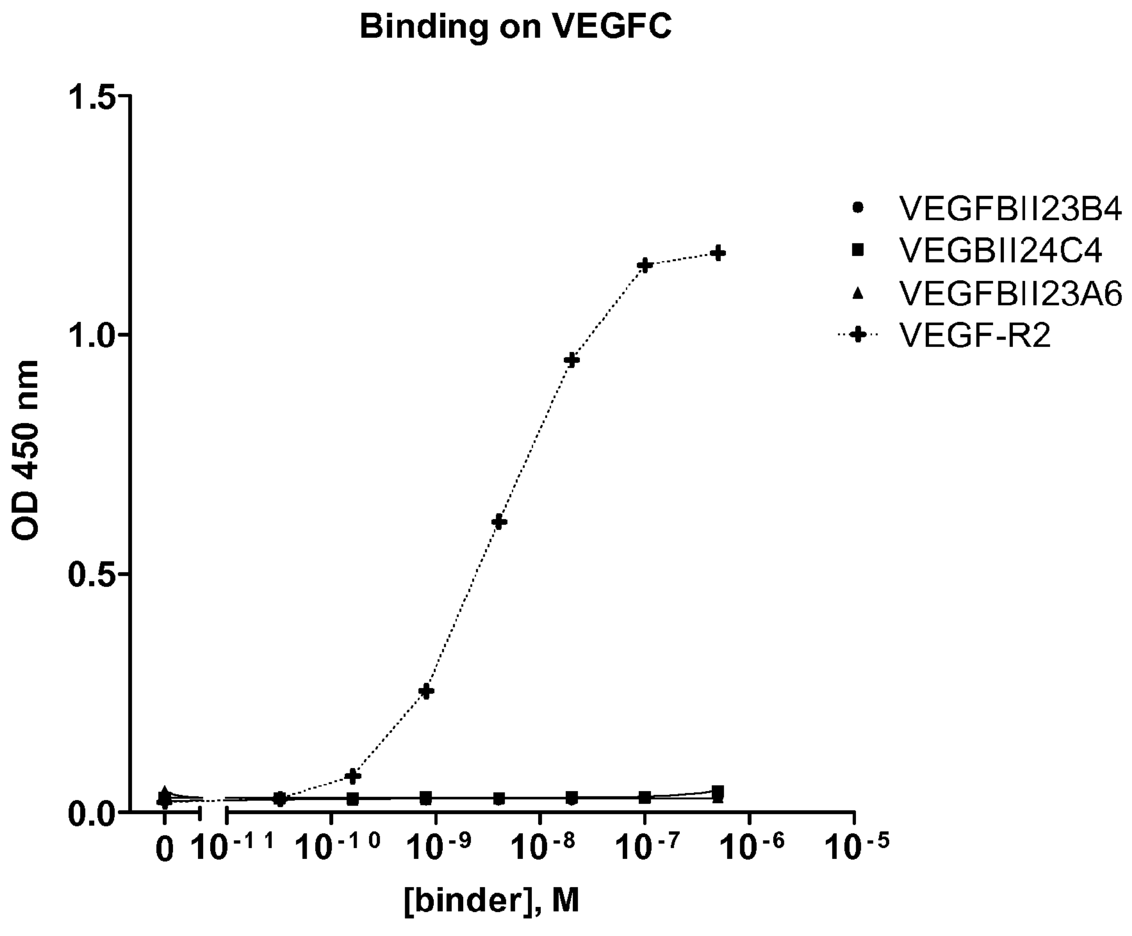
Binding on VEGFC
1.5
1.0
0.5
0.0
OD 450 nm
0
$10^{-11}$
$10^{-10}$
$10^{-9}$
$10^{-8}$
$10^{-7}$
$10^{-6}$
$10^{-5}$
[binder], M
VEGFBII23B4
VEGBII24C4
VEGFBII23A6
VEGF-R2

**Fig. 7-3**

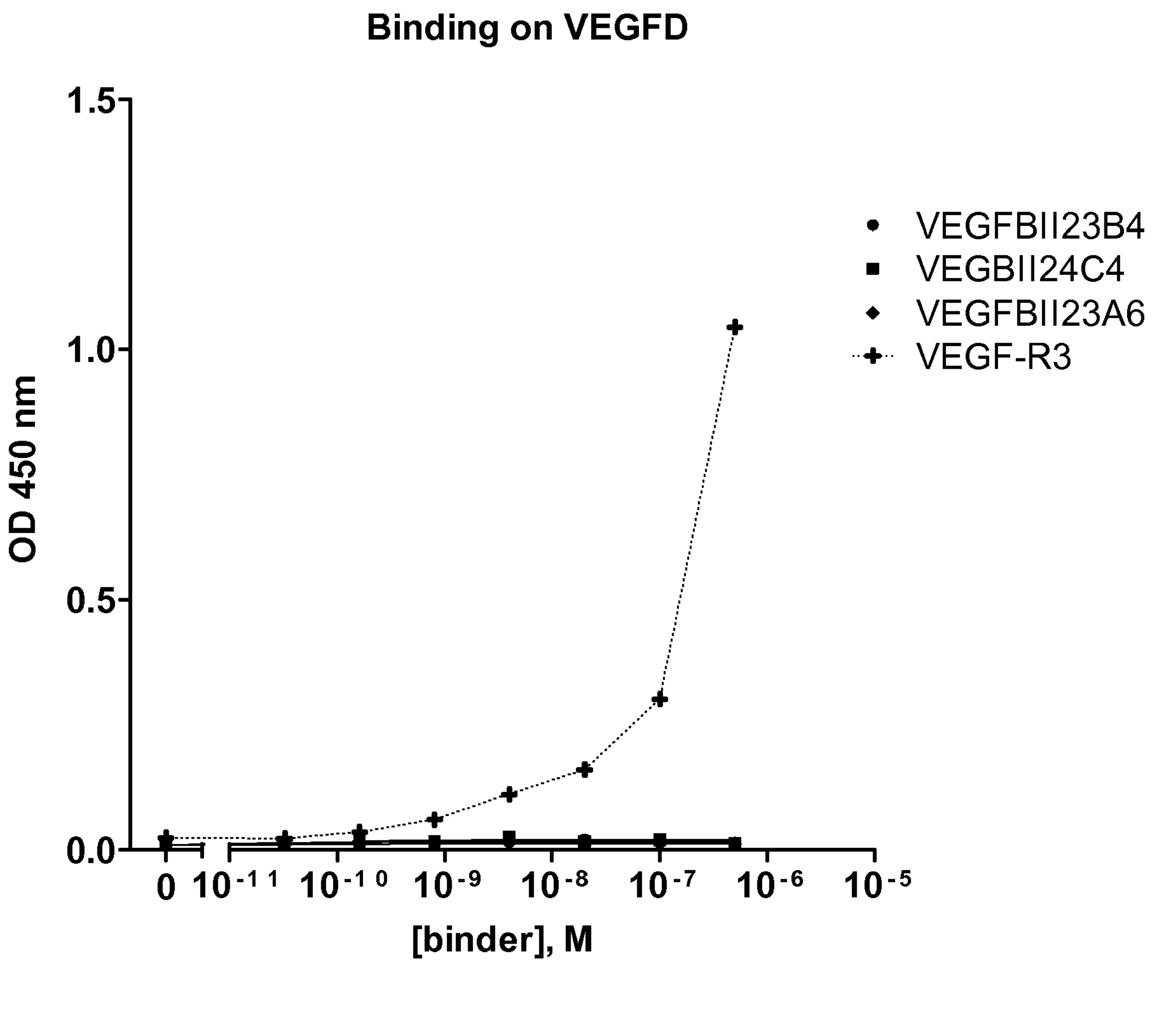
Binding on VEGFD
1.5
1.0
0.5
0.0
OD 450 nm
0
10-11
10-10
10-9
10-8
10-7
10-6
10-5
[binder], M
VEGFBII23B4
VEGBII24C4
VEGFBII23A6
VEGF-R3

Fig. 7-4

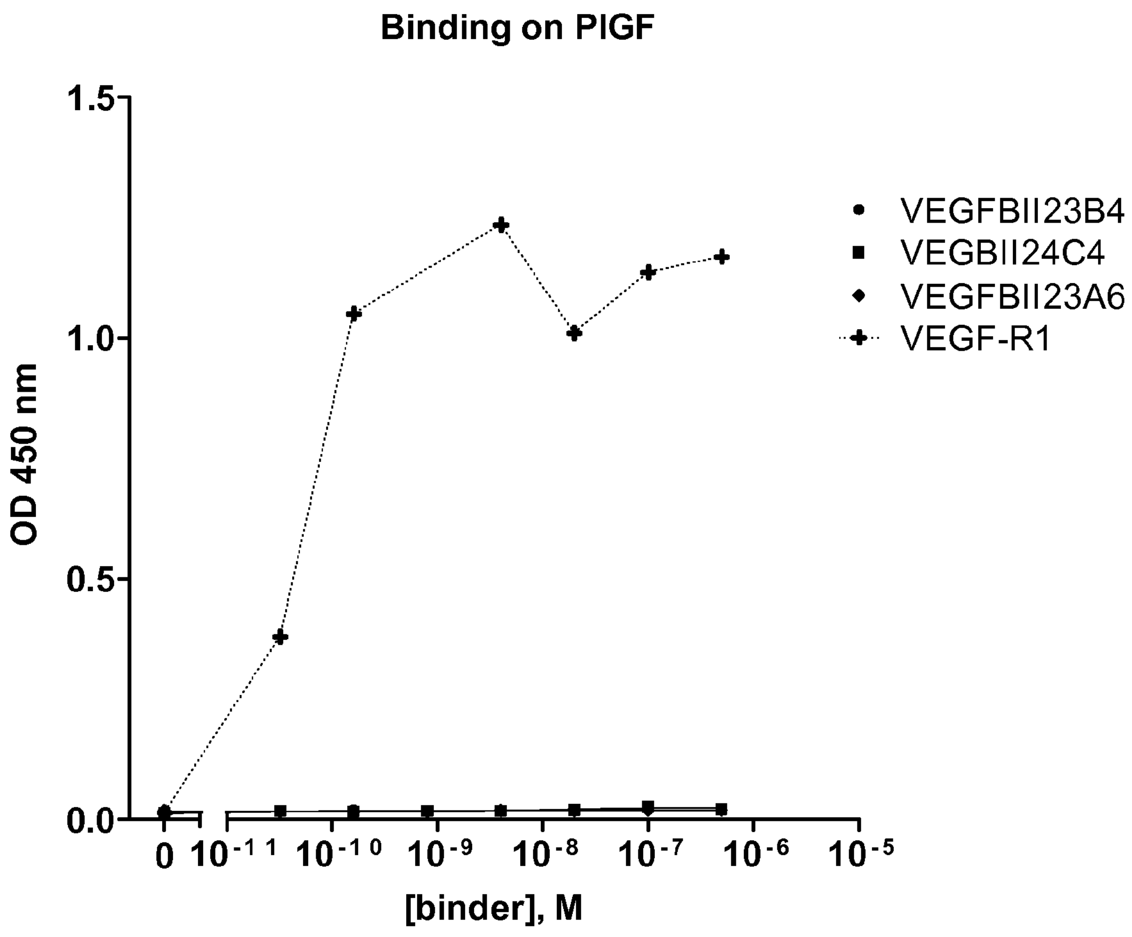

Binding on PIGF
1.5
1.0
0.5
0.0
OD 450 nm
0
10⁻¹¹
10⁻¹⁰
10⁻⁹
10⁻⁸
10⁻⁷
10⁻⁶
10⁻⁵
[binder], M
VEGFBII23B4
VEGBII24C4
VEGFBII23A6
VEGF-R1

**Fig. 8-1**

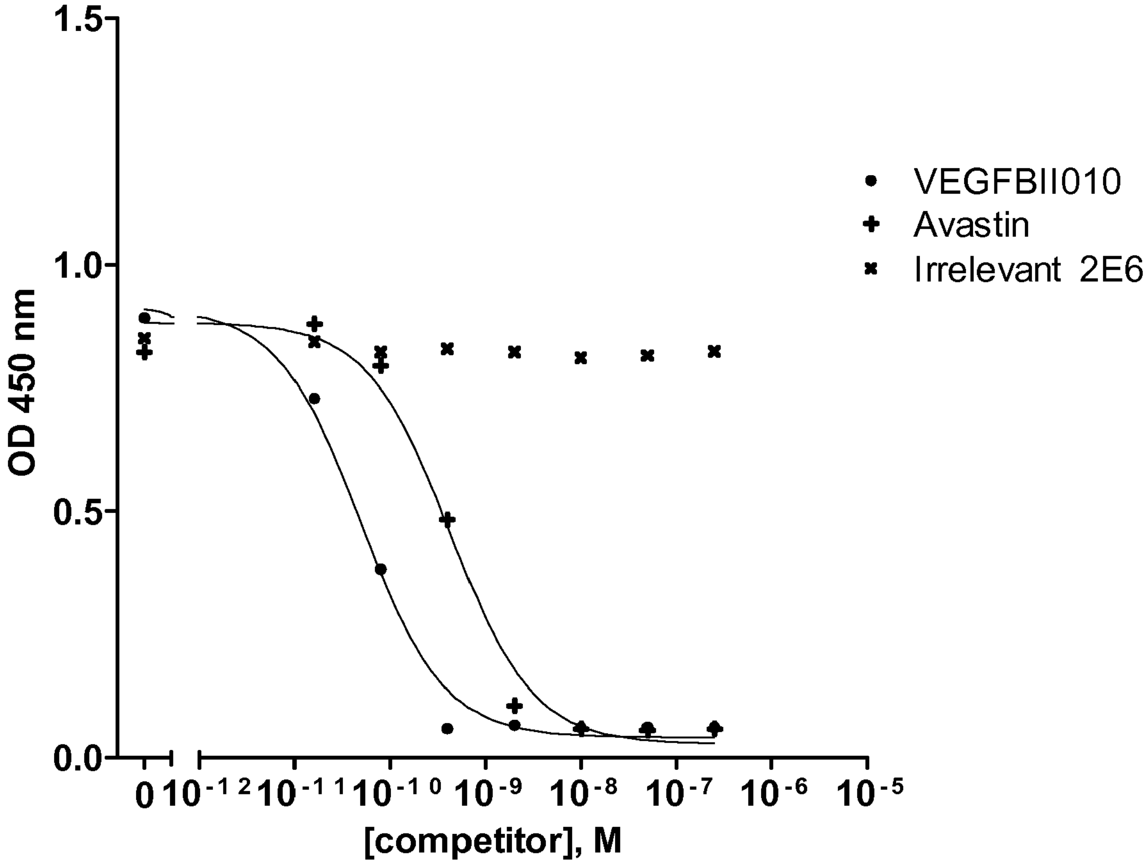
1.5
1.0
0.5
0.0
OD 450 nm
0
10⁻¹²
10⁻¹¹
10⁻¹⁰
10⁻⁹
10⁻⁸
10⁻⁷
10⁻⁶
10⁻⁵
[competitor], M
VEGFBII010
Avastin
Irrelevant 2E6

Fig. 8-2

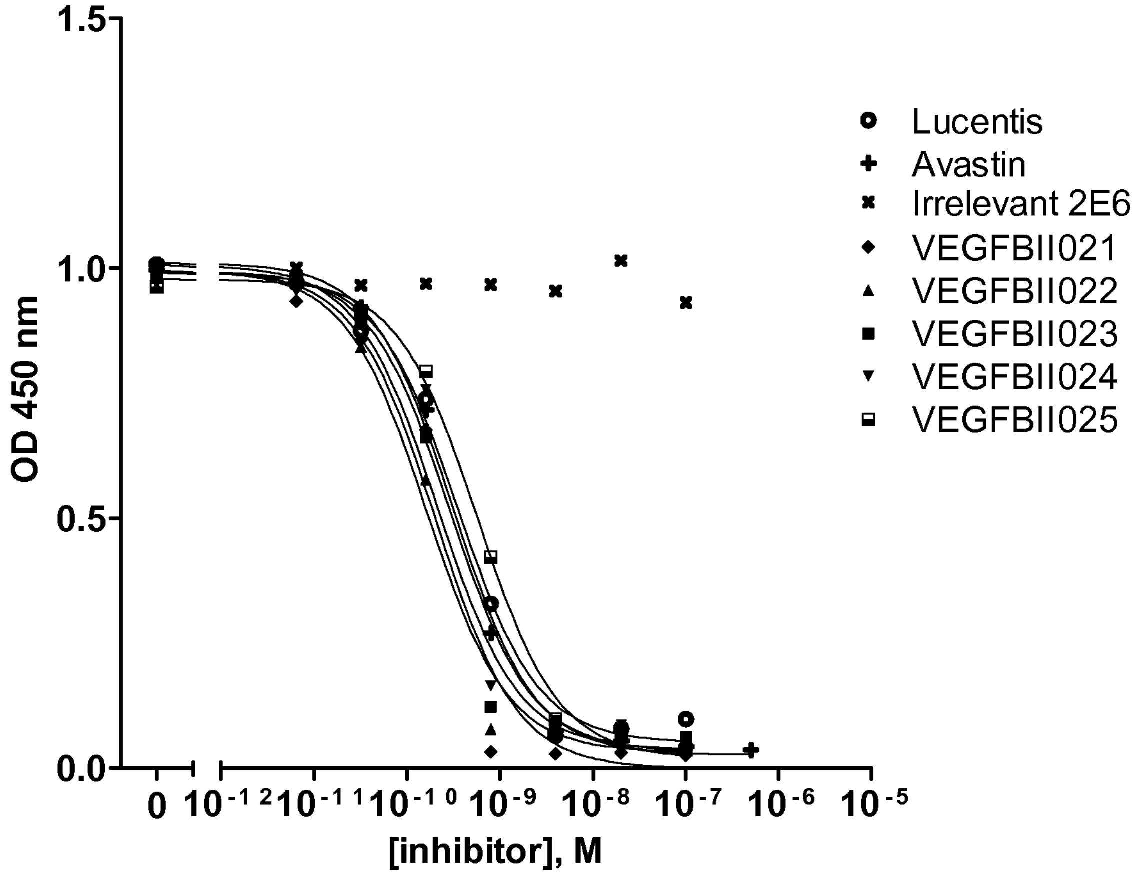
1.5
1.0
0.5
0.0
OD 450 nm
0
10⁻¹²
10⁻¹¹
10⁻¹⁰
10⁻⁹
10⁻⁸
10⁻⁷
10⁻⁶
10⁻⁵
[inhibitor], M
Lucentis
Avastin
Irrelevant 2E6
VEGFBII021
VEGFBII022
VEGFBII023
VEGFBII024
VEGFBII025

Fig. 9-1

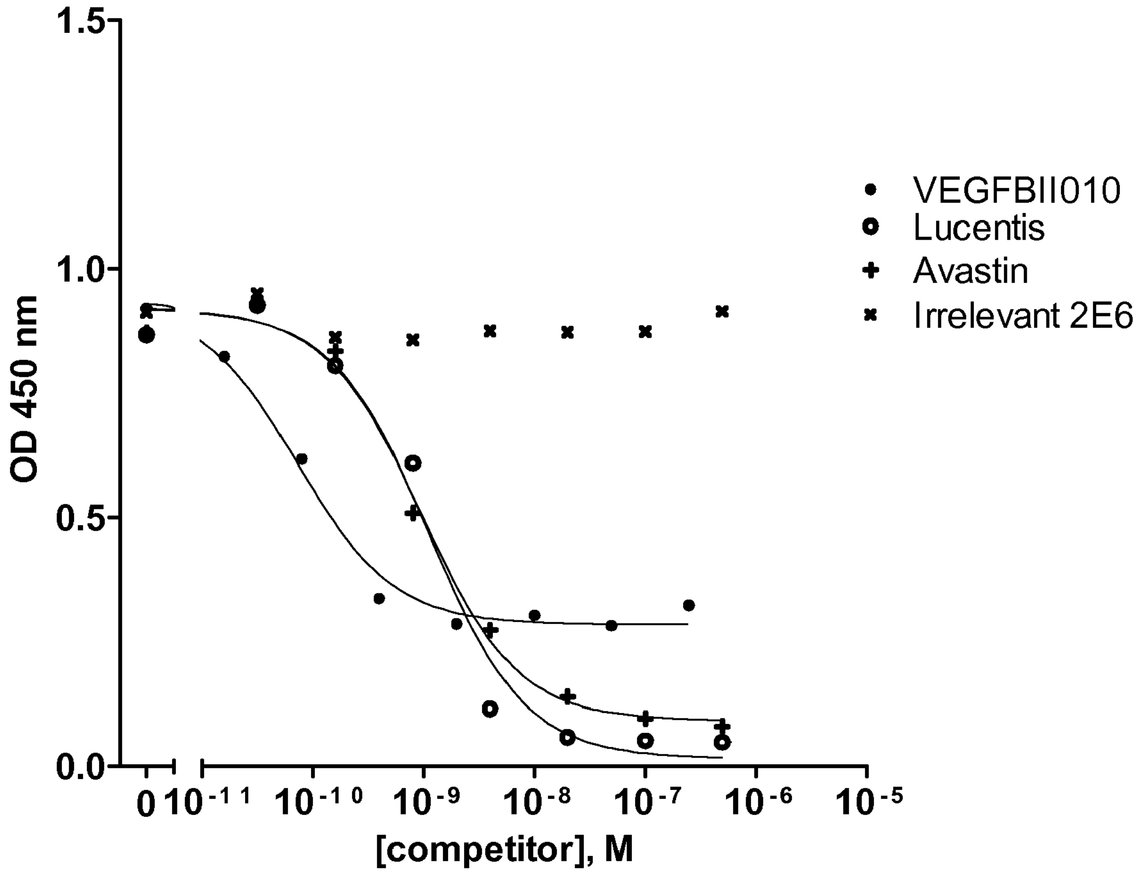
1.5
1.0
0.5
0.0
OD 450 nm
0
$10^{-11}$
$10^{-10}$
$10^{-9}$
$10^{-8}$
$10^{-7}$
$10^{-6}$
$10^{-5}$
[competitor], M
VEGFBII010
Lucentis
Avastin
Irrelevant 2E6

**Fig. 9-2**

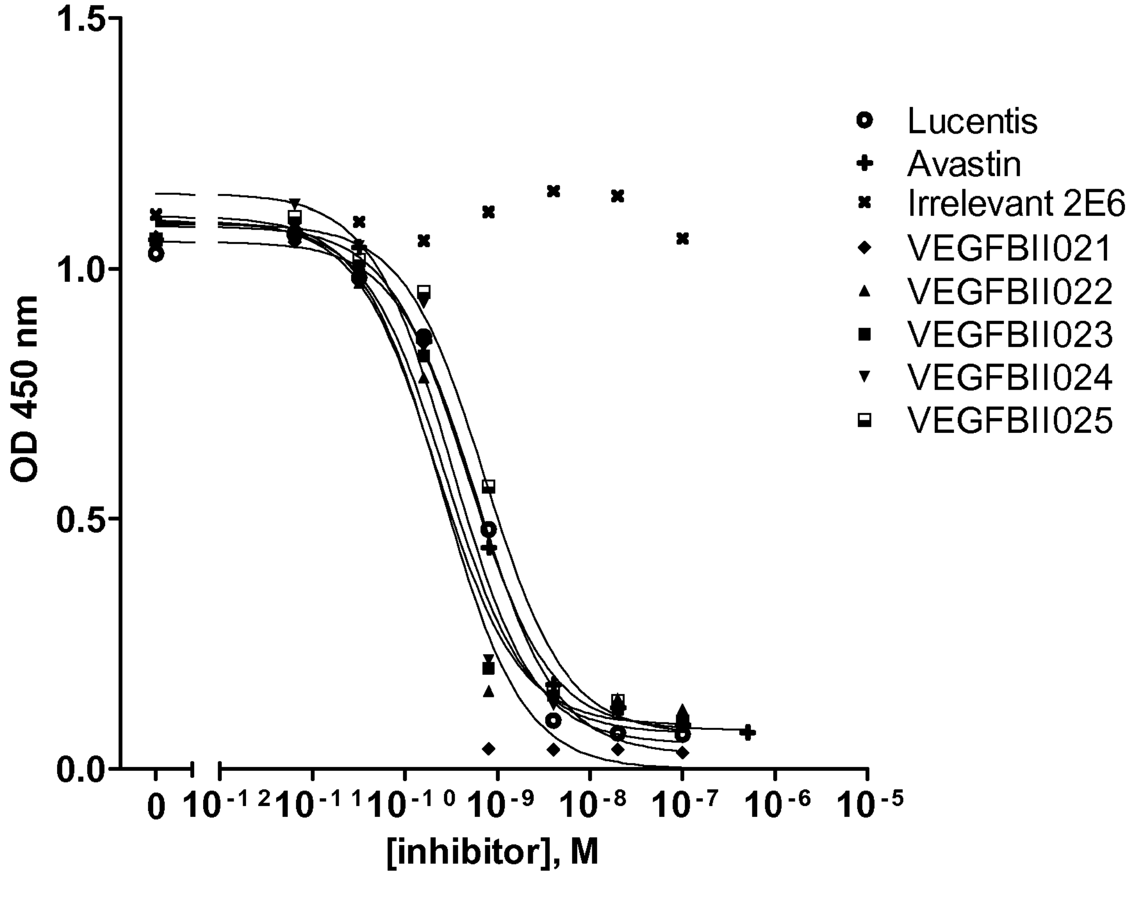
1.5
1.0
0.5
0.0
OD 450 nm
0
10⁻¹²
10⁻¹¹
10⁻¹⁰
10⁻⁹
10⁻⁸
10⁻⁷
10⁻⁶
10⁻⁵
[inhibitor], M
Lucentis
Avastin
Irrelevant 2E6
VEGFBII021
VEGFBII022
VEGFBII023
VEGFBII024
VEGFBII025

**Fig. 10**

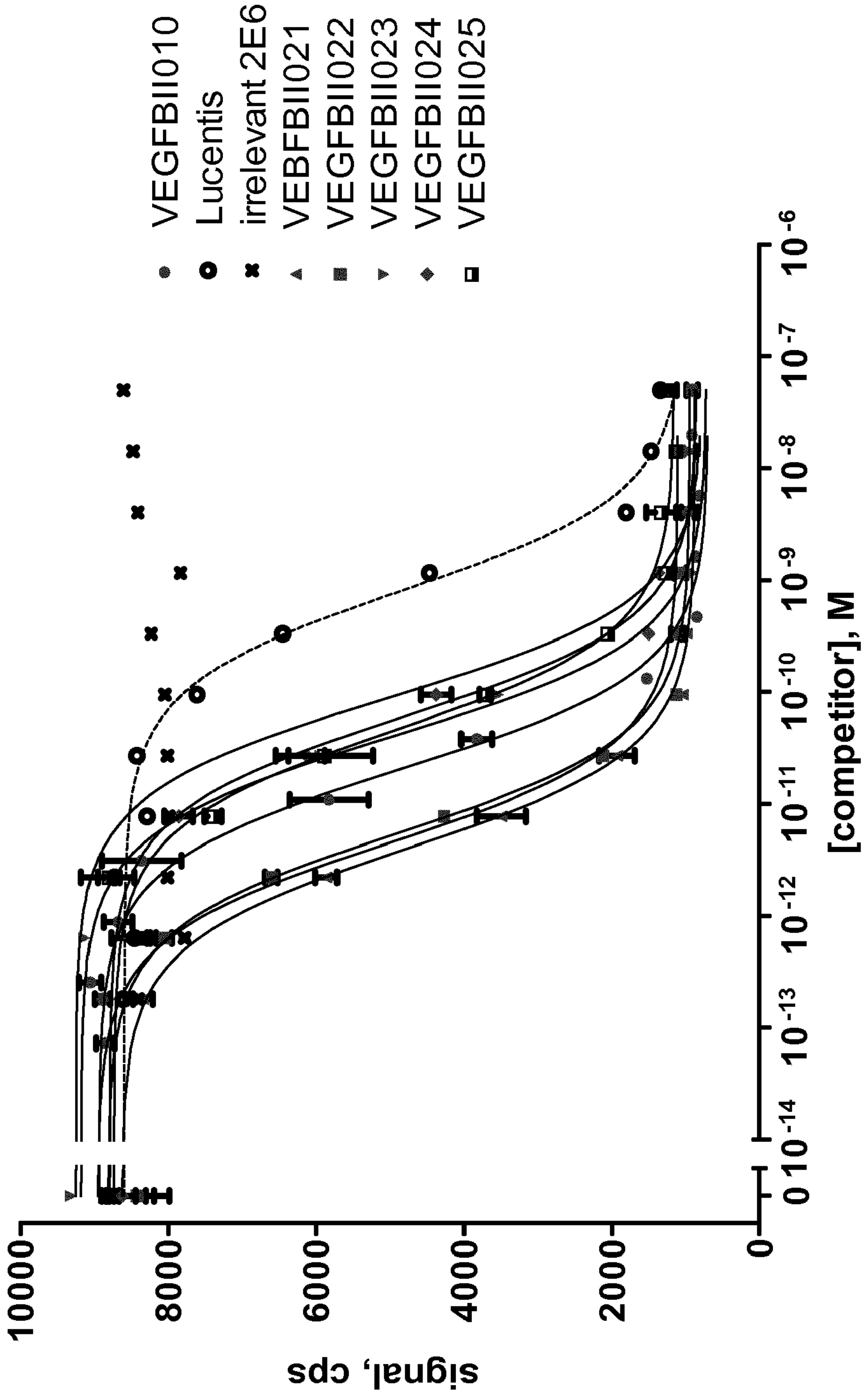
signal, cps
10000
8000
6000
4000
2000
0
[competitor], M
0
$10^{-14}$
$10^{-13}$
$10^{-12}$
$10^{-11}$
$10^{-10}$
$10^{-9}$
$10^{-8}$
$10^{-7}$
$10^{-6}$
VEGFBII010
Lucentis
irrelevant 2E6
VEBFBII021
VEGFBII022
VEGFBII023
VEGFBII024
VEGFBII025

**Fig. 11**

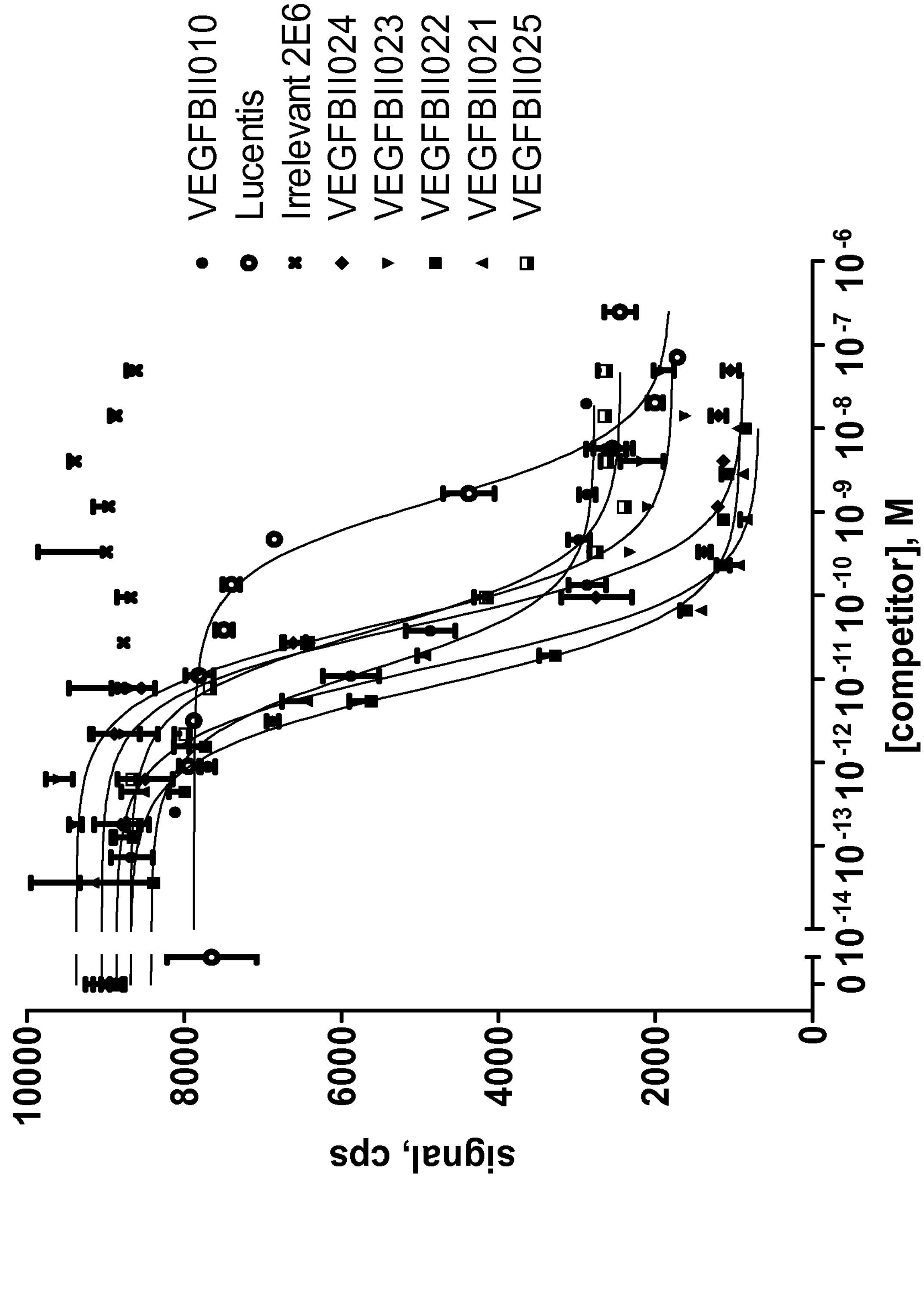

VEGFBII010
Lucentis
Irrelevant 2E6
VEGFBII024
VEGFBII023
VEGFBII022
VEGFBII021
VEGFBII025
signal, cps
10000
8000
6000
4000
2000
0
[competitor], M
0
10-14
10-13
10-12
10-11
10-10
10-9
10-8
10-7
10-6

**Fig. 12**

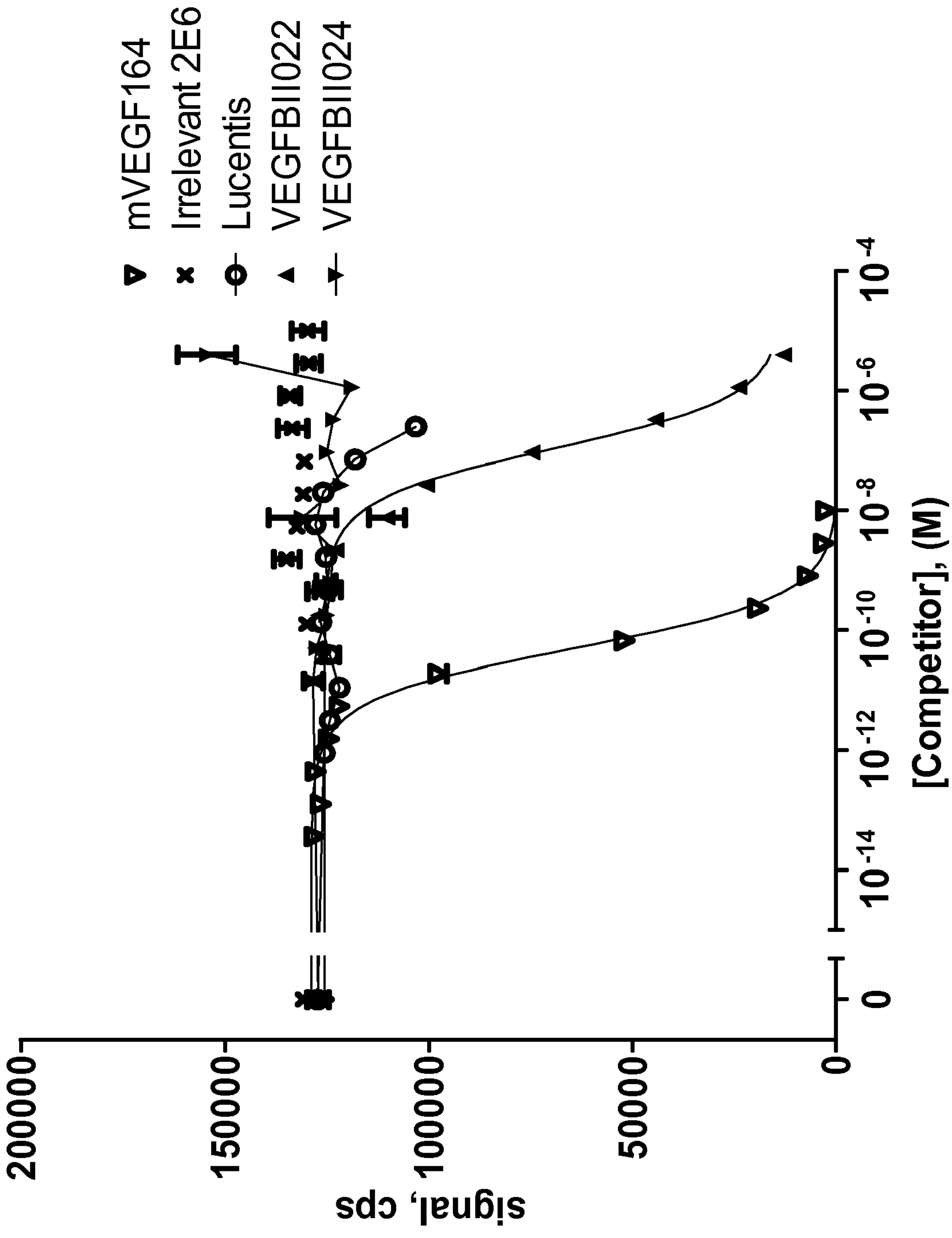
mVEGF164
Irrelevant 2E6
Lucentis
VEGFBII022
VEGFBII024
signal, cps
200000
150000
100000
50000
0
0
$10^{-14}$
$10^{-12}$
$10^{-10}$
$10^{-8}$
$10^{-6}$
$10^{-4}$
[Competitor], (M)

**Fig. 13-1**

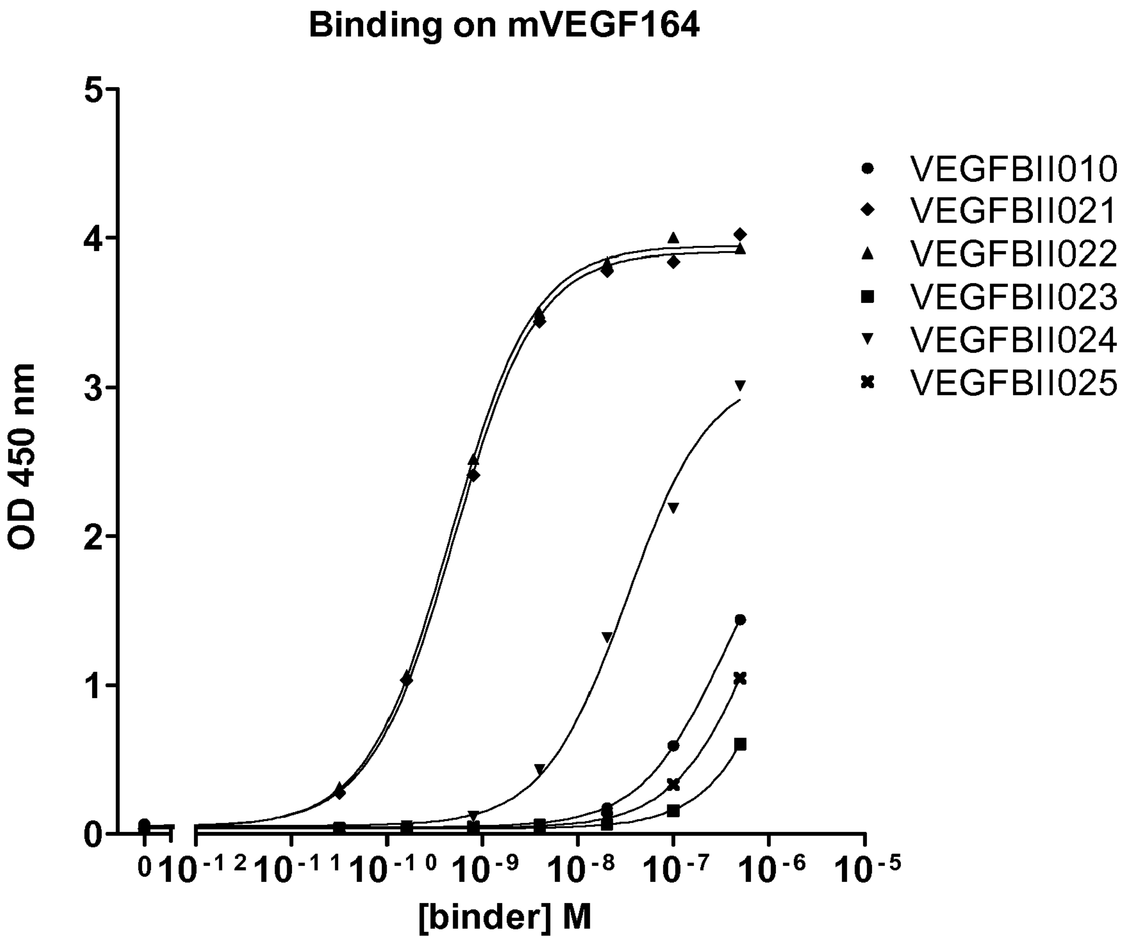

Binding on mVEGF164
OD 450 nm
[binder] M
0
1
2
3
4
5
0
10-12
10-11
10-10
10-9
10-8
10-7
10-6
10-5
VEGFBII010
VEGFBII021
VEGFBII022
VEGFBII023
VEGFBII024
VEGFBII025

Fig. 13-2

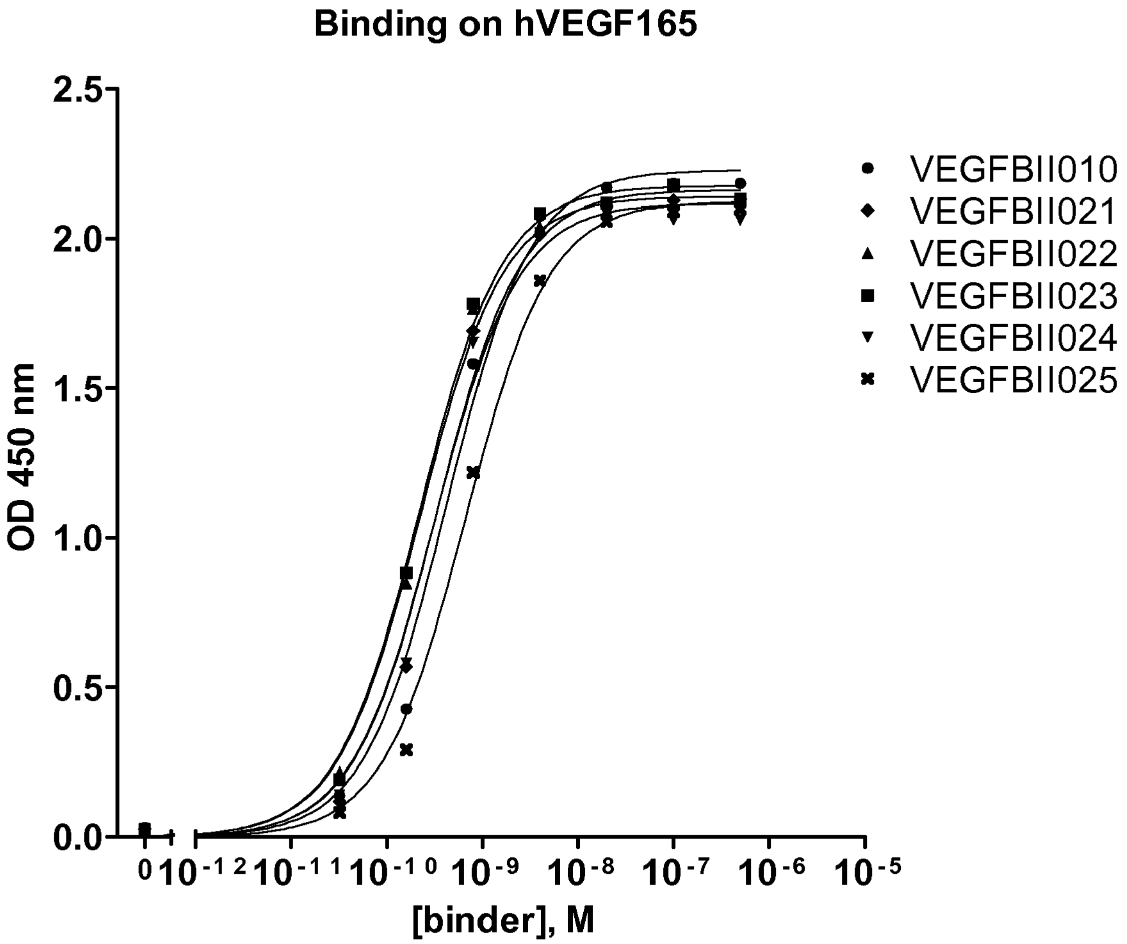
Binding on hVEGF165
OD 450 nm
2.5
2.0
1.5
1.0
0.5
0.0
0
10⁻¹²
10⁻¹¹
10⁻¹⁰
10⁻⁹
10⁻⁸
10⁻⁷
10⁻⁶
10⁻⁵
[binder], M
VEGFBII010
VEGFBII021
VEGFBII022
VEGFBII023
VEGFBII024
VEGFBII025

**Fig. 14-1**

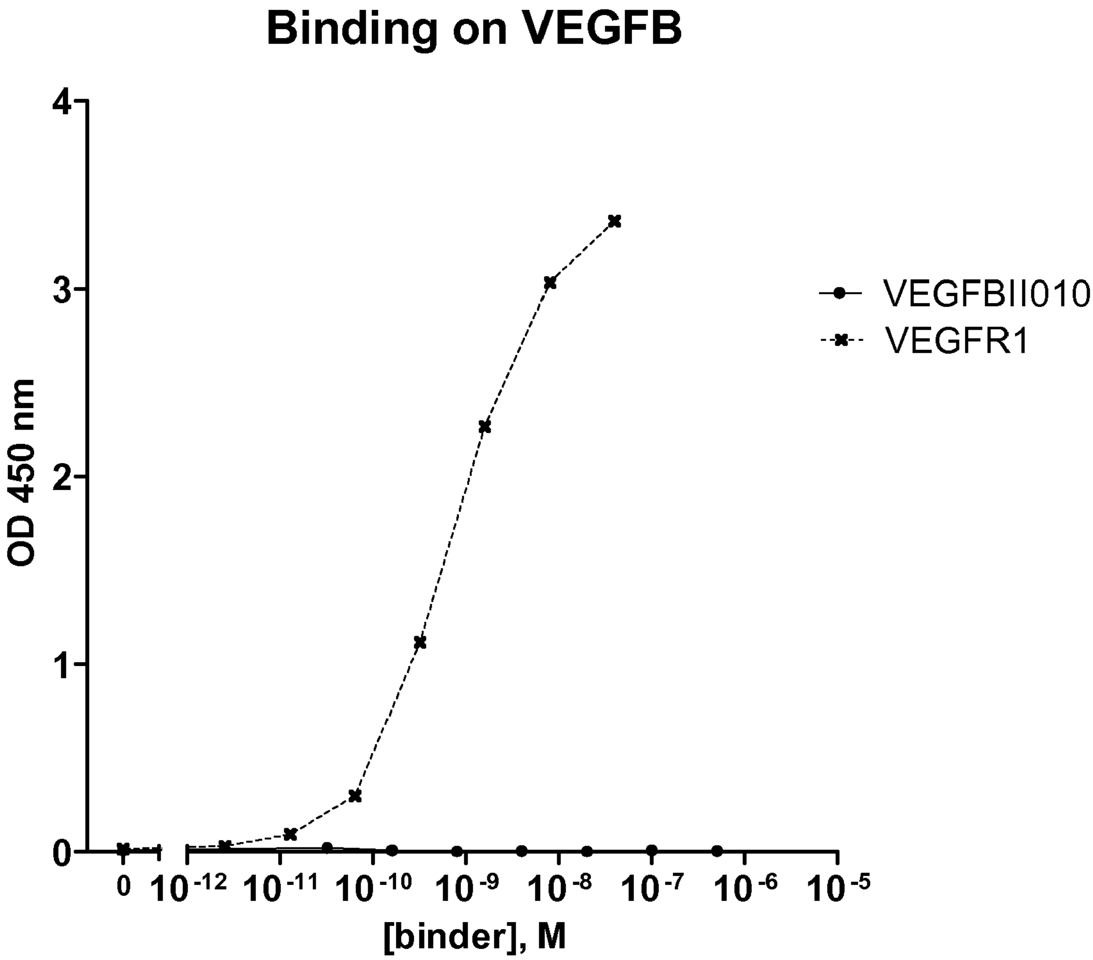
Binding on VEGFB
4
3
2
1
0
OD 450 nm
0
10 -12
10 -11
10 -10
10 -9
10 -8
10 -7
10 -6
10 -5
[binder], M
VEGFBII010
VEGFR1

**Fig. 14-2**

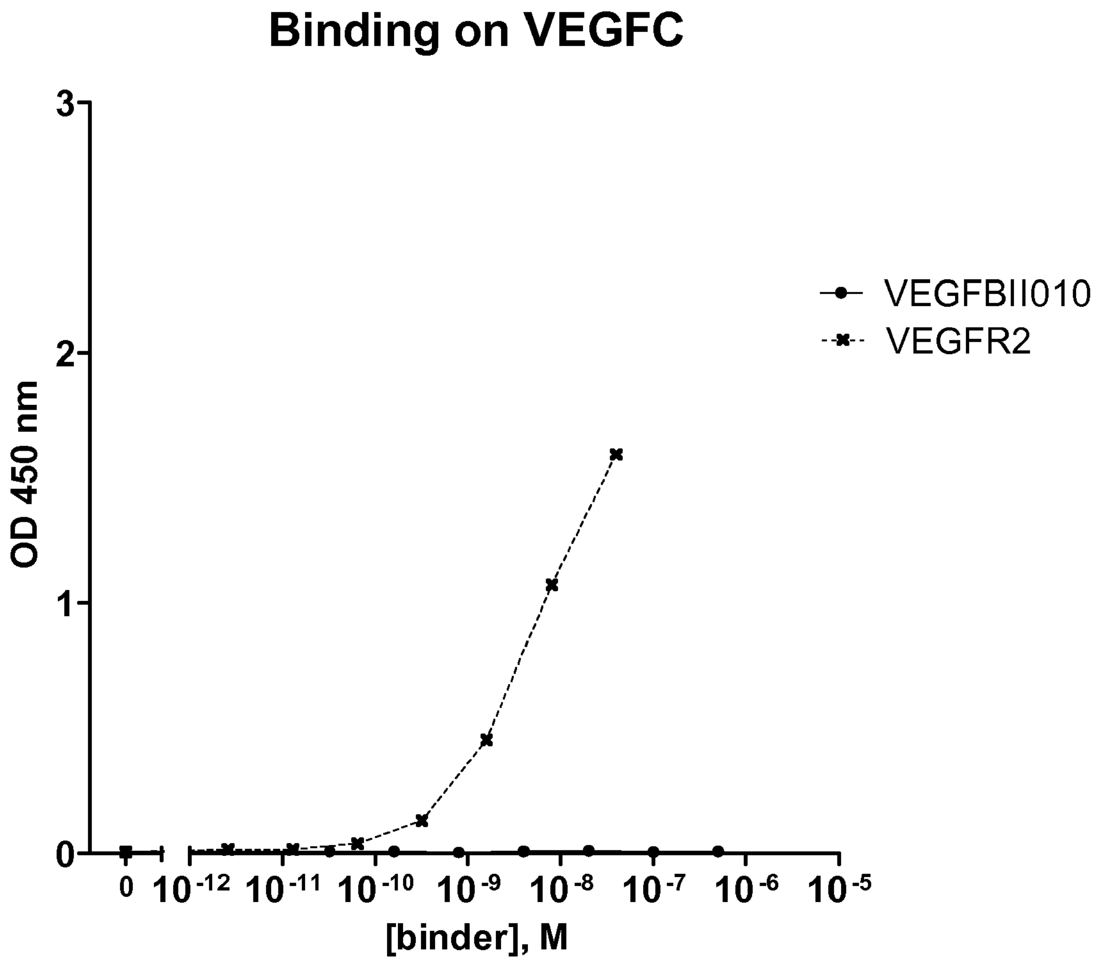

Binding on VEGFC
OD 450 nm
3
2
1
0
0
$10^{-12}$
$10^{-11}$
$10^{-10}$
$10^{-9}$
$10^{-8}$
$10^{-7}$
$10^{-6}$
$10^{-5}$
[binder], M
VEGFBII010
VEGFR2

Fig. 14-3

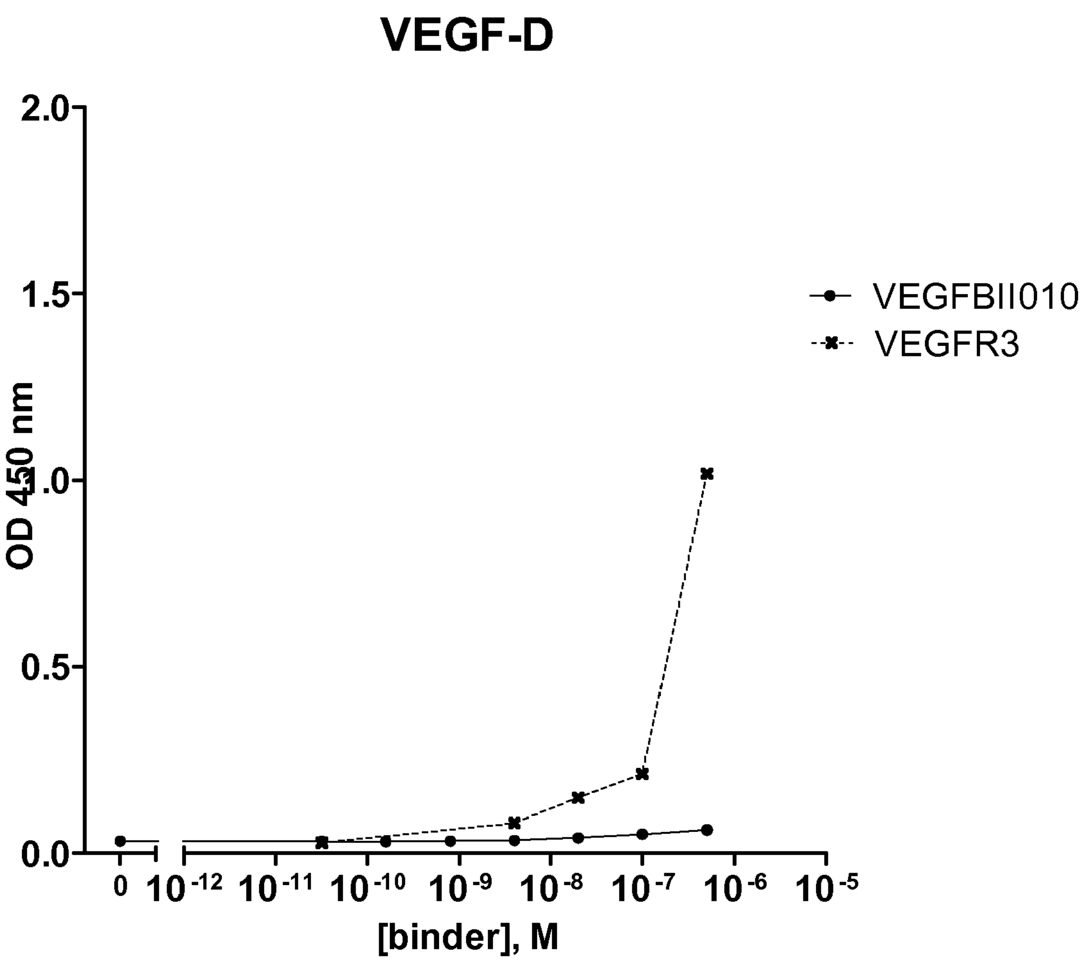
VEGF-D
2.0
1.5
1.0
0.5
0.0
OD 450 nm
VEGFBII010
VEGFR3
0
10 -12
10 -11
10 -10
10 -9
10 -8
10 -7
10 -6
10 -5
[binder], M

Fig. 14-4

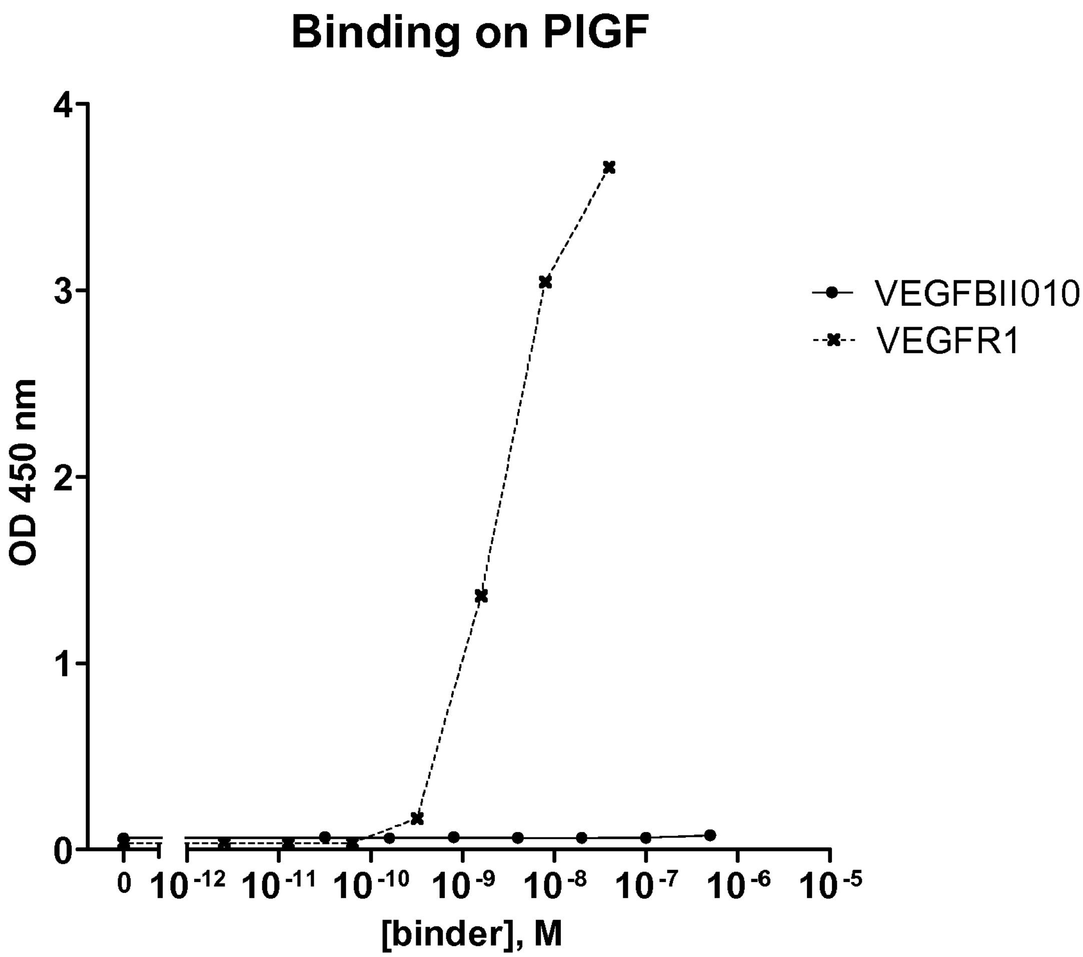
Binding on PIGF
OD 450 nm
4
3
2
1
0
0
10-12
10-11
10-10
10-9
10-8
10-7
10-6
10-5
[binder], M
VEGFBII010
VEGFR1

Fig. 14-5

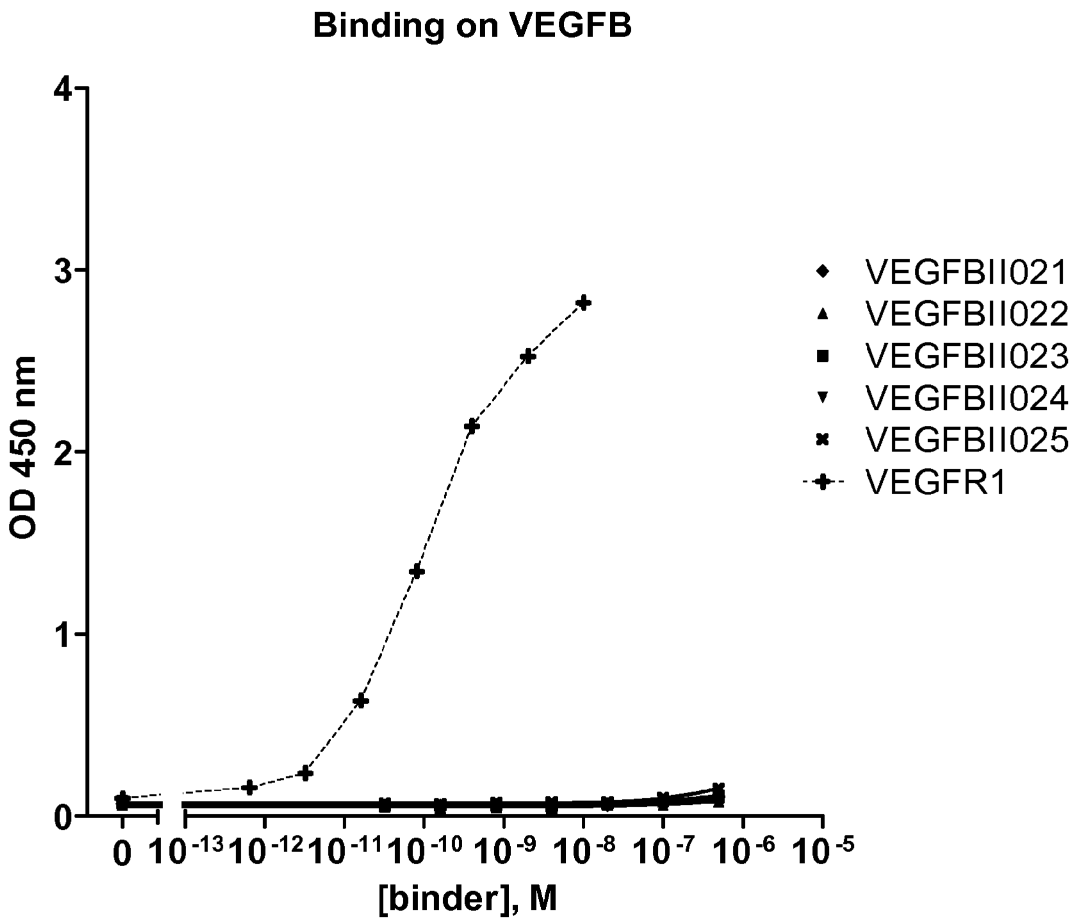
Binding on VEGFB
OD 450 nm
4
3
2
1
0
0
10 -13
10 -12
10 -11
10 -10
10 -9
10 -8
10 -7
10 -6
10 -5
[binder], M
VEGFBII021
VEGFBII022
VEGFBII023
VEGFBII024
VEGFBII025
VEGFR1

Fig. 14-6

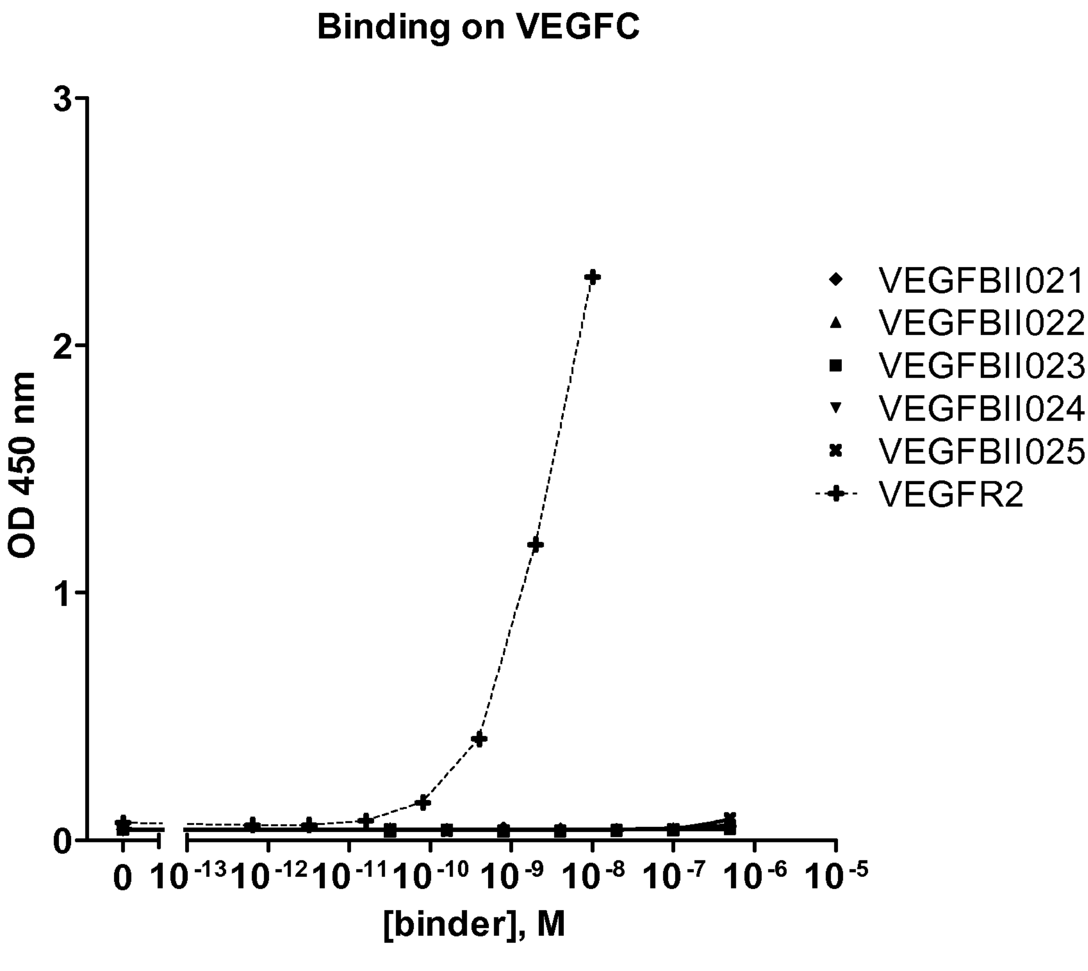
Binding on VEGFC
OD 450 nm
3
2
1
0
0
10^-13
10^-12
10^-11
10^-10
10^-9
10^-8
10^-7
10^-6
10^-5
[binder], M
VEGFBII021
VEGFBII022
VEGFBII023
VEGFBII024
VEGFBII025
VEGFR2

Fig. 14-7

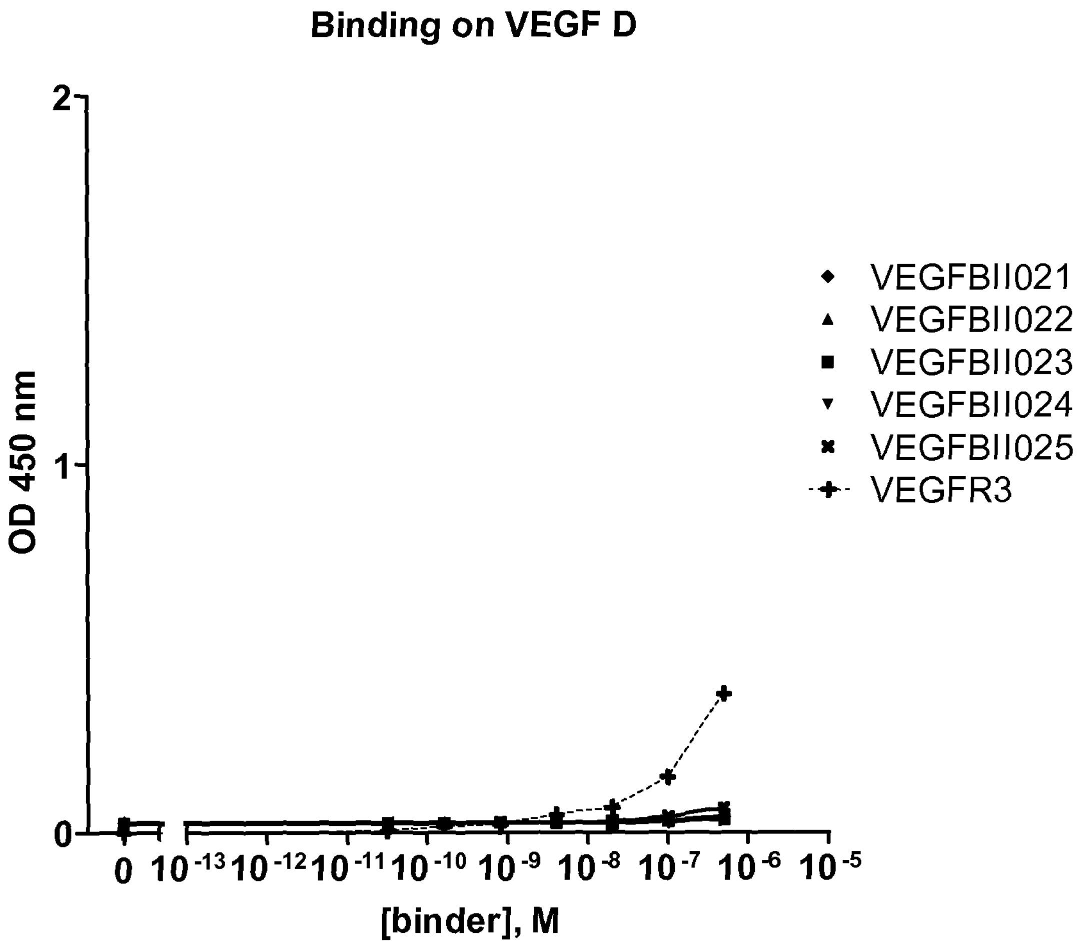
Binding on VEGF D
OD 450 nm
2
1
0
0 10 -13 10 -12 10 -11 10 -10 10 -9 10 -8 10 -7 10 -6 10 -5
[binder], M
VEGFBII021
VEGFBII022
VEGFBII023
VEGFBII024
VEGFBII025
VEGFR3

Fig. 14-8

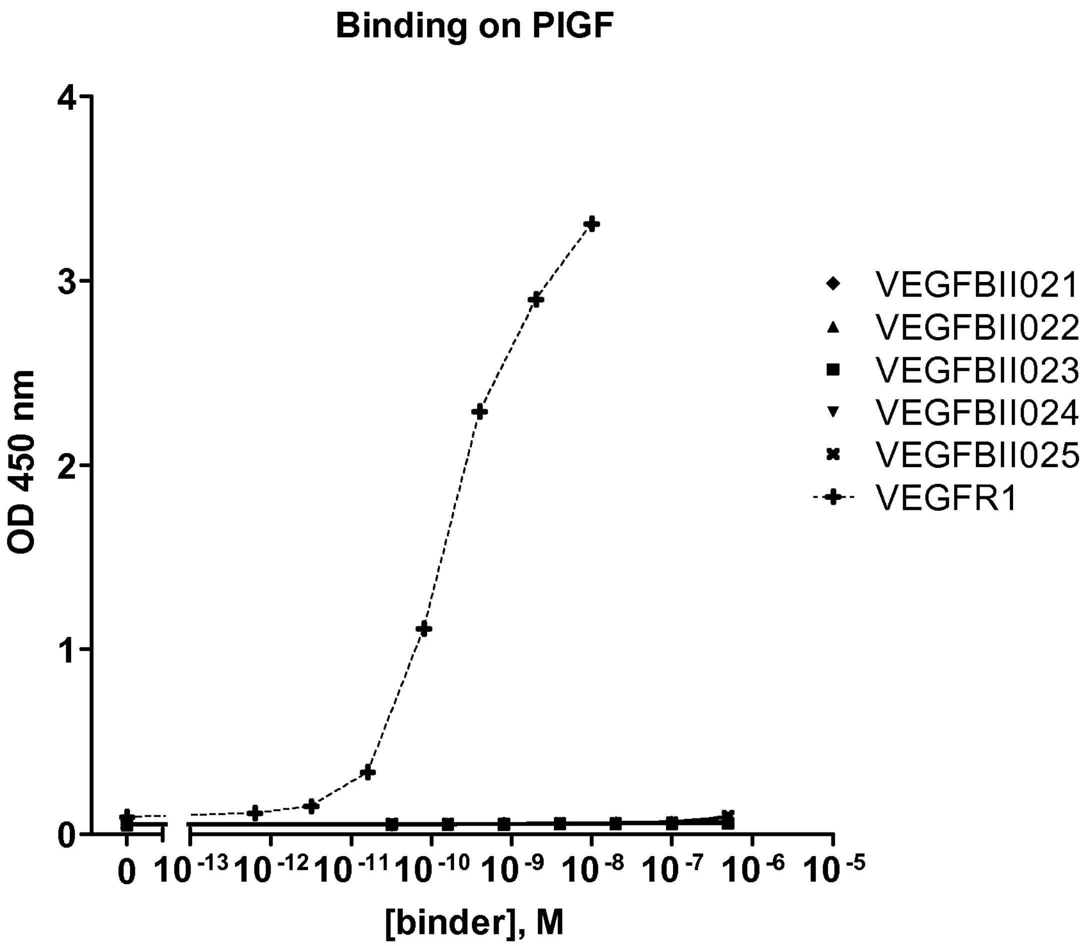
Binding on PlGF
OD 450 nm
4
3
2
1
0
0
10 -13
10 -12
10 -11
10 -10
10 -9
10 -8
10 -7
10 -6
10 -5
[binder], M
VEGFBII021
VEGFBII022
VEGFBII023
VEGFBII024
VEGFBII025
VEGFR1

**Fig. 15**

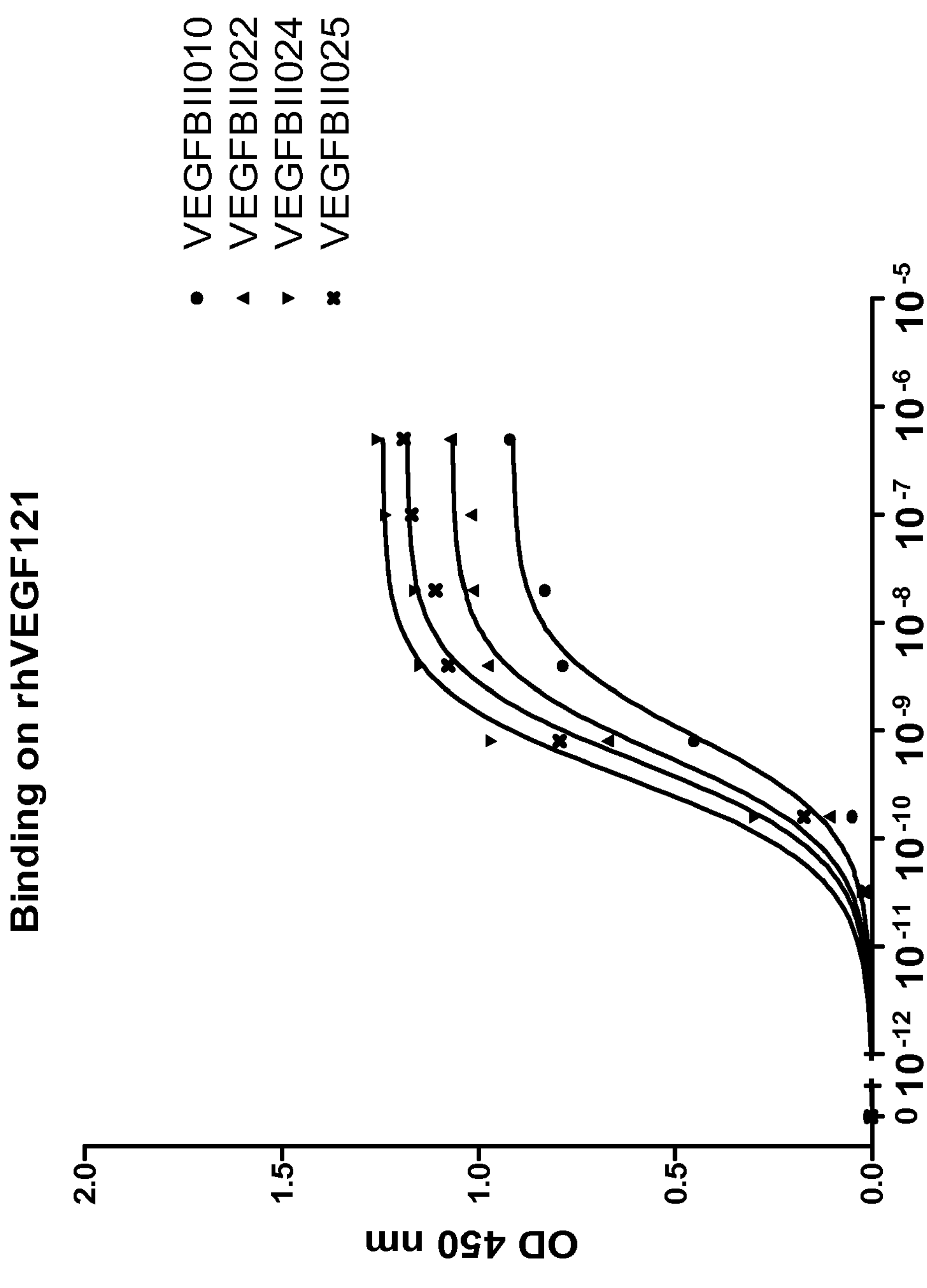
Binding on rhVEGF121
OD 450 nm
0.0
0.5
1.0
1.5
2.0
0
10^-12
10^-11
10^-10
10^-9
10^-8
10^-7
10^-6
10^-5
[binder], M
VEGFBII010
VEGFBII022
VEGFBII024
VEGFBII025

Fig. 16

```
                            10        20        30        40
Kabat#       :   .........|.........|.........|.........|
VH3-23/JH5   :   EVQLVESGGGLVQPGGSLRLSCAASGFTFSSYAMSWVRQA
VEGFBII23B04 :   .............T.D.......EV..R.....S.G.F...

                           50         60        70        80
Kabat#       :   .........|..a.......|.........|.........|
VH3-23/JH5   :   PGKGLEWVSAISGSGGSTYYADSVKGRFTISRDNSKNTLYL
VEGFBII23B04 :   Q..ER.F.V...-K..YK.DSV.LE......K..A...V..

                             90         100               110
Kabat#       :   ..abc.......|.........|abcdefghi.........|...
VH3-23/JH5   :   QMNSLRAEDTAVYYCAK----------------WGQGTLVTVSS
VEGFBII23B04 :   .I...KP.........SSRAYGSSRLRLADTYEY.....Q.....
```

Fig. 17

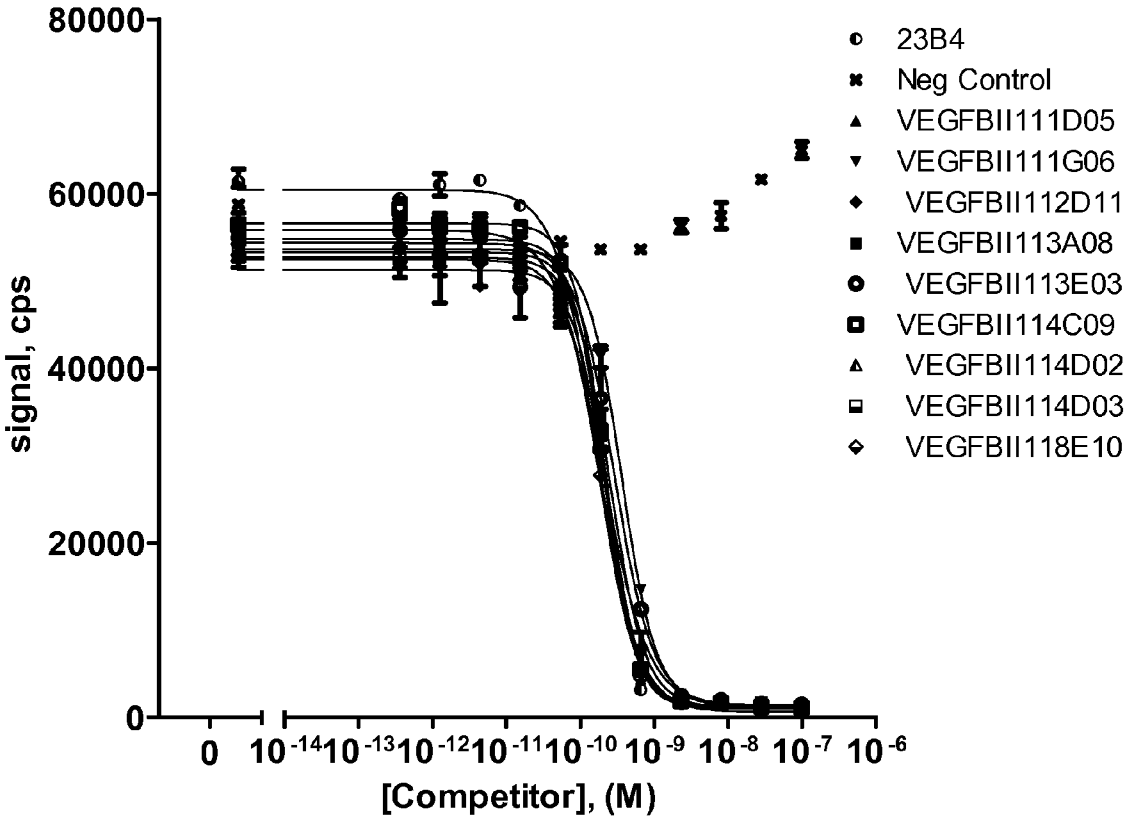
80000
60000
40000
20000
0
signal, cps
0
10 -14 10 -13 10 -12 10 -11 10 -10 10 -9 10 -8 10 -7 10 -6
[Competitor], (M)
23B4
Neg Control
VEGFBII111D05
VEGFBII111G06
VEGFBII112D11
VEGFBII113A08
VEGFBII113E03
VEGFBII114C09
VEGFBII114D02
VEGFBII114D03
VEGFBII118E10

Fig. 18

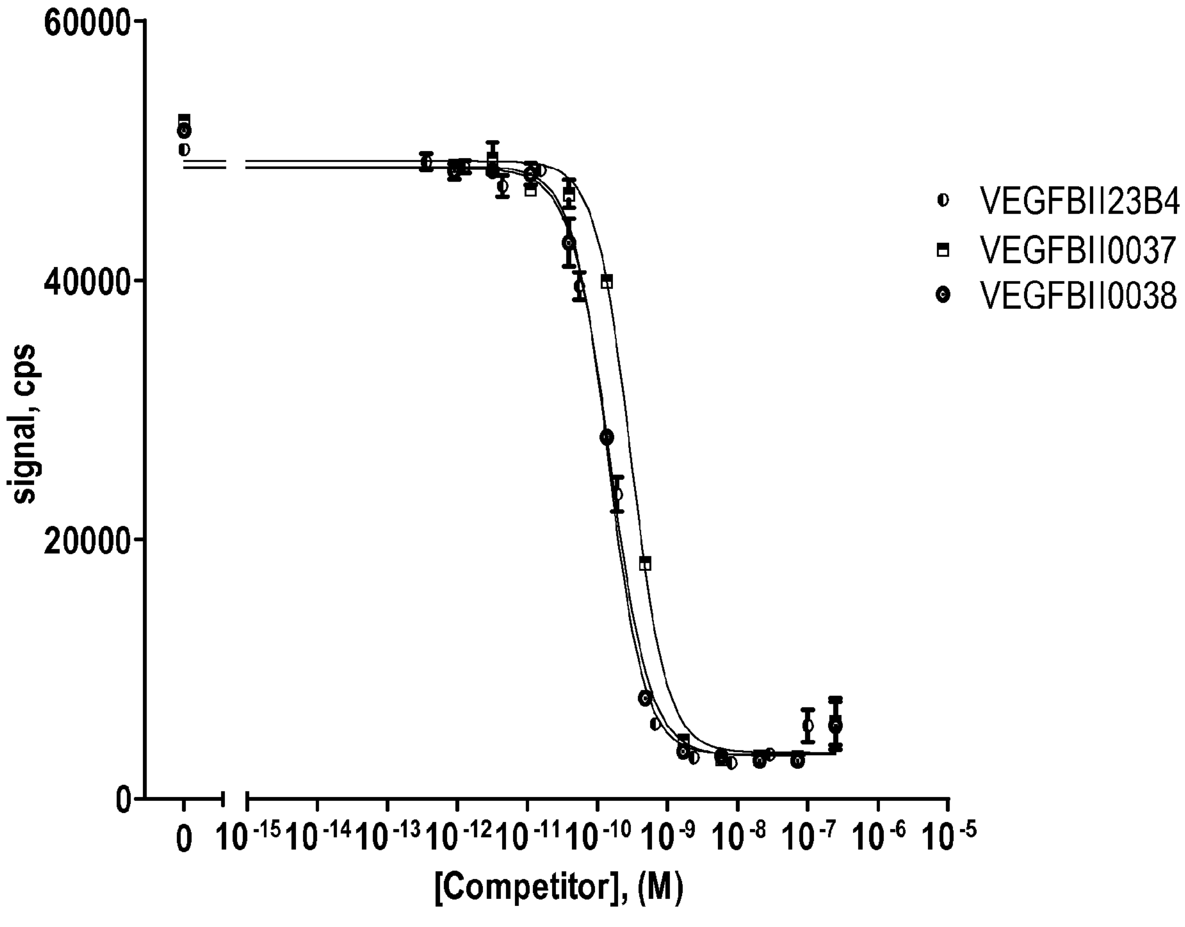
60000
40000
20000
0
signal, cps
0
10 -15
10 -14
10 -13
10 -12
10 -11
10 -10
10 -9
10 -8
10 -7
10 -6
10 -5
[Competitor], (M)
VEGFBII23B4
VEGFBII0037
VEGFBII0038

**Fig. 19**

```
                           10        20        30        40
Kabat#         : .........|.........|.........|.........|
VH3-23/JH5     : EVQLVESGGGLVQPGGSLRLSCAASGFTFSSYAMSWVRQA
PVEGFBIIPMP5B5 : ......................V...IR.MSM.--.Y...

                           50          60        70        80
Kabat#         : .........|..a.......|.........|.........|
VH3-23/JH5     : PGKGLEWVSAISGSGGSTYYADSVKGRFTISRDNSKNTLYL
PVEGFBIIPMP5B5 : ...HR.L.AR..-...T.A.V.................V..

                              90       101      110
Kabat#         : ..abc.......|.........|........|...
VH3-23/JH5     : QMNSLRAEDTAVYYCAK-------WGQGTLVTVSS
PVEGFBIIPMP5B5 : .....KA........NTFSSRPNP..A..Q.....
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080014196 A
- WO 2008101985 A
- WO 2009109635 A
- WO 2006040153 A
- WO 2006122786 A
- WO 2003002609 A
- WO 02056910 A
- WO 9849185 A
- WO 08020079 A
- WO 2006004678 A
- WO 9404678 A
- WO 9634103 A
- WO 2004081026 A
- WO 2009095489 A
- WO 0179271 A
- WO 0359934 A
- WO 2004060965 A
- WO 04041862 A
- WO 04041863 A
- WO 04041865 A
- WO 04041867 A
- WO 05044858 A


### Non-patent literature cited in the description


- **FERRARA.** *Endocrine Rev.,* 2004, vol. 25, 581-611
- **HOEBEN et al.** *Pharmacol. Rev.,* vol. 56, 549-580
- **FERRARA ; DAVIS-SMYTH.** *Endocrine Rev.,* 1997, vol. 18, 4-25
- **FERRARA J.** *MoL Med.,* 1999, vol. 77, 527-543
- **FOLKMAN et al.** *Nature,* 1989, 339-58
- **FERRARA et al.** *Nat Rev Drug Discov.,* May 2004, vol. 3 (5), 391-400
- **SCHLAEPPI ; WOOD.** *Cancer Metastasis Rev.,* 1999, vol. 18, 473-481
- **KIM et al.** *Nature,* 1993, vol. 362, 841-844
- **FOLKMAN.** *Nat. Med.,* 1995, vol. 1, 27-31
- **PRESTA et al.** *Cancer Res.,* 1997, vol. 57, 4593-4599
- **KANAI et al.** *Int. J. Cancer,* 1998, vol. 77, 933-936
- **FERRARA ; ALITALO.** *Nat. Med.,* 1999, vol. 5, 1359-1364320, 340
- **CAMPOCHIARO ; HACKETT.** *Oncogene,* 2003, vol. 22, 6537-6548
- **SAMBROOK et al.** Molecular Cloning: A Laboratory Manual. Cold Spring Harbor Laboratory Press, 1989, vol. 1-3
- **LEWIN.** Genes IV. Oxford University Press, 1990
- **ROITT et al.** Immunology. Gower Medical Publishing, 1989
- **HAMERS-CASTERMAN C ; ATARHOUCH T ; MUYLDERMANS S ; ROBINSON G ; HAMERS C ; SONGA EB ; BENDAHMAN N ; HAMERS R.** Naturally occurring antibodies devoid of light chains. *Nature,* 1993, vol. 363, 446-448
- **KABAT et al.** Sequence of proteins of immunological interest. US Public Health Services, NIH Bethesda, MD, Publication No. 91
- **RIECHMANN ; MUYLDERMANS.** *J. Immunol. Methods,* 1999, vol. 231, 25-38
- **WARD, E.S. et al.** Binding activities of a repertoire of single immunoglobulin variable domains secreted from Escherichia coli. *Nature,* 1989, vol. 341, 544-546
- **HOLT, L.J. et al.** Domain antibodies: proteins for therapy. *TRENDS in Biotechnology,* 2003, vol. 21 (11), 484-490
- **MARKS et al.** *Biotechnology,* 1992, vol. 10, 779-783
- **BARBAS et al.** *Proc. Nat. Acad. Sci, USA,* 1994, vol. 91, 3809-3813
- **SHIER et al.** *Gene,* 1995, vol. 169, 147-155
- **YELTON et al.** *Immunol.,* 1995, vol. 155, 1994-2004
- **JACKSON et al.** *J. Immunol.,* 1995, vol. 154 (7), 3310-9
- **HAWKINS et al.** *J. Mol. Biol.,* 1992, vol. 226 (3), 889-896
- **KS JOHNSON ; RE HAWKINS.** Affinity maturation of antibodies using phage display. Oxford University Press, 1996
- **WARD et al.** *Nature,* 1989, vol. 341, 544-546
- **CHAPMAN.** *Nat. Biotechnol.,* 2002, vol. 54, 531-545

- **VERONESE ; HARRIS.** *Adv. Drug Deliv. Rev.,* 2003, vol. 54, 453-456
- **HARRIS ; CHESS.** *Nat. Rev. Drug. Discov.,* 2003, 2
- **YANG et al.** *Protein Engineering,* 2003, vol. 16, 761-770
- **DAUGHERTY et al.** *Nucleic Acids Research,* 1991, vol. 19 (9), 2471-2476
- **NICAISE et al.** *Science,* 2004, vol. 13, 1882-1891
- Remington's Pharmaceutical Sciences. Mack Publishing Company, 1990
- **REMINGTON.** Science and Practice of Pharmacy. Lippincott Williams and Wilkins, 2005
- Handbook of Therapeutic Antibodies. Wiley, 2007, 252-255
- **DALEY et al.** *Clin. Diagn. Lab. Imm.,* 2005, vol. 12, 380-386
- **ERICSSON et al.** *Anal. Biochem.,* 2006, vol. 357, 289-298